# EUROPÄISCHE PATENTSCHRIFT

(11) **EP 1 165 547 B1**
(45) Veröffentlichungstag und Bekanntmachung des Hinweises auf die Patenterteilung: **27.08.2003**
(21) Anmeldenummer: 00912609.5
(22) Anmeldetag: 15.03.2000
(51) Int. Cl.: C07D 403/10, A01N 43/653, A01N 43/56, C07D 401/10, C07D 417/10, C07D 471/04, C07D 231/20, C07D 413/10, C07D 487/04

(54) **SUBSTITUIERTE BENZOYLPYRAZOLE ALS HERBIZIDE**
SUBSTITUTED BENZOYLPYRAZOLES AS HERBICIDES
BENZOYLPYRAZOLES SUBSTITUES EN TANT QU'HERBICIDES

(30) Priorität: 27.03.1999 DE 19914140
(43) Veröffentlichungstag der Anmeldung: 02.01.2002
(73) Patentinhaber: Bayer CropScience AG, 40789 Monheim (DE)
(72) Erfinder: MÜLLER, Klaus-Helmut, D-40593 Düsseldorf (DE); LEHR, Stefan, D-40764 Langenfeld (DE); SCHALLNER, Otto, D-40789 Monheim (DE); SCHWARZ, Hans-Georg, D-40764 Langenfeld (DE); WROBLOWSKY, Heinz-Jürgen, D-40764 Langenfeld (DE); DREWES, Mark, Wilhelm, D-40764 Langenfeld (DE); FEUCHT, Dieter, D-40789 Monheim (DE); PONTZEN, Rolf, D-42799 Leichlingen (DE); WETCHOLOWSKY, Ingo, CEP-13280-000 Vinhedo, SP (BR)
(86) Internationale Anmeldenummer: EP0002292
(87) Internationale Veröffentlichungsnummer: WO00058306

(56) Entgegenhaltungen:
- EP-A- 0 900 795
- WO-A-98/31681
- WO-A-98/42678
- WO-A-99/07697
- US-A- 5 846 907

## Beschreibung

Die Erfindung betrifft neue substituierte Benzoylpyrazole, Verfahren zu ihrer Herstellung und ihre Verwendung als Herbizide.

Es ist bereits bekannt, dass bestimmte substituierte Benzoylpyrazole herbizide Eigenschaften aufweisen (vgl. EP-A-352543, WO-A-96/26206, WO-A-97/35850, WO-A-97/41105, WO-A-97/41116, WO-A-97/41117, WO-A-97/41118, WO-A-97/46530, WO-A-98/28981, WO-A-98/31681, WO-A-98/31682, WO-A-99/07697). Die Wirkung dieser Verbindungen ist jedoch nicht in allen Belangen zufriedenstellend.

Es wurden nun die neuen substituierten Benzoylpyrazole der allgemeinen Formel (I), 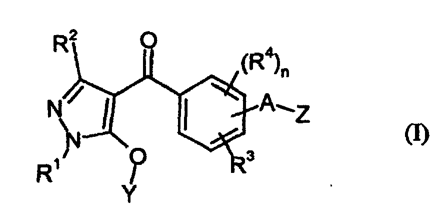 in welcher
- n: für die Zahlen 0, 1, 2 oder 3 steht,
- A: für Alkandiyl (Alkylen) steht,
- R¹: für jeweils gegebenenfalls substituiertes Alkyl, Alkenyl, Alkinyl oder Cycloalkyl steht,
- R²: für Wasserstoff, Cyano, Carbamoyl, Thiocarbamoyl, Halogen, oder für jeweils gegebenenfalls substituiertes Alkyl, Alkoxy, Alkylthio, Alkoxycarbonyl oder Cycloalkyl steht,
- R³: für Wasserstoff, Nitro, Cyano, Carboxy, Carbamoyl, Thiocarbamoyl, Halogen, oder für jeweils gegebenenfalls substituiertes Alkyl, Alkoxy, Alkylthio, Alkylsulfinyl, Alkylsulfonyl, Alkylamino, Dialkylamino oder Dialkylaminosulfonyl steht,
- R⁴: für Nitro, Cyano, Carboxy, Carbamoyl, Thiocarbamoyl, Halogen, oder für jeweils gegebenenfalls substituiertes Alkyl, Alkoxy, Alkylthio, Alkylsulfinyl, Alkylsulfonyl, Alkylamino, Dialkylamino oder Dialkylaminosulfonyl steht,
- Y: für Wasserstoff oder für jeweils gegebenenfalls substituiertes Alkyl, Alkylcarbonyl, Alkoxycarbonyl, Alkylsulfonyl, Alkylaminocarbonyl, Dialkylaminocarbonyl, Alkenyl, Alkenylcarbonyl, Alkenylsulfonyl, Alkinyl, Alkinylcarbonyl, Cycloalkyl, Cycloalkylcarbonyl, Cycloalkylalkyl, Phenylcarbonyl, Phenylsulfonyl, Phenylalkyl oder Phenylcarbonylalkyl steht, und
- Z: für eine der nachstehenden heterocyclischen Gruppierungen steht 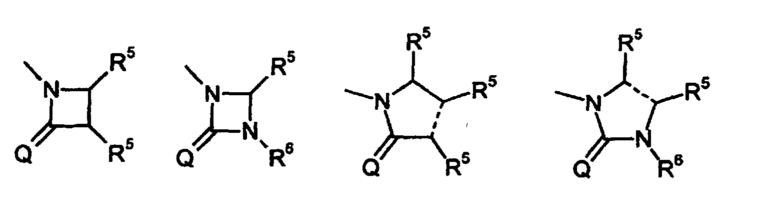 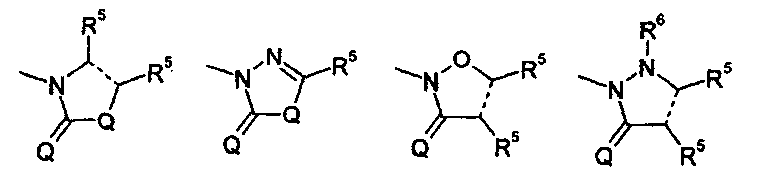 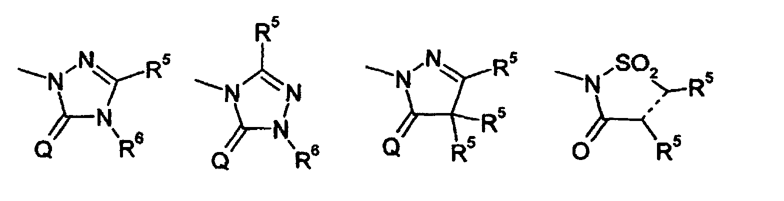 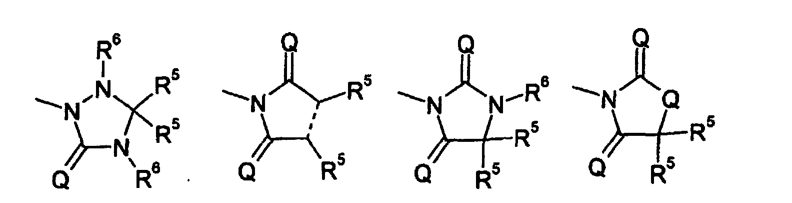 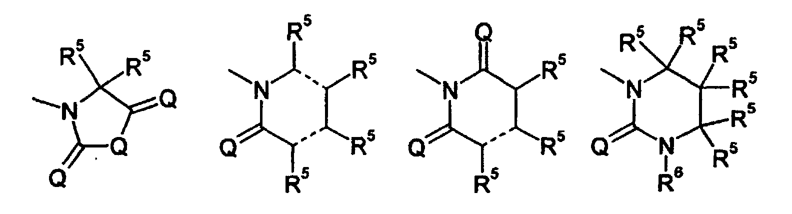 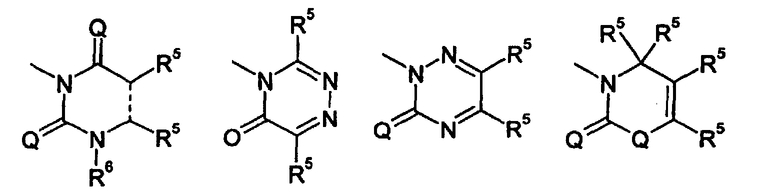 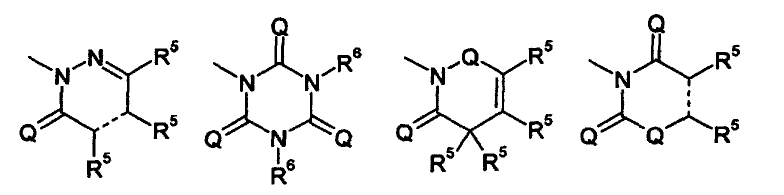 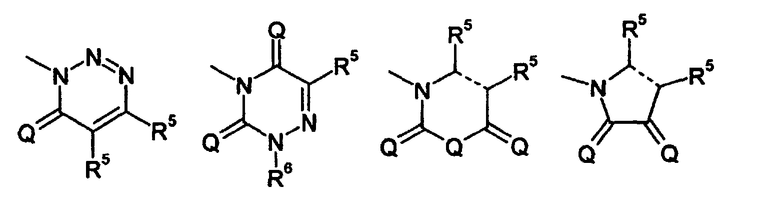 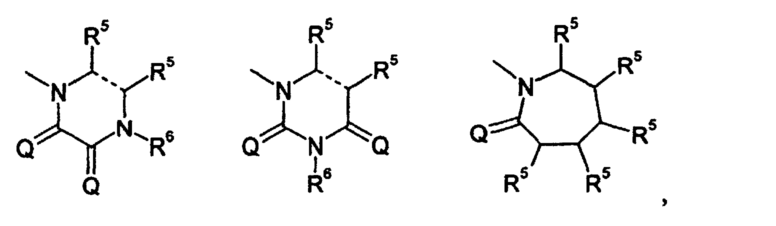 worin jeweils die gestrichelt gezeichnete Bindung eine Einfachbindung oder eine Doppelbindung ist,
Q für Sauerstoff oder Schwefel steht,
R⁵ für Wasserstoff, Hydroxy, Mercapto, Cyano, Halogen, für jeweils gegebenenfalls durch Cyano, Halogen, C₁-C₄-Alkoxy, C₁-C₄-Alkylthio, C₁-C₄-Alkylsulfinyl oder C₁-C₄-Alkylsulfonyl substituiertes Alkyl, Alkylcarbonyl, Alkoxy, Alkoxycarbonyl, Alkylthio, Alkylsulfinyl oder Alkylsulfonyl mit jeweils bis zu 6 Kohlenstoffatomen in den Alkylgruppen, für Propadienylthio, für jeweils gegebenenfalls durch Halogen substituiertes Alkylamino oder Dialkylamino mit jeweils bis zu 6 Kohlenstoffatomen in den Alkylgmppen, für jeweils gegebenenfalls durch Halogen substituiertes Alkenyl, Alkinyl, Alkenyloxy, Alkenylthio oder Alkenylamino mit jeweils bis zu 6 Kohlenstoffatomen in den Alkenyl- bzw. Alkinylgruppen, für jeweils gegebenenfalls durch Halogen substituiertes Cycloalkyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in den Cycloalkylgruppen und gegebenenfalls bis zu 4 Kohlenstoffatomen im Alkylteil, für jeweils gegebenenfalls durch Halogen, C₁-C₄-Alkyl oder C₁-C₄-Alkoxy substituiertes Phenyl, Phenyloxy, Phenylthio, Phenylamino, Benzyl, Benzyloxy, Benzylthio oder Benzylamino steht, für Pyrrolidino, Piperidino oder Morpholino steht, oder - für den Fall, dass zwei benachbarte Reste R⁵ und R⁵ sich an einer Doppelbindung befinden - zusammen mit dem benachbarten Rest R⁵ auch für eine Benzogruppierung steht, und
R⁶ für Wasserstoff, Hydroxy, Amino, Alkylidenamino mit bis zu 4 Kohlenstoffatomen, für jeweils gegebenenfalls durch Halogen oder C₁-C₄-Alkoxy substituiertes Alkyl, Alkoxy, Alkylamino, Dialkylamino oder Alkanoylamino mit jeweils bis zu 6 Kohlenstoffatomen in den Alkylgruppen, für jeweils gegebenenfalls durch Halogen substituiertes Alkenyl, Alkinyl oder Alkenyloxy mit jeweils bis zu 6 Kohlenstoffatomen in den Alkenyl- bzw. Alkinylgruppen, für jeweils gegebenenfalls durch Halogen substituiertes Cycloalkyl, Cycloalkylalkyl oder Cycloalkylamino mit jeweils 3 bis 6 Kohlenstoffatomen ii den Cycloalkylgruppen und gegebenenfalls bis zu 3 Kohlenstoff atomen im Alkylteil, oder für jeweils gegebenenfalls durch Halogen C₁-C₄-Alkyl oder C₁-C₄-Alkoxy substituiertes Phenyl oder Benzy steht, oder zusammen mit einem benachbarten Rest R⁵ oder R⁶ für gegebenenfalls durch Halogen oder C₁-C₄-Alkyl substituiertes Alkandiyl mit 3 bis 5 Kohlenstoffatomen steht,

- wobei die einzelnen Reste R⁵ und R⁶, soweit mehrere davon an gleiche heterocyclische Gruppierungen gebunden sind, gleiche oder verschiedene Bedeutungen im Rahmen der obigen Definition haben können,
- einschließlich aller möglichen tautomeren Formen der Verbindungen der allgemeinen Formel (I) und der möglichen Salze der Verbindungen der allgemeinen Formel (I) - gefunden.

In den Definitionen sind die Kohlenwasserstoffketten, wie Alkyl oder Alkandiyl - auch in Verbindung mit Heteroatomen, wie in Alkoxy - jeweils geradkettig oder verzweigt.
- n: steht bevorzugt für die Zahlen 0, 1 oder 2.
- A: steht bevorzugt für Alkandiyl (Alkylen) mit 1 bis 4 Kohlenstoffatomen.
- R¹: steht bevorzugt für gegebenenfalls durch Cyano, Carboxy, Carbamoyl, Halogen, C₁-C₄-Alkoxy, C₁-C₄-Alkyl-carbonyl, C₁-C₄-Alkoxy-carbonyl, C₁-C₄-Alkylthio, C₁-C₄-Alkylsulfinyl oder C₁-C₄-Alkylsulfonyl substituiertes Alkyl mit 1 bis 6 Kohlenstoffatomen, für jeweils gegebenenfalls durch Cyano, Carboxy, Carbamoyl, Halogen oder C₁-C₄-Alkoxy-carbonyl substituiertes Alkenyl oder Alkinyl mit jeweils 2 bis 6 Kohlenstoffatomen, oder für gegebenenfalls durch Cyano, Carboxy, Carbamoyl, Halogen, C₁-C₄-Alkyl oder C₁-C₄-Alkoxy-carbonyl substituiertes Cycloalkyl mit 3 bis 6 Kohlenstoffatomen.
- R²: steht bevorzugt für Wasserstoff, Cyano, Carbamoyl, Thiocarbamoyl, Halogen, für jeweils gegebenenfalls durch Cyano, Halogen oder C₁-C₄-Alkoxy substituiertes Alkyl, Alkoxy oder Alkoxycarbonyl mit jeweils bis zu 6 Kohlenstoffatomen, für gegebenenfalls durch Halogen substituiertes Alkylthio mit 1 bis 6 Kohlenstoffatomen, oder für gegebenenfalls durch Cyano, Halogen oder C₁-C₄-Alkyl substituiertes Cycloalkyl mit 3 bis 6 Kohlenstoffatomen.
- R³: steht bevorzugt für Wasserstoff, Nitro, Cyano, Carboxy, Carbamoyl, Thiocarbamoyl, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkoxy, Alkylthio, Alkylsulfinyl oder Alkylsulfonyl mit jeweils bis zu 4 Kohlenstoffatomen in den Alkylgruppen, oder für Alkylamino, Dialkylamino oder Dialkylaminosulfonyl mit jeweils bis zu 4 Kohlenstoffatomen in den Alkylgruppen.
- R⁴: steht bevorzugt für Nitro, Cyano, Carboxy, Carbamoyl, Thiocarbamoyl, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkoxy, Alkylthio, Alkylsulfinyl oder Alkylsulfonyl mit jeweils bis zu 4 Kohlenstoffatomen in den Alkylgruppen, oder für Alkylamino, Dialkylamino oder Dialkylaminosulfonyl mit jeweils bis zu 4 Kohlenstoffatomen in den Alkylgruppen.
- Y: steht bevorzugt für Wasserstoff, für jeweils gegebenenfalls durch Cyano, Carboxy, Carbamoyl, Halogen oder C₁-C₄-Alkoxy-carbonyl substituiertes Alkyl, Alkylcarbonyl oder Alkoxycarbonyl mit jeweils bis zu 6 Kohlenstoffatomen, für jeweils gegebenenfalls durch Halogen substituiertes Alkylsulfonyl, Alkylaminocarbonyl oder Dialkylaminocarbonyl mit jeweils bis zu 6 Kohlenstoffatomen in den Alkylgruppen, für jeweils gegebenenfalls durch Cyano, Carboxy, Carbamoyl, Halogen oder C₁-C₄-Alkoxy-carbonyl substituiertes Alkenyl, Alkenylcarbonyl, Alkinyl oder Alkinylcarbonyl mit jeweils 2 bis 6 Kohlenstoffatomen, für gegebenenfalls durch Halogen substituiertes Alkenylsulfonyl mit bis zu 6 Kohlenstoffatomen für jeweils gegebenenfalls durch Cyano, Halogen oder C₁-C₄-Alkyl substituiertes Cycloalkyl, Cycloalkylcarbonyl oder Cycloalkylalkyl mit jeweils 3 bis 6 Kohlenstoffatomen in den Cycloalkylgruppen und gegebenenfalls 1 bis 3 Kohlenstoffatomen im Alkylteil, oder für jeweils gegebenenfalls durch Nitro, Cyano, Carboxy, Carbamoyl, Halogen, C₁-C₄-Alkyl, C₁-C₄-Halogenalkyl, C₁-C₄-Alkoxy oder C₁-C₄-Halogenalkoxy substituiertes Phenylcarbonyl, Phenylsulfonyl, Phenyl-C₁-C₄-alkyl oder Phenylcarbonyl-C₁-C₄-alkyl.
Q steht bevorzugt für Sauerstoff.
R⁵ steht bevorzugt für Wasserstoff, Hydroxy, Mercapto, Cyano, Fluor, Chlor, Brom, Iod, für jeweils gegebenenfalls durch Fluor, Chlor, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, für Methylamino, Ethylamino, n- oder i-Propylamino, n-, i-, s- oder t-Butylamino, Dimethylamino, Diethylamino, Di-n-propylamino oder Di-i-propylamino, für jeweils gegebenenfalls durch Fluor und/oder Chlor substituiertes Ethenyl, Propenyl, Butenyl, Ethinyl, Propinyl, Butinyl, Propenyloxy, Butenyloxy, Propenylthio, Butenylthio, Propenylamino oder Butenylamino, für jeweils gegebenenfalls durch Fluor und/oder Chlor substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, für jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl, Phenyloxy, Phenylthio, Phenylamino, Benzyl, Benzyloxy, Benzylthio oder Benzylamino, für Pyrrolidino, Piperidino oder Morpholino steht, oder - für den Fall, dass zwei benachbarte Reste R⁵ und R⁵ sich an einer Doppelbindung befinden - zusammen mit dem benachbarten Rest R⁵ auch für eine Benzogruppierung.
R⁶ steht bevorzugt für Wasserstoff, Hydroxy, Amino, für jeweils gegebenenfalls durch Fluor und/oder Chlor, Methoxy oder Ethoxy substituiertes Methyl, Ethyl, n- oder i-Propyl, n-, i- oder s-Butyl, Methoxy, Ethoxy, n- oder i-Propoxy, Methylamino, Ethylamino oder Dimethylamino, für jeweils gegebenenfalls durch Fluor und/oder Chlor substituiertes Ethenyl, Propenyl, Ethinyl, Propinyl oder Propenyloxy, für jeweils gegebenenfalls durch Fluor und/oder Chlor substituiertes Cyclopropyl, Cyclobutyl, Cyclopentyl, Cyclohexyl, Cyclopropylmethyl, Cyclobutylmethyl, Cyclopentylmethyl, Cyclohexylmethyl, oder für jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl oder Benzyl steht, oder zusammen mit einem benachbarten Rest R⁵ oder R⁶ für jeweils gegebenenfalls durch Methyl und/oder Ethyl substituiertes Propan-1,3-diyl (Trimethylen), Butan-1,4-diyl (Tetramethylen) oder Pentan-1,5-diyl (Pentamethylen).
- n: steht besonders bevorzugt für die Zahlen 0 oder 1.
- A: steht besonders bevorzugt für Methylen, Ethyliden (Ethan-1,1-diyl) oder Dimethylen (Ethan-1,2-diyl).
- R¹: steht besonders bevorzugt für jeweils gegebenenfalls durch Fluor, Chlor, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, oder für jeweils gegebenenfalls durch Cyano, Fluor, Chlor, Brom, Methyl oder Ethyl substituiertes Cyclopropyl, Cyclobutyl, Cyclopentyl oder Cyclohexyl.
- R²: steht besonders bevorzugt für Wasserstoff, Cyano, Carbamoyl, Thiocarbamoyl, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, Methoxycarbonyl, Ethoxycarbonyl, n- oder i-Propoxycarbonyl, für jeweils gegebenenfalls durch Fluor und/oder Chlor substituiertes Methylthio, Ethylthio, n- oder i-Propylthio, oder für jeweils gegebenenfalls durch Cyano, Fluor, Chlor, Brom, Methyl oder Ethyl substituiertes Cyclopropyl, Cyclobutyl, Cyclopentyl oder Cyclohexyl.
- R³: steht besonders bevorzugt für Wasserstoff, Nitro, Cyano, Carboxy, Carbamoyl, Thiocarbamoyl, Fluor, Chlor, Brom, Iod, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl.
- R⁴: steht besonders bevorzugt für Nitro, Cyano, Carboxy, Carbamoyl, Thiocarbamoyl, Fluor, Chlor, Brom, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl.
- R⁵: steht besonders bevorzugt für Wasserstoff, Hydroxy, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Fluorethyl, Chlorethyl, Difluorethyl, Dichlorethyl, Fluor-n-propyl, Fluor-i-propyl, Chlor-n-propyl, Chlor-i-propyl, Methoxymethyl, Ethoxymethyl, Methoxyethyl, Ethoxyethyl, Methoxy, Ethoxy, n- oder i-Propoxy, n-, i-, s- oder t-Butoxy, Fluorethoxy, Chlorethoxy, Difluorethoxy, Dichlorethoxy, Trifluorethoxy, Trichlorethoxy, Chlorfluorethoxy, Chlordifluorethoxy, Fluordichlorethoxy, Methylthio, Ethylthio, n- oder i-Propylthio, Fluorethylthio, Chlorethylthio, Difluorethylthio, Dichlorethylthio, Chlorfluorethylthio, Chlordifluorethylthio, Fluordichlorethylthio, Methylsulfinyl, Ethylsulfinyl, n- oder i-Propylsulfinyl, Methylsulfonyl, Ethylsulfonyl, n- oder i-Propylsulfonyl, Dimethylamino, Propenylthio, Butenylthio, Propinylthio, Butinylthio, Cyclopropyl, Cyclopropylmethyl, Cyclopropylmethoxy, Phenyl oder Phenoxy.
- R⁶: steht besonders bevorzugt für Amino, Methyl, Ethyl, n- oder i-Propyl, n-, i-, s- oder t-Butyl, Methoxy, Ethoxy, Methylamino, Dimethylamino, Cyclopropyl oder Cyclopropylmethyl steht, oder zusammen mit R⁵ für Propan-1,3-diyl (Trimethylen), Butan-1,4-diyl (Tetramethylen) oder Pentan-1,5-diyl (Pentamethylen).
- Y: steht besonders bevorzugt für Wasserstoff, für jeweils gegebenenfalls durch Cyano, Fluor, Chlor, Methoxy oder Ethoxy substituiertes Methyl, Ethyl, n- oder i-Propyl, Acetyl, Propionyl, n- oder i-Butyroyl, Methoxycarbonyl oder Ethoxycarbonyl, für jeweils gegebenenfalls durch Fluor, Chlor und/oder Brom substituiertes Methylsulfonyl, Ethylsulfonyl, n- oder i-Propylsulfonyl, n-, i-, s- oder t-Butylsulfonyl, Methylaminocarbonyl, Ethylaminocarbonyl, n-oder i-Propylaminocarbonyl, Dimethylaminocarbonyl oder Diethylaminocarbonyl, für jeweils gegebenenfalls durch Fluor, Chlor oder Brom substituiertes Propenyl, Butenyl, Propenylcarbonyl, Butenylcarbonyl, Propenylsulfonyl, Butenylsulfonyl, Propinyl, Butinyl, Propinylcarbonyl oder Butinylcarbonyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylcarbonyl, Cyclobutylcarbonyl, Cyclopentylcarbonyl, Cyclohexylcarbonyl, Cyclopropylmethyl, Cyclobutylmethyl, Cyclopentylmethyl oder Cyctohexylmethyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenylcarbonyl, Phenylsulfonyl, Benzyl oder Phenylcarbonylmethyl.
- Z: steht besonders bevorzugt für 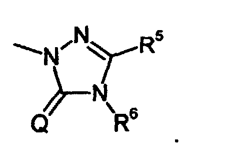
- n: steht ganz besonders bevorzugt für 0.
- A: steht ganz besonders bevorzugt für Methylen.
- R¹: steht ganz besonders bevorzugt für jeweils gegebenenfalls durch Fluor, Chlor, Methoxy, Ethoxy, Methylthio, Ethylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, oder für gegebenenfalls durch Cyano, Fluor, Chlor, Brom, Methyl oder Ethyl substituiertes Cyclopropyl.
- R²: steht ganz besonders bevorzugt für Wasserstoff, Cyano, Carbamoyl, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxycarbonyl, Ethoxycarbonyl, n- oder i-Propoxycarbonyl, für jeweils gegebenenfalls durch Fluor und/oder Chlor substituiertes Methylthio, Ethylthio, n- oder i-Propylthio, oder für Cyclopropyl.
- R³: steht ganz besonders bevorzugt für Wasserstoff, Nitro, Cyano, Fluor, Chlor, Brom, Iod, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl.
- R⁴: steht ganz besonders bevorzugt für Nitro, Cyano, Fluor, Chlor, Brom, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl, Dimethylamino oder Dimethylaminosulfonyl.
- R⁶: steht ganz besonders bevorzugt für Methyl, Cyclopropyl, Dimethylamino, Methoxy oder Ethoxy.
- Y: steht ganz besonders bevorzugt für Wasserstoff.
- R¹: steht am meisten bevorzugt für Methyl oder Ethyl.
- R²: steht am meisten bevorzugt für Wasserstoff oder Methyl.
- R³: steht am meisten bevorzugt für Wasserstoff, Fluor, Chlor, Brom, Trifluormethyl oder Methylsulfonyl.
- R⁴: steht am meisten bevorzugt für (2-)Chlor, (4-)Chlor, (6-)Trifluormethyl oder (2-)Methylsulfonyl.

Erfindungsgemäß bevorzugt sind die Verbindungen der Formel (I), in welchen eine Kombination der vorstehend als bevorzugt aufgeführten Bedeutungen vorliegt

Erfindungsgemäß besonders bevorzugt sind die Verbindungen der Formel (I), in welchen eine Kombination der vorstehend als besonders bevorzugt aufgeführten Bedeutungen vorliegt.

Erfindungsgemäß ganz besonders bevorzugt sind die Verbindungen der Formel (I), in welchen eine Kombination der vorstehend als ganz besonders bevorzugt aufgeführten Bedeutungen vorliegt

Erfindungsgemäß am meisten bevorzugt sind die Verbindungen der Formel (I), in welchen eine Kombination der vorstehend als am meisten bevorzugt aufgeführten Bedeutungen vorliegt.

Die Verbindungen der allgemeinen Formeln (IA), (IB) und (IC) sind insbesondere Gegenstand der vorliegenen Erfindung: 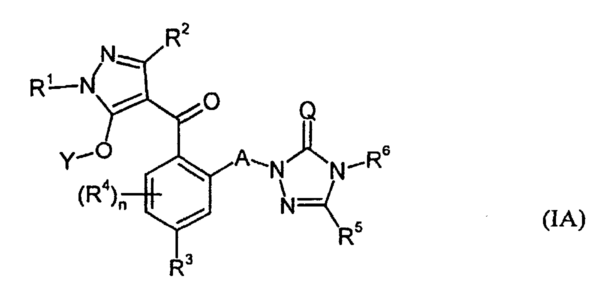 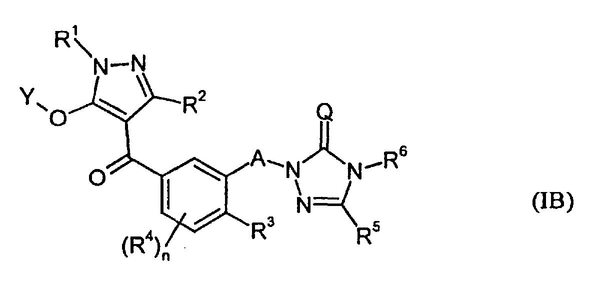 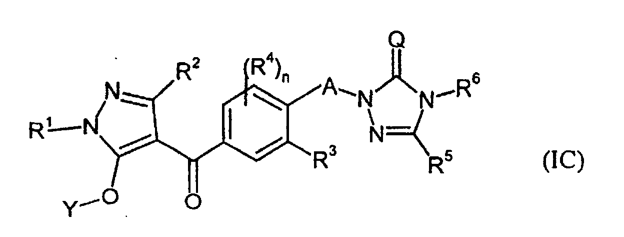 in welchen
- n, A, Q, R¹, R², R³, R⁴, R⁵, R⁶ und Y: die vorausstehend angegebene Bedeutung haben.

Gegenstand der Erfindung sind vorzugsweise auch Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzylammonium-Salze von Verbindungen der Formel (I), in welcher n, A, R¹, R², R³, R⁴, Y und Z die oben angegebenen Bedeutungen haben.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Beispiele für die erfindungsgemäßen Verbindungen der allgemeinen Formel (I) sind in den nachstehenden Gruppen aufgeführt.

### Gruppe 1

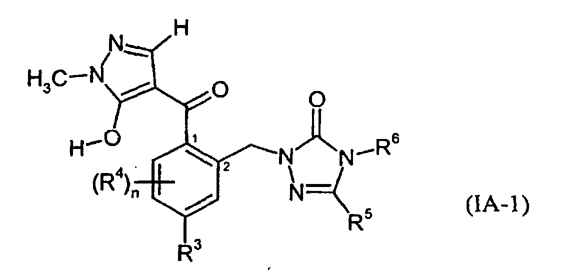

R³, (R⁴)ₙ, R⁵ und R⁶ haben hierbei beispielhaft die in der nachstehenden Tabelle angegebenen Bedeutungen:

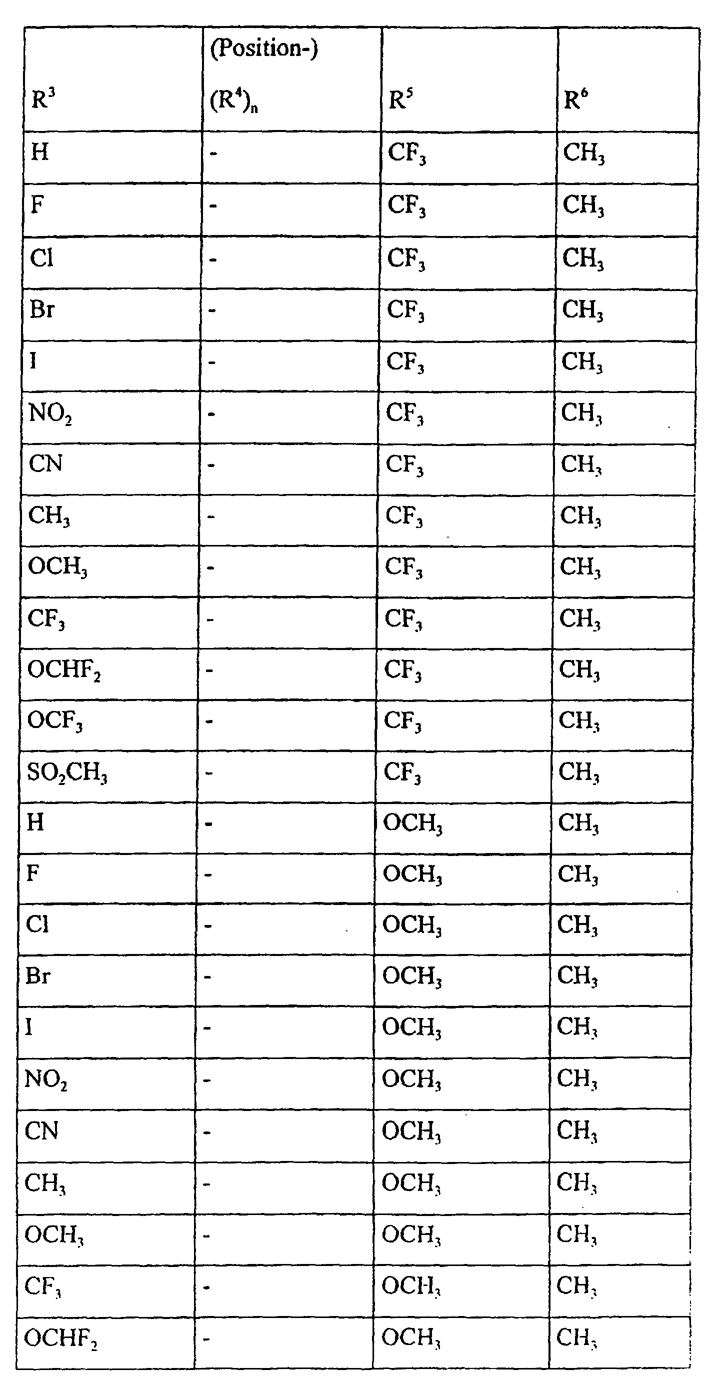

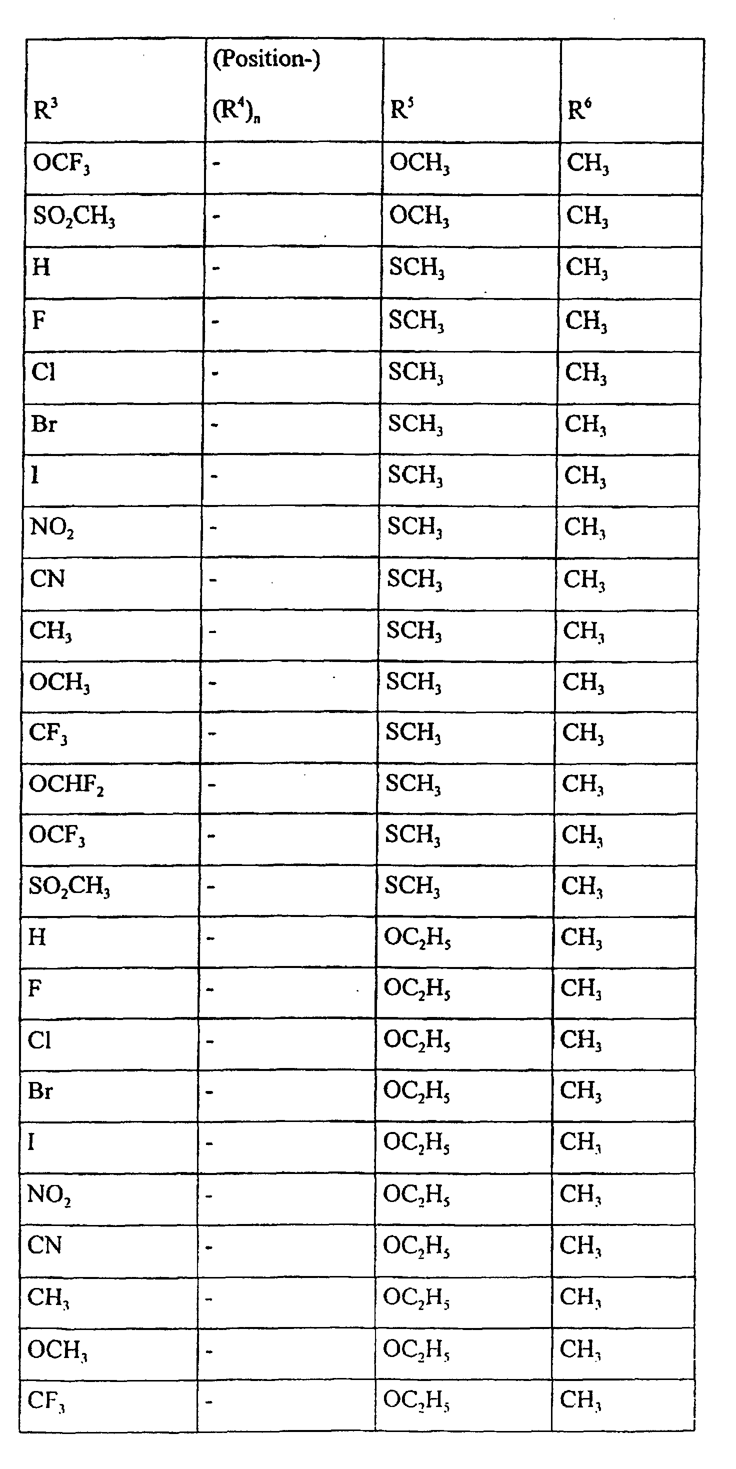

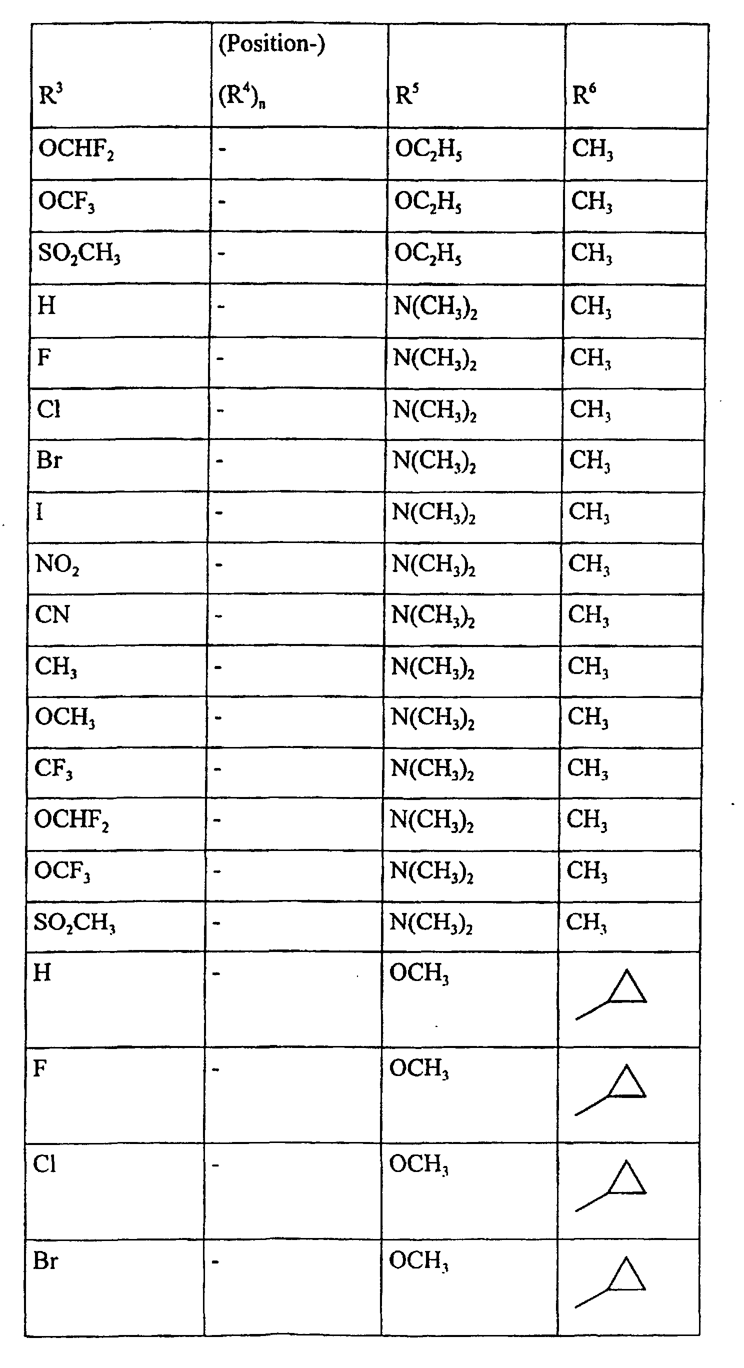

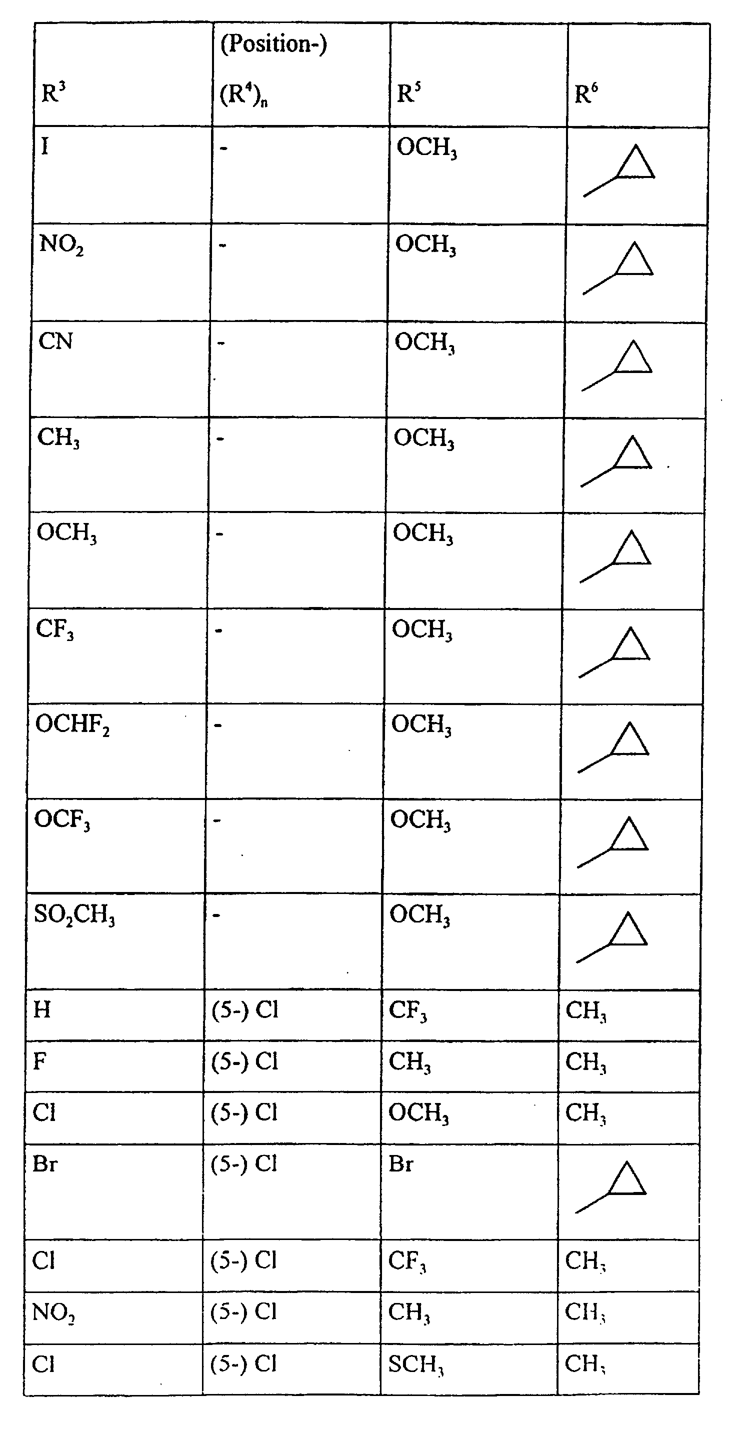

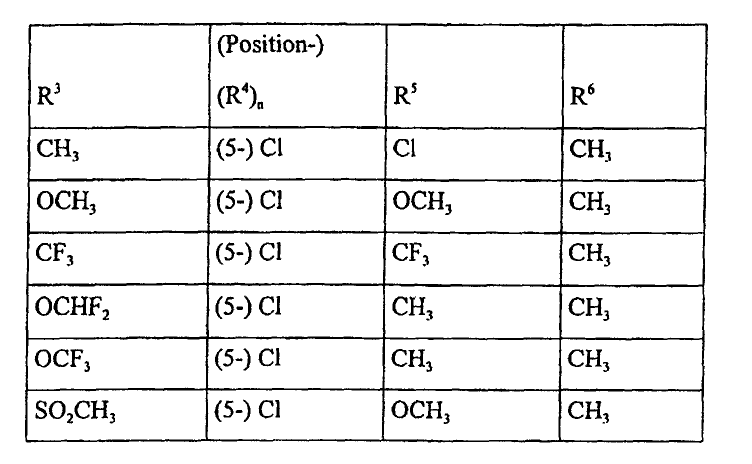

### Gruppe 2

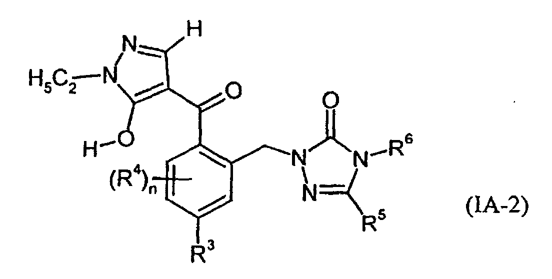

R³, (R⁴)ₙ, R⁵ und R⁶ haben hierbei beispielhaft die oben in Gruppe 1 angegenenen Bedeutungen.

### Gruppe 3

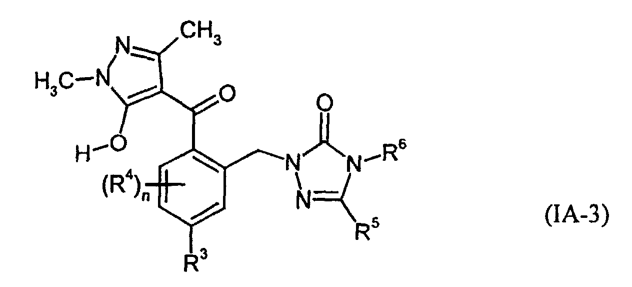

R³, (R⁴)ₙ, R⁵ und R⁶ haben hierbei beispielhaft die oben in Gruppe 1 angegenenen Bedeutungen.

### Gruppe 4

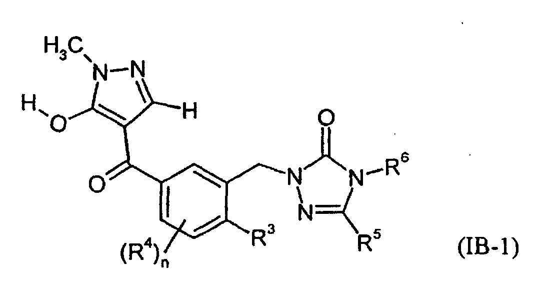

R³, (R⁴)ₙ, R⁵ und R⁶ haben hierbei beispielhaft die in der nachstehenden Tabelle angegenenen Bedeutungen:

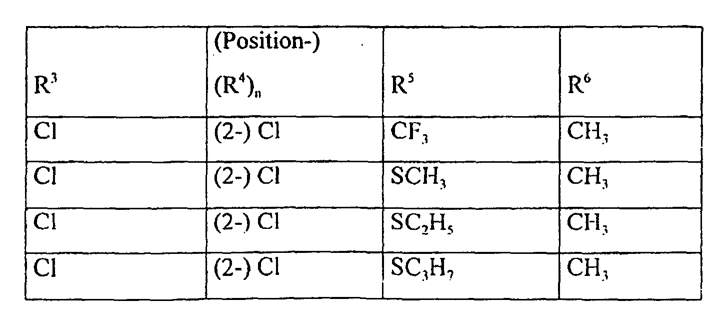

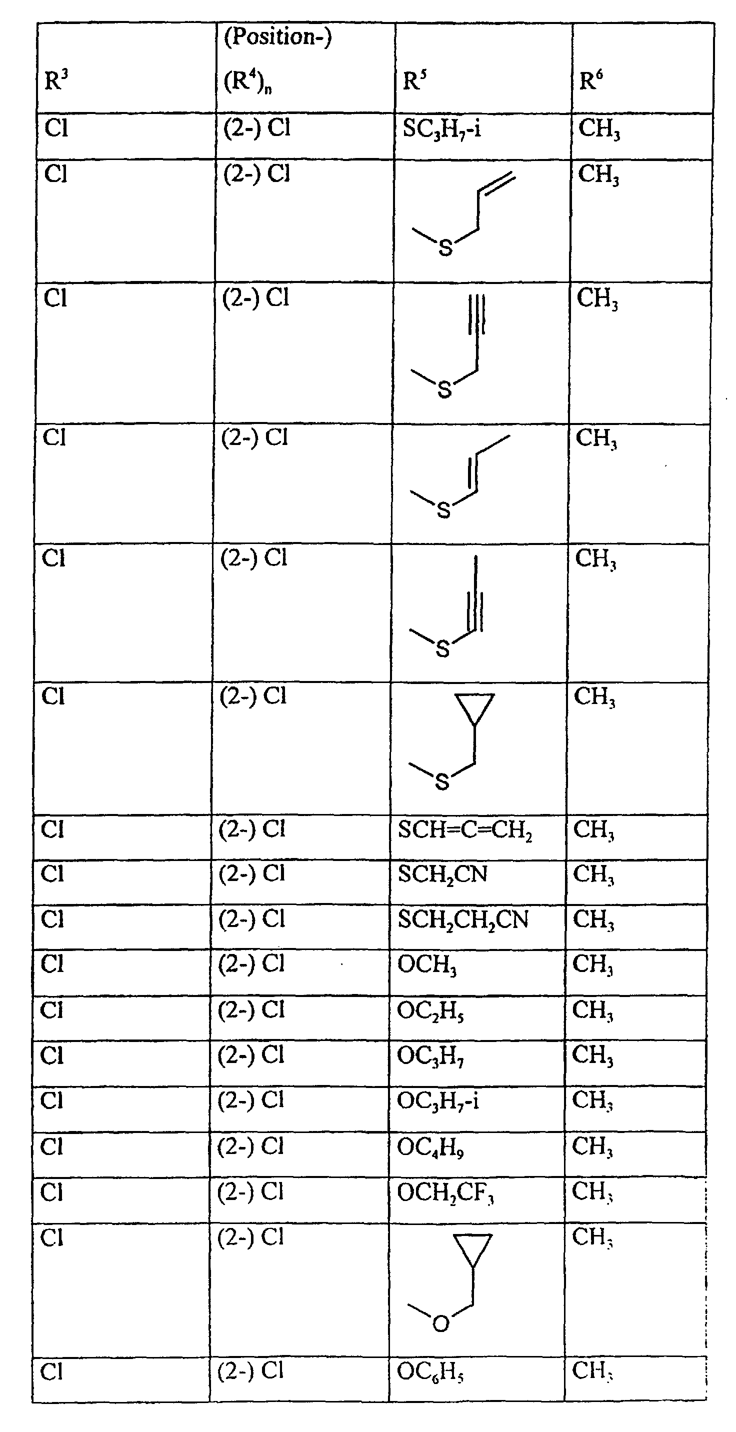

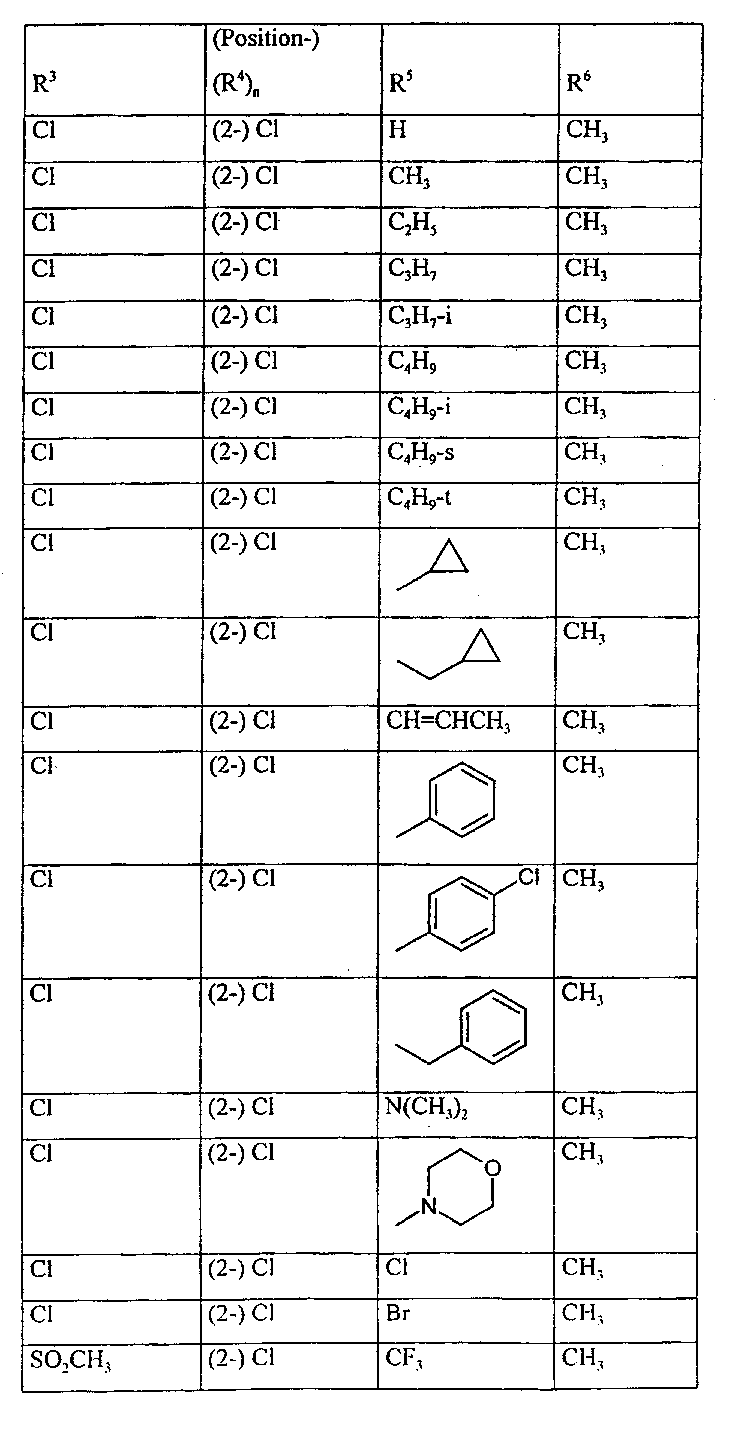

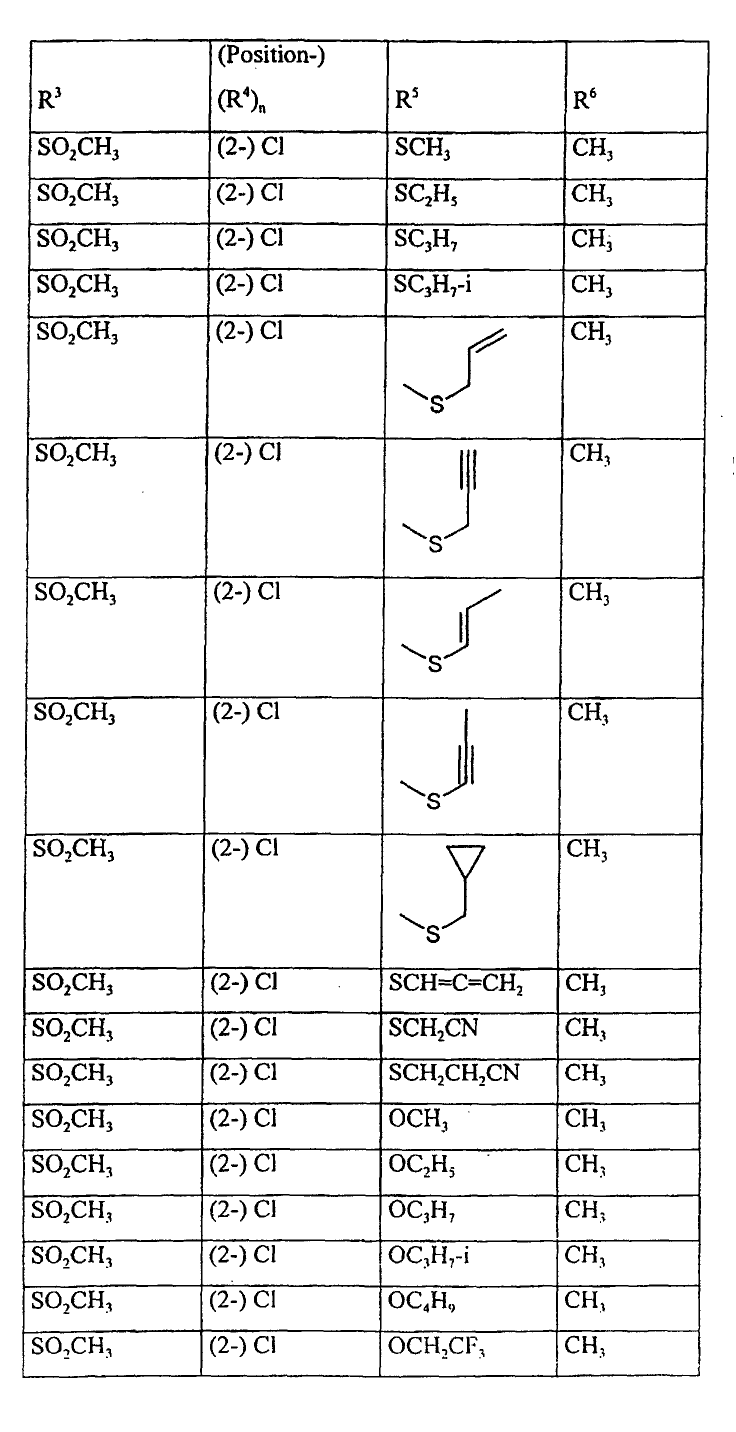

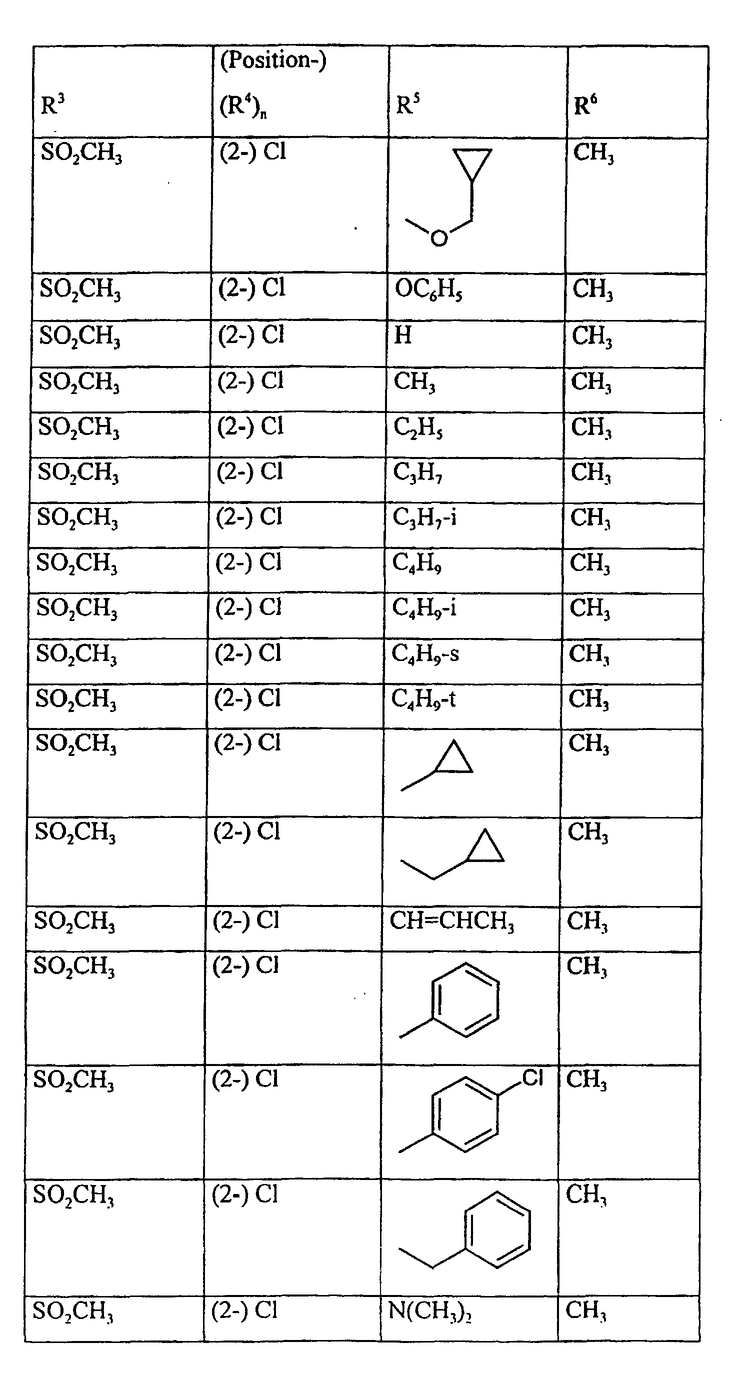

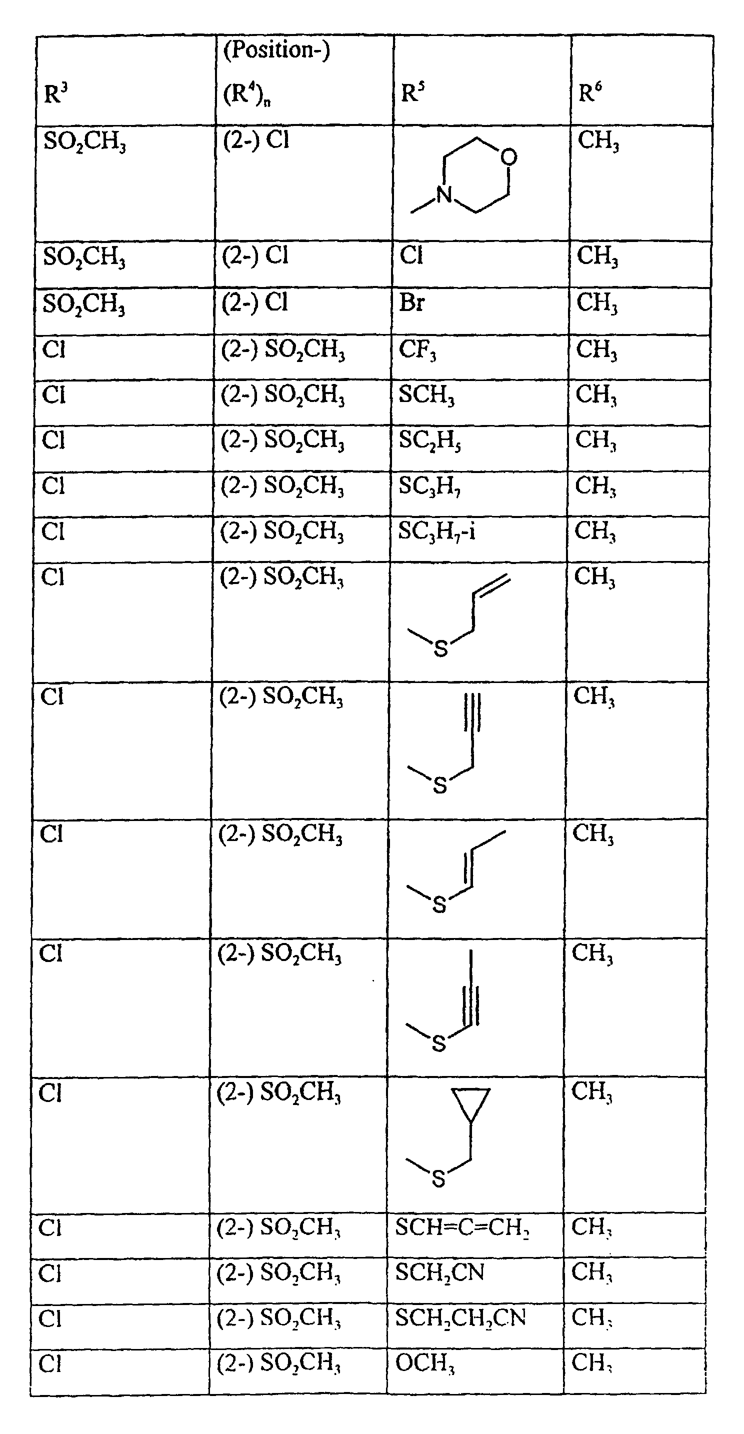

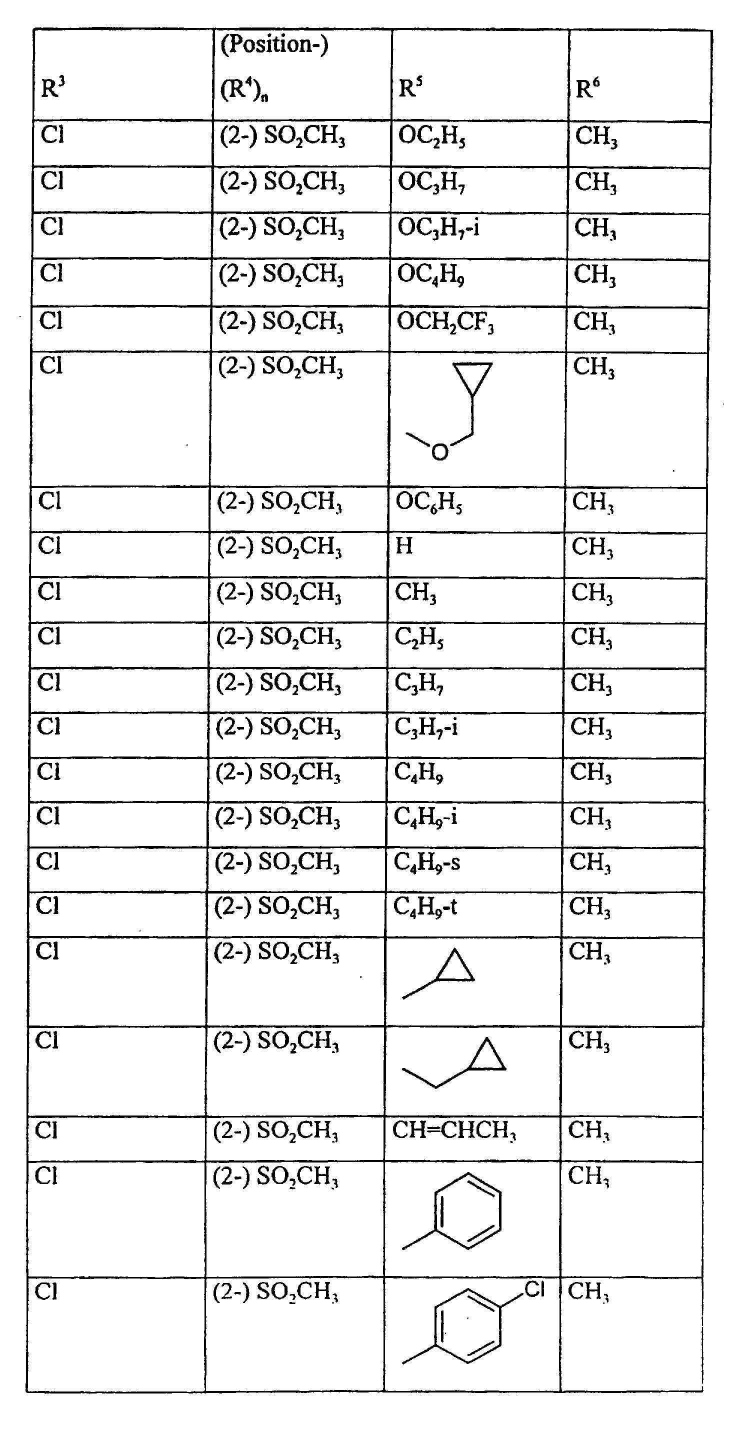

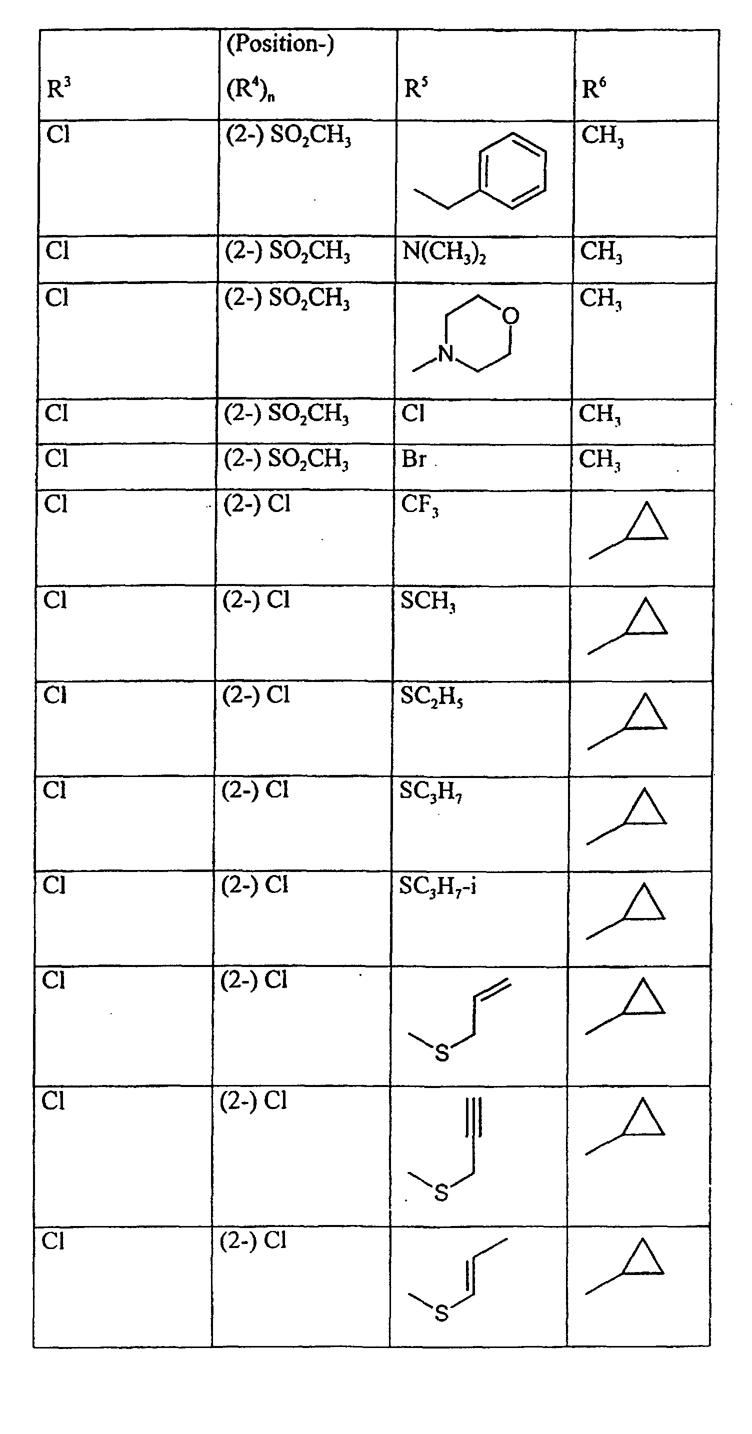

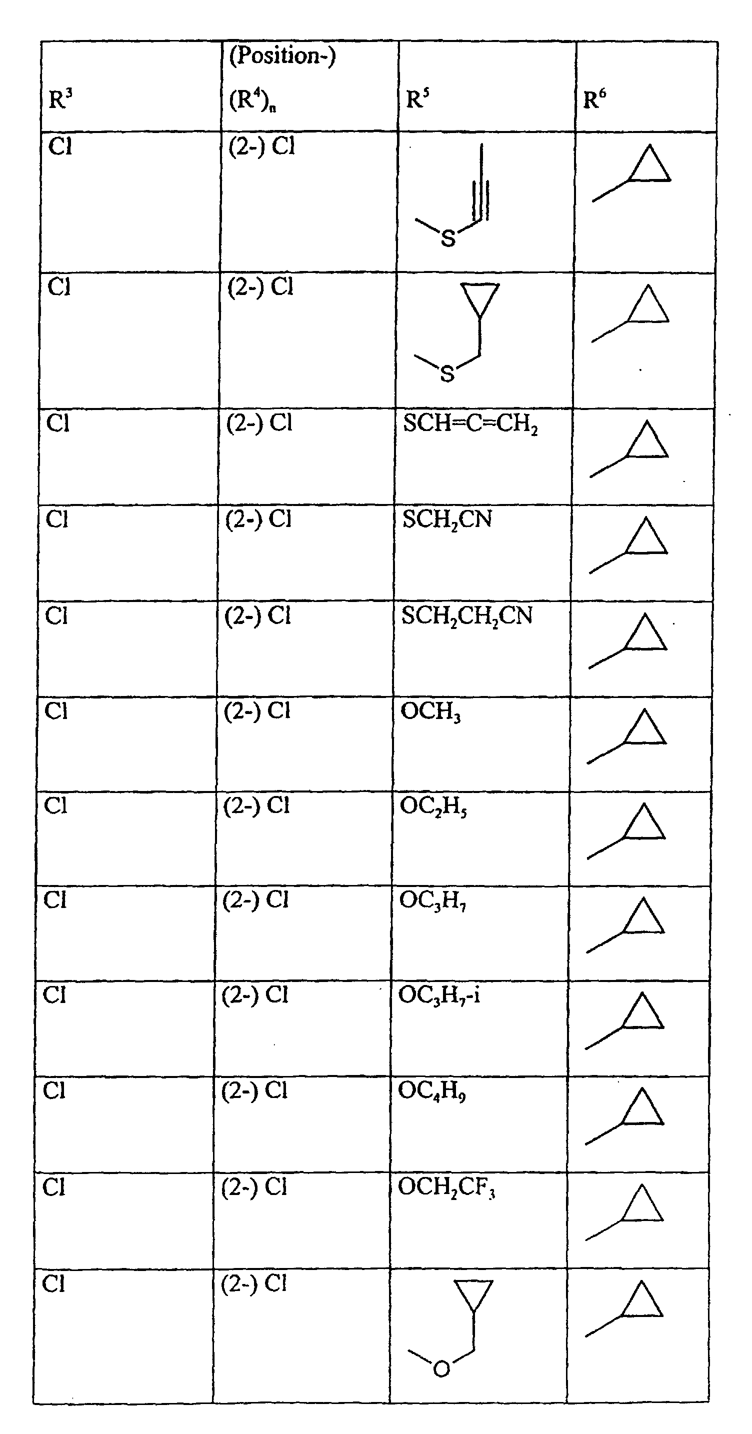

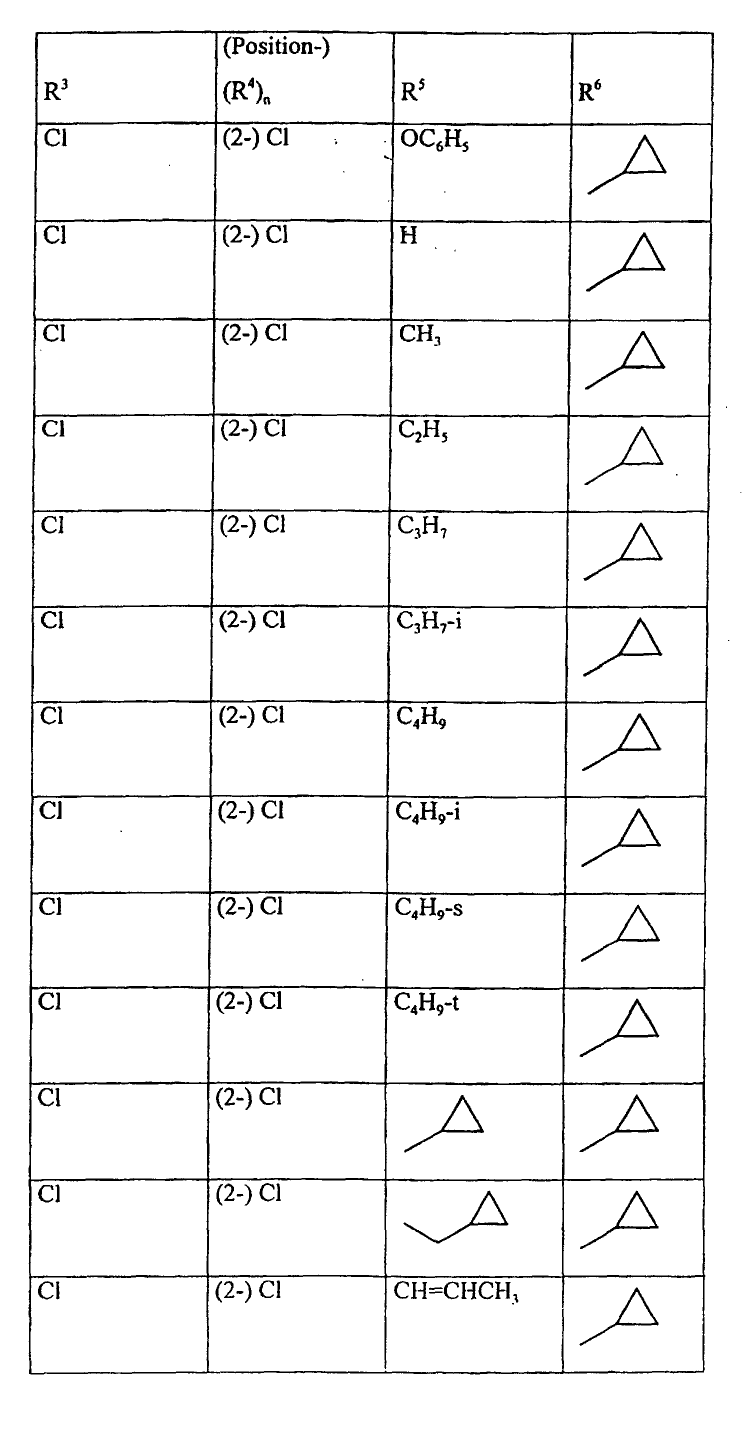

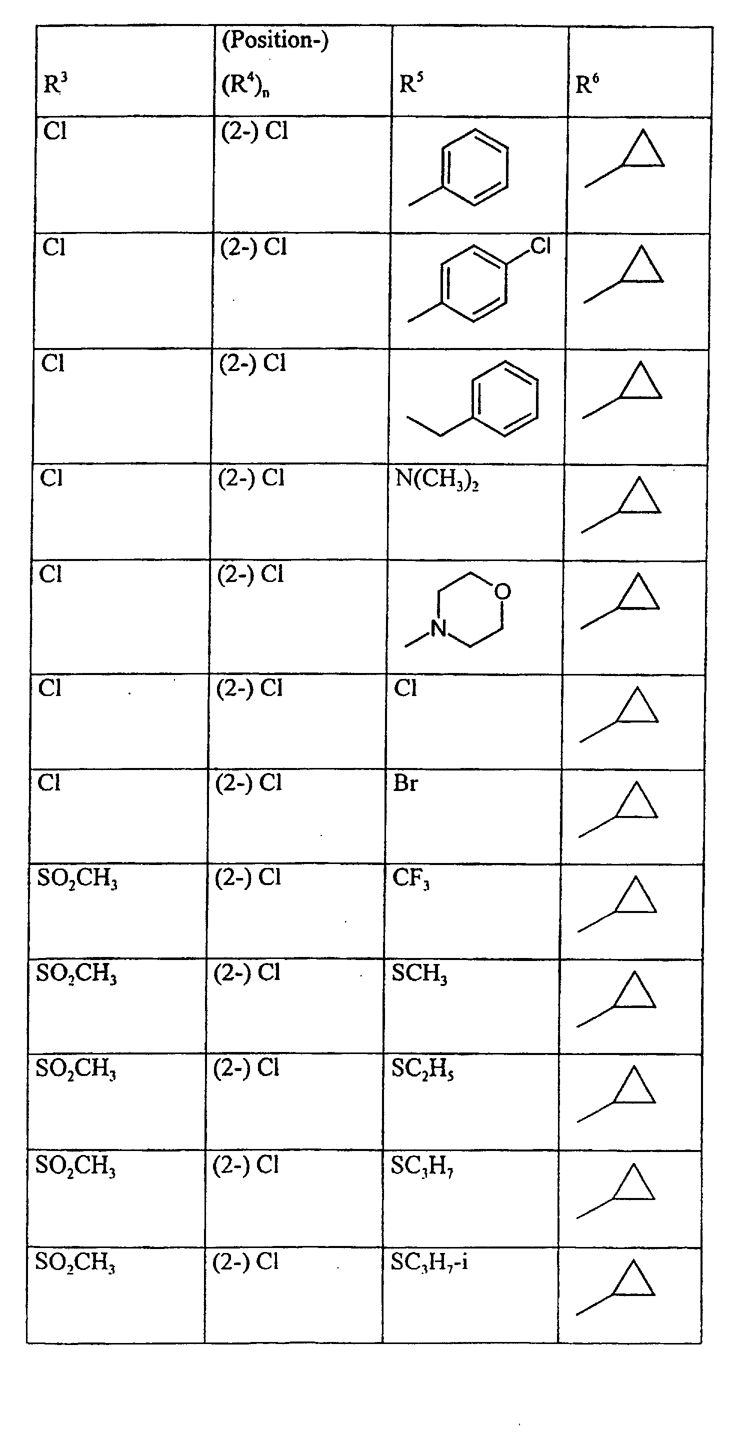

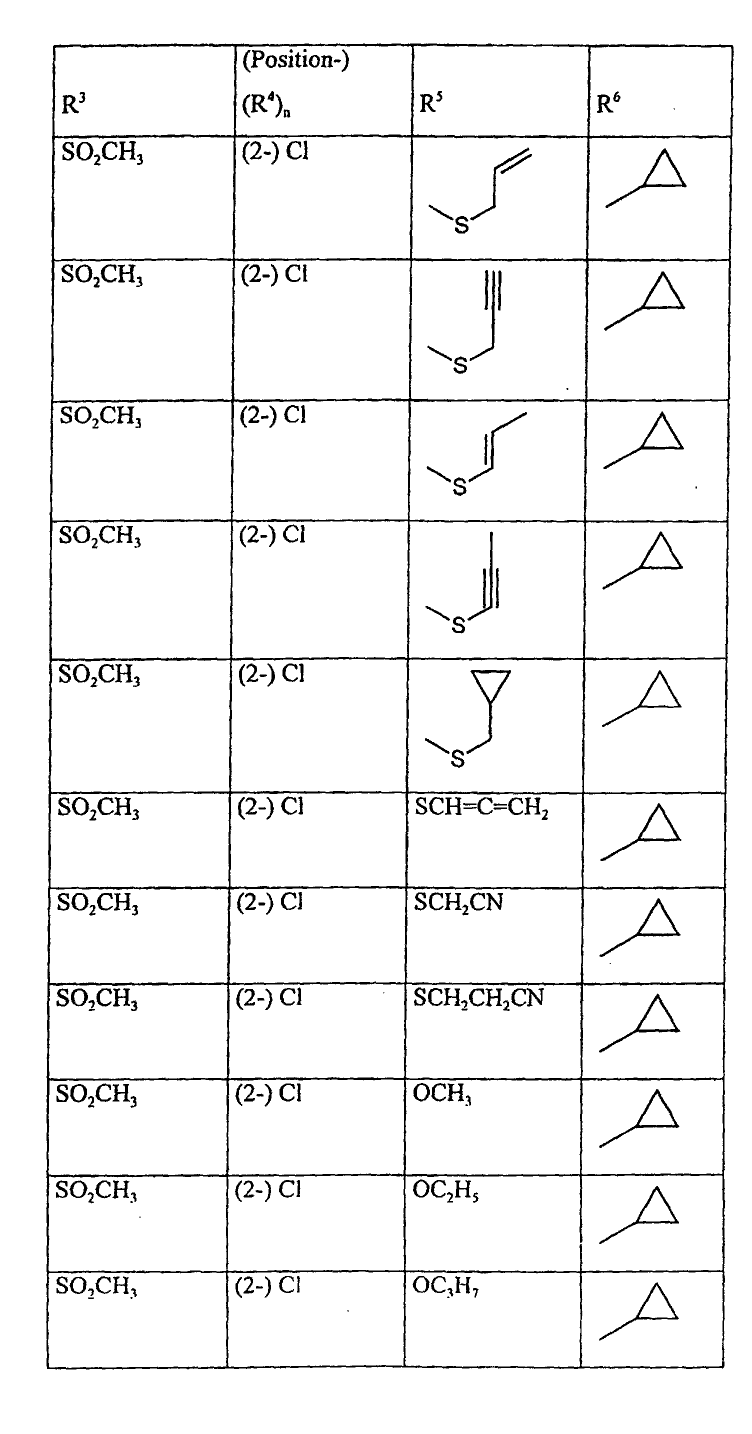

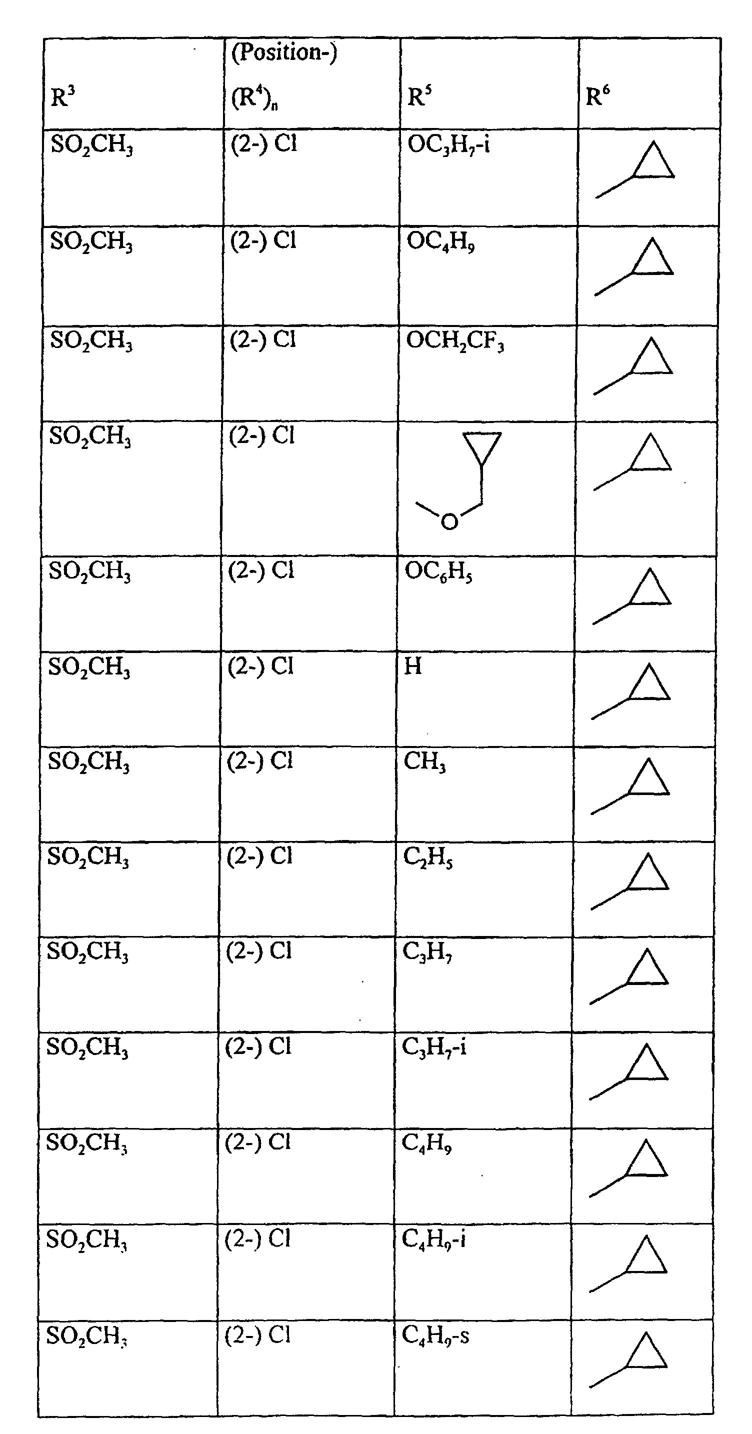

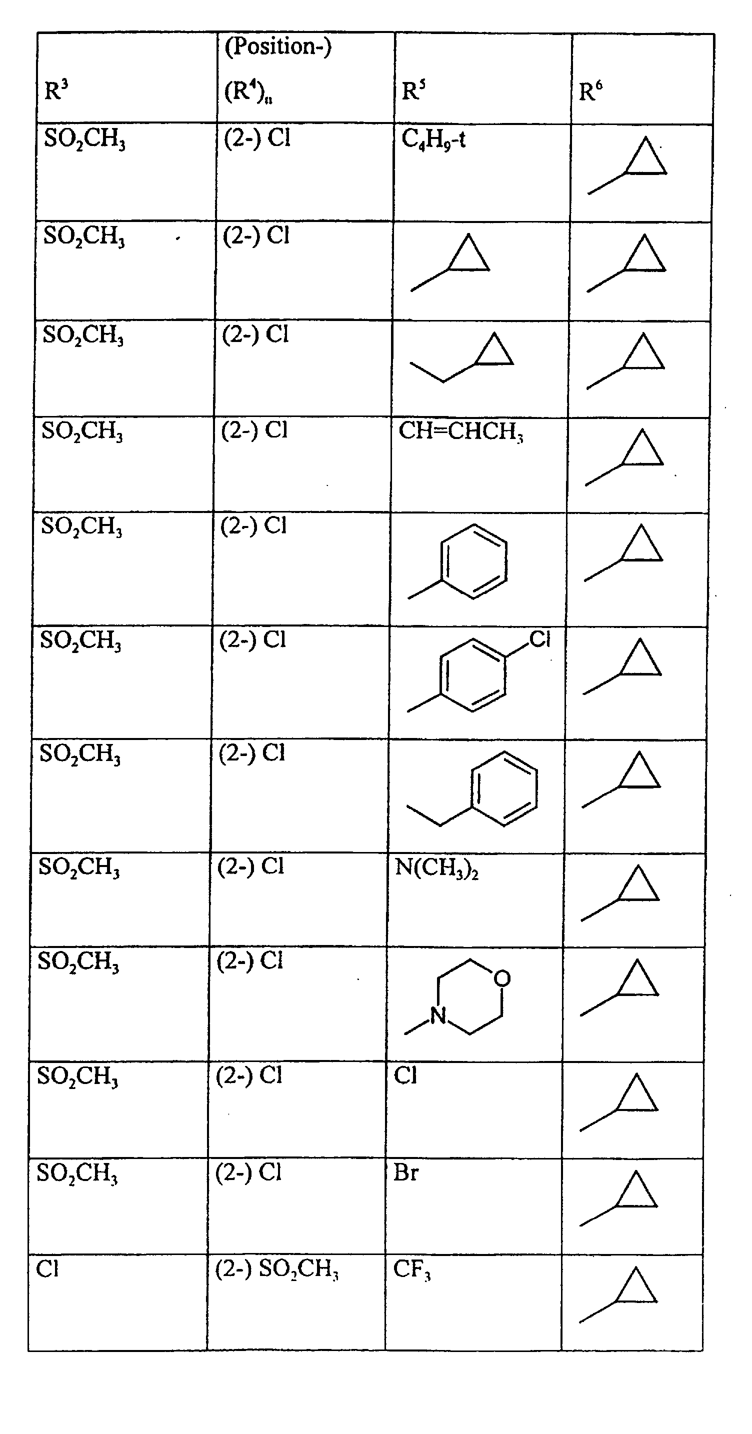

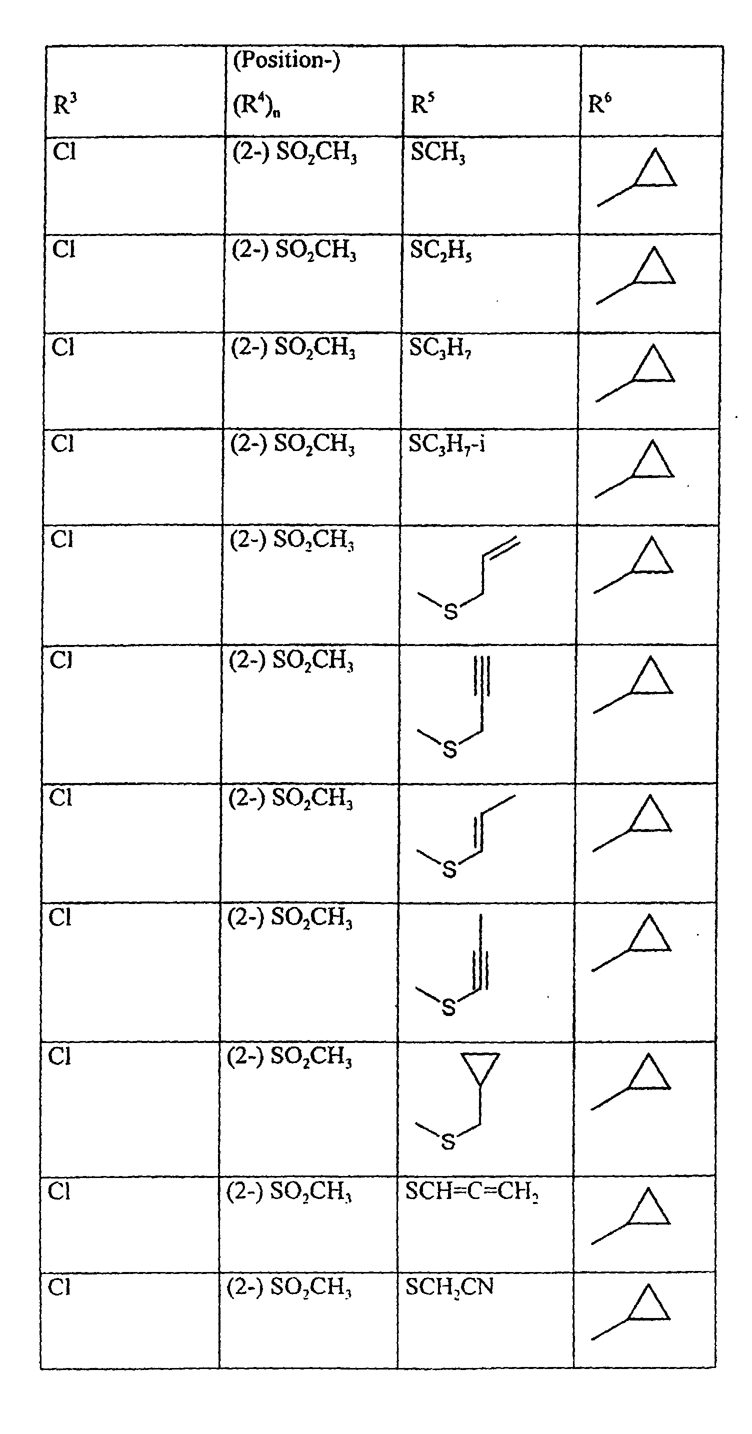

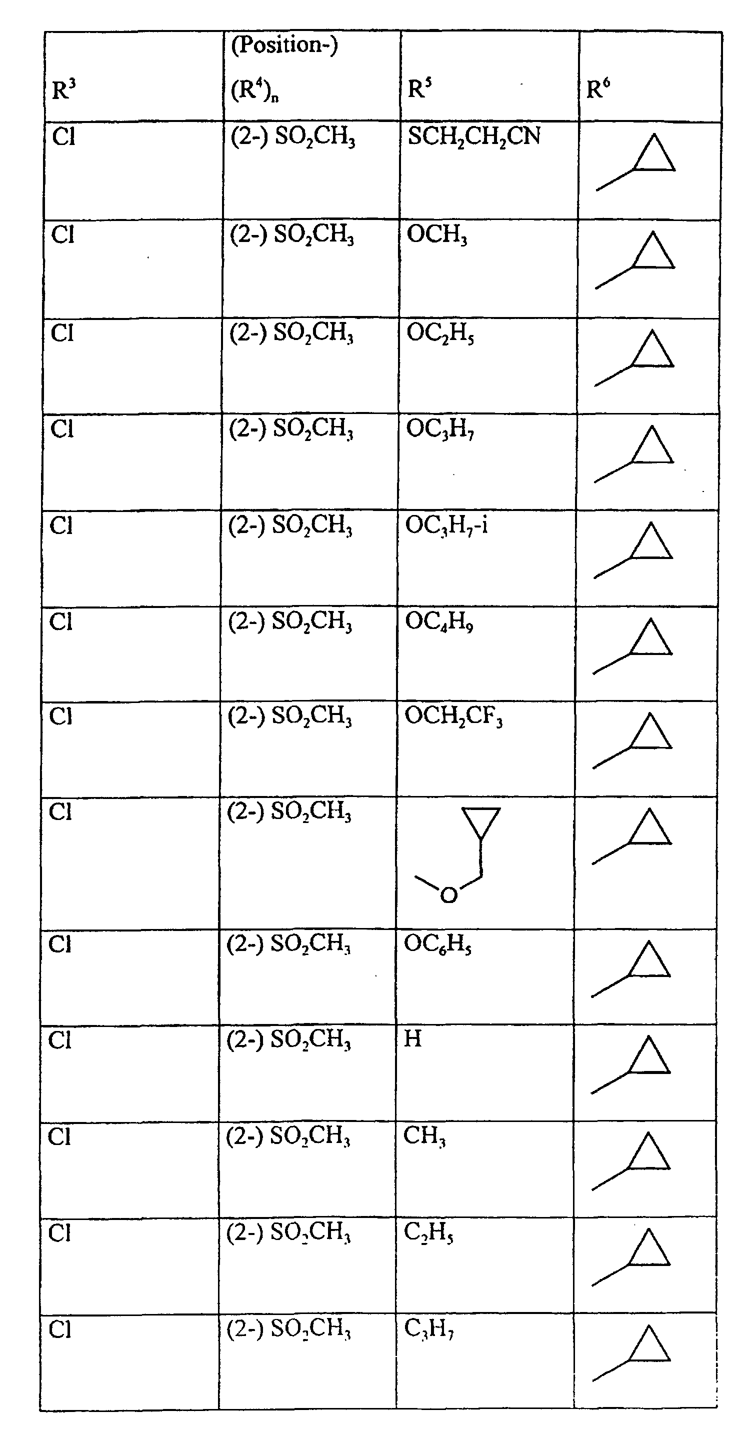

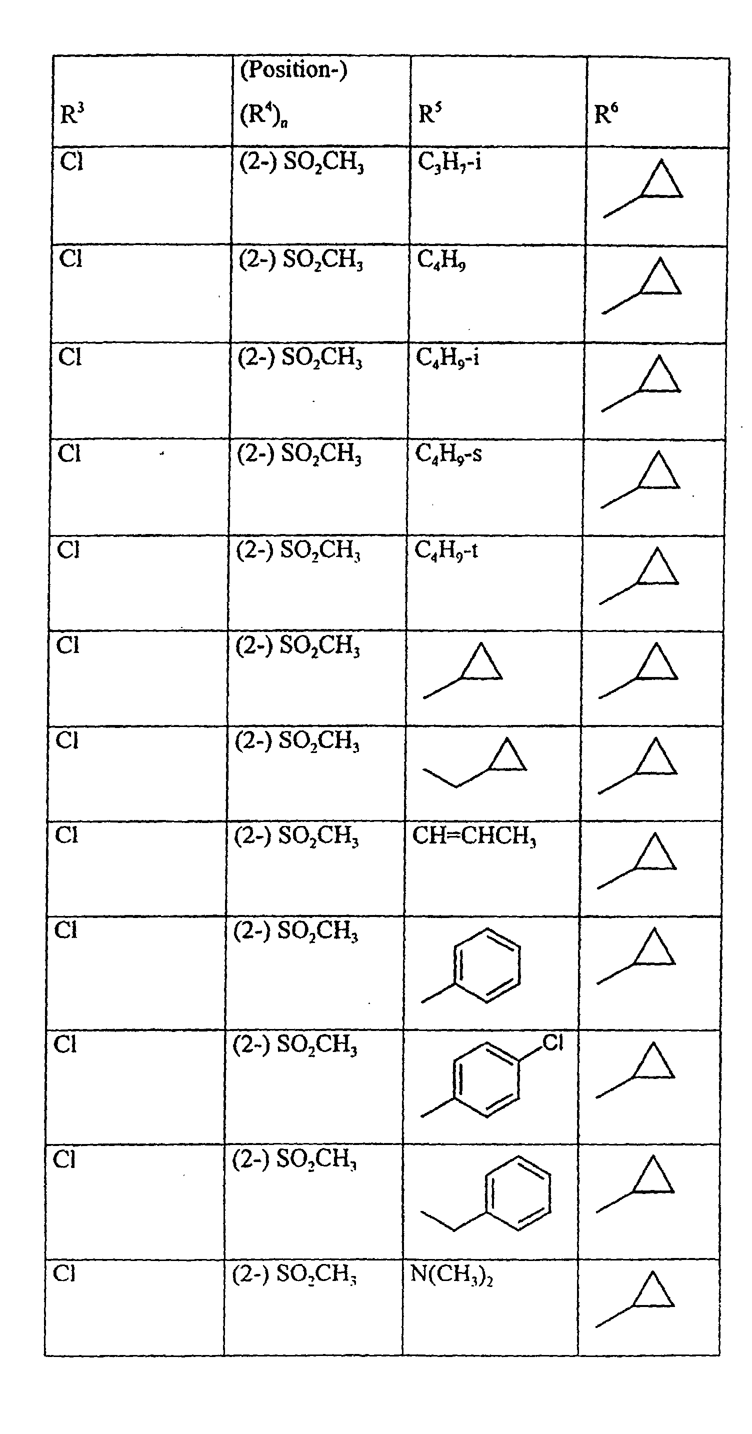

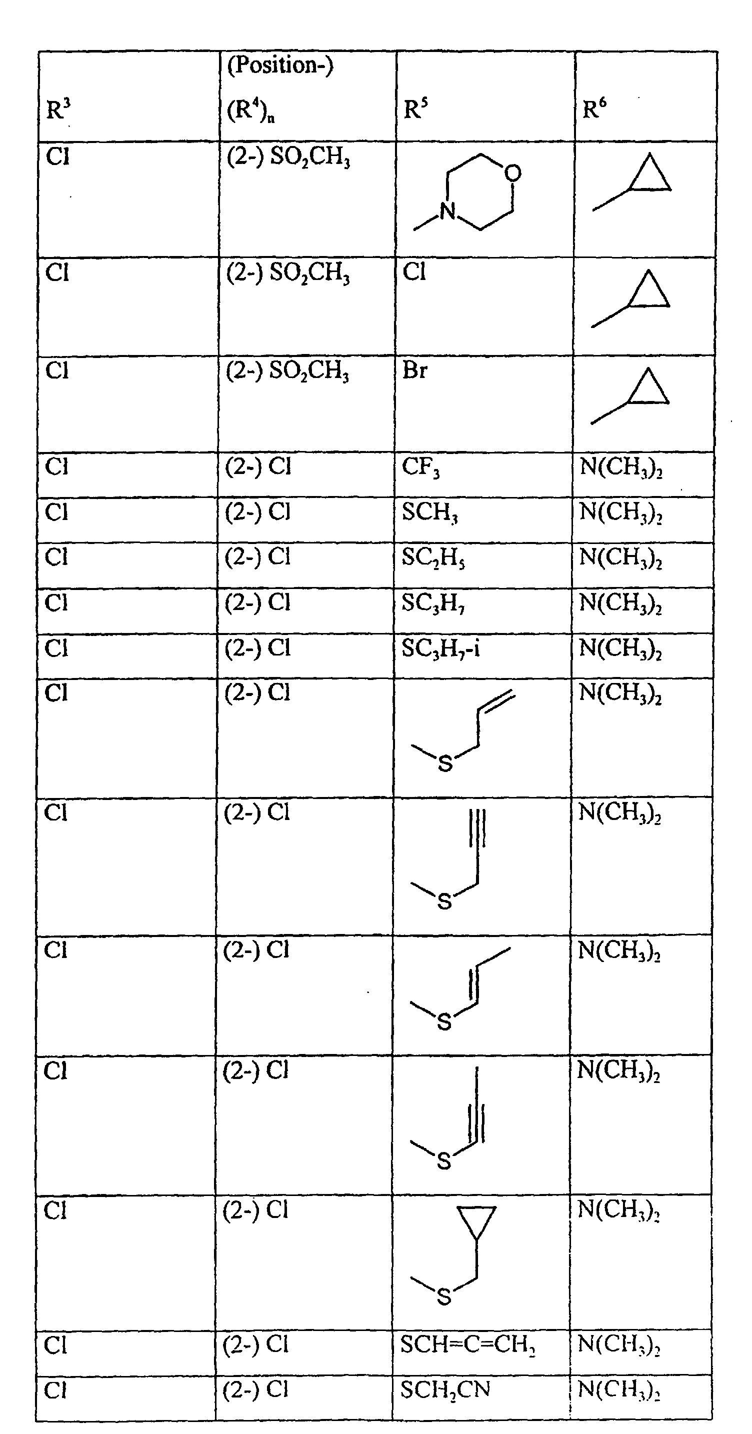

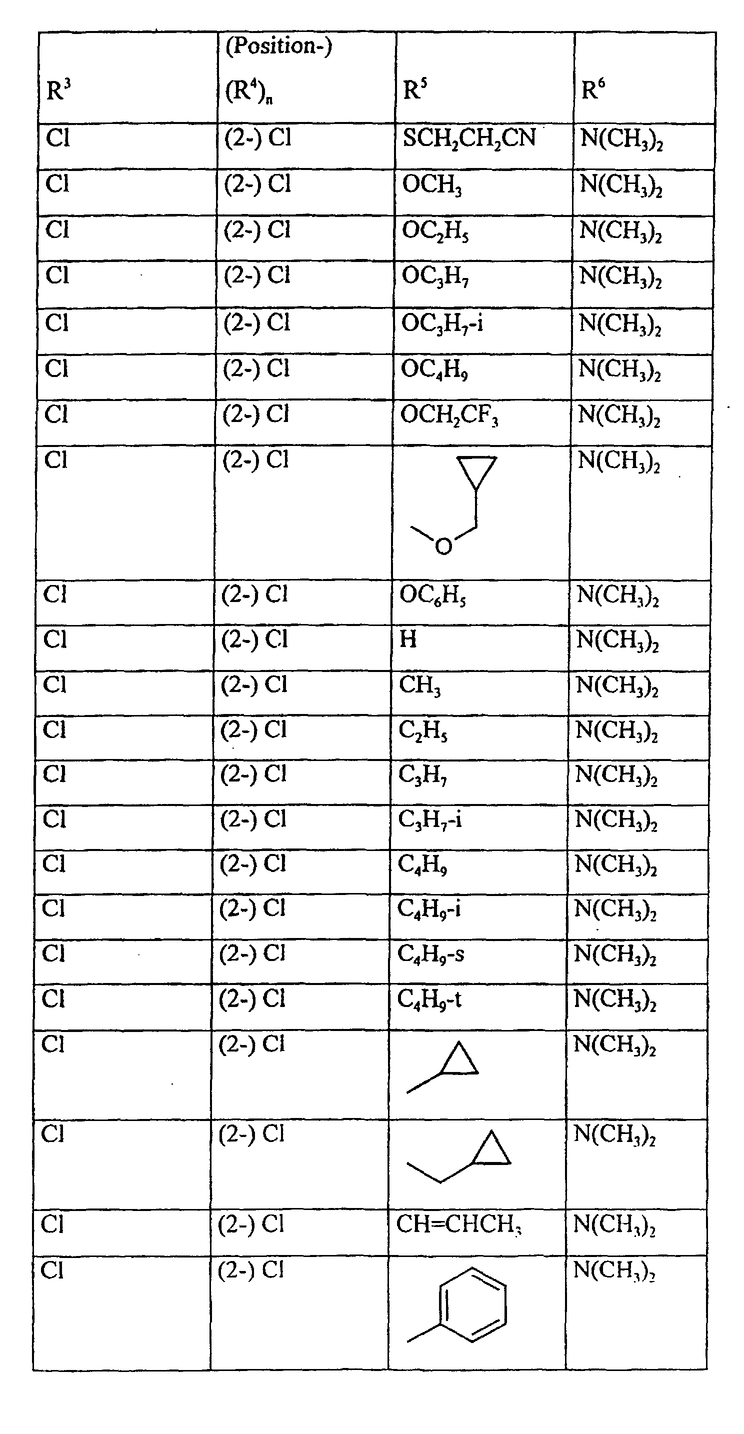

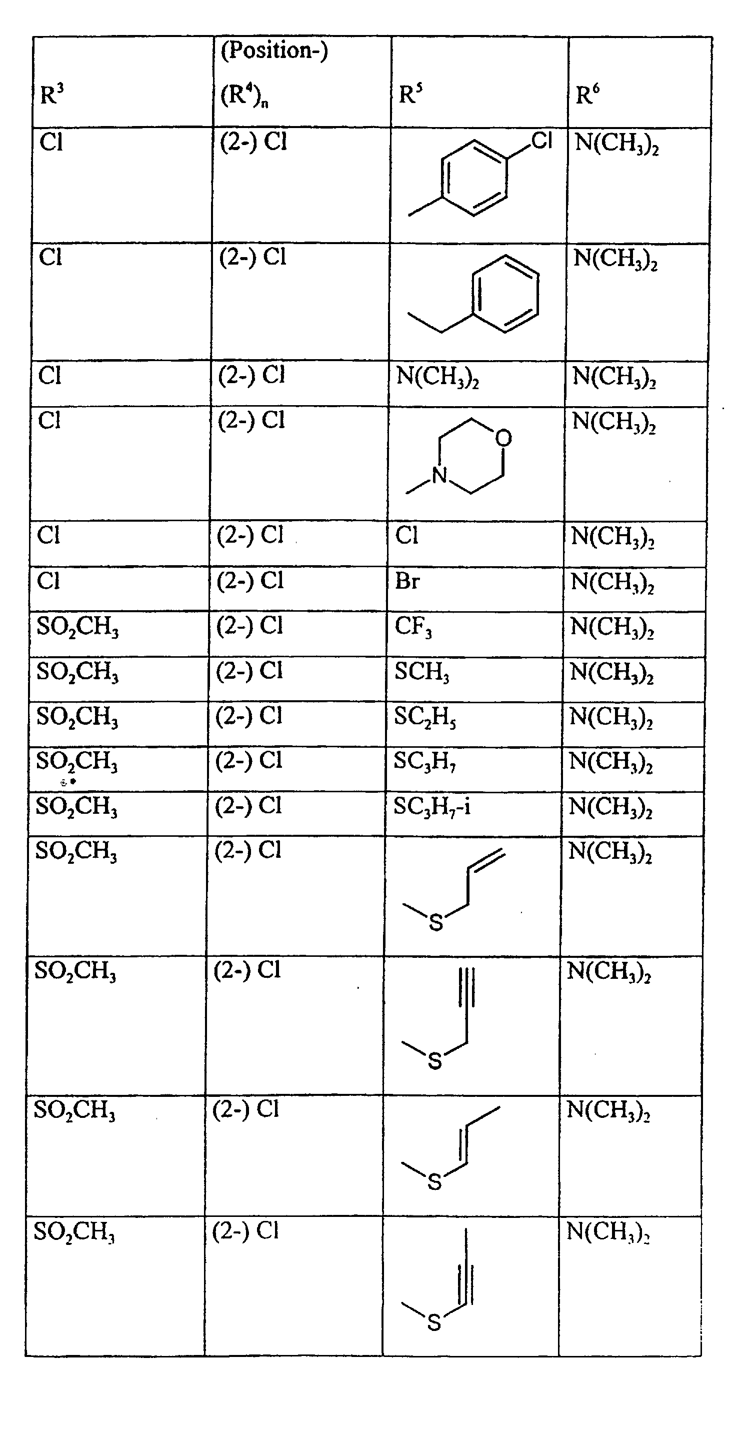

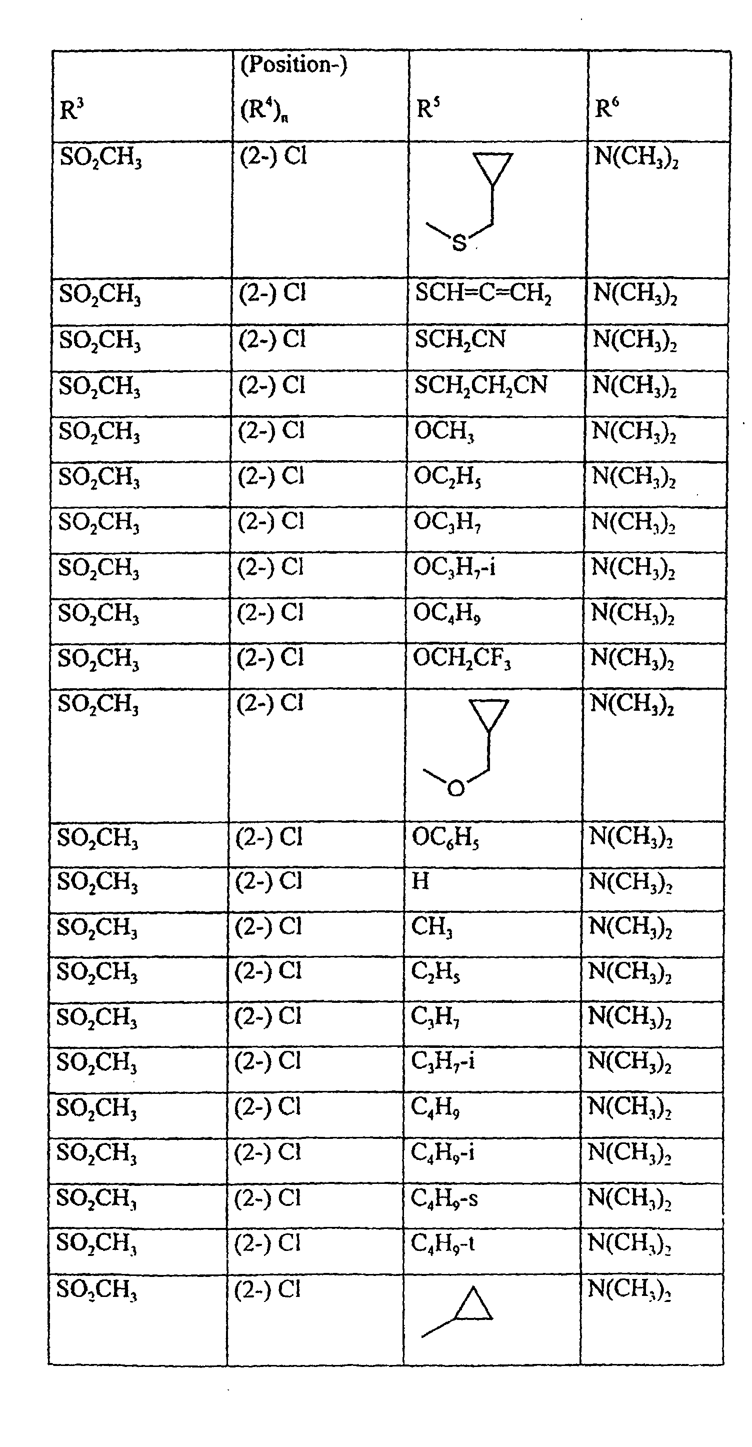

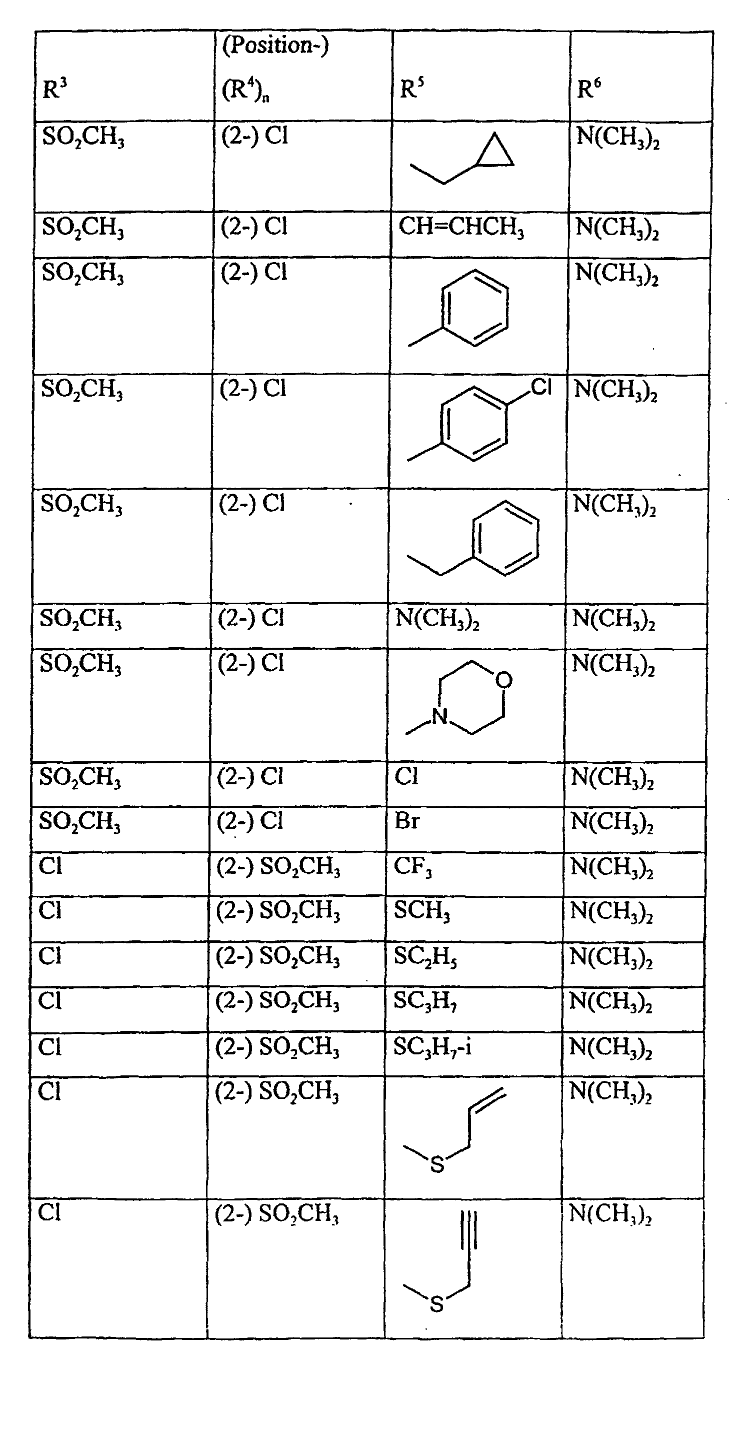

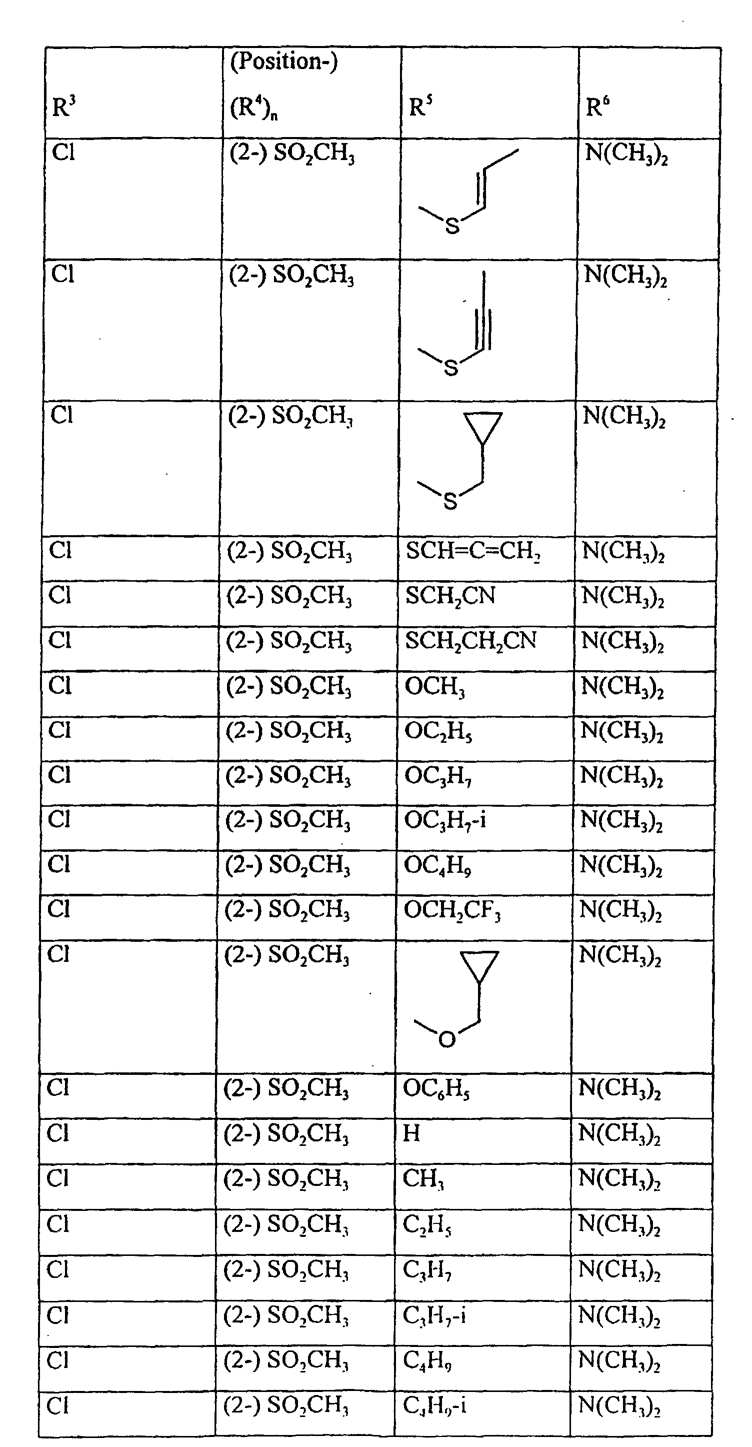

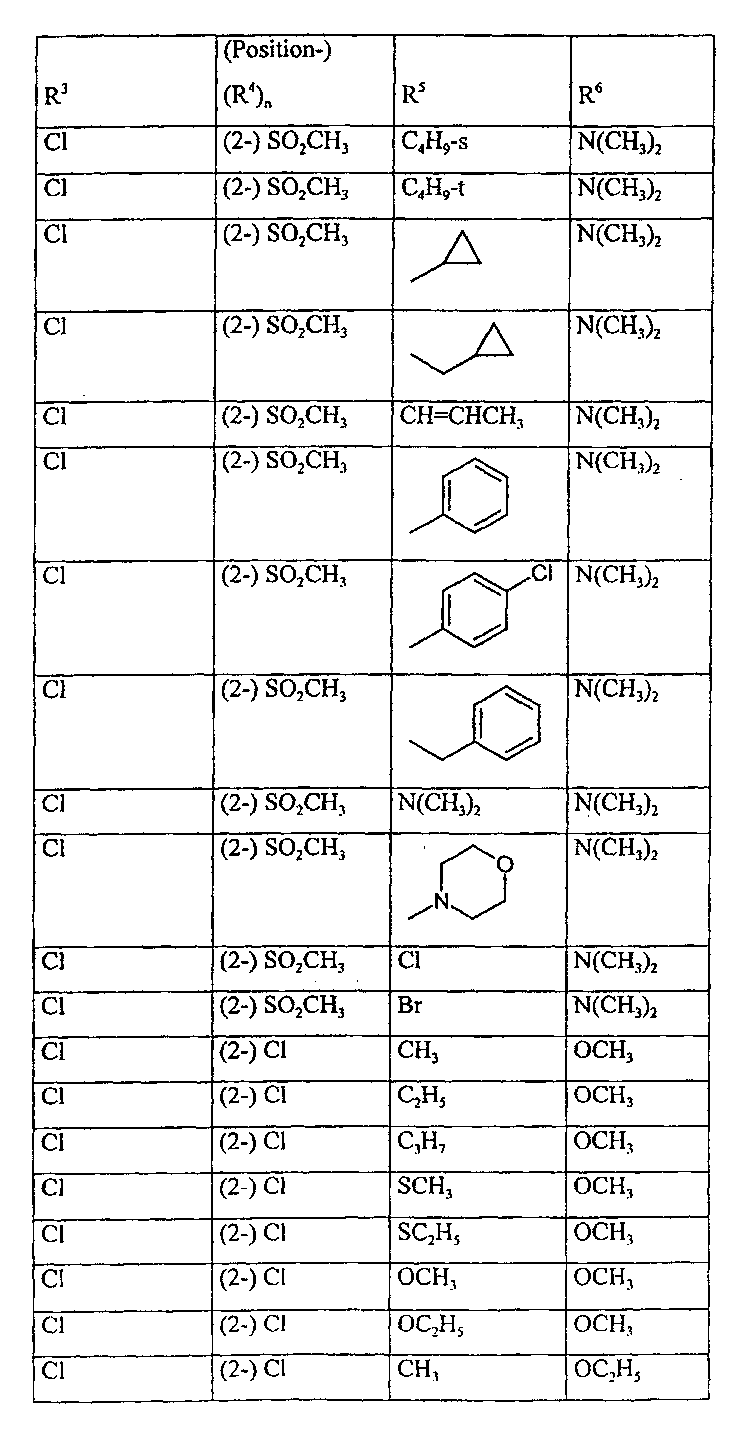

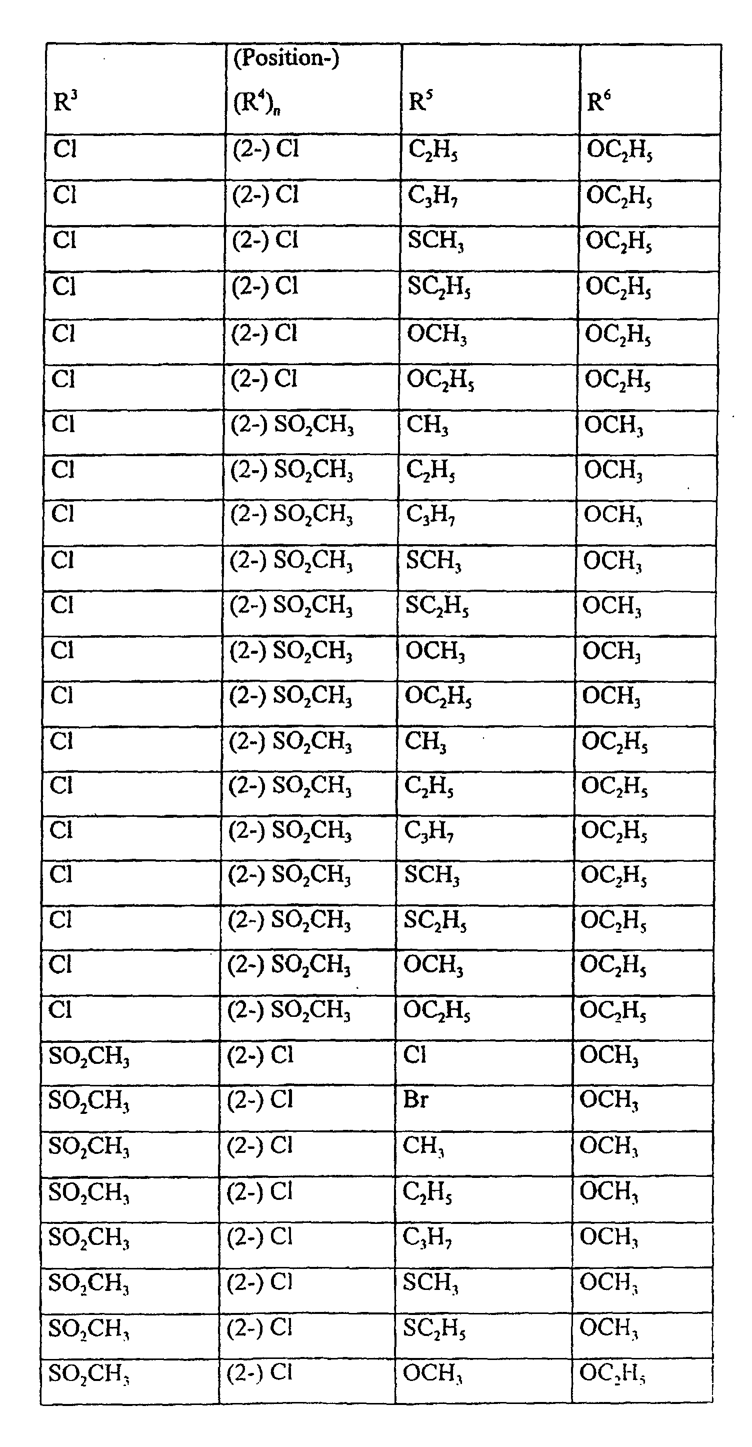

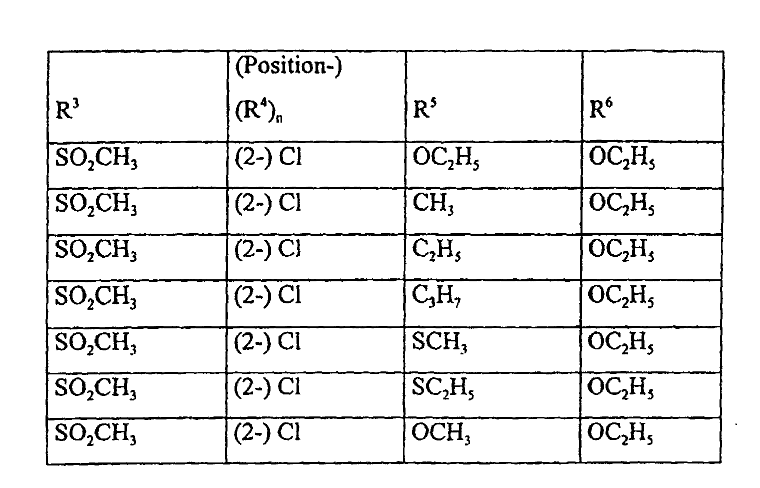

### Gruppe 5

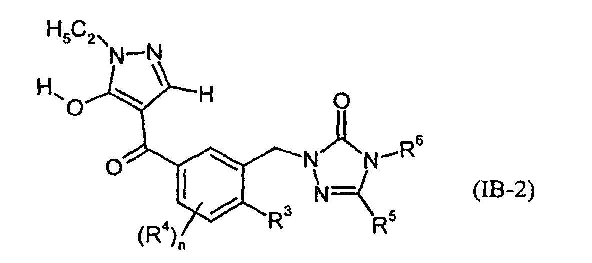

R³, (R⁴)ₙ, R⁵ und R⁶ haben hierbei beispielhaft die oben in Gruppe 4 angegebenen Bedeutungen.

### Gruppe 6

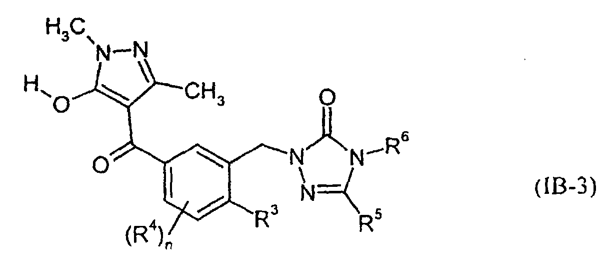

R³, (R⁴)ₙ, R⁵ und R⁶ haben hierbei beispielhaft die oben in Gruppe 4 angegebenen Bedeutungen.

### Gruppe 7

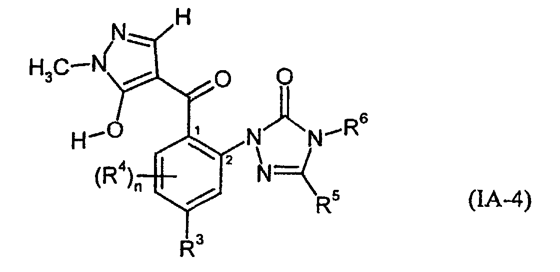

R³, (R⁴)ₙ, R⁵ und R⁶ haben hierbei beispielhaft die in der nachstehenden Tabelle angegebenen Bedeutungen:

| R³ | (Position-) (R⁴)ₙ | R⁵ | R⁶ |
|---|---|---|---|
| H | - | CF₃ | CH₃ |
| F | - | CF₃ | CH₃ |
| Cl | - | CF₃ | CH₃ |
| Br | - | CF₃ | CH₃ |

### Gruppe 8

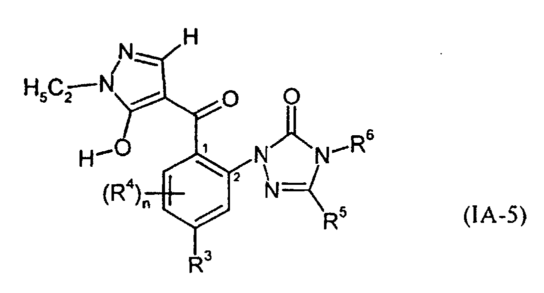

R³, (R⁴)ₙ, R⁵ und R⁶ haben hierbei beispielhaft die oben in Gruppe 7 angegebenen Bedeutungen.

### Gruppe 9

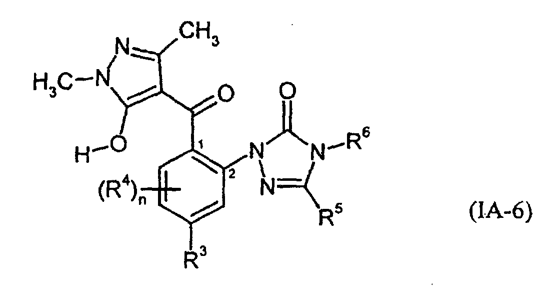

R³, (R⁴)ₙ, R⁵ und R⁶ haben hierbei beispielhaft die oben in Gruppe 7 angegebenen Bedeutungen.

### Gruppe 10

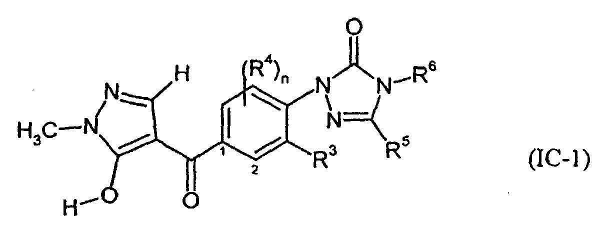

R³, (R⁴)ₙ, R⁵ und R⁶ haben hierbei beispielhaft die in der nachstehenden Tabelle angegebenen Bedeutungen:

| R³ | (Position-) (R⁴)ₙ | R⁵ | R⁶ |
|---|---|---|---|
| H | (2-) F | CF₃ | CH₃ |
| H | (2-) Cl | CF₃ | CH₃ |
| H | (2-) Br | CF₃ | CH₃ |
| H | - | CF₃ | CH₃ |

### Gruppe 11

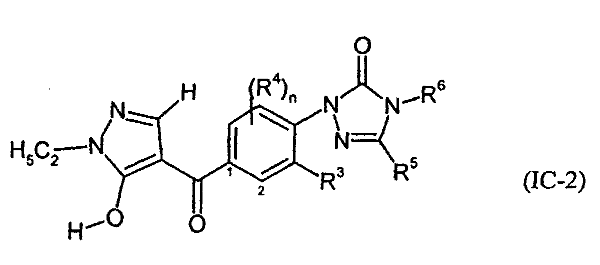

R³, (R⁴)ₙ, R⁵ und R⁶ haben hierbei beispielhaft die oben in Gruppe 10 angegebenen Bedeutungen.

### Gruppe 12

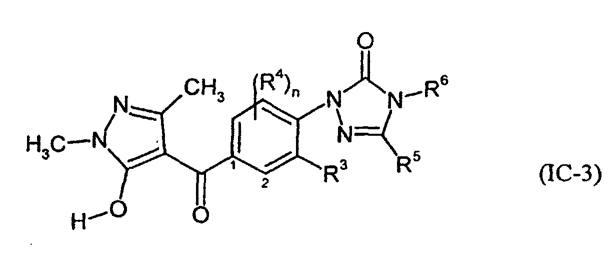

R³, (R⁴)ₙ, R⁵ und R⁶ haben hierbei beispielhaft die oben in Gruppe 10 angegebenen Bedeutungen.

Die neuen substituierten Benzoylpyrazole der allgemeinen Formel (I) zeichnen sich durch starke und selektive herbizide Wirksamkeit aus.

Man erhält die neuen substituierten Benzoylpyrazole der allgemeinen Formel (I), wenn man
(a) Pyrazole der allgemeinen Formel (II) 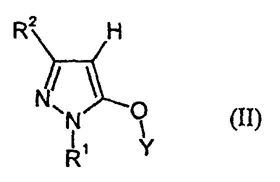 in welcher
   - R¹, R² und Y: die oben angegebene Bedeutung haben,
   mit substituierten Benzoesäuren der allgemeinen Formel (III), 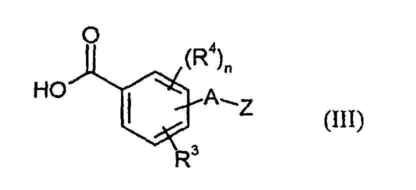 in welcher
   - n, A, R³, R⁴ und Z: die oben angegebene Bedeutung haben,
   in Gegenwart eines Dehydratisierungsmittels, gegebenenfalls in Gegenwart eines oder mehrerer Reaktionshilfsmittel und gegebenenfalls in Gegenwart eines Verdünnungsmittels umsetzt,
   oder wenn man
(b) Pyrazole der allgemeinen Formel (II) 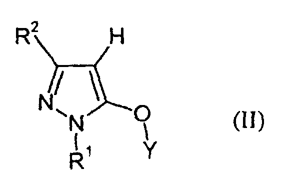 in welcher
   - R¹, R² und Y: die oben angegebene Bedeutung haben,
   mit substituierten Benzoesäurederivaten der allgemeinen Formel (IV) 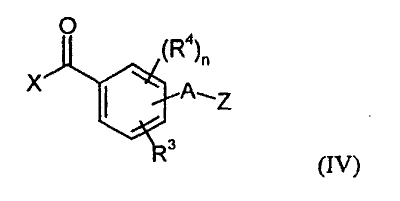 in welcher
   - n, A, R³, R⁴ und Z: die oben angegebene Bedeutung haben, und
   - X: für Cyano, Halogen oder Alkoxy steht,
   - oder mit entsprechenden Carbonsäureanhydriden -
   gegebenenfalls in Gegenwart eines oder mehrerer Reaktionshilfsmittel und gegebenenfalls in Gegenwart eines Verdünnungsmittels umsetzt,
   oder wenn man
(c) substituierte Benzoyipyrazole der allgemeinen Formel (Ia) 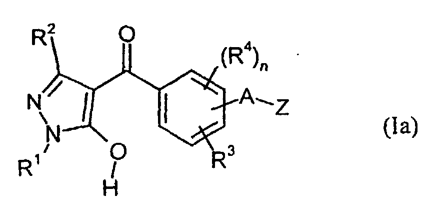 in welcher
   - n, A, R¹, R², R³, R⁴ und Z: die oben angegebene Bedeutung haben,
   mit Verbindungen der allgemeinen Formel (V)

   Cl - Y (V)

   in welcher
   - Y: mit Ausnahme von Wasserstoff die oben angegebene Bedeutung hat,
   - oder gegebenenfalls mit entsprechenden Isocyanaten oder Isothiocyanaten -
   gegebenenfalls in Gegenwart eines oder mehrerer Reaktionshilfsmittel und gegebenenfalls in Gegenwart eines Verdünnungsmittels umsetzt,
und gegebenenfalls im Anschluss daran an den so erhaltenen Verbindungen der Formel (I) im Rahmen der Substituentendefinition auf übliche Weise elektrophile oder nucleophile bzw. Oxidations- oder Reduktionsreaktionen durchführt oder die Verbindungen der Formel (I) auf übliche Weise in Salze überführt.

Die Verbindungen der Formel (I) können nach üblichen Methoden in andere Verbindungen der Formel (I) gemäß obiger Definition umgewandelt werden, beispielsweise durch nucleophile Substitution (z.B. R⁵: Cl → OC₂H₅, SCH₃) oder durch Oxidation (z.B. R⁵: CH₂SCH₃ → CH₂S(O)CH₃).

Verwendet man beispielsweise 3-Chlor-5-hydroxy-1-methyl-pyrazol und 2-(3-Carboxy-5-fluor-benzyl)-5-ethyl-4-methoxy-2,4-dihydro-3H-1,2,4-triazol-3-on als Ausgangsstoffe, so kann der Reaktionsablauf beim erfindungsgemäßen Verfahren (a) durch das folgende Formelschema skizziert werden:

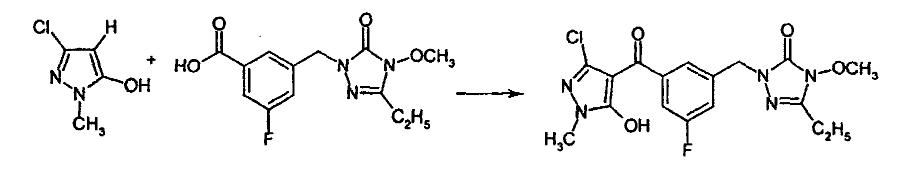

Verwendet man beispielsweise 3-Cyano-5-hydroxy-1-ethyl-pyrazol und 2-(3-Methoxycarbonyl-5-chlor-benzyl)-4-ethyl-5-methylthio-2,4-dihydro-3H-1,2,4-triazol-3-on als Ausgangsstoffe, so kann der Reaktionsablauf beim erfindungsgemäßen Verfahren (b) durch das folgende Formelschema skizziert werden:

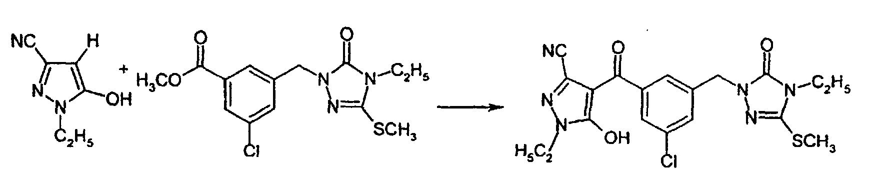

Verwendet man beispielsweise 4-Methyl-5-trifluormethyl-2-[3-chlor-4-(1-ethyl-5-hydroxy-pyrazol-4-yl-carbonyl)-phenyl]-2,4-dihydro-3H-1,2,4-triazol-3-on und Benzoylchlorid als Ausgangsstoffe, so kann der Reaktionsablauf beim erfindungsgemäßen Verfahren (c) durch das folgende Formelschema skizziert werden:

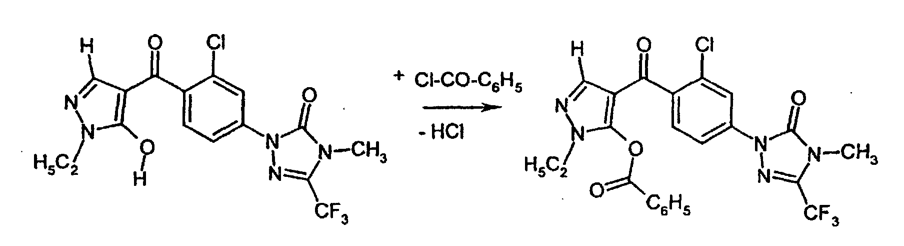

Die beim erfindungsgemäßen Verfahren (a) zur Herstellung von Verbindungen der allgemeinen Formel (I) als Ausgangsstoffe zu verwendenden Pyrazole sind durch die Formel (II) allgemein definiert. In der allgemeinen Formel (II) haben R¹. R² und Y vorzugsweise diejenigen Bedeutungen, die bereits oben im Zusammenhang mit der Beschreibung der erfindungsgemäßen Verbindungen der allgemeinen Formel (I) als bevorzugt, besonders bevorzugt, ganz besonders bevorzugt oder am meisten bevorzugt für R¹, R² und Y angegeben worden sind.

Die Ausgangsstoffe der allgemeinen Formel (II) sind bekannt und/oder können nach an sich bekannten Verfahren hergestellt werden (vgl. EP-A-240001).

Die beim erfindungsgemäßen Verfahren (a) weiter als Ausgangsstoffe zu verwendenden substituierten Benzoesäuren sind durch die Formel (III) allgemein definiert. In der Formel (III) haben n, A, R³, R⁴ und Z vorzugsweise diejenigen Bedeutungen, die bereits oben im Zusammenhang mit der Beschreibung der erfindungsgemäßen Verbindungen der Formel (I) als bevorzugt, besonders bevorzugt, ganz besonders bevorzugt oder am meisten bevorzugt für n, A, R³, R⁴ und Z angegeben wurden.

Die Ausgangsstoffe der allgemeinen Formel (III) sind mit Ausnahme von 2-(5-Carboxy-2,4-dichlor-phenyl)-4-difluormethyl-5-methyl-2,4-dihydro-3H-1,2,4-triazol-3-on - alias 2,4-Dichlor-5-(4-difluormethyl-4,5-dihydro-3-methyl-5-oxo-1H-1,2,4-triazol-1-yl)-benzoesäure (CAS-Reg.-Nr. 90208-77-8) und 2-(5-Carboxy-2,4-dichlor-phenyl)-4,5-dimethyl-2,4-dihydro-3H-1,2,4-triazol-3-on- alias 2,4-Dichlor-5-(4,5-dihydro-3,4-dimethyl-5-oxo-1H-1,2,4-triazol-1-yl)-benzoesäure (CAS-Reg.-Nr. 90208-76-7) - noch nicht aus der Literatur bekannt. Sie sind jedoch unter Ausnahme von 2-(5-Carboxy-2,4-dichlor-phenyl)-4-difluormethyl-5-methyl-2,4-dihydro-3H-1,2,4-triazol-3-on und 2-(5-Carboxy-2,4-dichlor-phenyl)-4,5-dimethyl-2,4-dihydro-3H-1,2,4-triazol-3-on (vgl. JP-A-58225070 - zitiert in Chem. Abstracts 100:209881, JP-A-02015069 - zitiert in Chem. Abstracts 113:23929) Gegenstand einer vorgängigen, jedoch nicht vorveröffentlichten Anmeldung (vgl. DE-A-19833360).

Man erhält die substituierten Benzoesäuren der allgemeinen Formel (III). wenn man Benzoesäurederivate der allgemeinen Formel (VI), 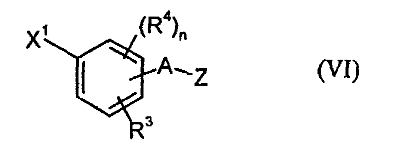 in welcher
- n, A, R³ und R⁴ und Z: die oben angegebene Bedeutung haben, und
- X¹: für Cyano, Carbamoyl, Halogencarbonyl oder Alkoxycarbonyl steht,
mit Wasser, gegebenenfalls in Gegenwart eines Hydrolysehilfsmittels, wie z.B. Schwefelsäure, bei Temperaturen zwischen 50°C und 120°C umsetzt (vgl. die Herstellungsbeispiele).

Die beim erfindungsgemäßen Verfahren (b) zur Herstellung von Verbindungen der allgemeinen Formel (I) als Ausgangsstoffe zu verwendenden substituierten Benzoesäurederivate sind durch die Formel (IV) allgemein definiert. In der allgemeinen Formel (IV) haben n, A, R³, R⁴ und Z vorzugsweise diejenigen Bedeutungen, die bereits oben im Zusammenhang mit der Beschreibung der erfindungsgemäßen Verbindungen der allgemeinen Formel (I) als bevorzugt, besonders bevorzugt, ganz besonders bevorzugt oder am meisten bevorzugt für n, A, R³, R⁴ und Z angegeben worden sind; X steht vorzugsweise für Cyano, Fluor, Chlor, Brom oder C₁-C₄-Alkoxy, insbesondere für Chlor, Methoxy oder Ethoxy.

Die Ausgangsstoffe der allgemeinen Formel (IV) - sowie die Vorprodukte der allgemeinen Formel (VI) - sind bekannt und/oder können nach an sich bekannten Verfahren hergestellt werden (vgl. DE-A-3839480, DE-A-4239296, EP-A-597360, EP-A-609734, DE-A-4303676, EP-A-617026, DE-A-4405614, US-A-5378681).

Die beim erfindungsgemäßen Verfahren (c) zur Herstellung von Verbindungen der allgemeinen Formel (I) als Ausgangsstoffe zu verwendenden substituierten Benzoylpyrazole sind durch die Formel (Ia) allgemein definiert. In der allgemeinen Formel (Ia) haben n, A, R¹, R², R³, R⁴ und Z vorzugsweise diejenigen Bedeutungen, die bereits oben im Zusammenhang mit der Beschreibung der erfindungsgemäßen Verbindungen der allgemeinen Formel (I) als bevorzugt, besonders bevorzugt, ganz besonders bevorzugt oder am meisten bevorzugt für n, A, R¹, R², R³, R⁴ und Z angegeben worden sind.

Die Ausgangsstoffe der allgemeinen Formel (Ia) sind endungsgemäße, neue Verbindungen; sie können nach den erfindungsgemäßen Verfahren (a) und (b) hergestellt werden.

Die beim erfindungsgemäßen (c) weiter als Ausgangsstoffe zu verwendenden Verbindungen sind durch die Formel (V) allgemein definiert. In der allgemeinen Formel (V) hat Y vorzugsweise diejenige Bedeutung, die bereits oben im Zusammenhang mit der Beschreibung der erfindungsgemäßen Verbindungen der allgemeinen Formel (I) als bevorzugt, besonders bevorzugt, ganz besonders bevorzugt oder am meisten bevorzugt für Y angegeben worden ist.

Die Ausgangsstoffe der allgemeinen Formel (V) sind bekannte Synthesechemikalien.

Das erfindungsgemäße Verfahren (a) zur Herstellung der neuen substituierten Benzoylpyrazole der allgemeinen Formel (I) wird unter Verwendung eines Dehydratisierungsmittels durchgeführt. Es kommen hierbei die üblichen zur Bindung von Wasser geeigneten Chemikalien in Betracht.

Als Beispiele hierfür seien Dicyclohexylcarbodiimid und Carbonyl-bis-imidazol genannt.

Als besonders gut geeignetes Dehydratisierungsmittel sei Dicyclohexylcarbodiimid genannt.

Das erfindungsgemäße Verfahren (a) zur Herstellung der neuen substituierten Benzoylpyrazole der allgemeinen Formel (I) wird gegebenenfalls unter Verwendung eines Reaktionshilfsmittels durchgeführt.

Als Beispiele hierfür seien Natriumcyanid, Kaliumcyanid, Acetoncyanhydrin, 2-Cyano-2-(trimethylsilyloxy)-propan und Trimethylsilylcyanid genannt.

Als besonders gut geeignetes Reaktionshilfsmittel sei Trimethylsilylcyanid genannt.

Das erfindungsgemäße Verfahren (b) zur Herstellung der neuen substituierten Benzoylpyrazole der allgemeinen Formel (I) wird gegebenenfalls unter Verwendung von Reaktionshilfsmitteln durchgeführt.

Als Beispiele hierfür seien (konz.) Schwefelsäure, Zinkchlorid, Aluminiumchlorid, und Borfluorid genannt.

Die erfindungsgemäßen Verfahren zur Herstellung der neuen substituierten Benzoylpyrazole der allgemeinen Formel (I) werden gegebenenfalls unter Verwendung weiterer Reaktionshilfsmittel durchgeführt. Als (weitere) Reaktionshilfsmittel für die erfindungsgemäßen Verfahren kommen im allgemeinen basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilm, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylaminopyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4.0]-undec-7-en (DBU) in Betracht.

Als Verdünnungsmittel zur Durchführung der erfindungsgemäßen Verfahren (a), (b) und (c) kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan oder 1,2-Dichlor-ethan; Ether, wie Diethylether, Diisopropylether, Dioxan, Tetrahydrofuran, Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid.

Die Reaktionstemperaturen können bei der Durchführung der erfindungsgemäßen Verfahren (a), (b) und (c) in einem größeren Bereich variiert werden. Im allgemeinen arbeitet man bei Temperaturen zwischen 0°C und 150°C, vorzugsweise zwischen 10°C und 120°C.

Die erfindungsgemäßen Verfahren (a), (b) und (c) werden im allgemeinen unter Normaldruck durchgeführt. Es ist jedoch auch möglich, die erfindungsgemäßen Verfahren unter erhöhtem oder vermindertem Druck - im allgemeinen zwischen 0,1 bar und 10 bar - durchzuführen.

Zur Durchführung der erfindungsgemäßen Verfahren (a), (b) und (c) werden die Ausgangsstoffe im allgemeinen in angenähert äquimolaren Mengen eingesetzt. Es ist jedoch auch möglich, eine der Komponenten in einem größeren Überschuss zu verwenden. Die Umsetzung wird im allgemeinen in einem geeigneten Verdünnungsmittel in Gegenwart eines Dehydratisierungsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera, Aegilops, Phalaris.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-. Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise
Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinidon(-ethyl), Cinmethylin, Cinosulfuron, Clethodim, Clodinafop(-propargyl), Clomazone, Clomeprop, Clopyralid, Clopyrasulfuron(-methyl), Cloransulam(methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epoprodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Flamprop(-isopropyl), Flamprop(-isopropyl-L), Flamprop(-methyl), Flazasulfuron, Fluazifop(-Pbutyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(isopropylammonium), Halosafen, Haloxyfop(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfuron, Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pentoxazone, Phenmedipham, Picolinafen, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(methyl), Thiobencarb, Tiocarbazil, Tralkoxydim, Triallate, Triasulfuron, Tribenuron(-methyl), Triclopyr, Tridiphane, Trifluralin, Triflusulfuron und Tritosulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die endungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

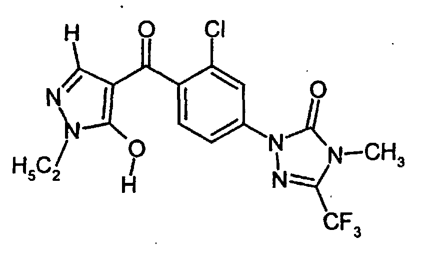

Eine Mischung aus 1,64 g (5 mMol) 4-Methyl-5-trifluormethyl-2-(3-chlor-4-carboxy-phenyl)-2,4-dihydro-3H-1,2,4-triazol-3-on, 0,62 g (5,5 mMol) 1-Ethyl-5-hydroxy-pyrazol und 40 ml Acetonitril wird bei Raumtemperatur (ca. 20°C) unter Rühren mit 1,13 g (5,5 mMol) Dicyclohexylcarbodiimid versetzt und die Reaktionsmischung wird 16 Stunden bei Raumtemperatur gerührt. Dann werden 1,0 g (10 mMol) Triethylamin und 0,2 g (2 mMol) Trimethylsilylcyanid dazu gegeben und die Mischung wird drei Tage bei Raumtemperatur gerührt. Anschließend werden 60 ml einer 2%igen wässrigen Natriumcarbonat-Lösung dazu gegeben und die Mischung wird drei Stunden bei Raumtemperatur gerührt. Der ausgefallene Dicyclohexylharnstoff wird durch Absaugen abgetrennt und die Mutterlauge zweimal mit Diethylether geschüttelt. Die wässrige Phase wird unter Rühren durch Zugabe von konz. Salzsäure auf einen pH-Wert von ca. 1 eingestellt. Das sich hierbei abscheidende ölige Produkt wird mit Methylenchlorid extrahiert, die Extraktionslösung mit Magnesiumsulfat getrocknet und filtriert. Vom Filtrat wird das Lösungsmittel im Wasserstrahlvakuum sorgfältig abdestilliert.

Man erhält 1,5 g (72% der Theorie) 4-Methyl-5-trifluormethyl-2-[3-chlor-4-(1-ethyl-5-hydroxy-pyrazol-4-yl-carbonyl)-phenyl]-2,4-dihydro-3H-1,2,4-triazol-3-on als amorphes Produkt.
logP (bei pH≈2 bestimmt): 2,63.

Analog zu Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I) - bzw. der Formeln (IA), (IB) oder (IC) - hergestellt werden.

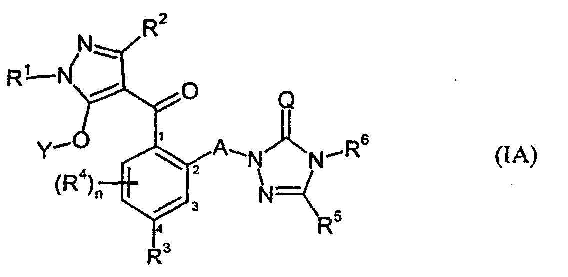

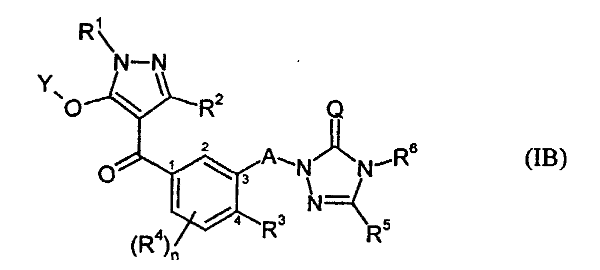

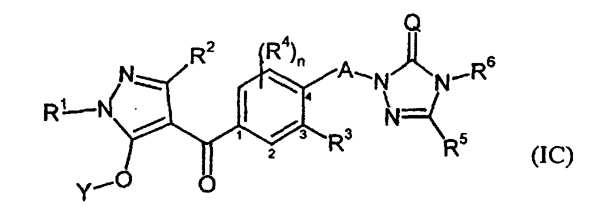

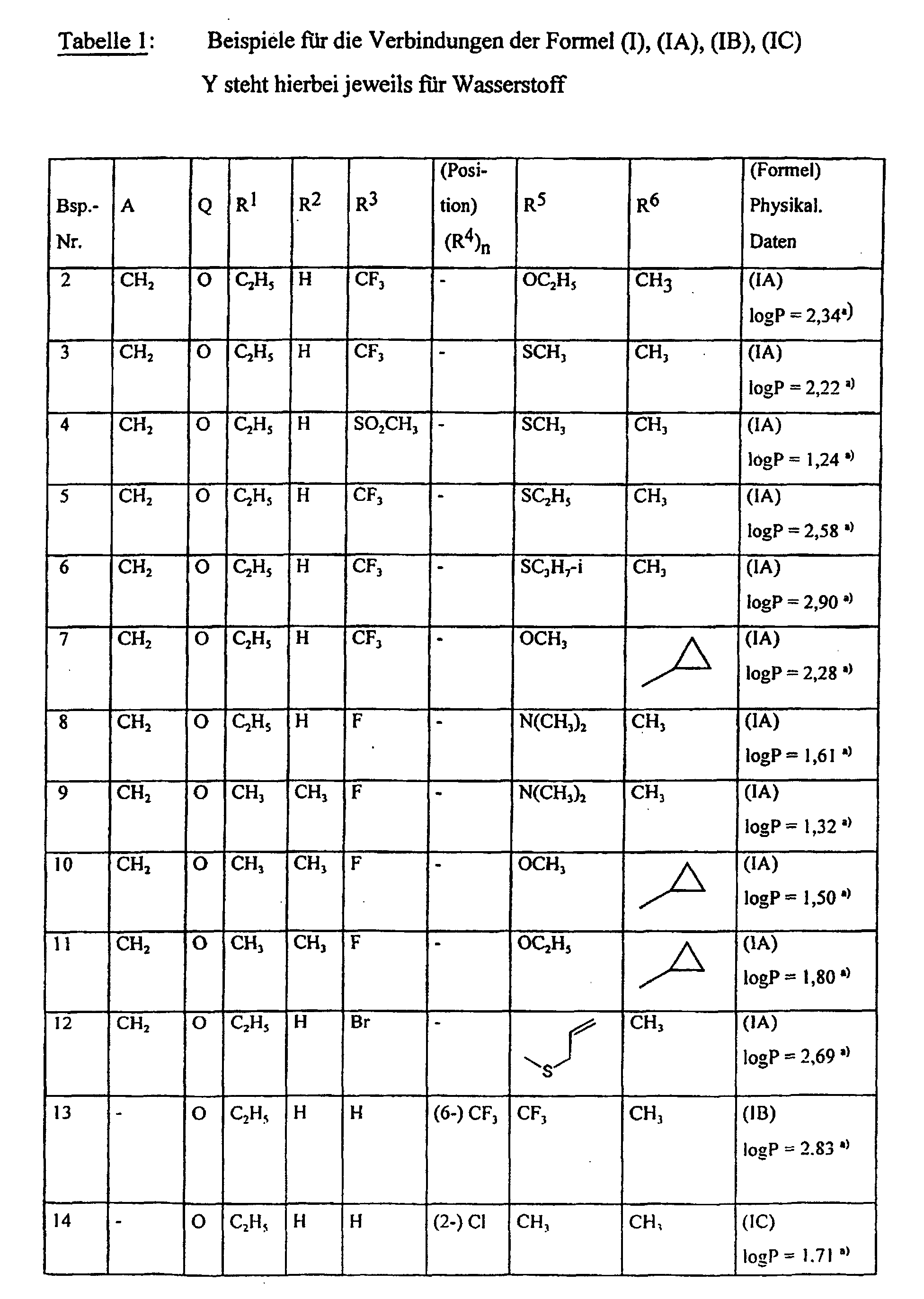

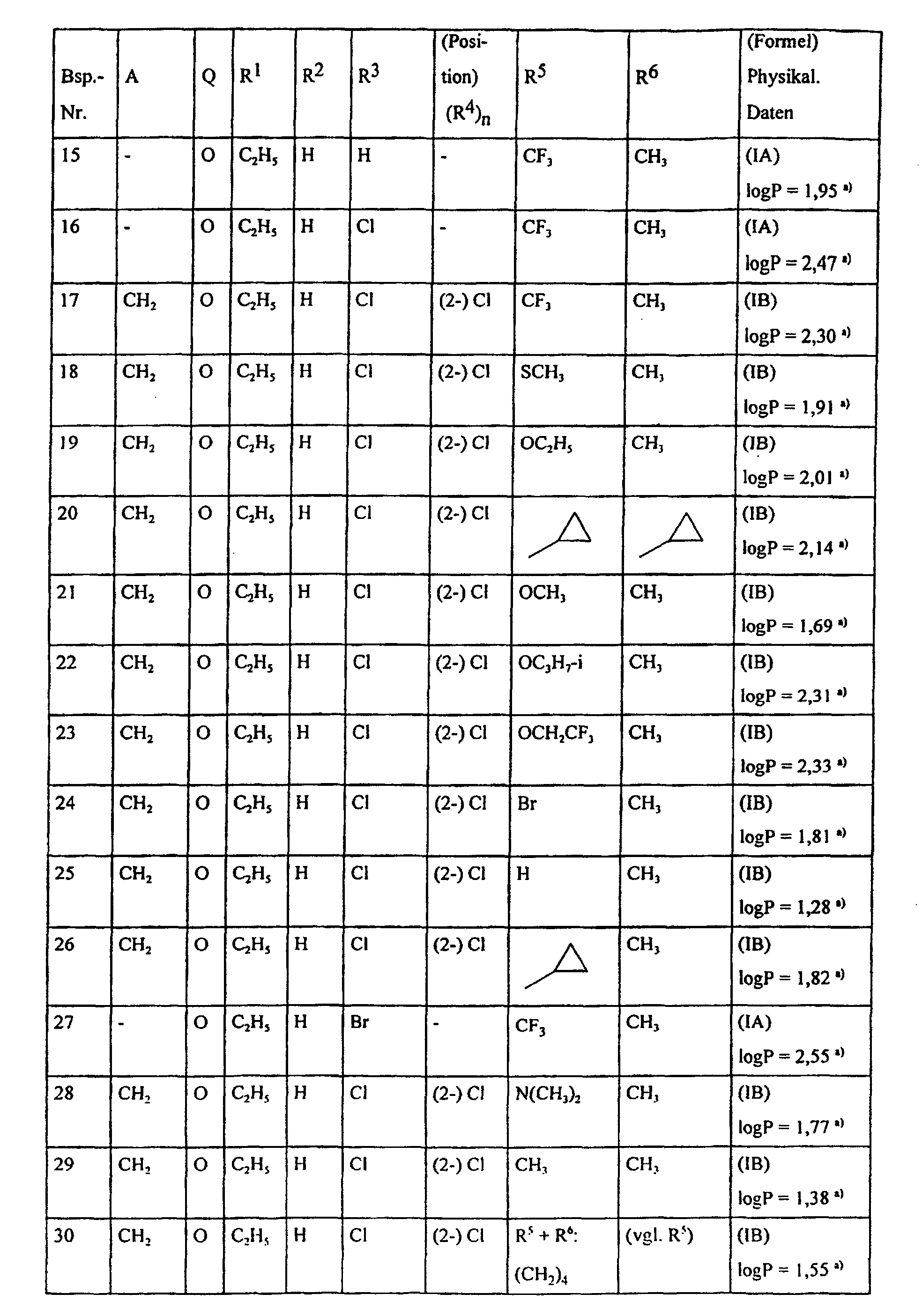

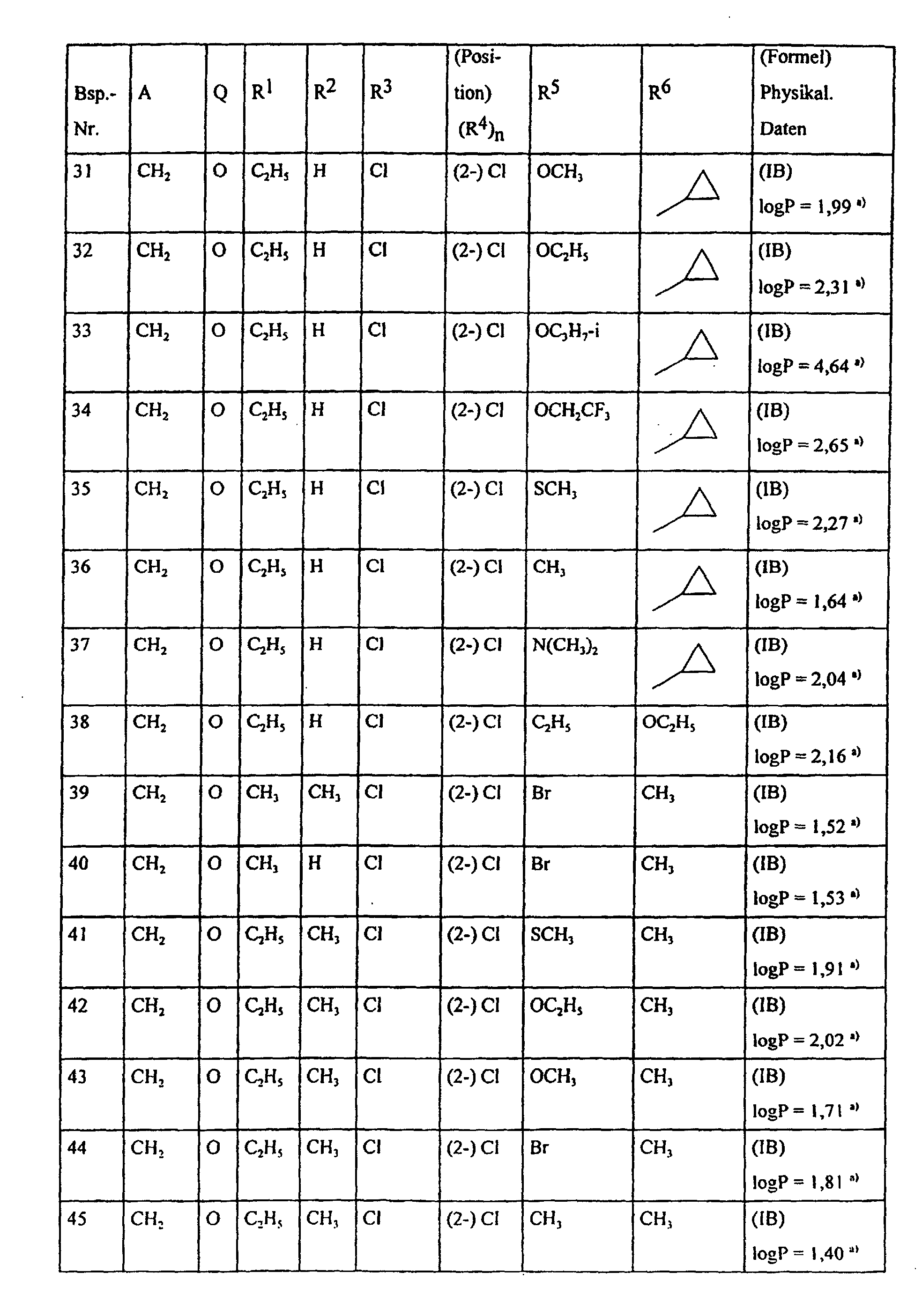

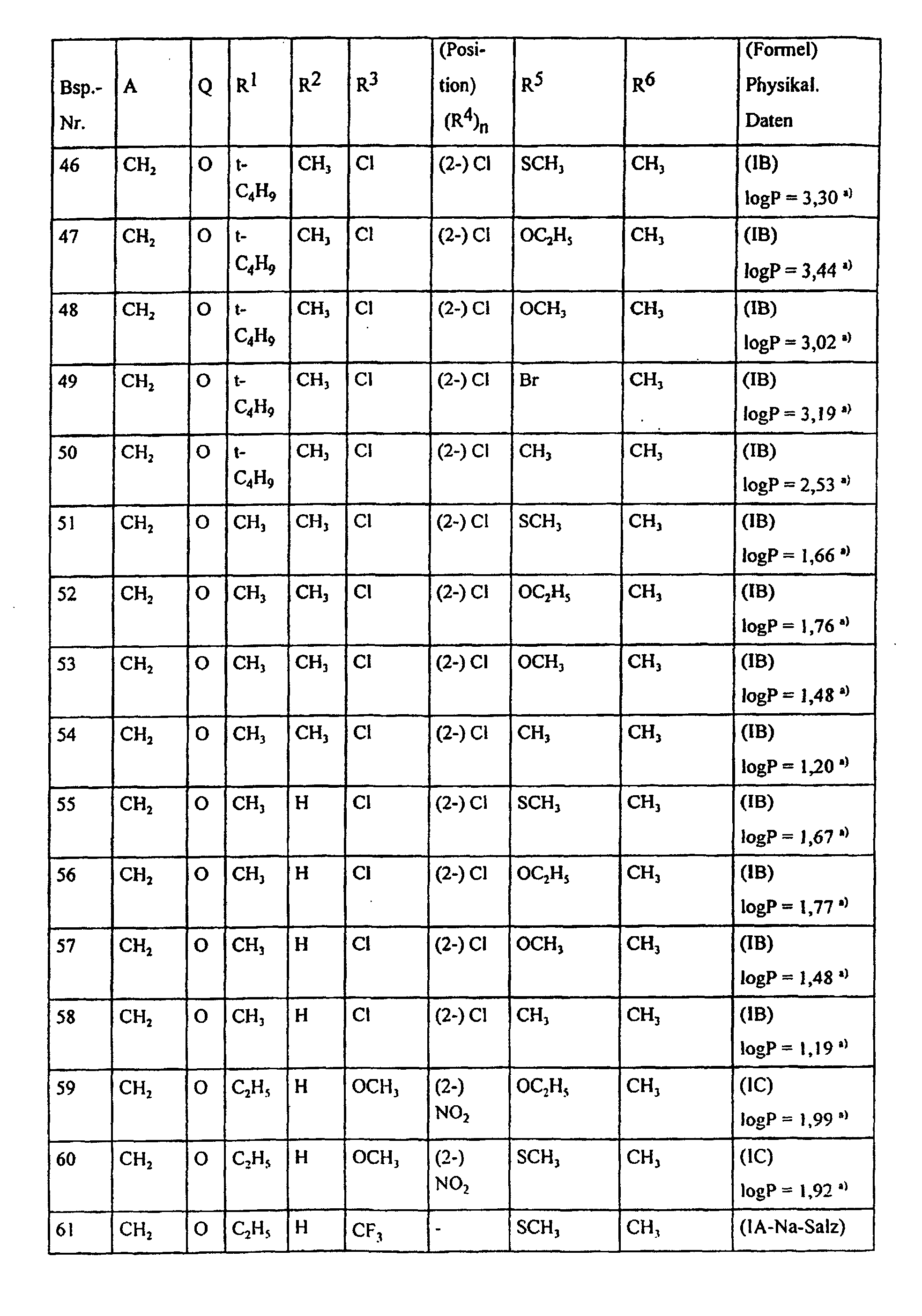

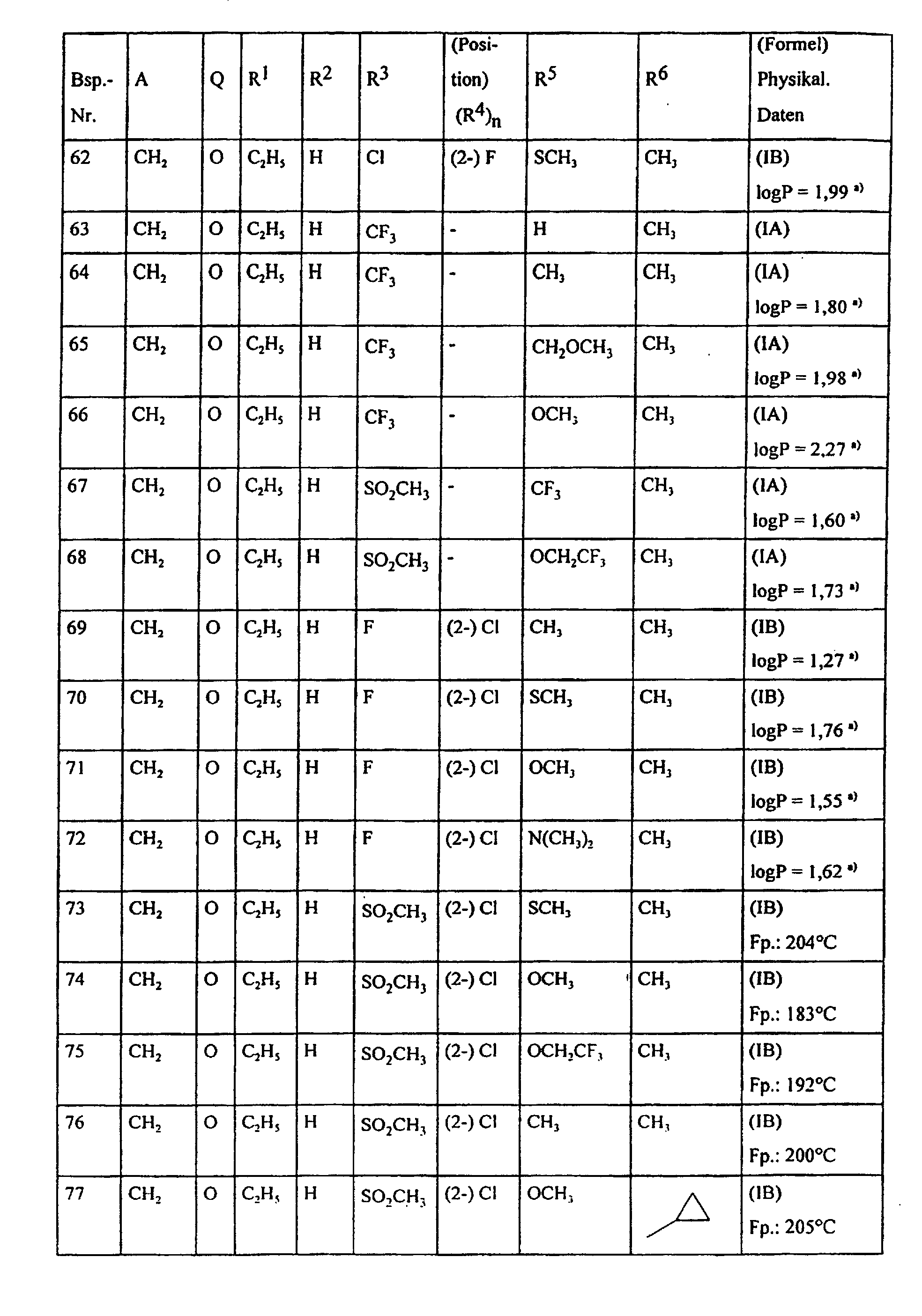

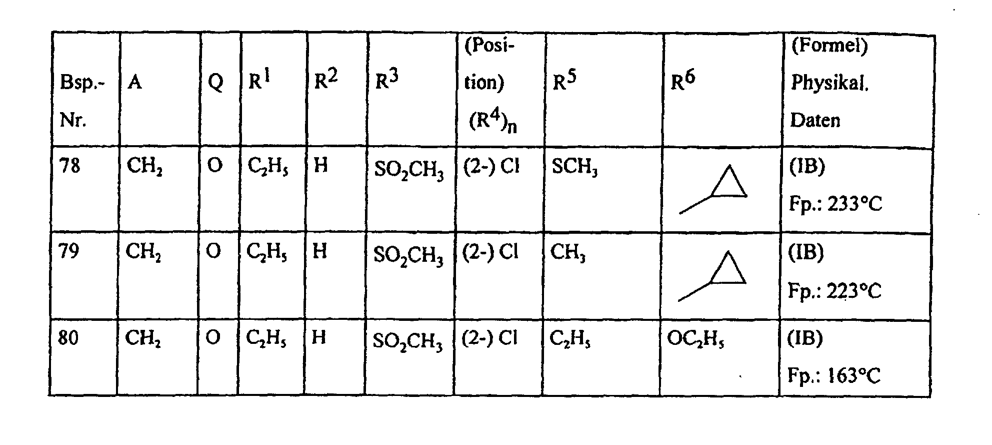

Analog zu Beispiel 1 sowie entsprechend der allgemeinen Beschreibung des erfindungsgemäßen Herstellungsverfahrens können beispielsweise auch die in der nachstehenden Tabelle 2 aufgeführten Verbindungen der allgemeinen Formel (I) - bzw. der Formel (ID) - hergestellt werden.

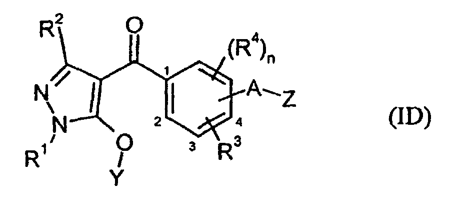

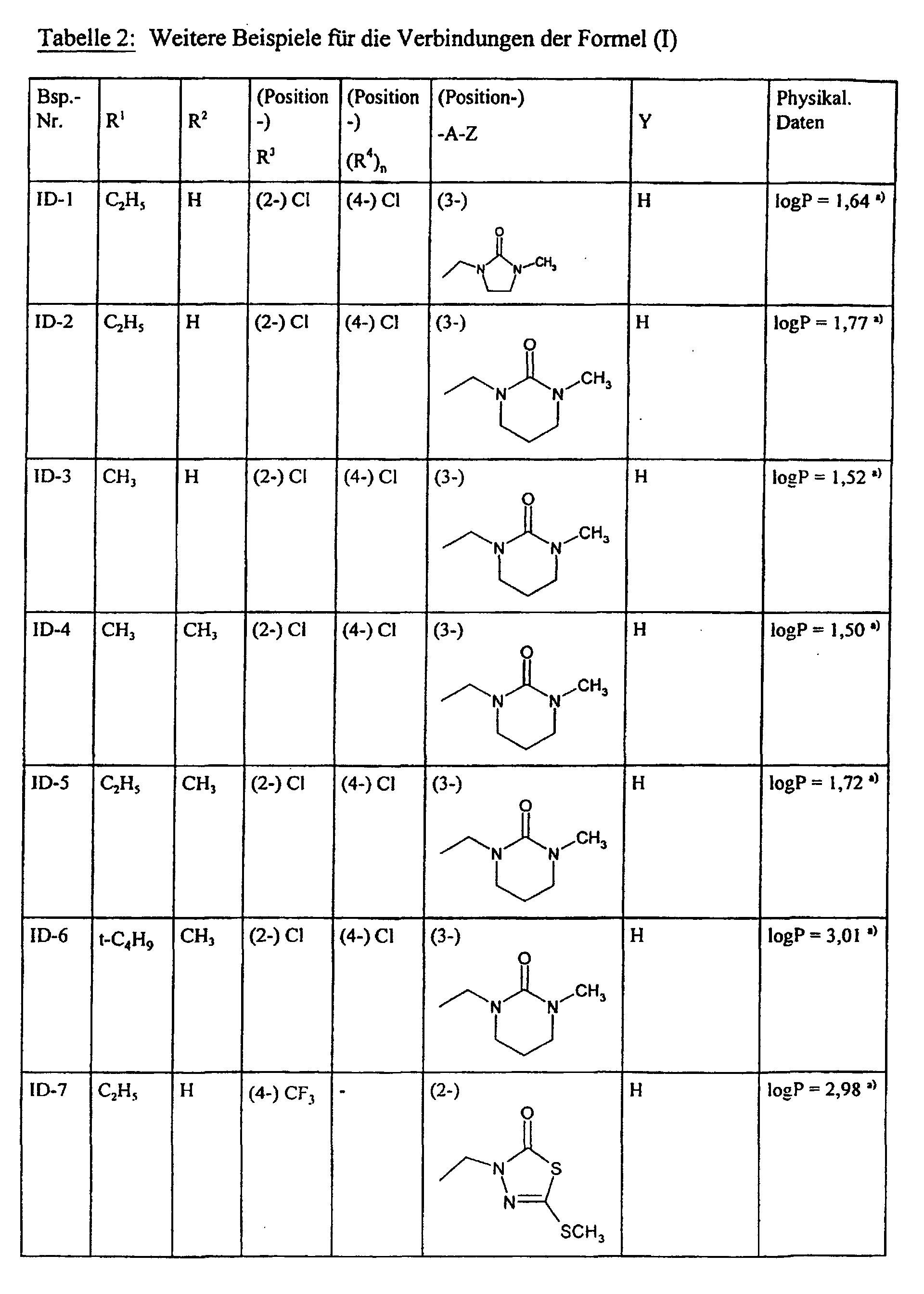

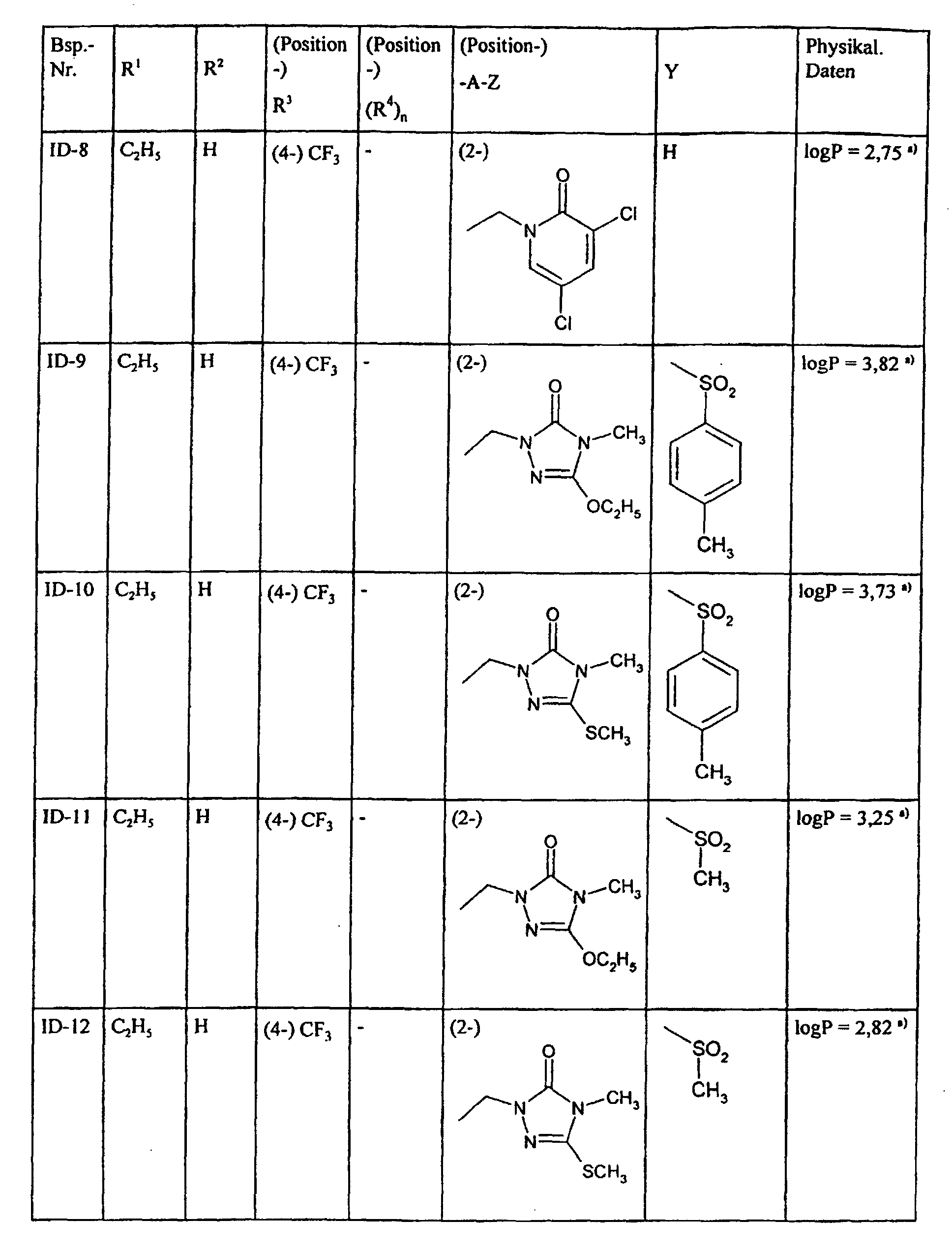

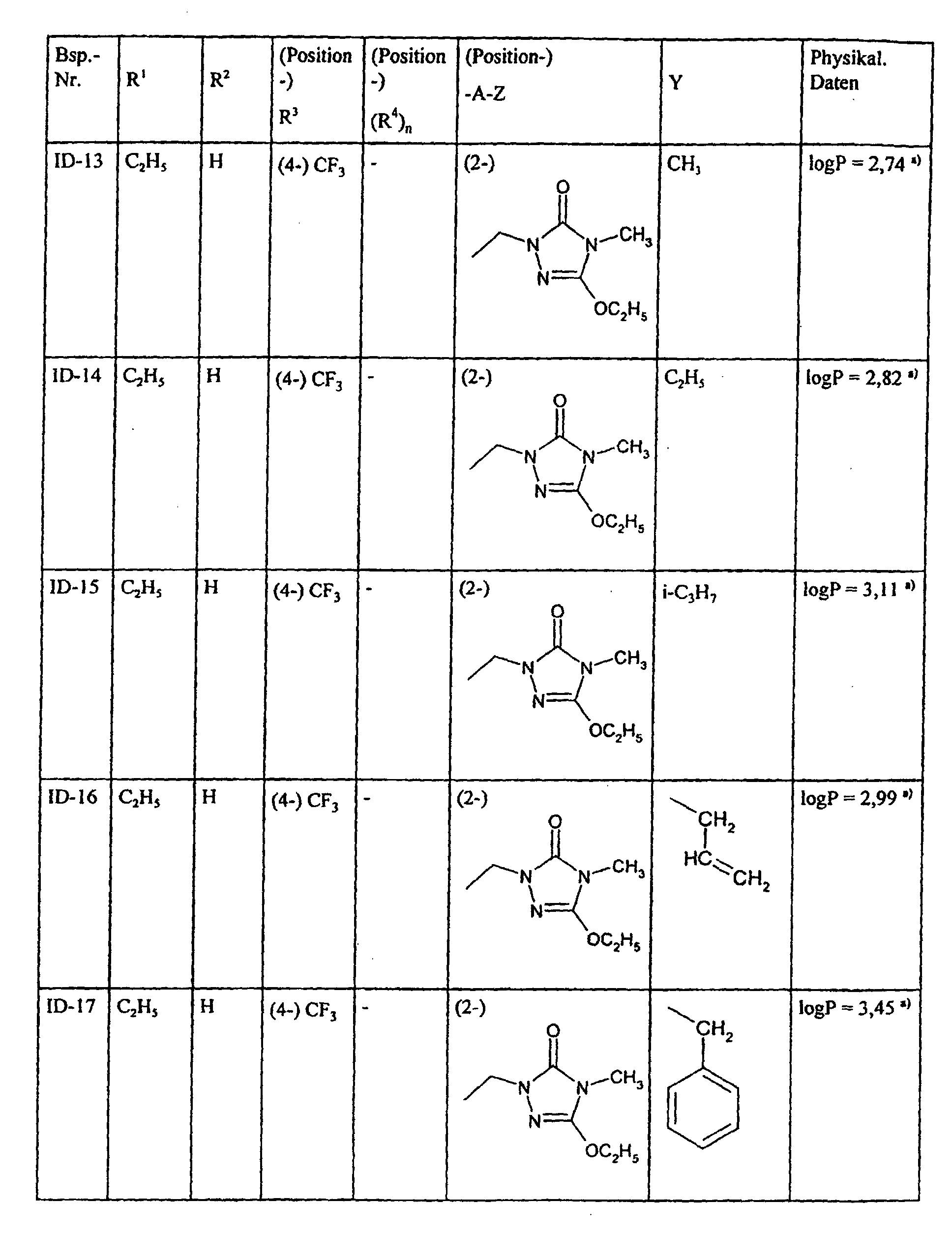

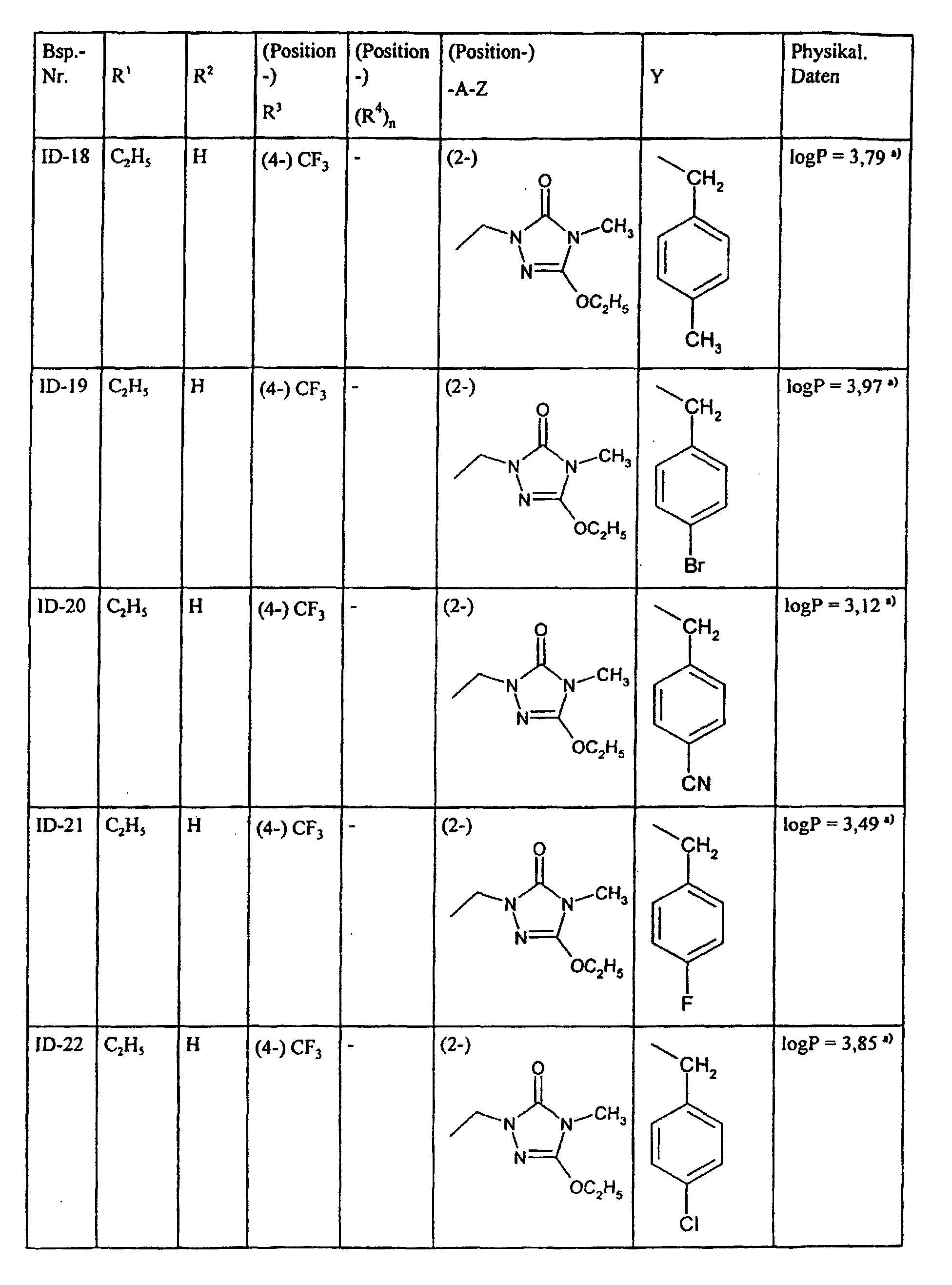

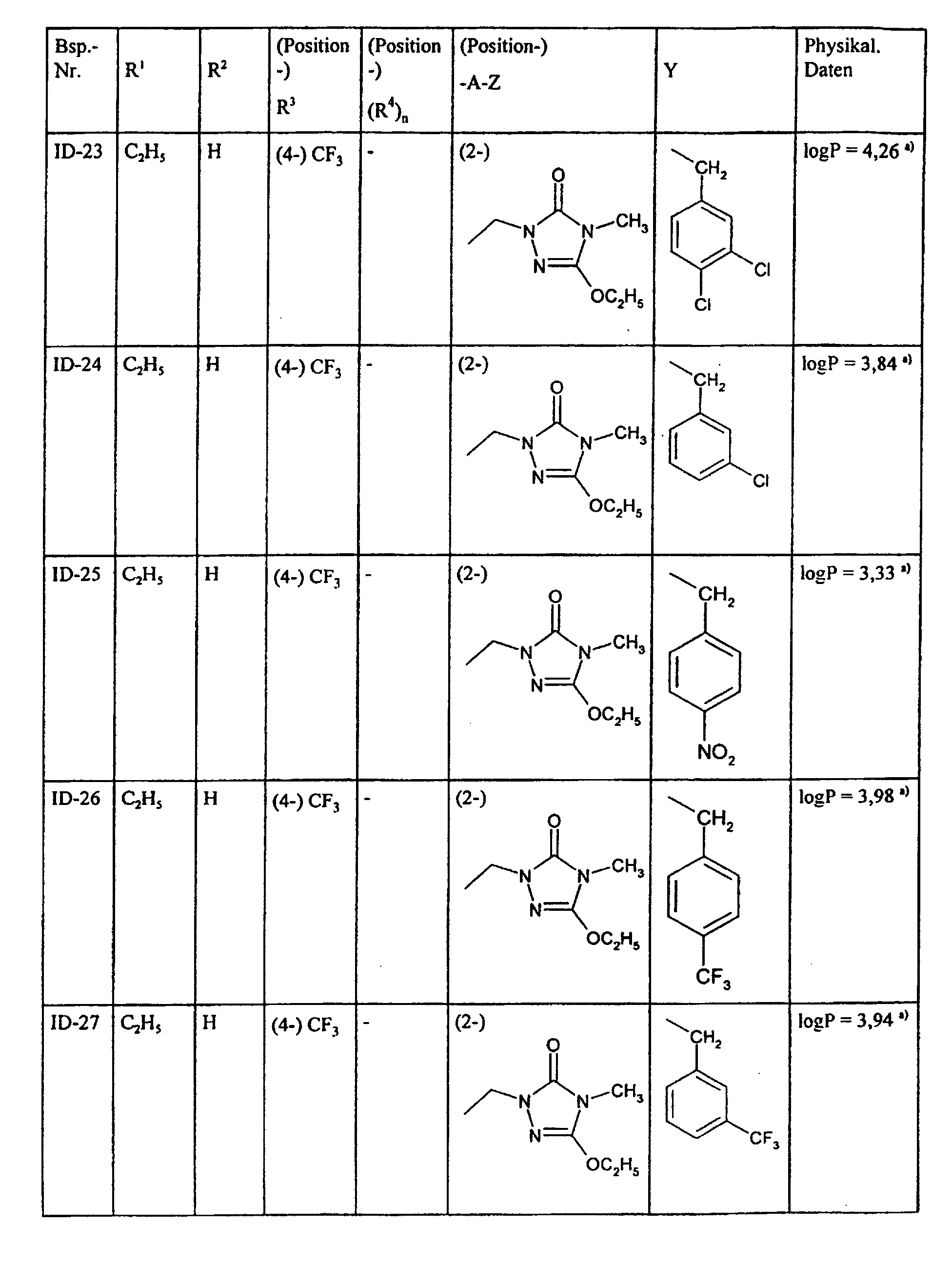

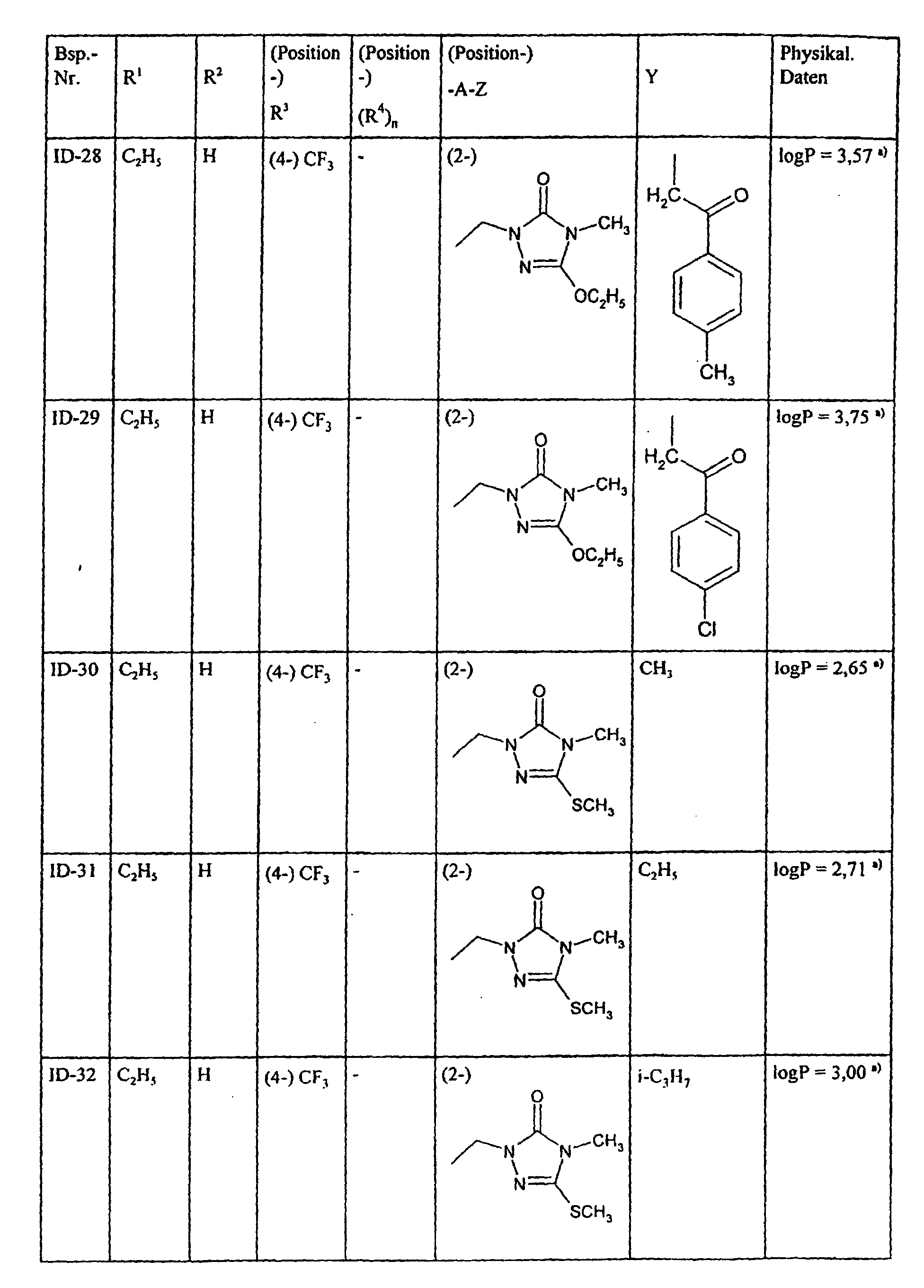

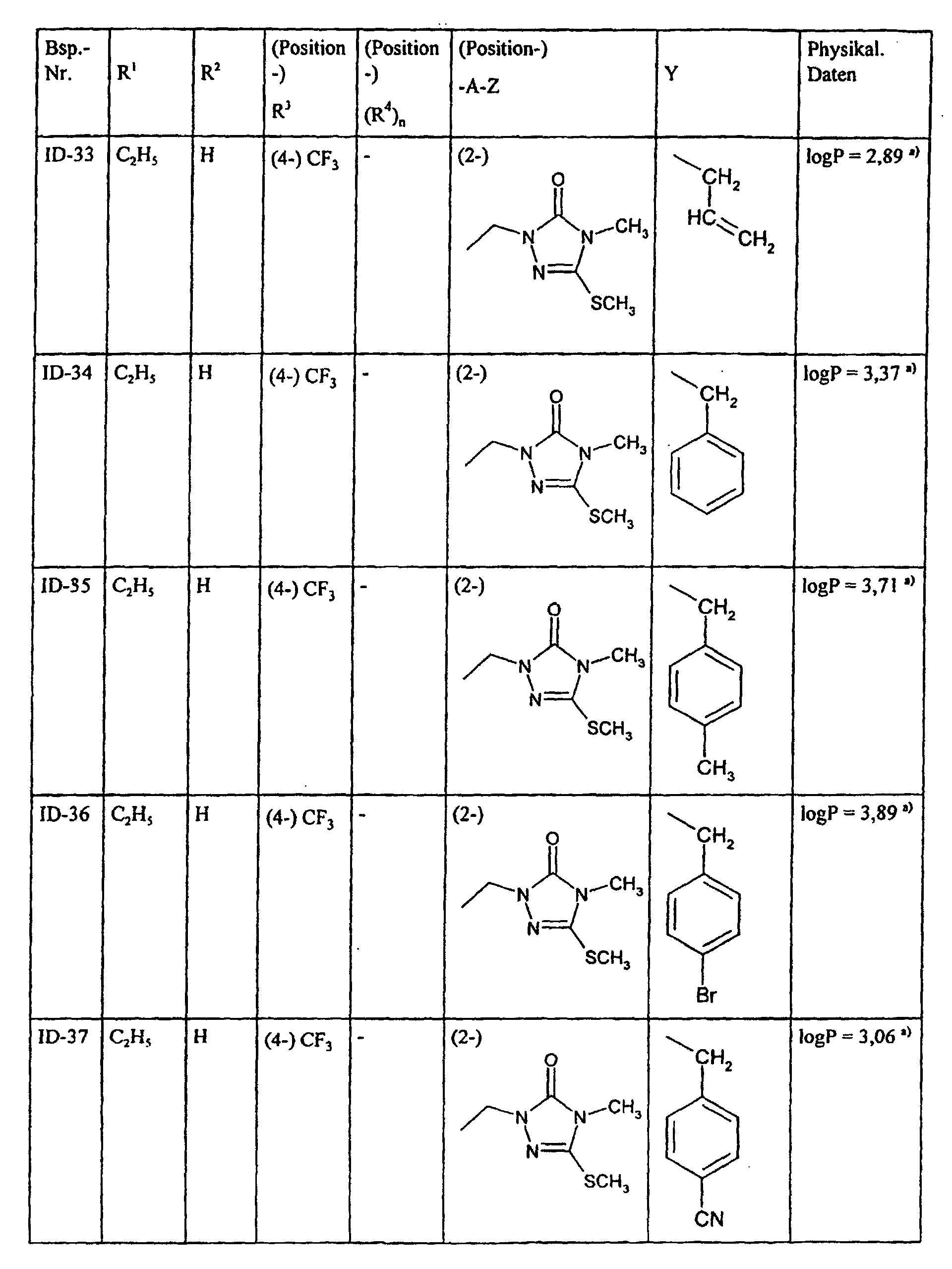

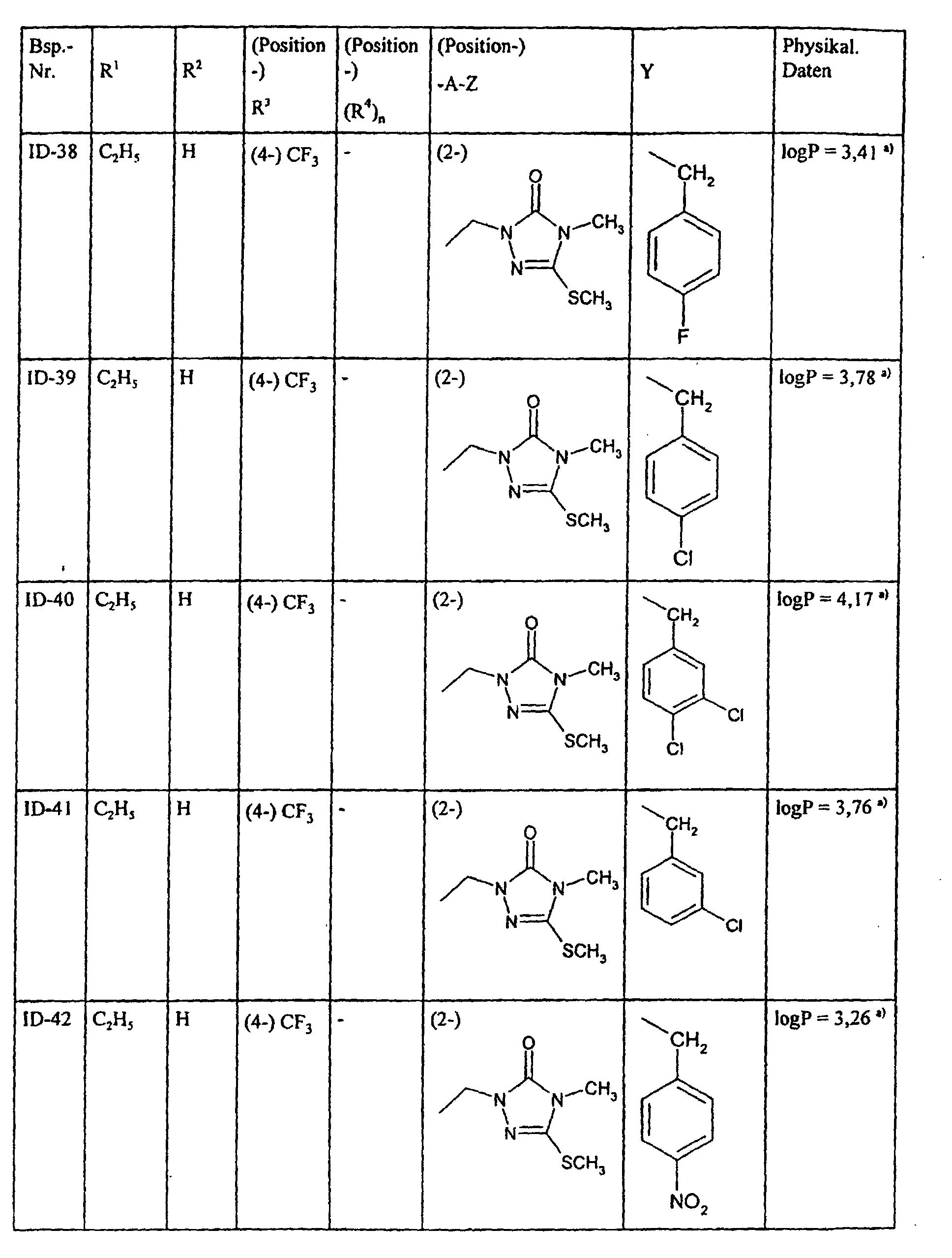

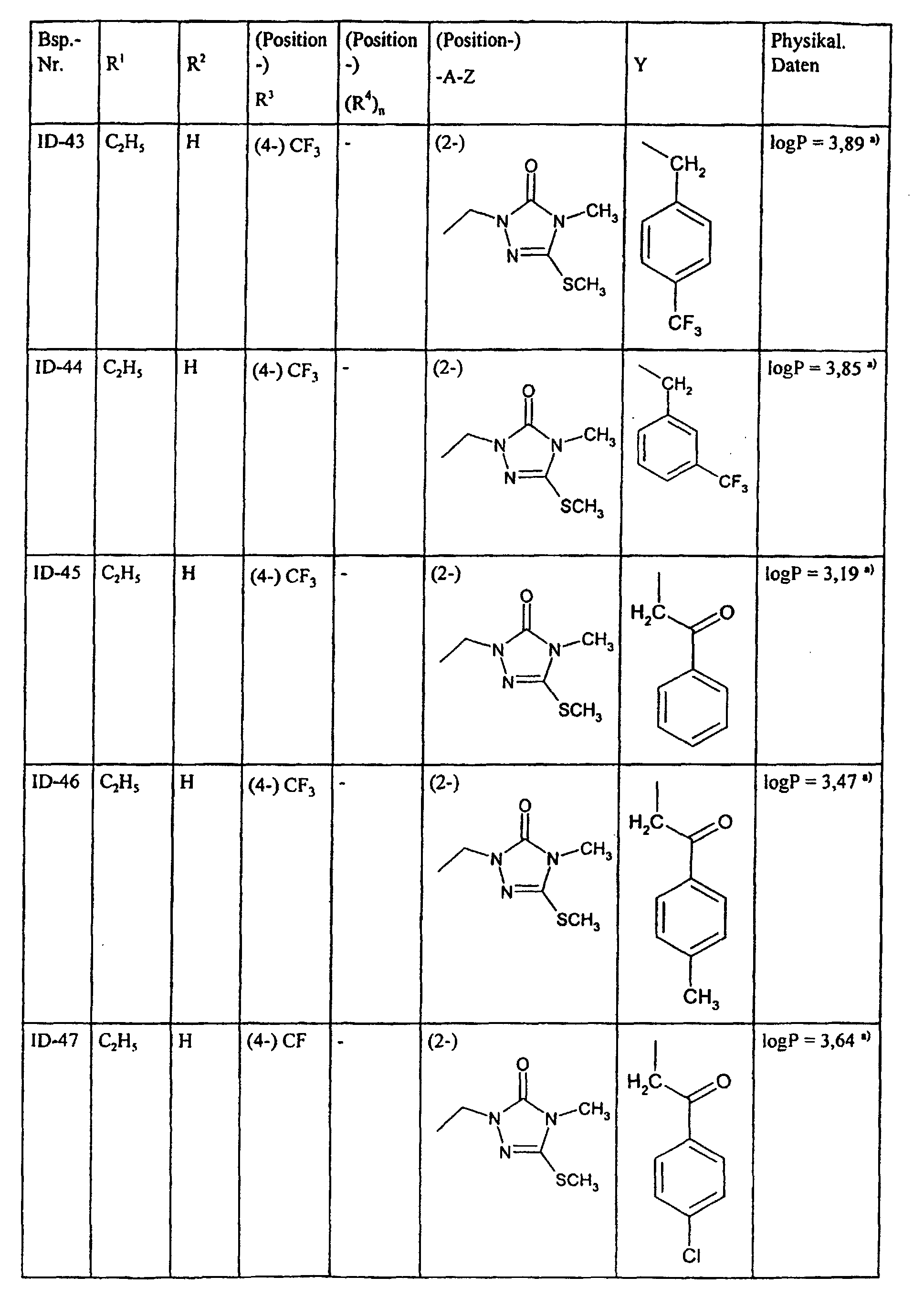

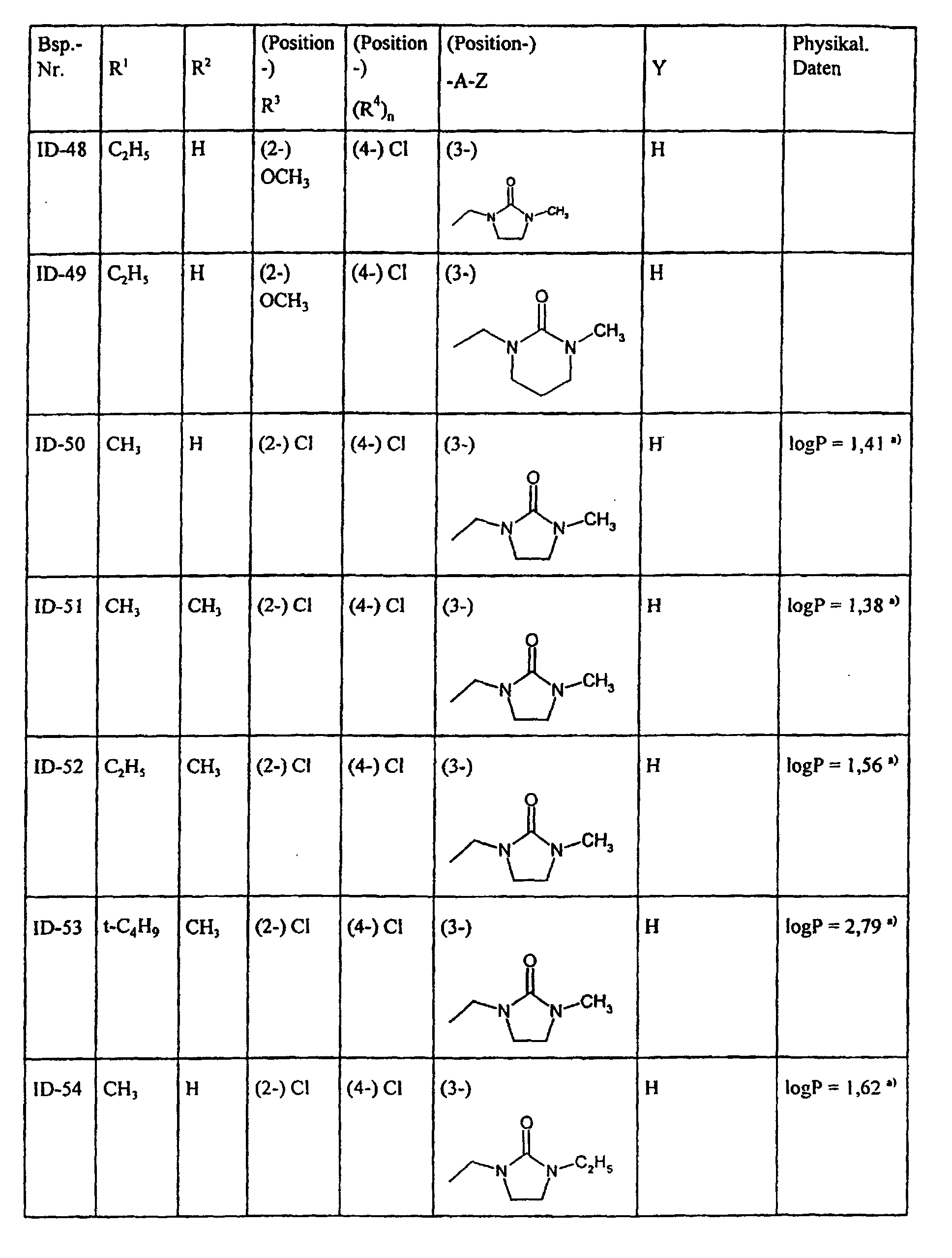

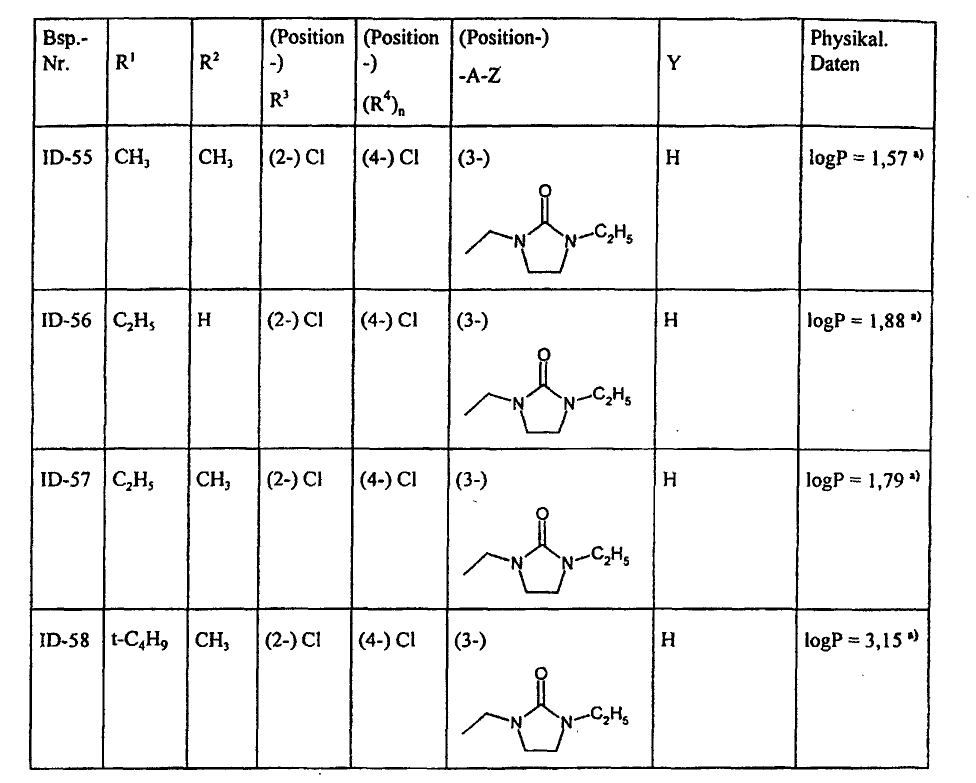

Die Bestimmung der in den Tabellen 1 und 2 angegebenenen logP-Werte erfolgte gemäß EEC-Directive 79/831 Annex V.A8 durch HPLC (High Performance Liquid Chromatography) an einer Phasenumkehrsäule (C 18), Temperatur: 43°C.
(a) Eluenten für die Bestimmung im sauren Bereich: 0,1% wässrige Phosphorsäure, Acetonitril; linearer Gradient von 10% Acetonitril bis 90% Acetonitril - entsprechende Messergebnisse sind in Tabelle 1 mit ^{a)} markiert.
(b) Eluenten für die Bestimmung im neutralen Bereich: 0,01-molare wässrige Phosphatpuffer-Lösung, Acetonitril; linearer Gradient von 10% Acetonitril bis 90% Acetonitril - entsprechende Messergebnisse sind in Tabelle 1 mit ^{b)} markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Ausgangsstoffe der Formel (III):

### Beispiel (III-1)

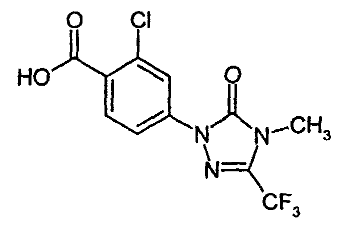

4,5 g (15 mMol) 2-(3-Chlor-4-cyano-phenyl)-4-methyl-5-trifluormethyl-2,4-dihydro-3H-1,2,4-triazol-3-on werden in 80 ml 60%iger Schwefelsäure aufgenommen und die Mischung wird 6 Stunden unter Rückfluss erhitzt. Nach Abkühlen auf Raumtemperatur wird das kristallin angefallene Produkt durch Absaugen isoliert.

Man erhält 4,5 g (91% der Theorie) 2-(3-Carboxy-4-Chlor-phenyl)-4-methyl-5-trifluormethyl-2,4-dihydro-3H-1,2,4-triazol-3-on vom Schmelzpunkt 223°C.

### Beispiel (III-2)

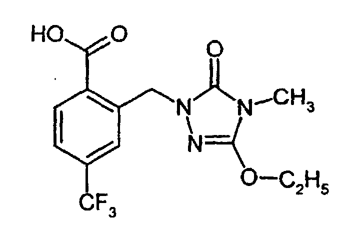

2 g (4,9 mMol) 5-Brom-4-methyl-2-(2-ethoxycarbonyl-5-trifluormethyl-benzyl)-2,4-dihydro-3H-1,2,4-triazol-3-an (vgl. Beispiel IV-1) werden in 30 ml 10%iger ethanolischer Kalilauge gelöst und 2 Stunden unter Rückfluss erhitzt. Das Reaktionsgemisch wird im Wasserstrahlvakuum eingeengt. in 20 ml Wasser aufgenommen und mit verdünnter Salzsäure angesäuert. Der ausfallende Feststoff wird filtriert und getrocknet.

Man erhält 1,2 g (71% der Theorie) 5-Ethoxy-4-methyl-2-(2-carboxy-5-trifluormethyl-benzyl)-2,4-dihydro-3H-1,2,4-triazol-3-on als festes Produkt.
logP: 2,18^{a)}

### Beispiel (III-3)

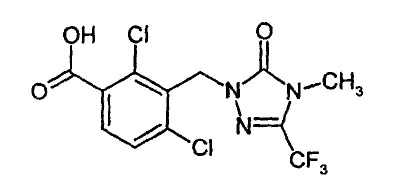

13,4 g (35 mMol) 4-Methyl-5-trifluormethyl-2-(2,6-dichlor-3-methoxycarbonylbenzyl)-2,4-dihydro-3H-1,2,4-triazol-3-on werden in 60 ml 1,4-Dioxan vorgelegt und eine Lösung von 1,54 g (38,5 mMol) Natriumhydroxid in 20 ml Wasser wird bei Raumtemperatur langsam eindosiert. Die Reaktionsmischung wird 150 Minuten bei 60°C gerührt und anschließend im Wasserstrahlvakuum eingeengt. Der Rückstand wird in 100 ml Wasser gelöst und durch Zugabe von konz. Salzsäure wird der pH-Wert der Lösung auf 1 eingestellt. Das hierbei kristallin angefallene Produkt wird durch Absaugen isoliert.

Man erhält 11,7 g (90% der Theorie) 4-Methyl-5-trifluormethyl-2-(2,6-dichlor-3-carboxy-benzyl)-2,4-dihydro-3H-1,2,4-triazol-3-on vom Schmelzpunkt 207°C.

Analog zu den Beispielen (III-1) bis (III-3) können beispielsweise auch die in der nachstehenden Tabelle 3 aufgeführten Verbindungen der allgemeinen Formel (III) hergestellt werden.

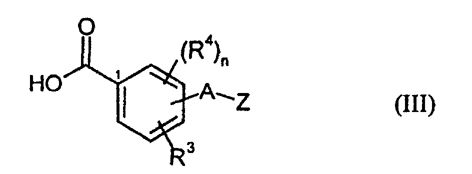

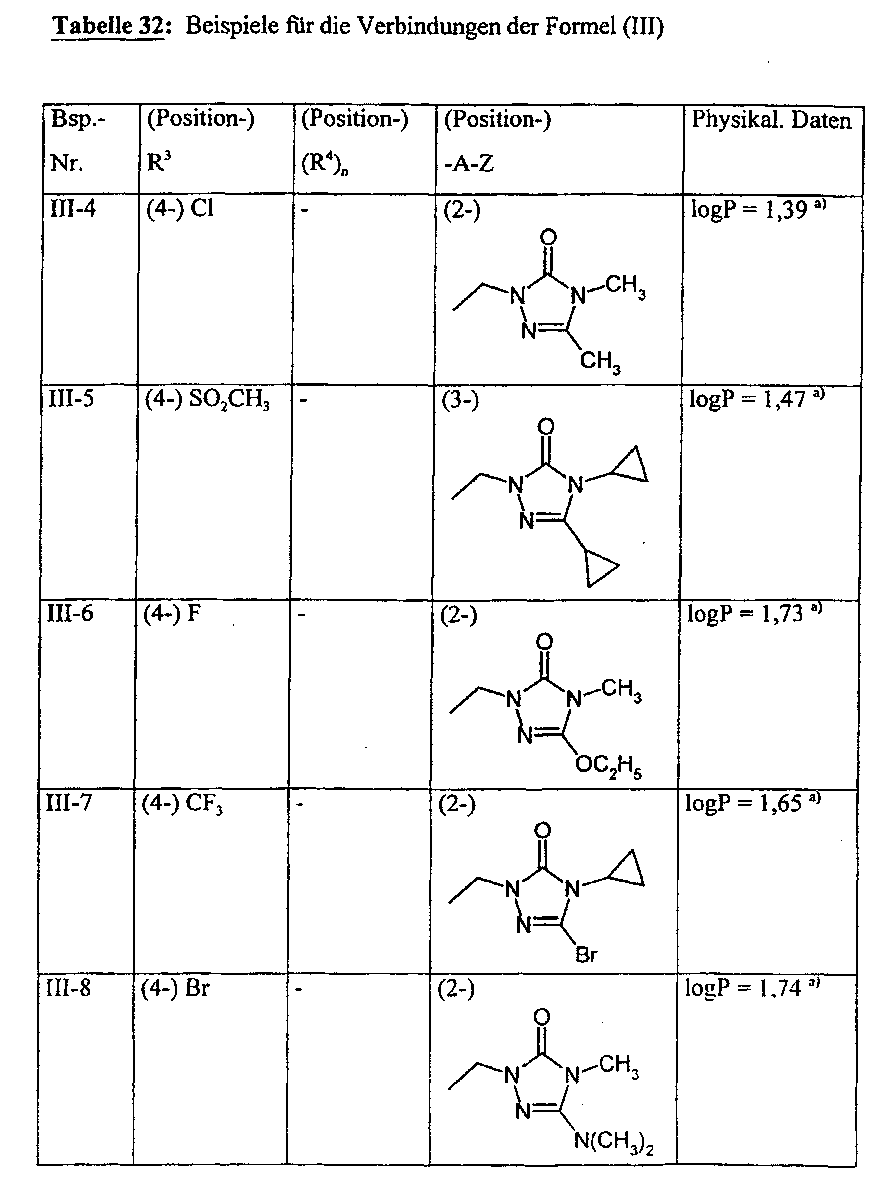

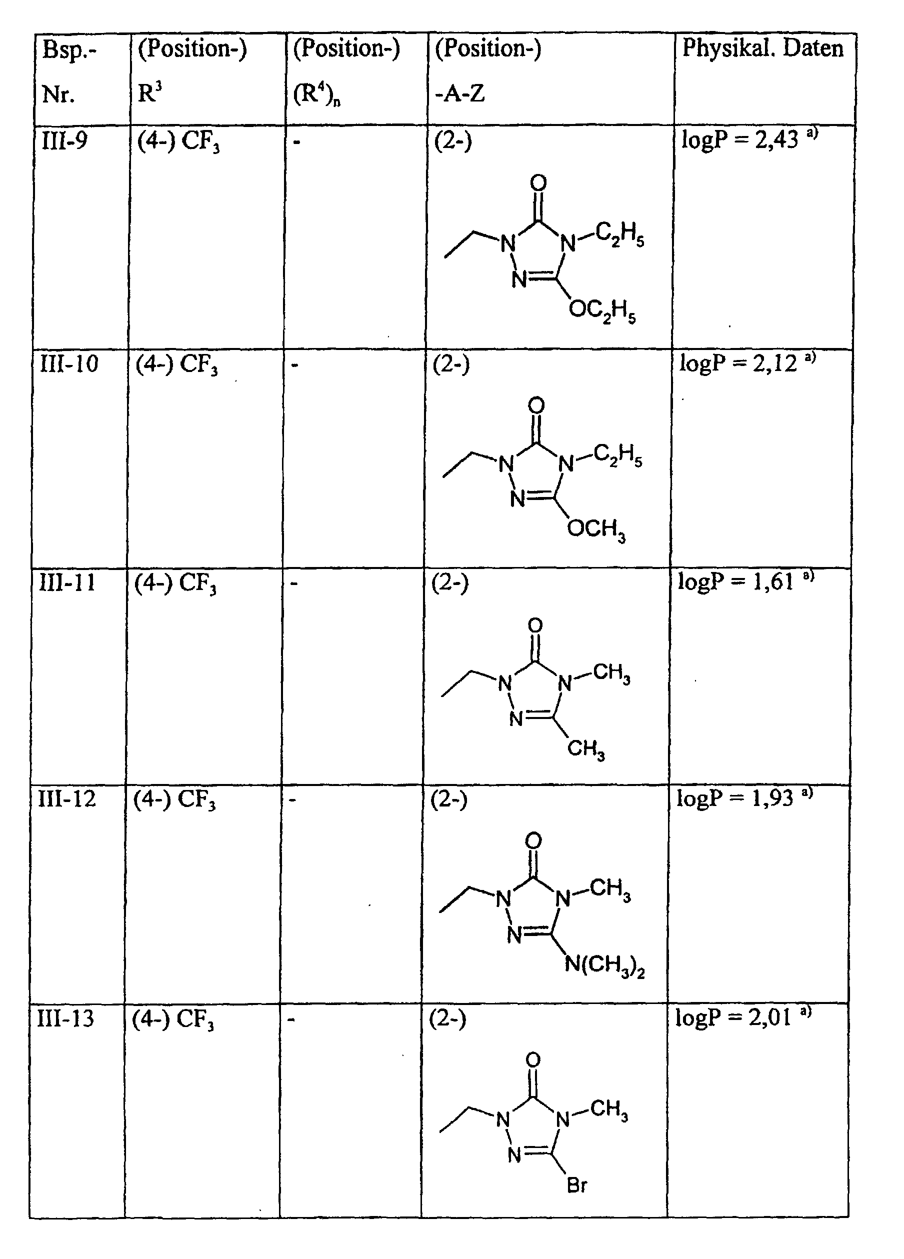

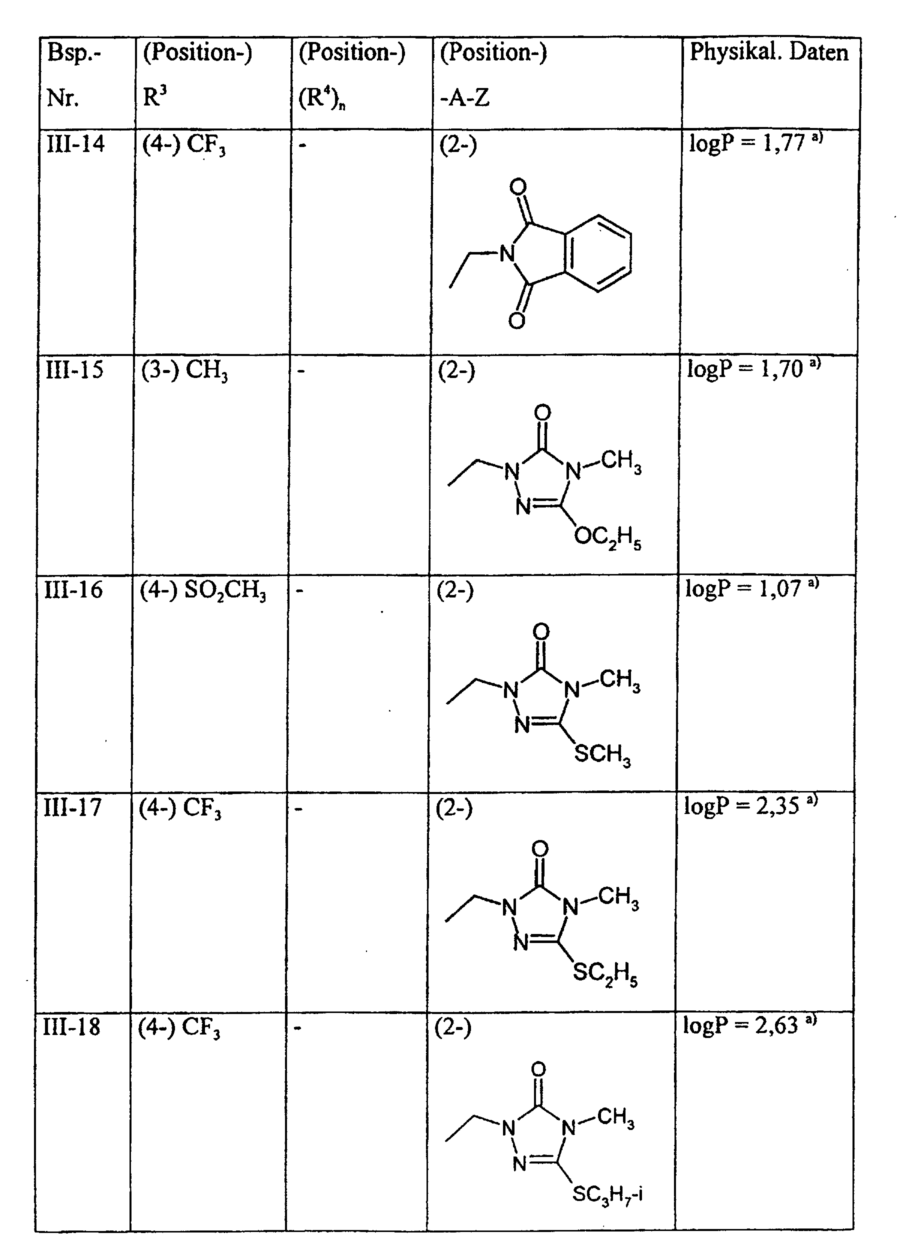

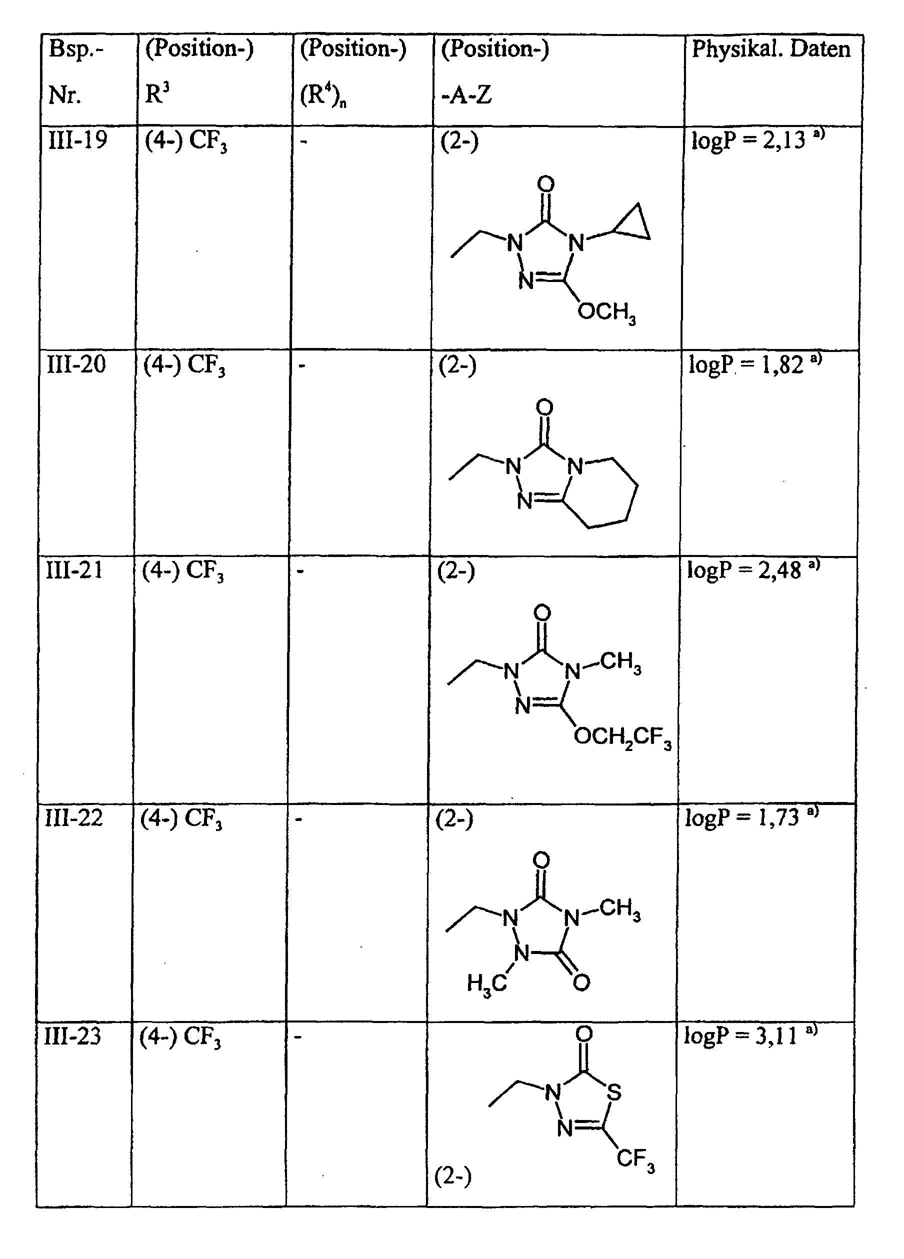

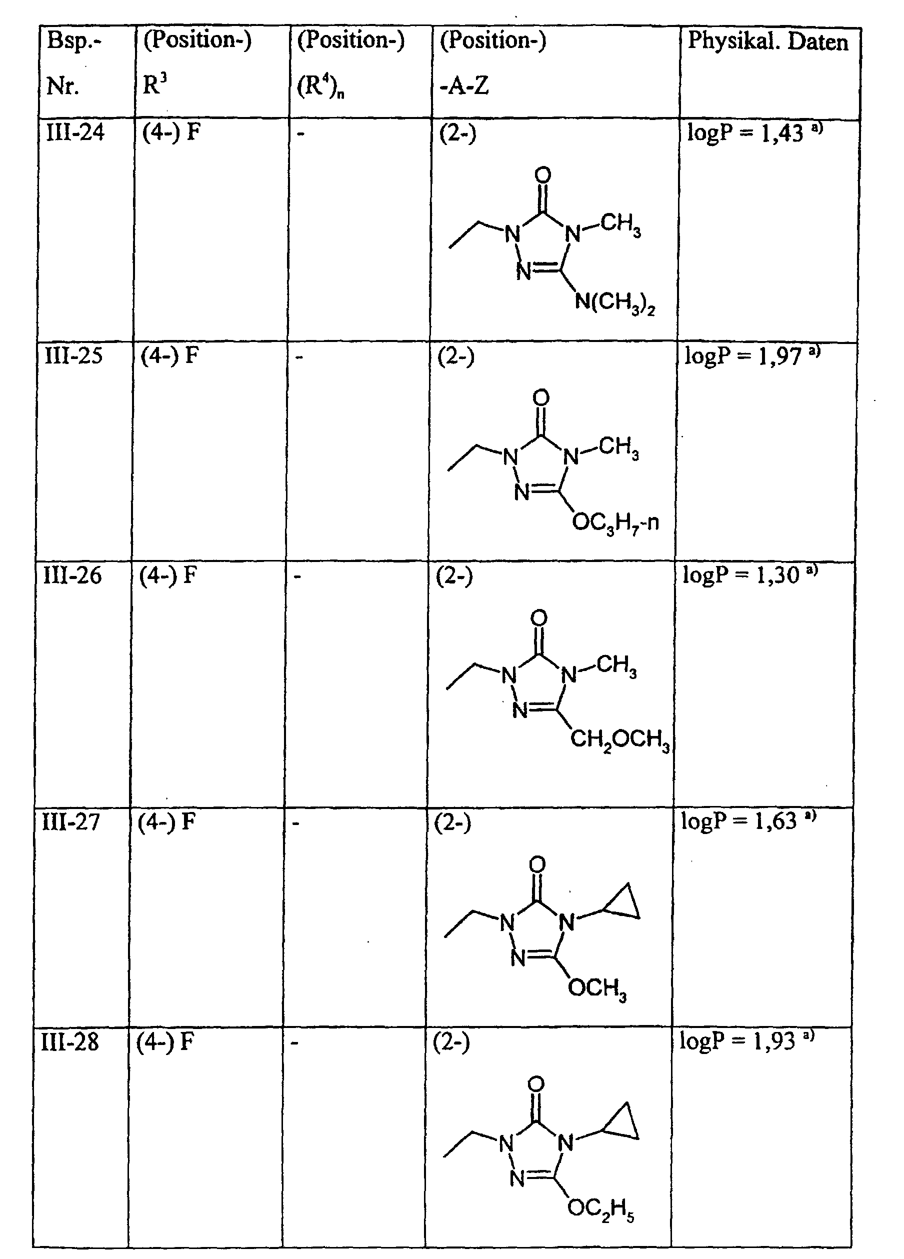

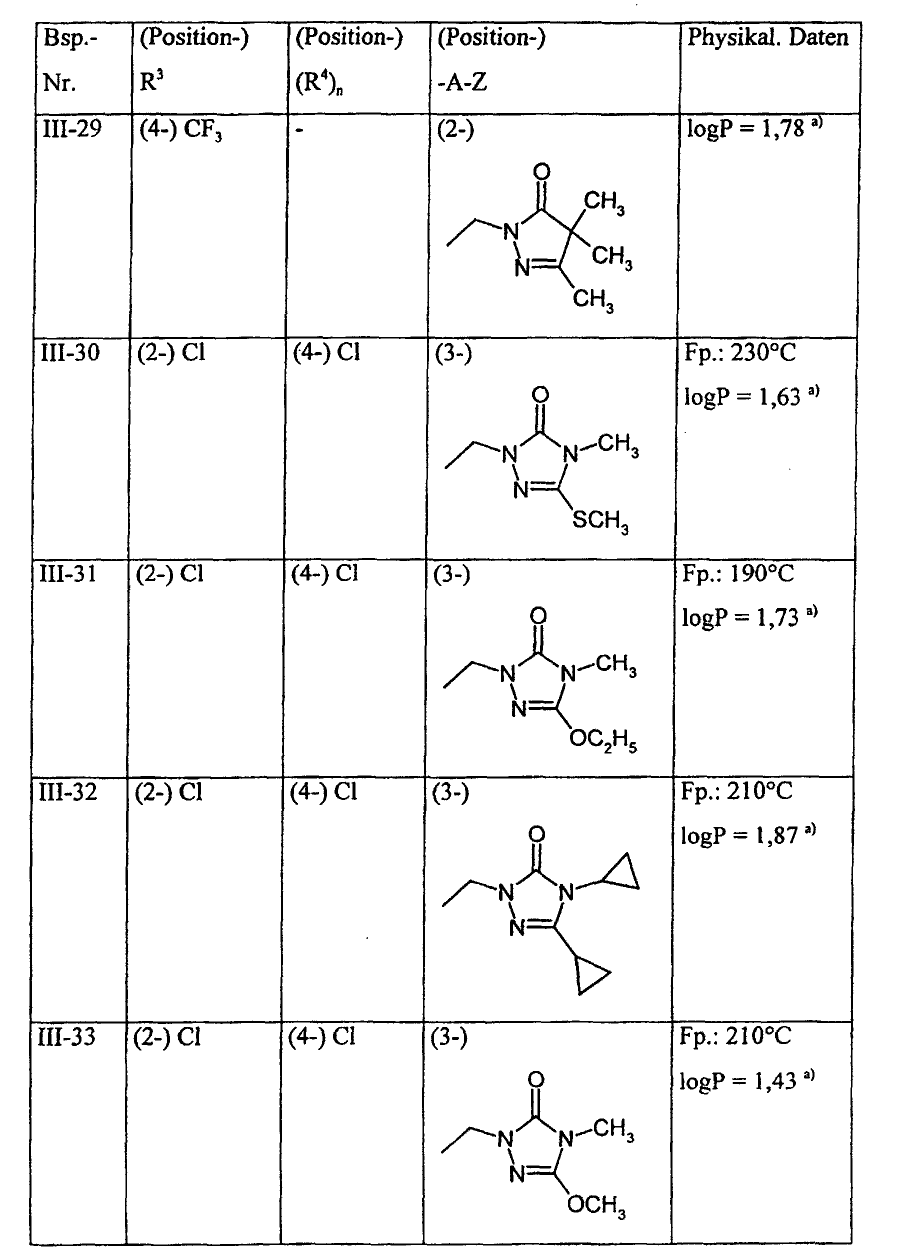

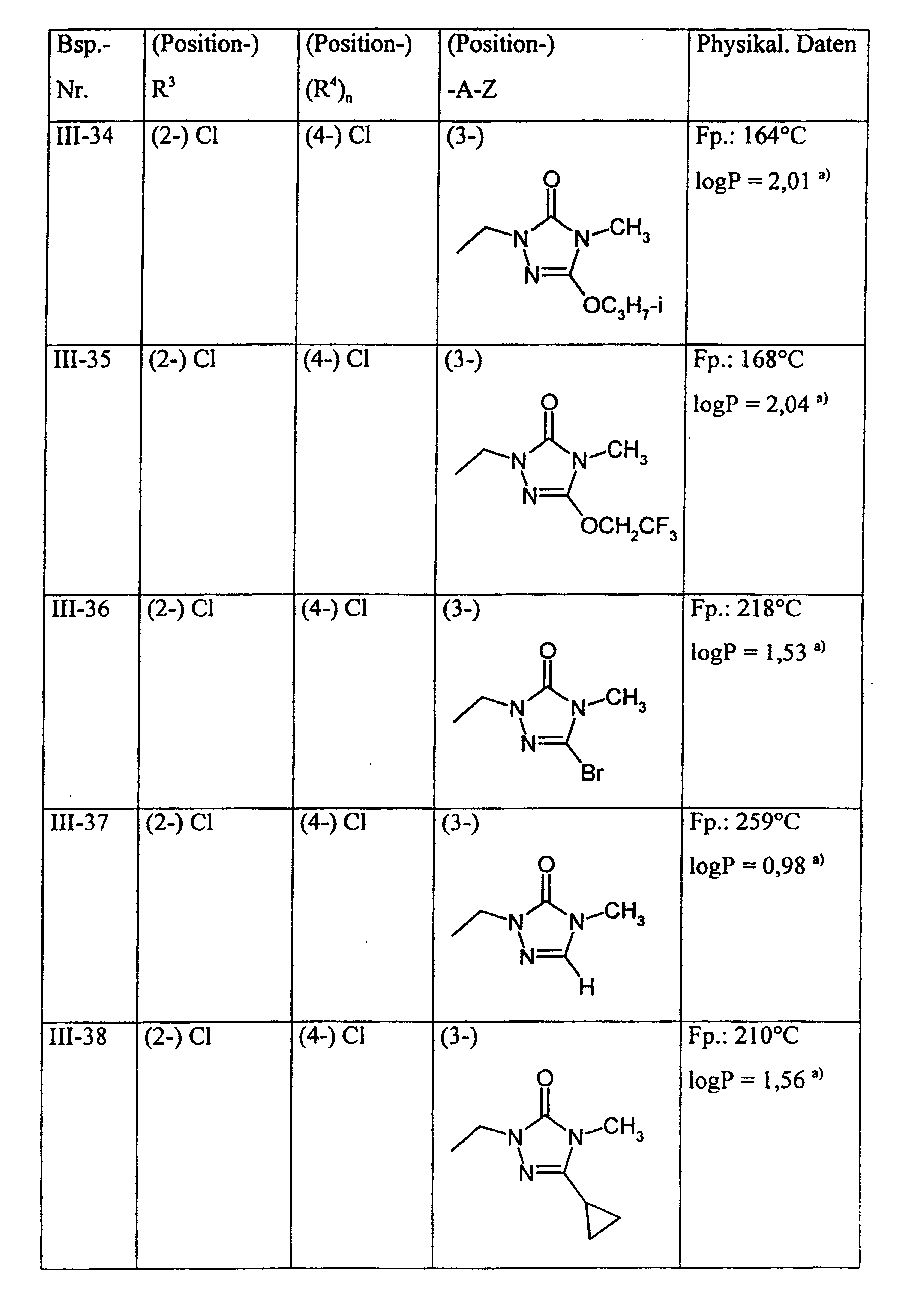

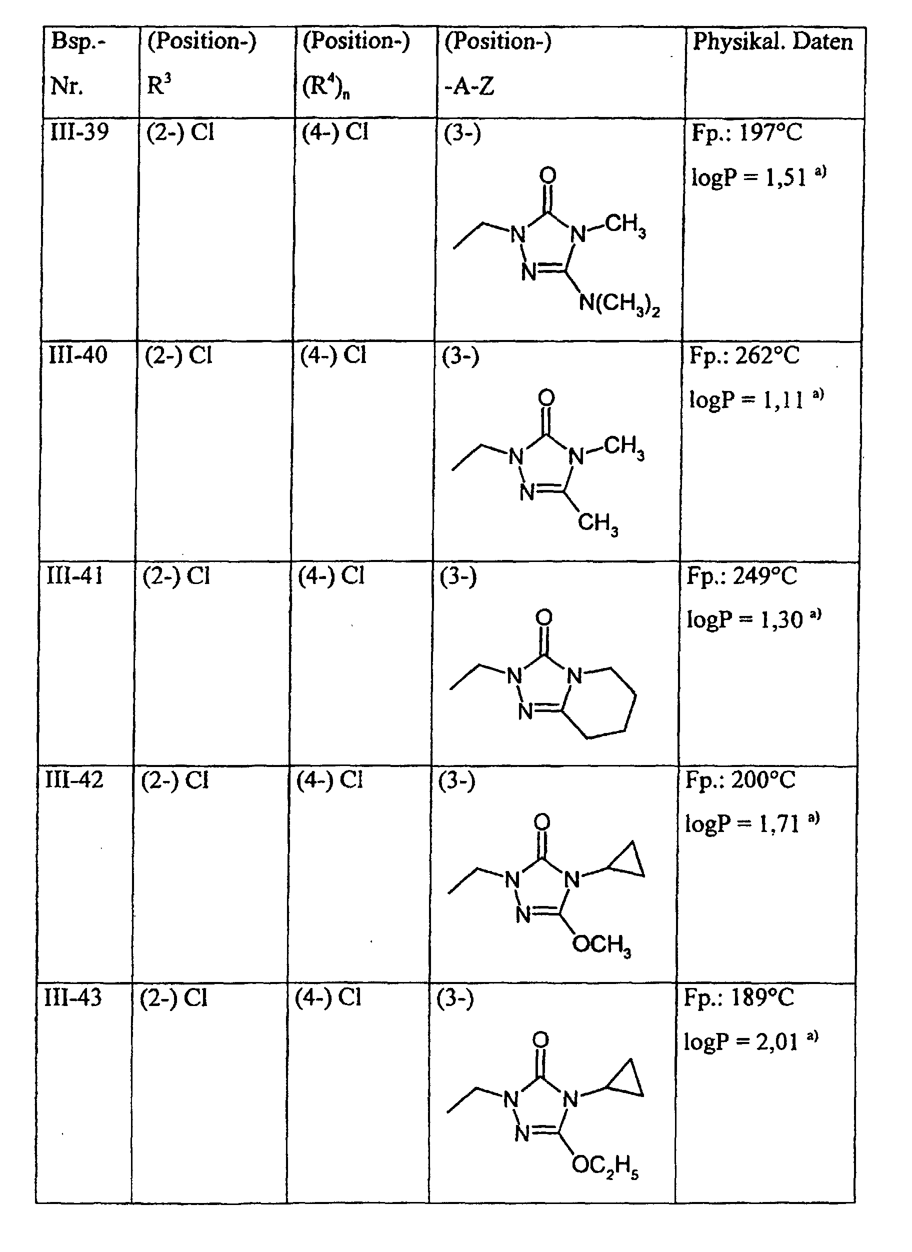

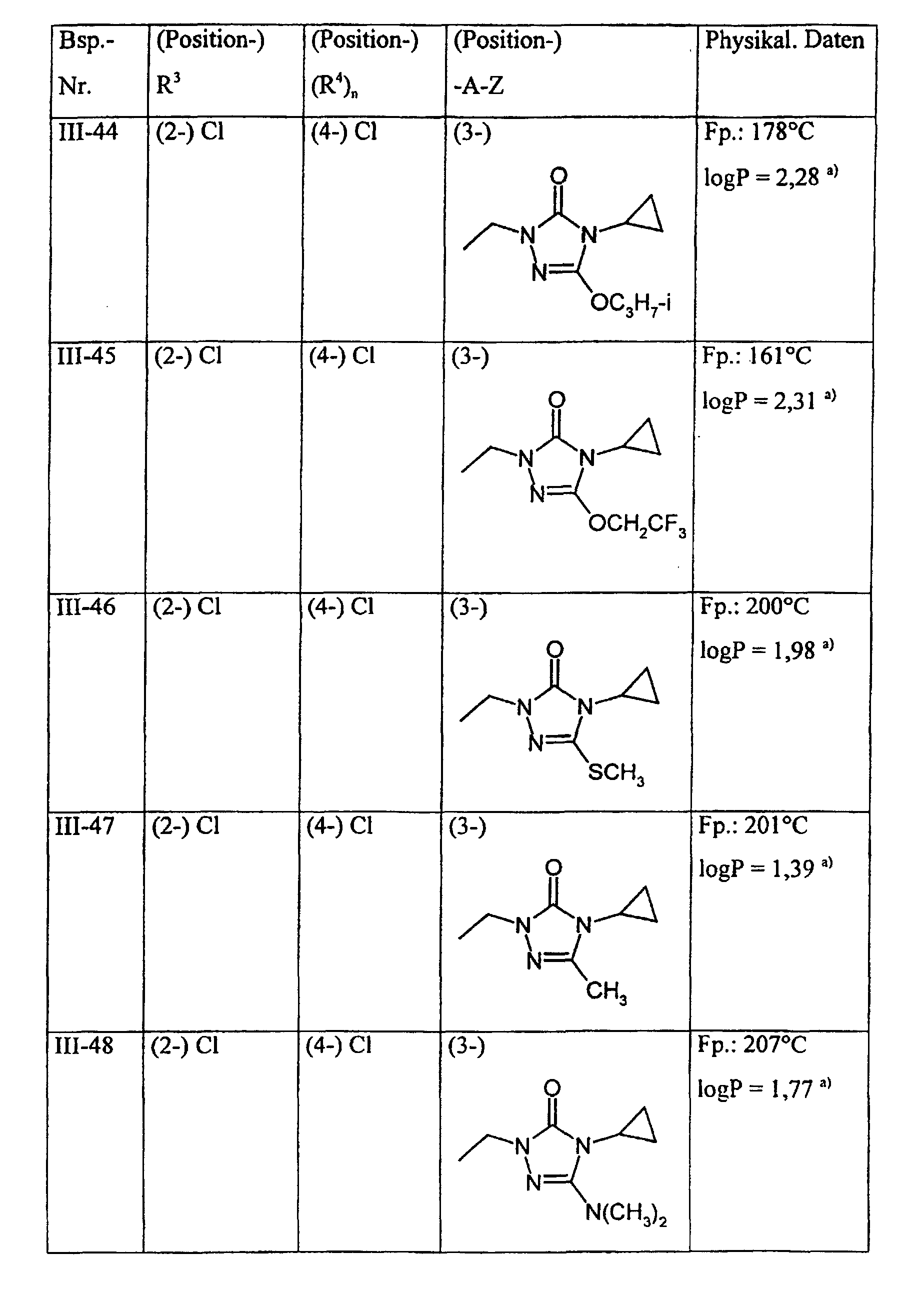

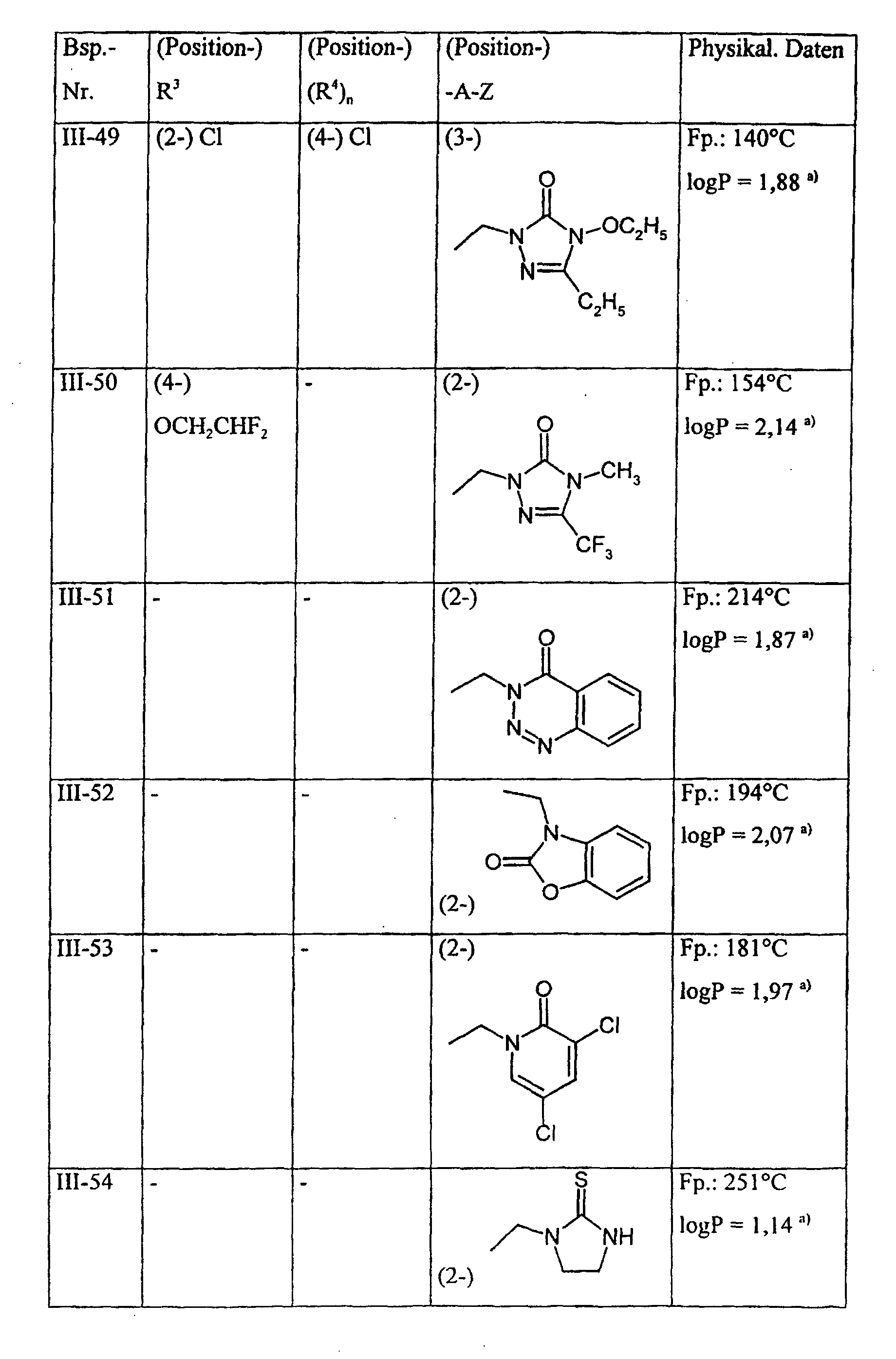

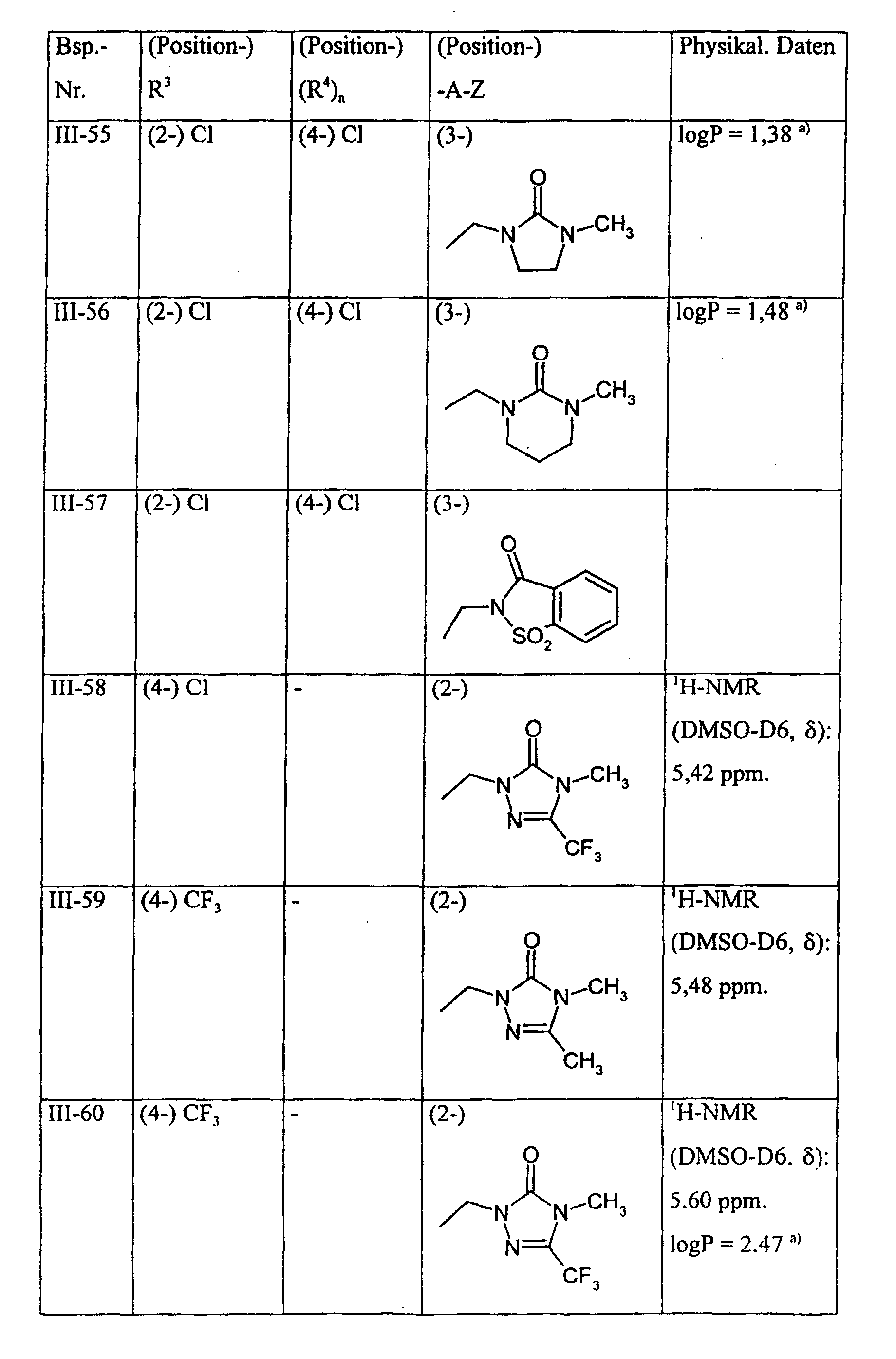

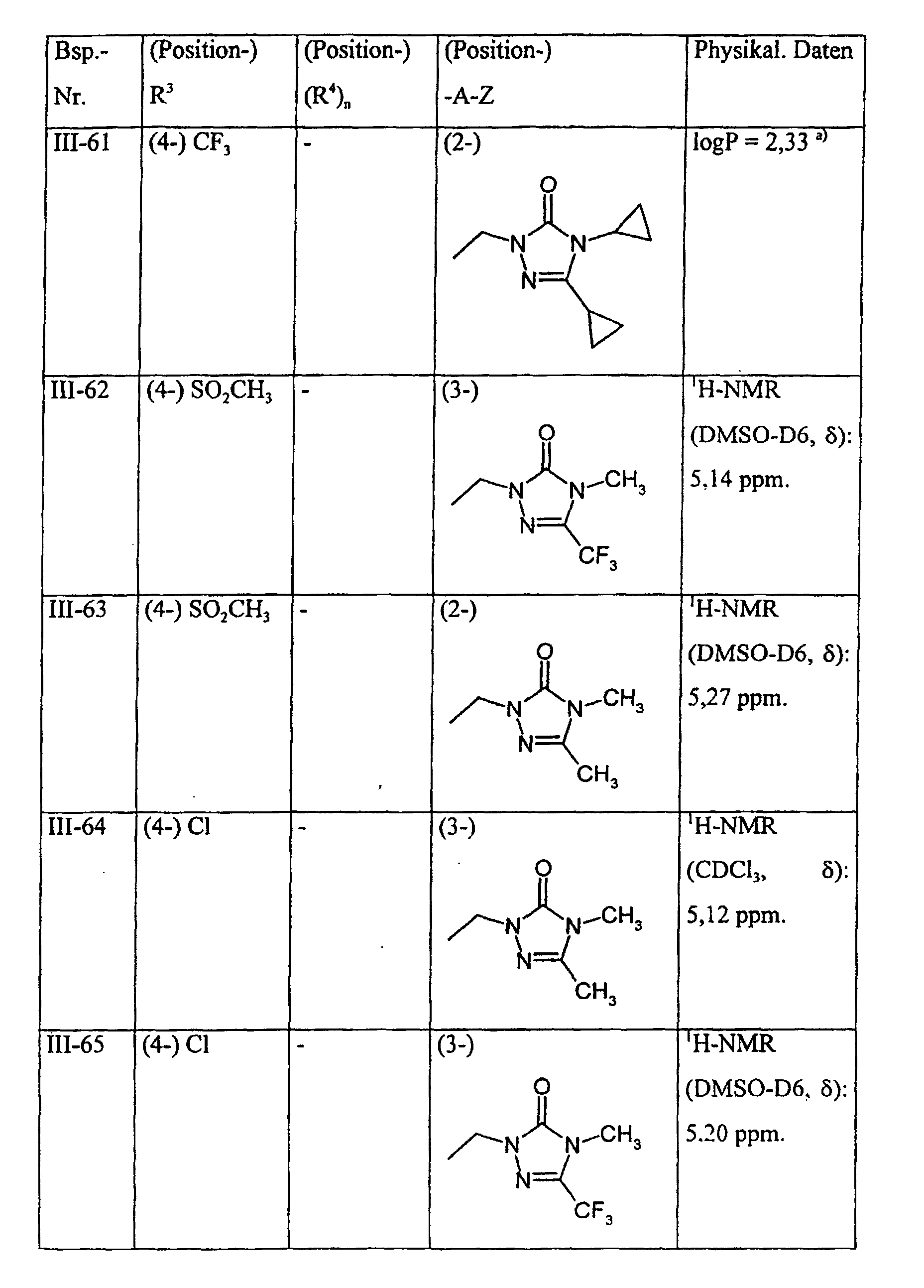

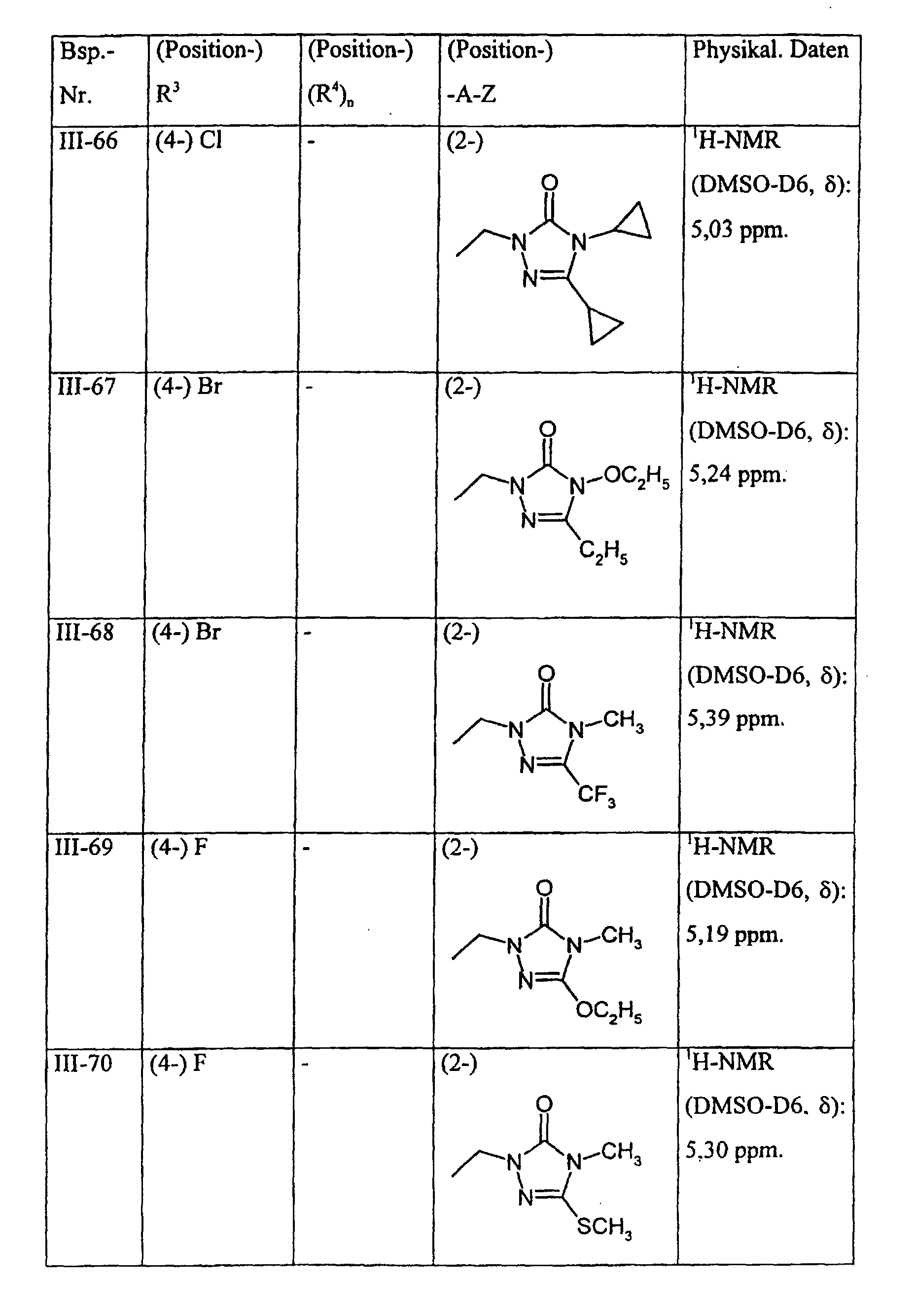

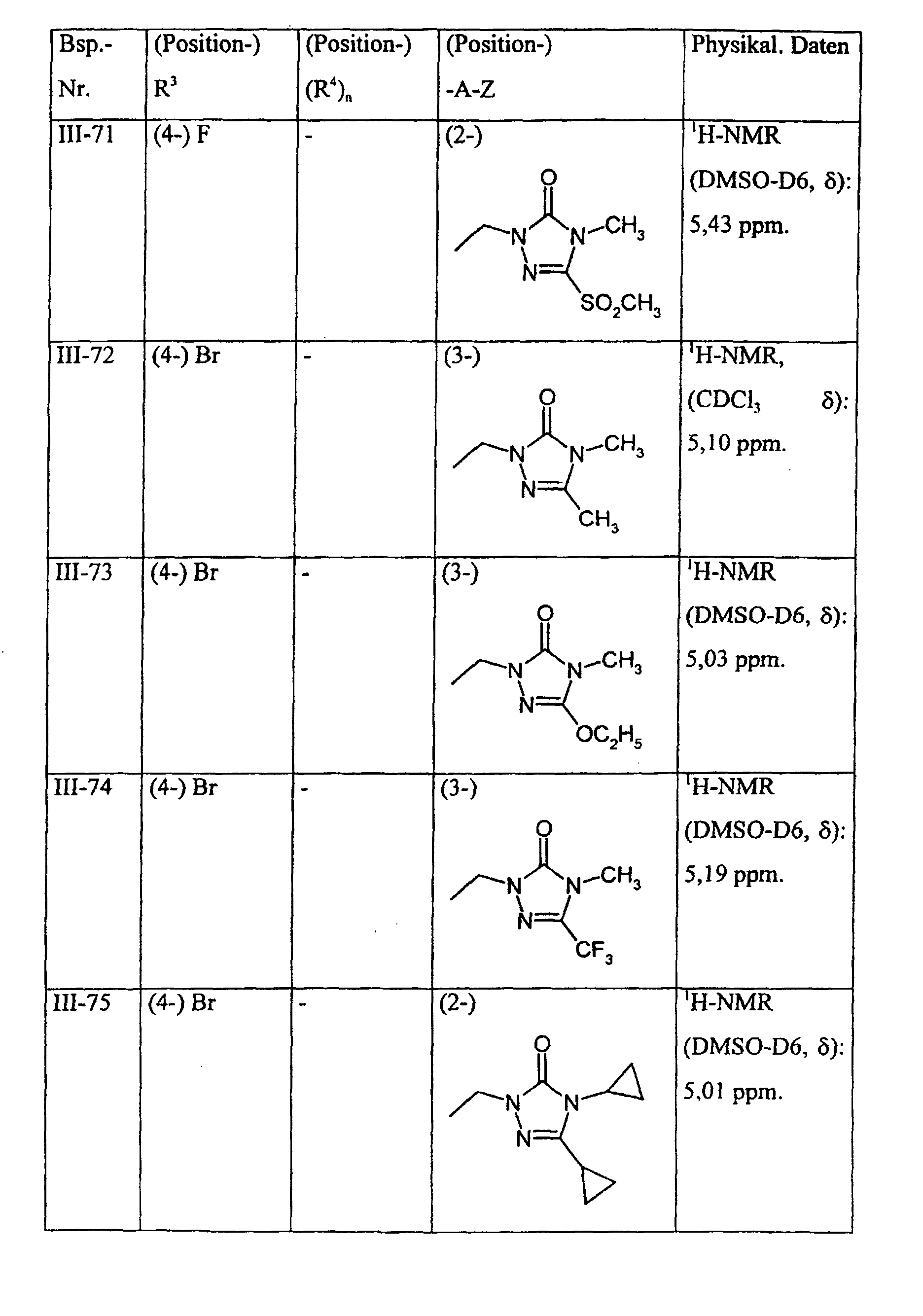

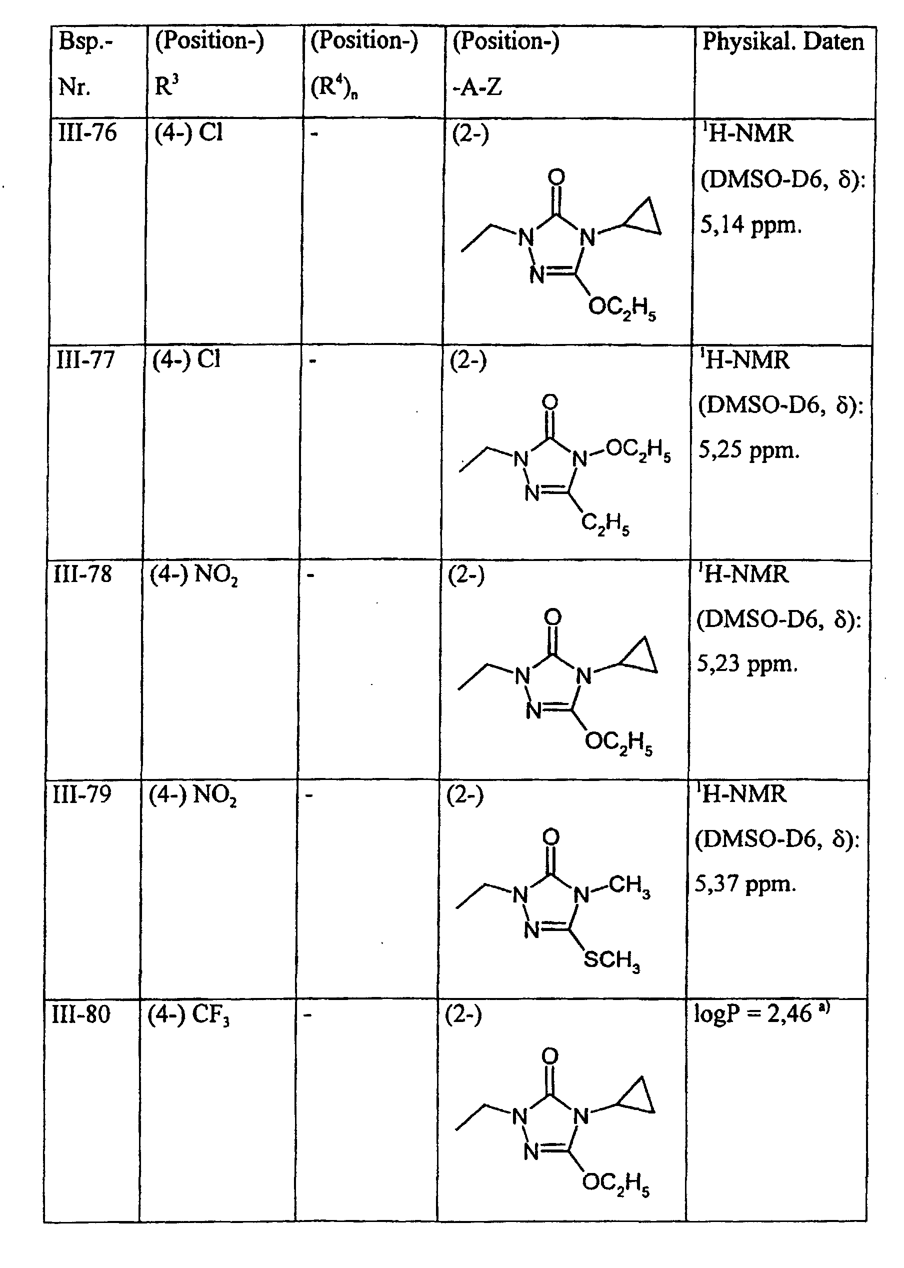

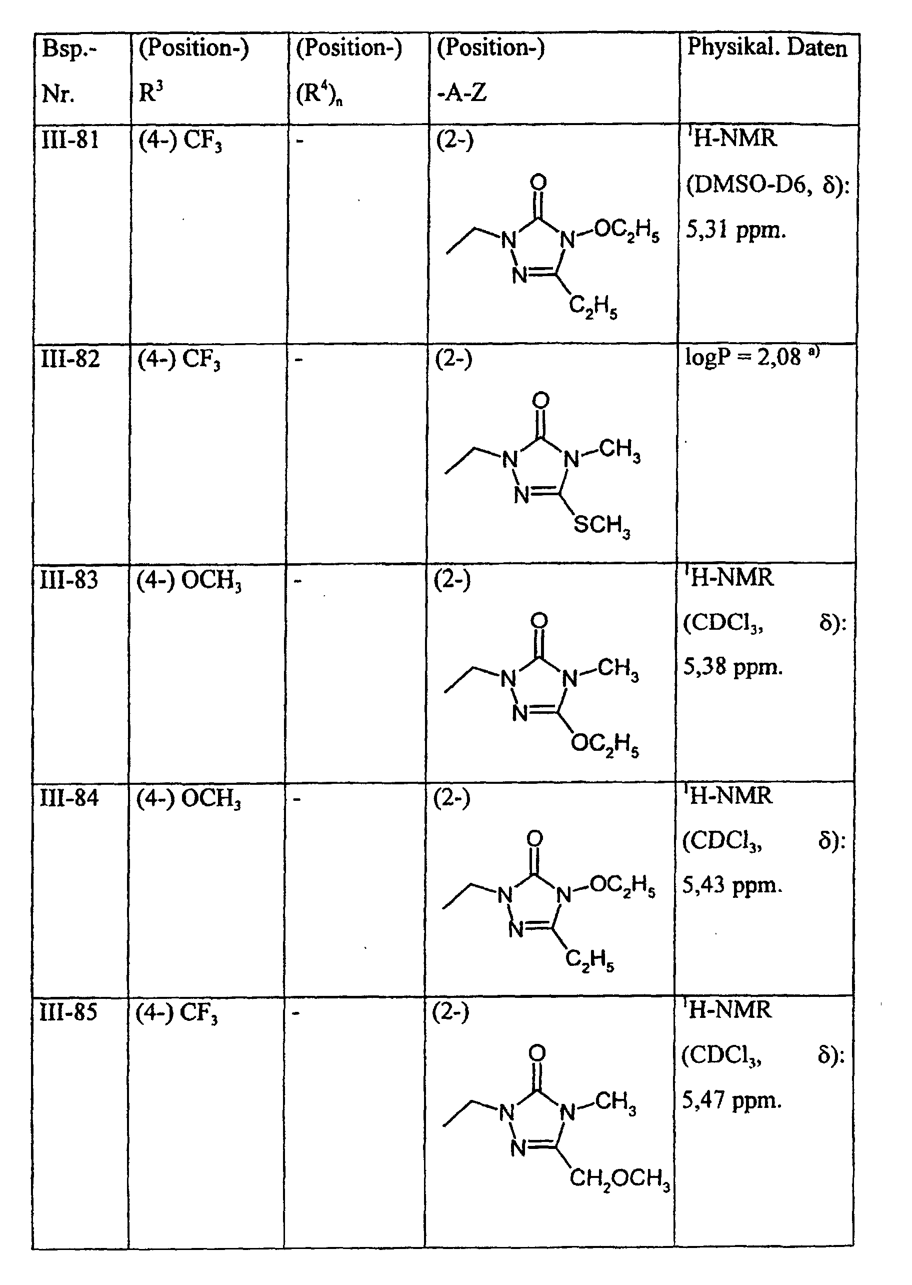

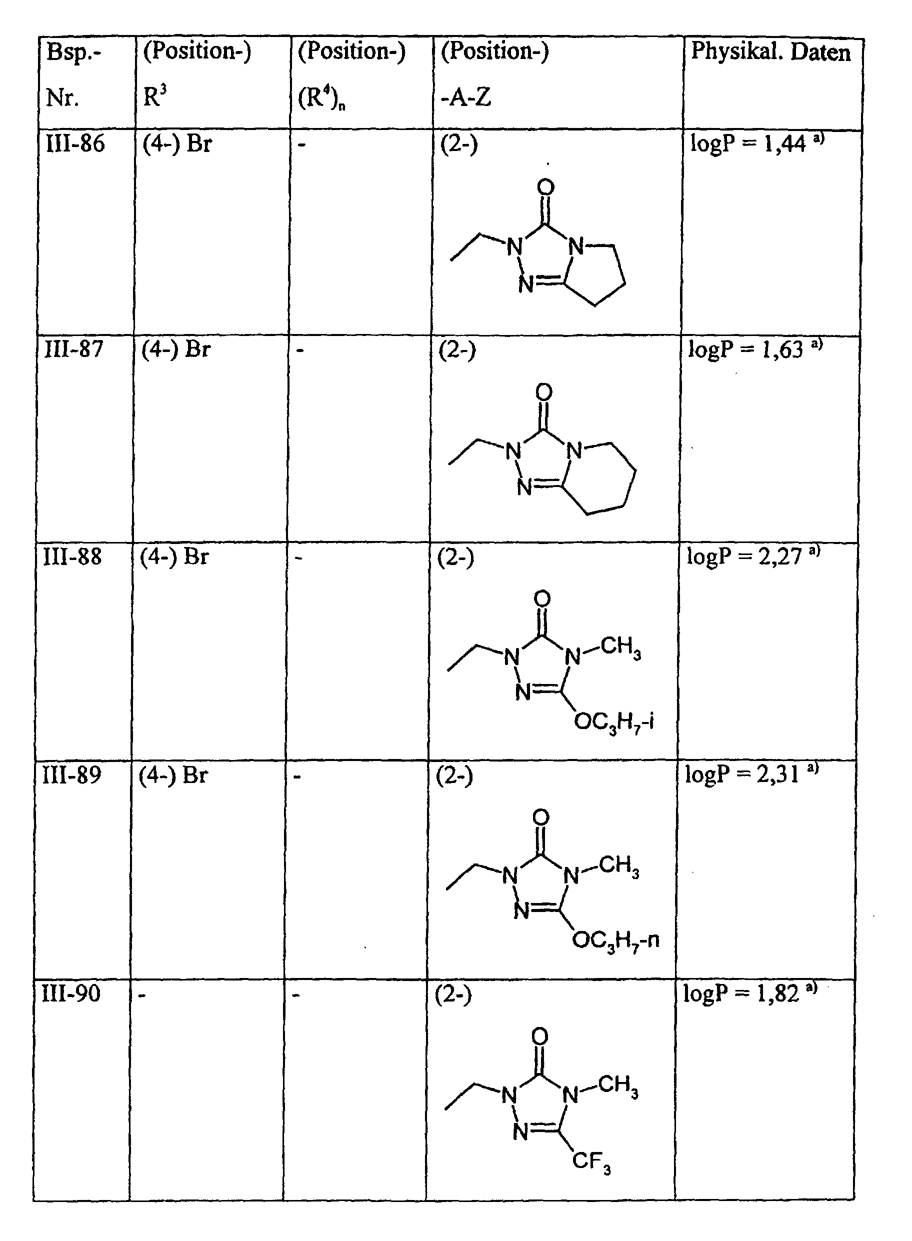

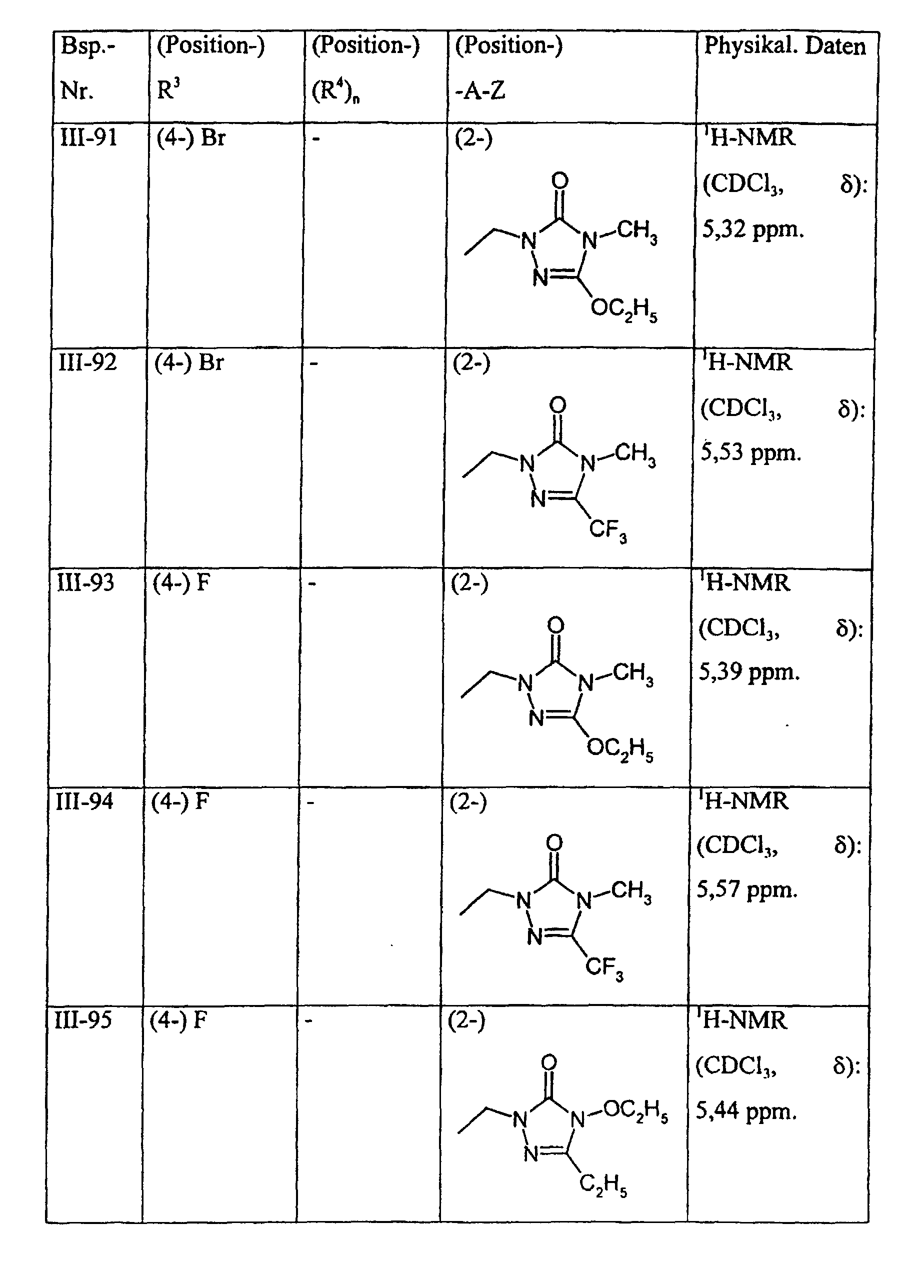

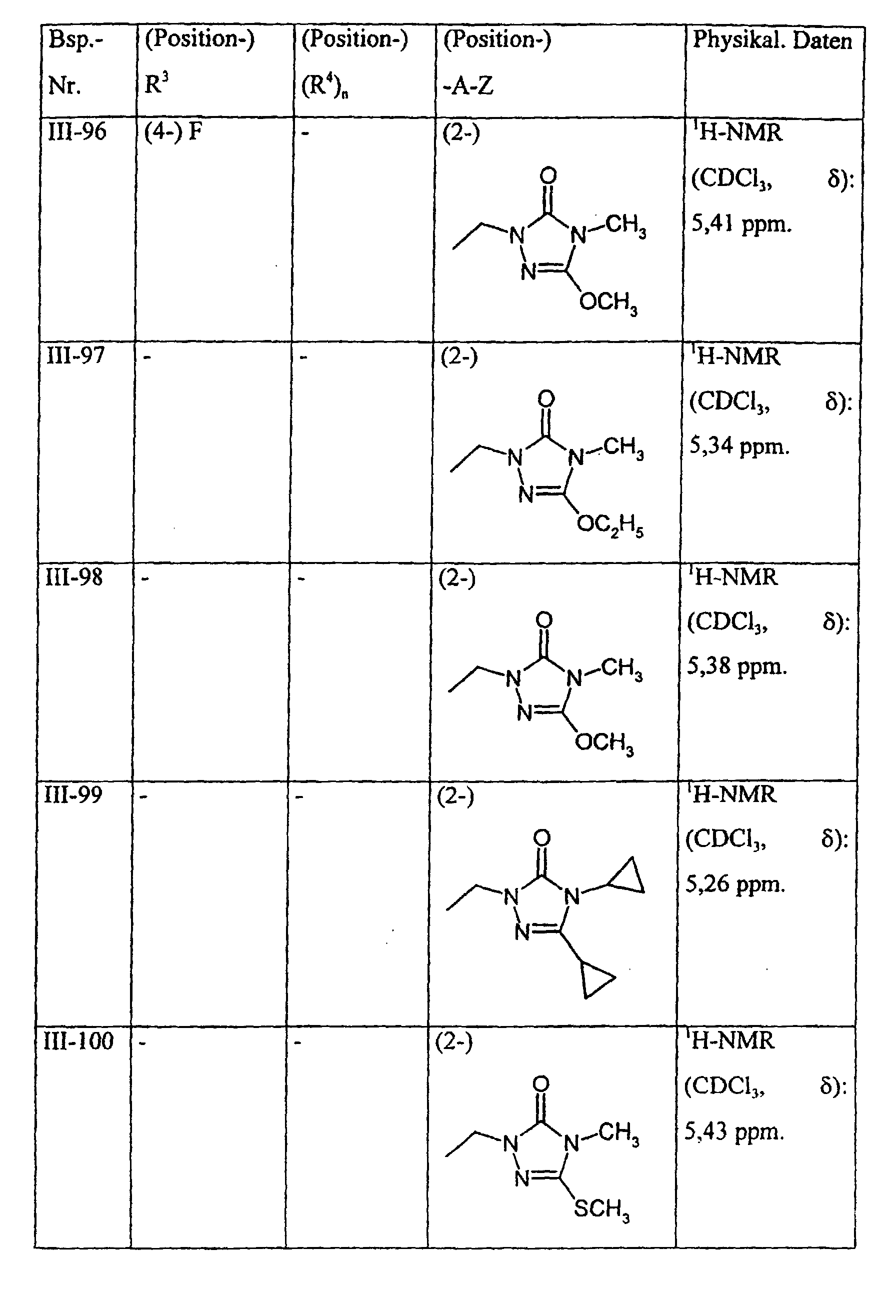

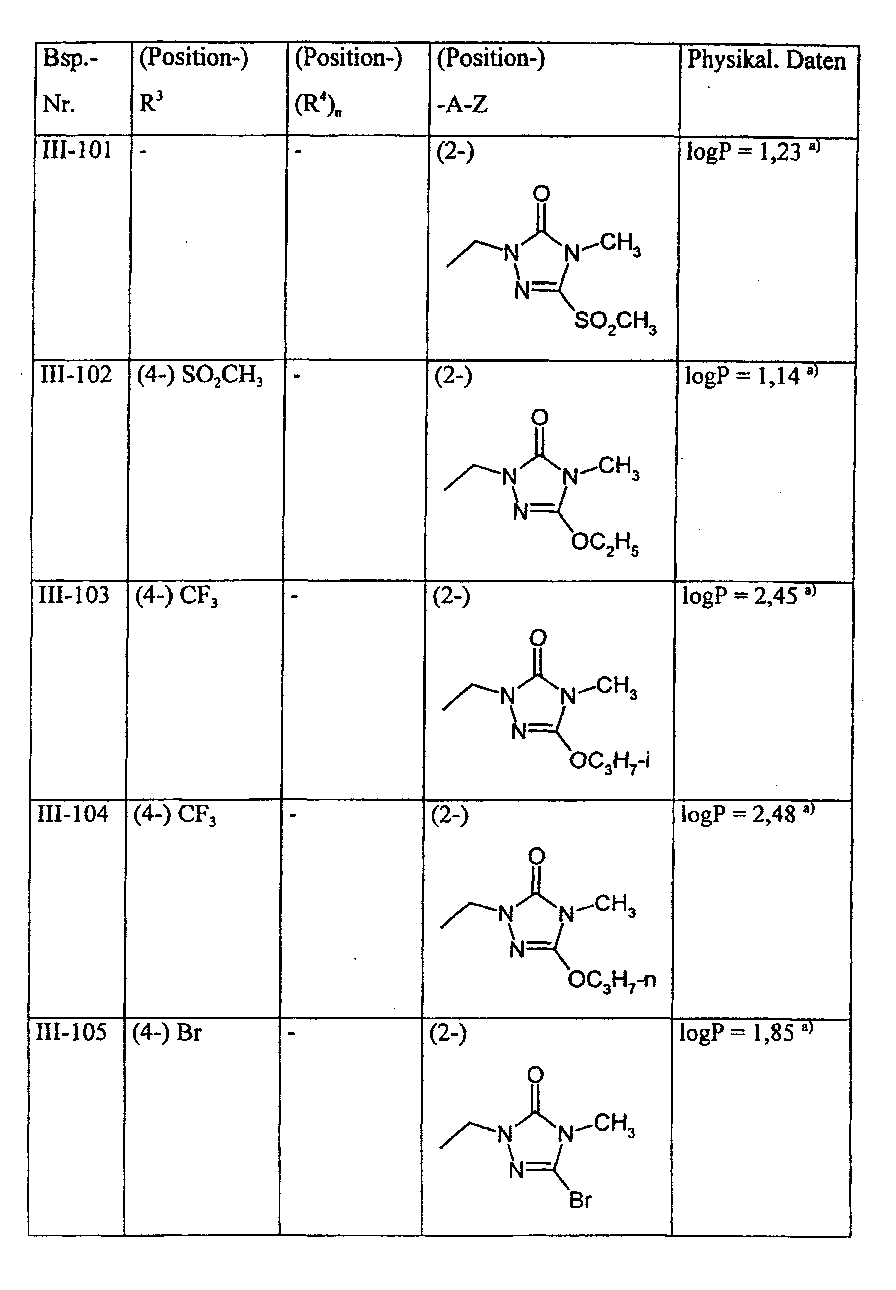

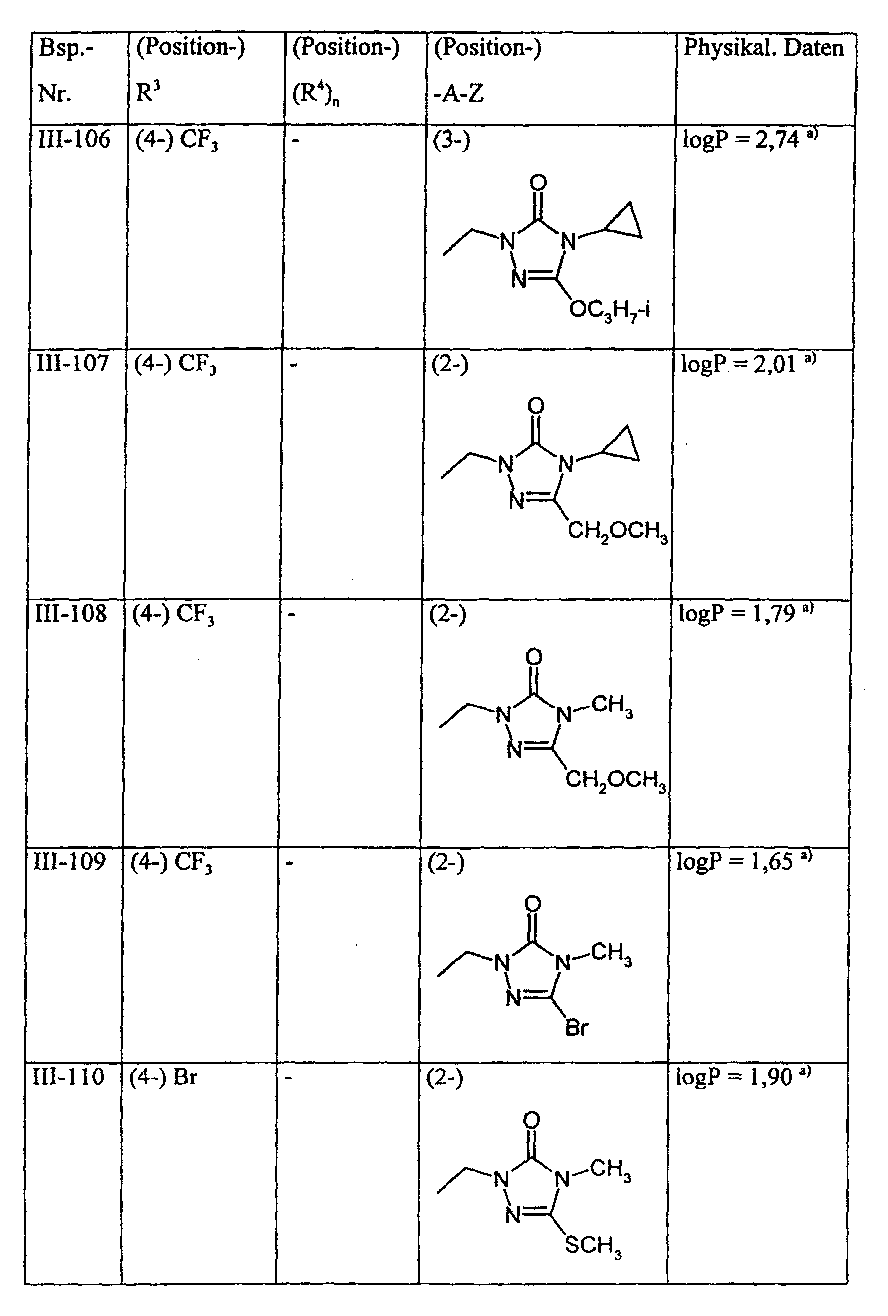

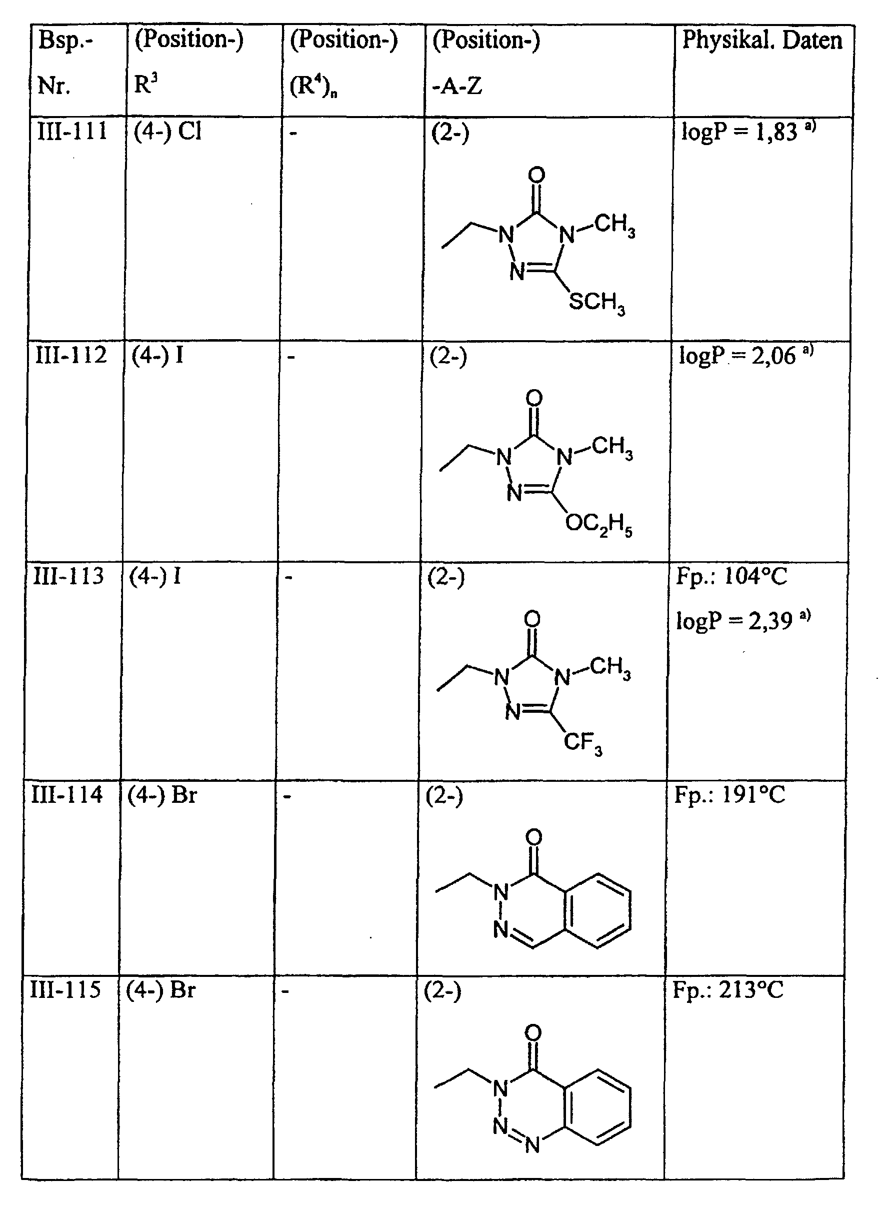

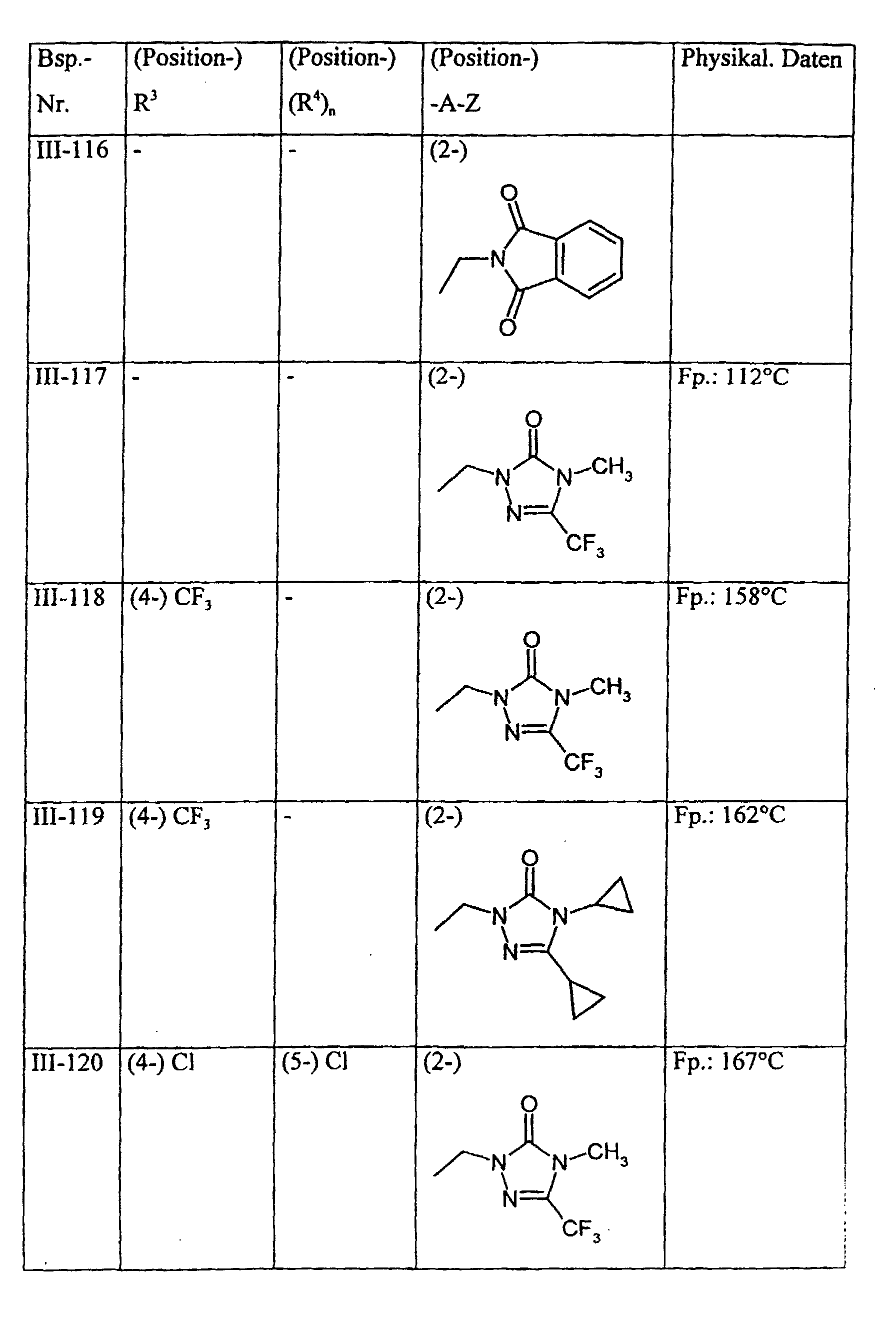

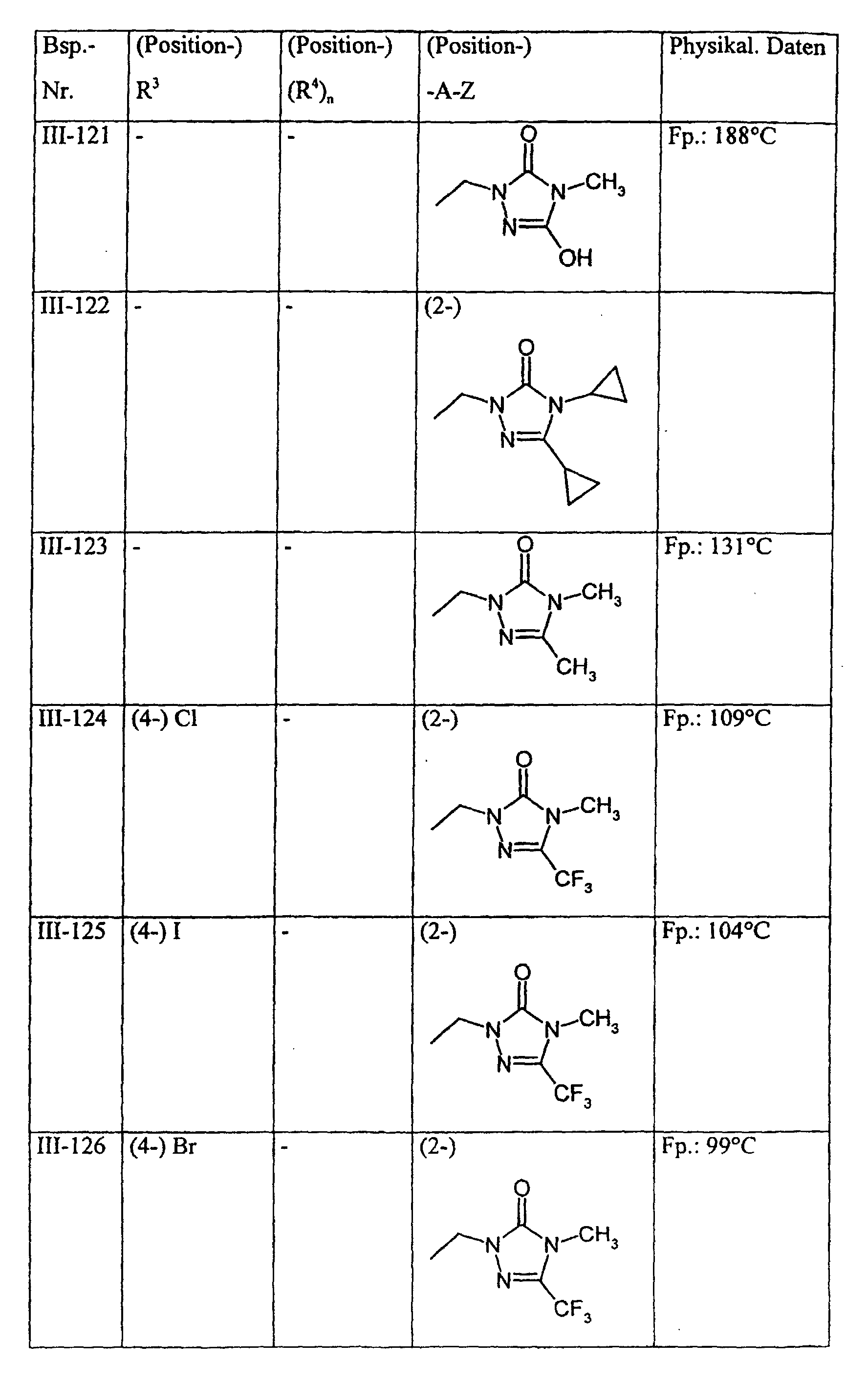

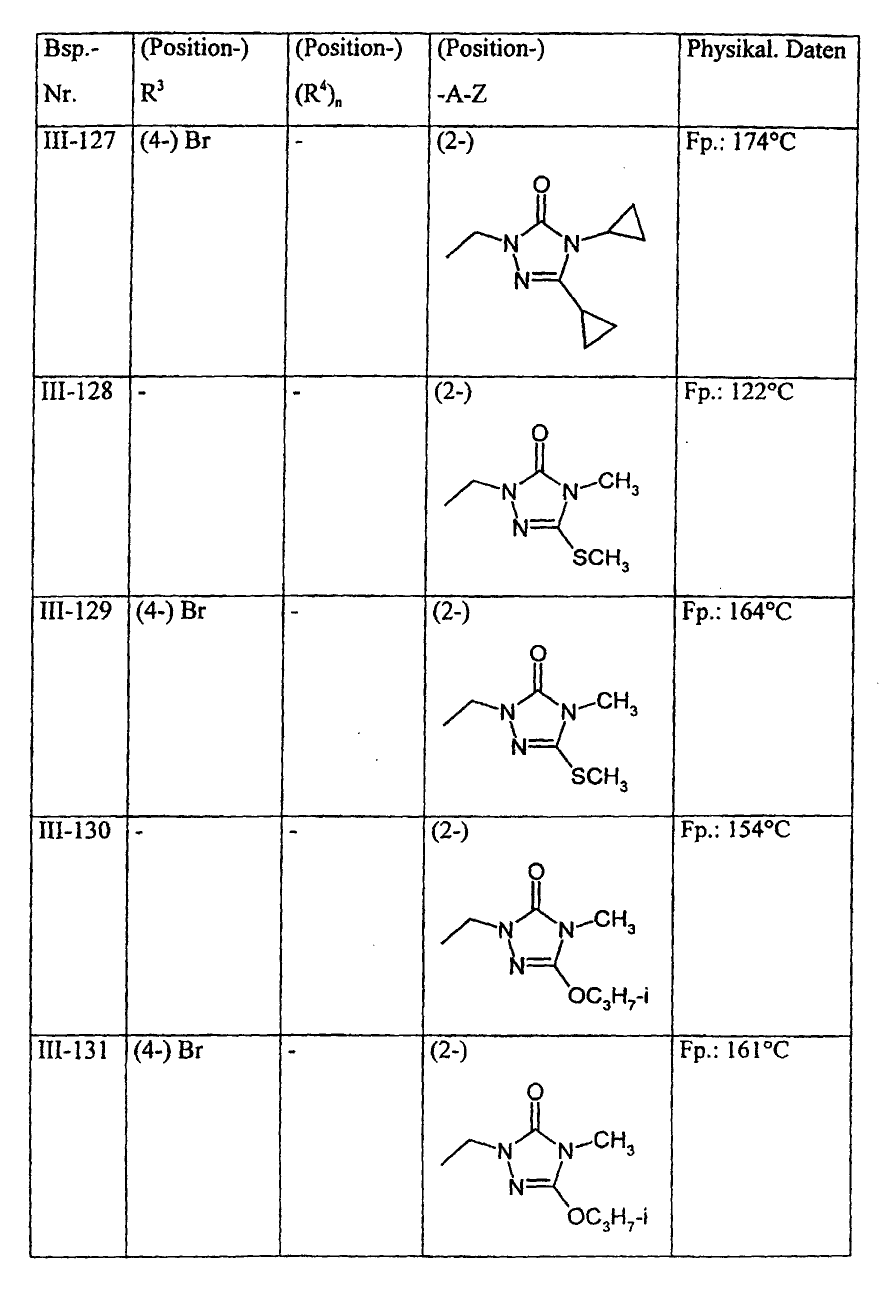

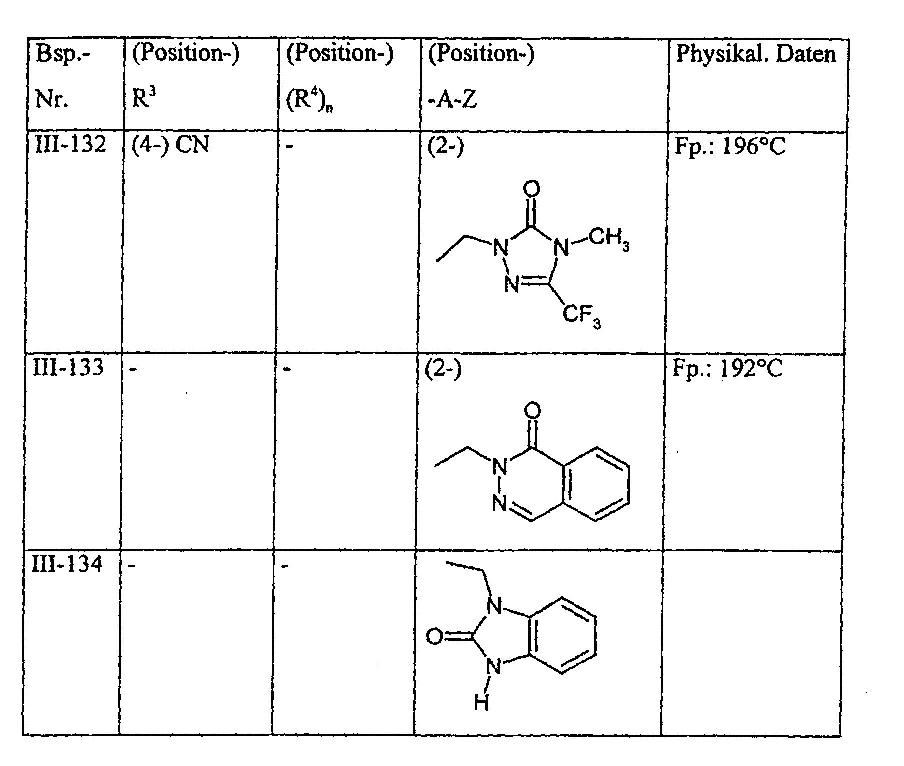

Die Bestimmung der in Tabelle 3 angegebenenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten für die Bestimmung im sauren Bereich: 0,1% wässrige Phosphorsäure, Acetonitril; linearer Gradient von 10% Acetonitril bis 90% Acetonitril - entsprechende Messergebnisse sind in Tabelle 1 mit ^{a)} markiert.
(b) Eluenten für die Bestimmung im neutralen Bereich: 0,01-molare wässrige Phosphatpuffer-Lösung, Acetonitril; linearer Gradient von 10% Acetonitril bis 90% Acetonitril - entsprechende Messergebnisse sind in Tabelle 1 mit ^{b)} markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Ausgangsstoffe der Formel (IV):

### Beispiel (IV-1)

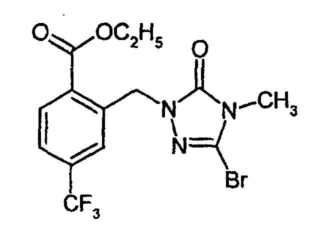

### Stufe 1

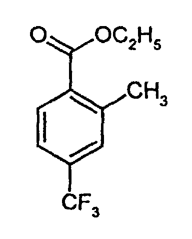

10 g (49 mMol) 2-Methyl-4-trifluormethyl-benzoesäure werden in 150 ml Ethanol gelöst und mit 1 ml konz. Schwefelsäure versetzt. Nach 24 Stunden Erhitzen unter Rückfluss wird die Lösung eingeengt, in Methylenchlorid aufgenommen und mit gesättigter wässriger Natriumhydrogencarbonat-Lösung extrahiert. Die Methylenchlorid-Phase wird über Natriumsulfat getrocknet und im Wasserstrahlvakuum eingeengt.

Man erhält 9 g (80% der Theorie) 2-Methyl-4-trifluormethyl-benzoesäure-ethylester als amorphen Rückstand.

### Stufe 2

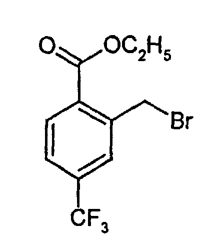

9 g (39 mMol) 2-Methyl-4-trifluormethyl-benzoesäure-ethylester werden in 200 ml Tetrachlormethan gelöst und mit 7 g (39 mMol) *N*-Brom-succinimid und 0.1 g Dibenzoylperoxid versetzt. Nach 6 Stunden Erhitzen unter Rückfluss wird das abgeschiedene Succinimid abfiltriert und das Filtrat im Wasserstrahlvakuum eingeengt.

Man erhält 12 g eines amorphen Rückstandes, der neben 2-Brommethyl-4-trifluormethyl-benzoesäure-ethylester noch 17% 2,2-Dibrommethyl-4-trifluormethylbenzoesäure-ethylester und 12% 2-Methyl-4-trifluormethyl-benzoesäure-ethylester enthält.

### Stufe 3

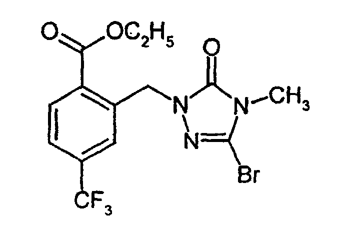

4 g 2-Brommethyl-4-trifluormethyl-benzoesäure-ethylester (ca. 70%ig) und 2.28 g (12,8 mMol) 5-Brom-4-methyl-2,4-dihydro-3H-1,2,4-triazol-3-on werden in 150 ml Acetonitril gelöst, mit 5.3 g (38,4 mMol) Kaliumcarbonat versetzt und unter kräftigem Rühren 2 Stunden zum Rückfluss erhitzt. Das Reaktionsgemisch wird in Wasser aufgenommen und mit Methylenchlorid mehrfach extrahiert. Die gesammelten Methylenchlorid-Phasen werden über Natriumsulfat getrocknet, im Wasserstrahlvakuum eingeengt und chromatographiert.

Man erhält 2 g (38 % der Theorie) 5-Brom-4-methyl-2-(2-ethoxycarbonyl-5-trifluormethyl-benzyl)-2,4-dihydro-3H-1,2,4-triazol-3-on als amorphes Produkt.
¹H-NMR (CDCl₃, δ): 5,46 ppm.

### Beispiel (IV-2)

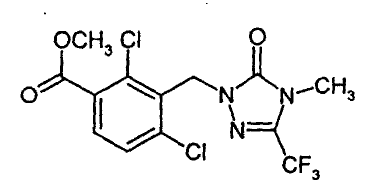

6,7 g (40 mMol) 4-Methyl-5-trifluormethyl-2,4-dihydro-3H-1,2,4-triazol-3-on werden in 150 ml Acetonitril vorgelegt und mit 11 g (80 mMol) Kaliumcarbonat verrührt. Nach Erwärmen der Mischung auf 50°C wird dann eine Lösung von 13,1 g (44 mMol) 3-Brommethyl-2,4-dichlor-benzoesäure-methylester in 20 ml Acetonitril unter Rühren tropfenweise dazu gegeben und die Reaktionsmischung wird noch 15 Stunden unter Rühren zum Rückfluss erhitzt. Anschließend wird im Wasserstrahlvakuum eingeengt, der Rückstand in Methylenchlorid aufgenommen, mit 1N-Salzsäure gewaschen, mit Natriumsulfat getrocknet und filtriert. Das Filtrat wird unter vermindertem Druck eingeengt, der Rückstand mit Petrolether digeriert und das kristallin angefallene Produkt durch Absaugen isoliert.

Man erhält 14,9 g (97% der Theorie) 4-Methyl-5-trifluormethyl-2-(2,6-dichlor-3-methoxycarbonyl-benzyl)-2,4-dihydro-3H-1,2,4-triazol-3-on vom Schmelzpunkt 109°C.

Analog zu den Beispielen (IV-1) und (IV-2) können beispielsweise auch die in der nachstehenden Tabelle 4 aufgeführten Verbindungen der allgemeinen Formel (IV) hergestellt werden.

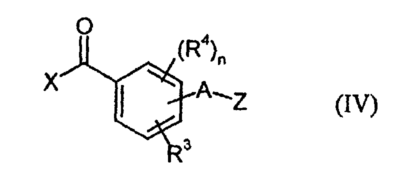

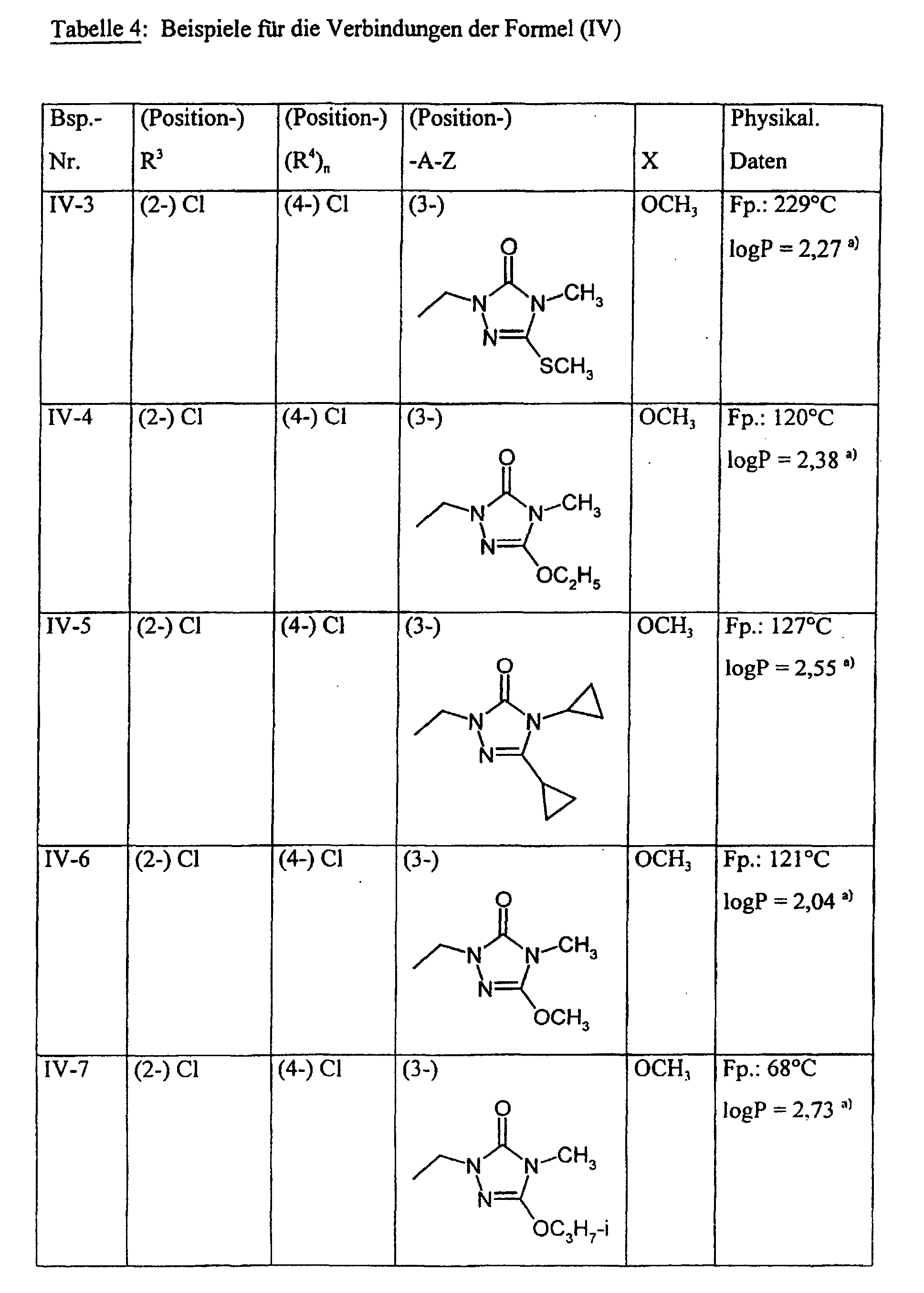

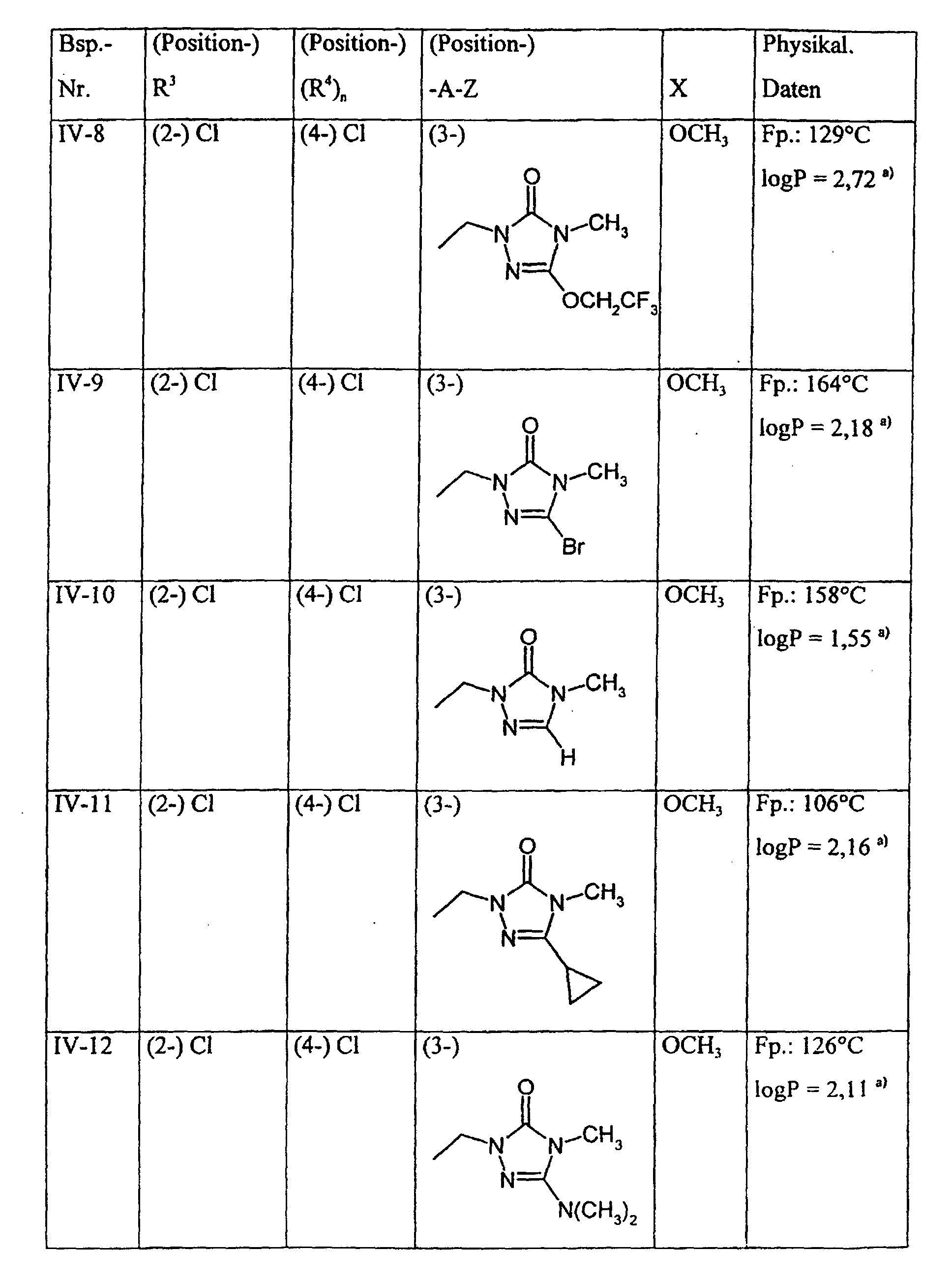

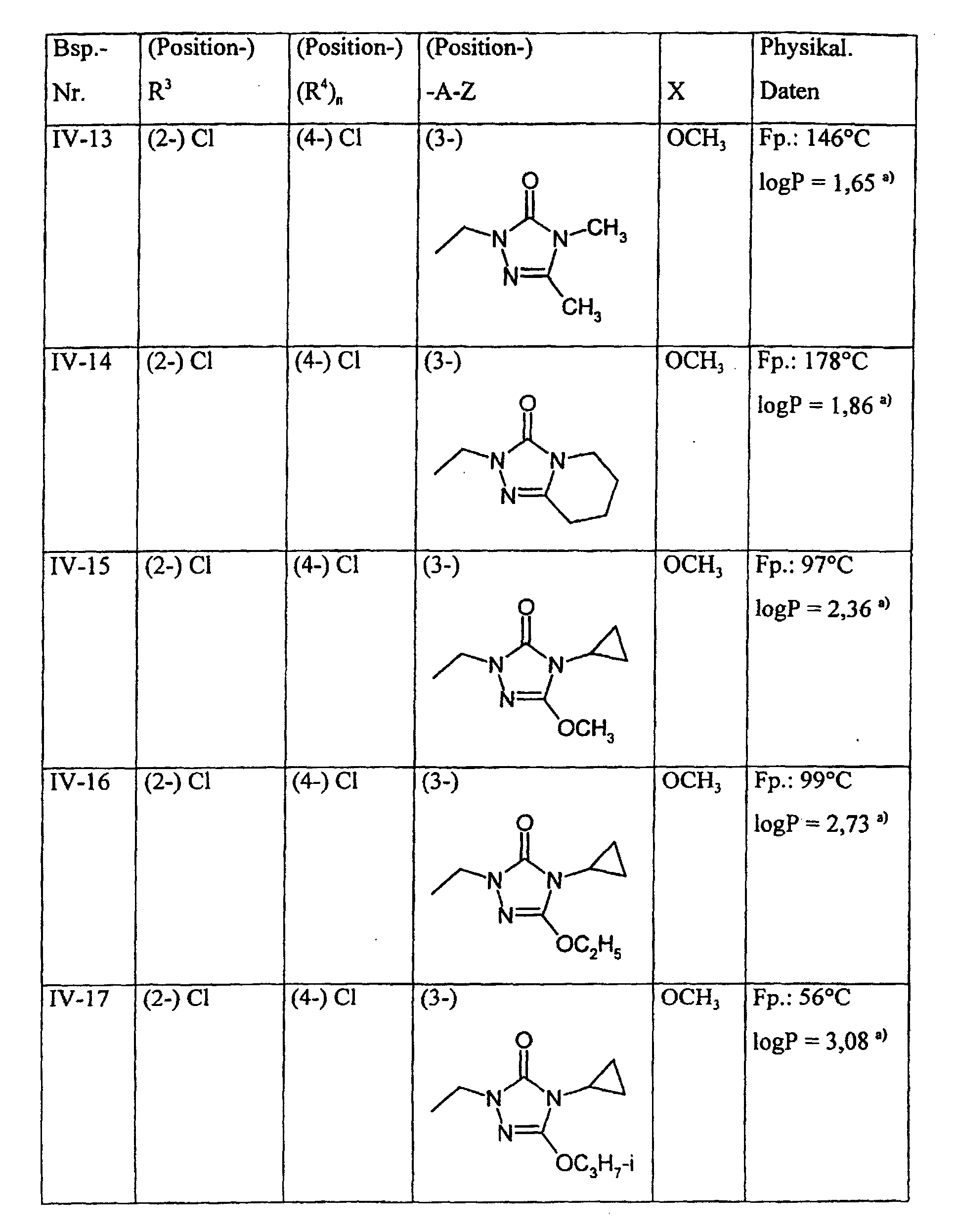

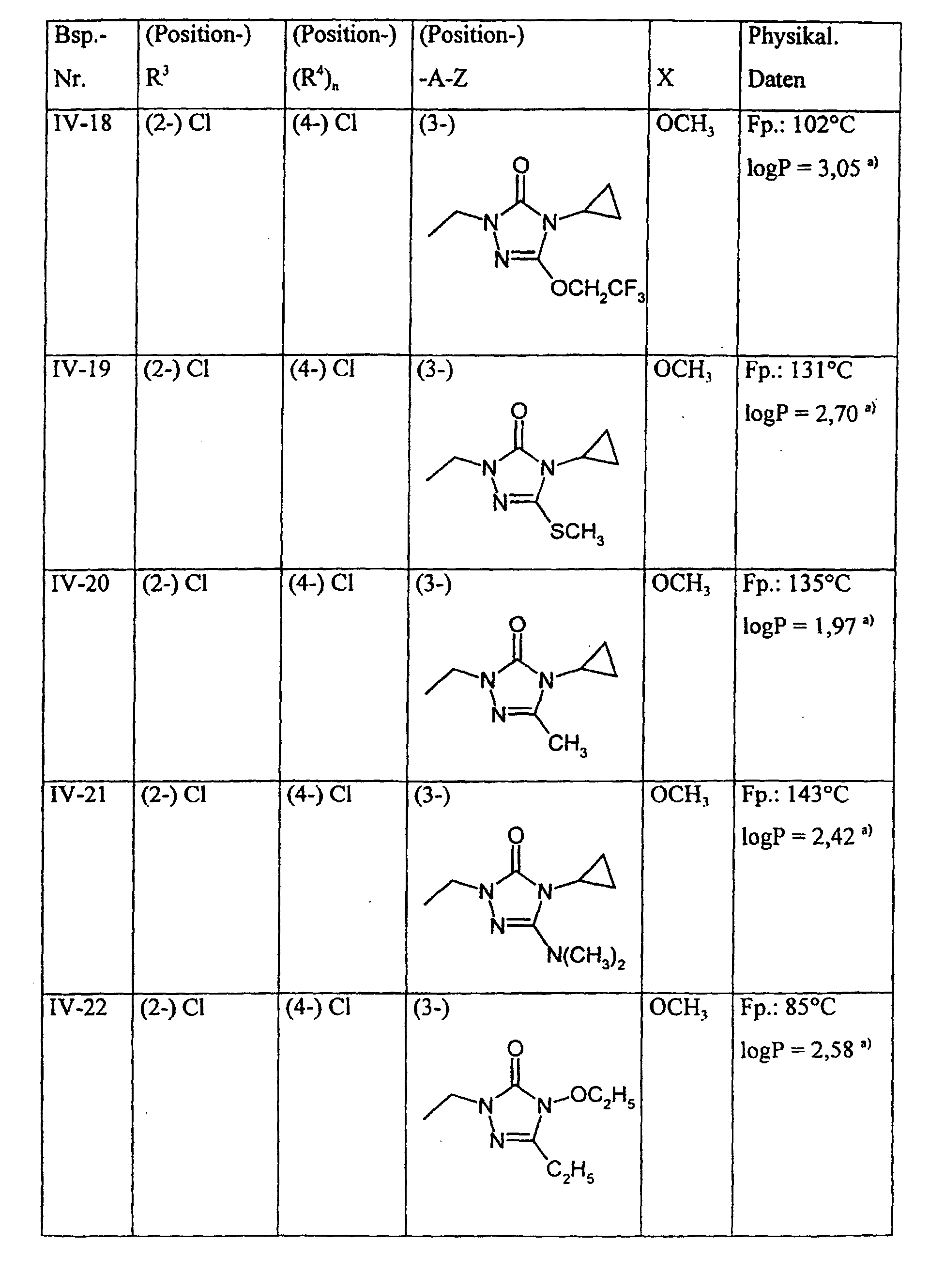

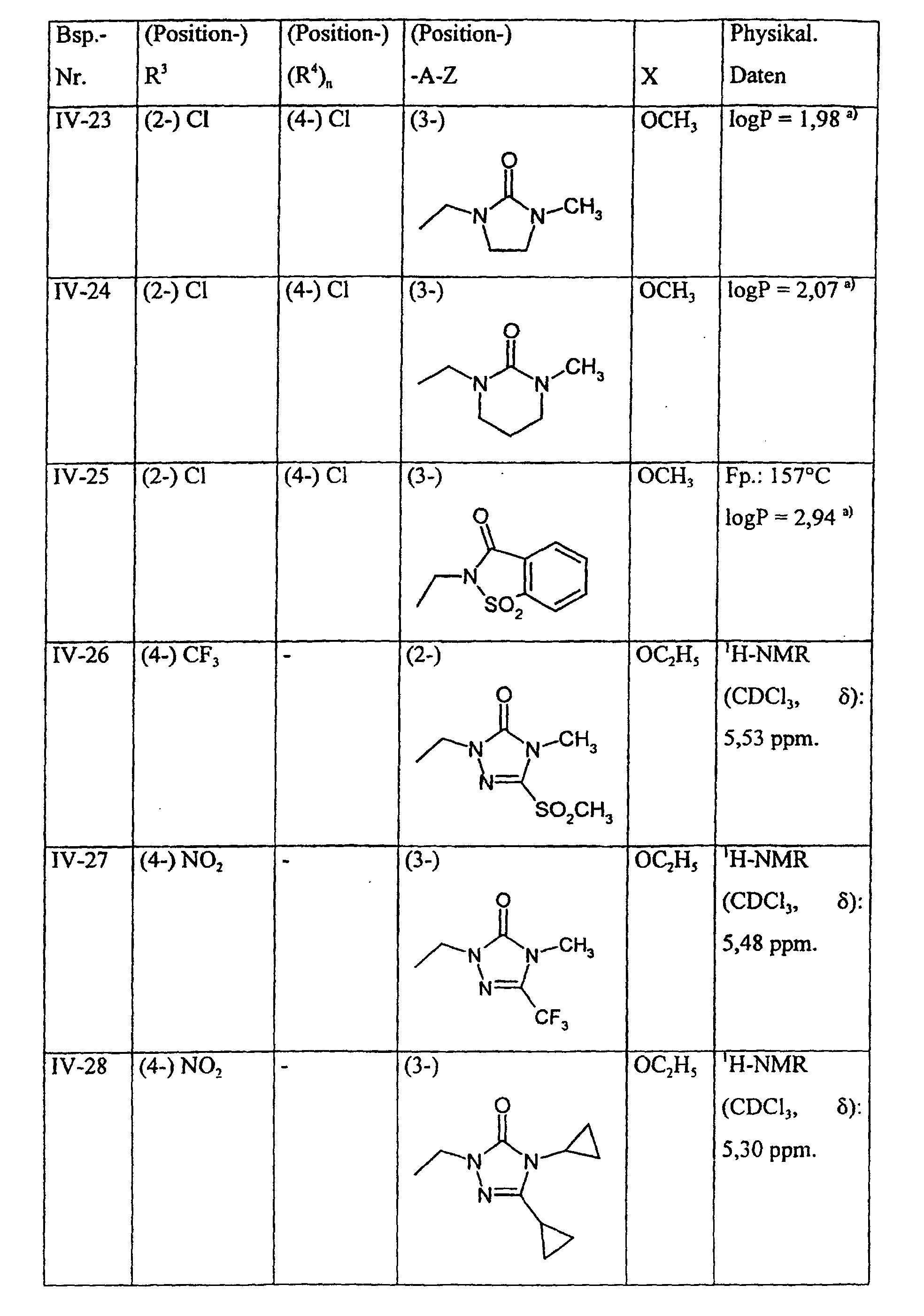

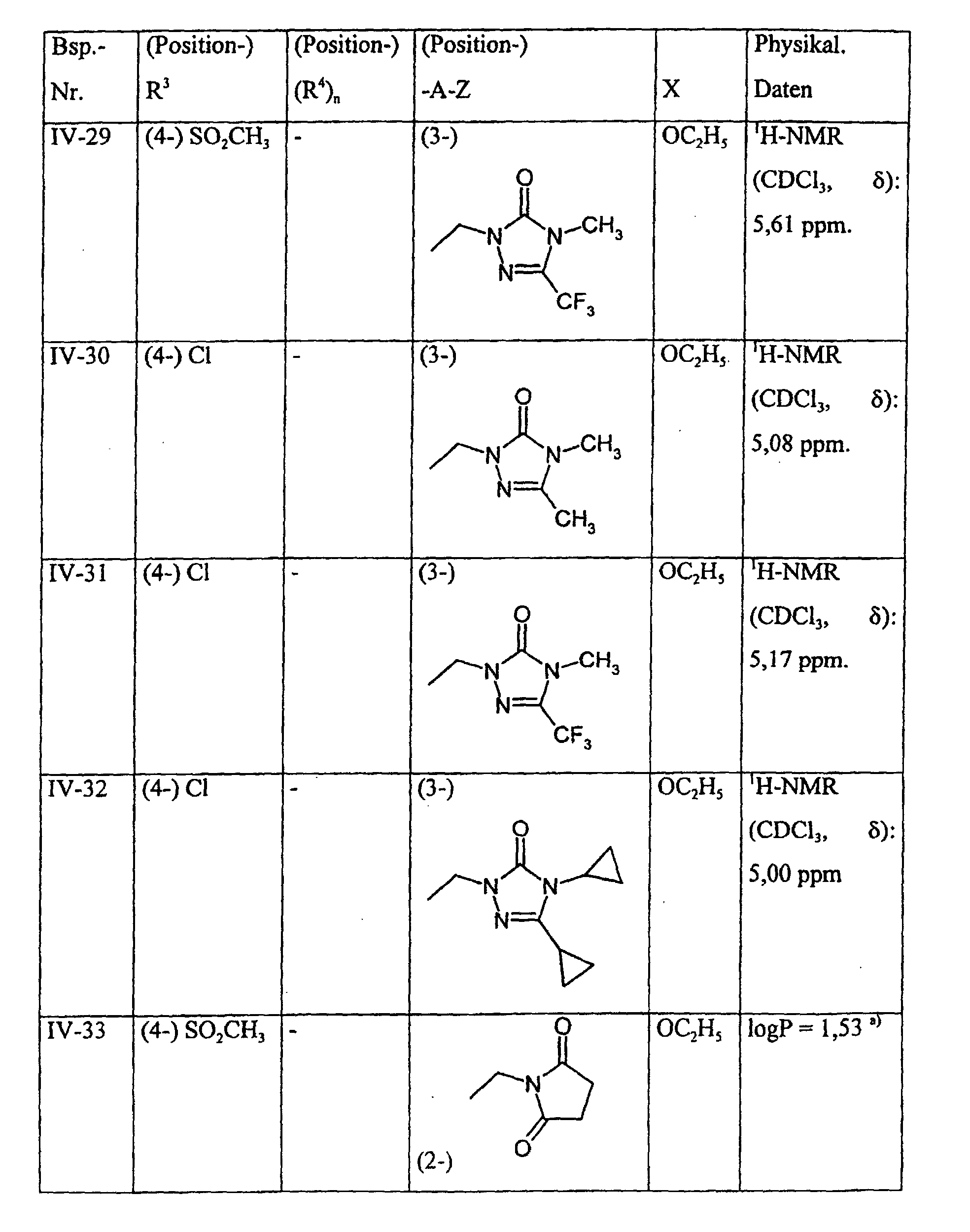

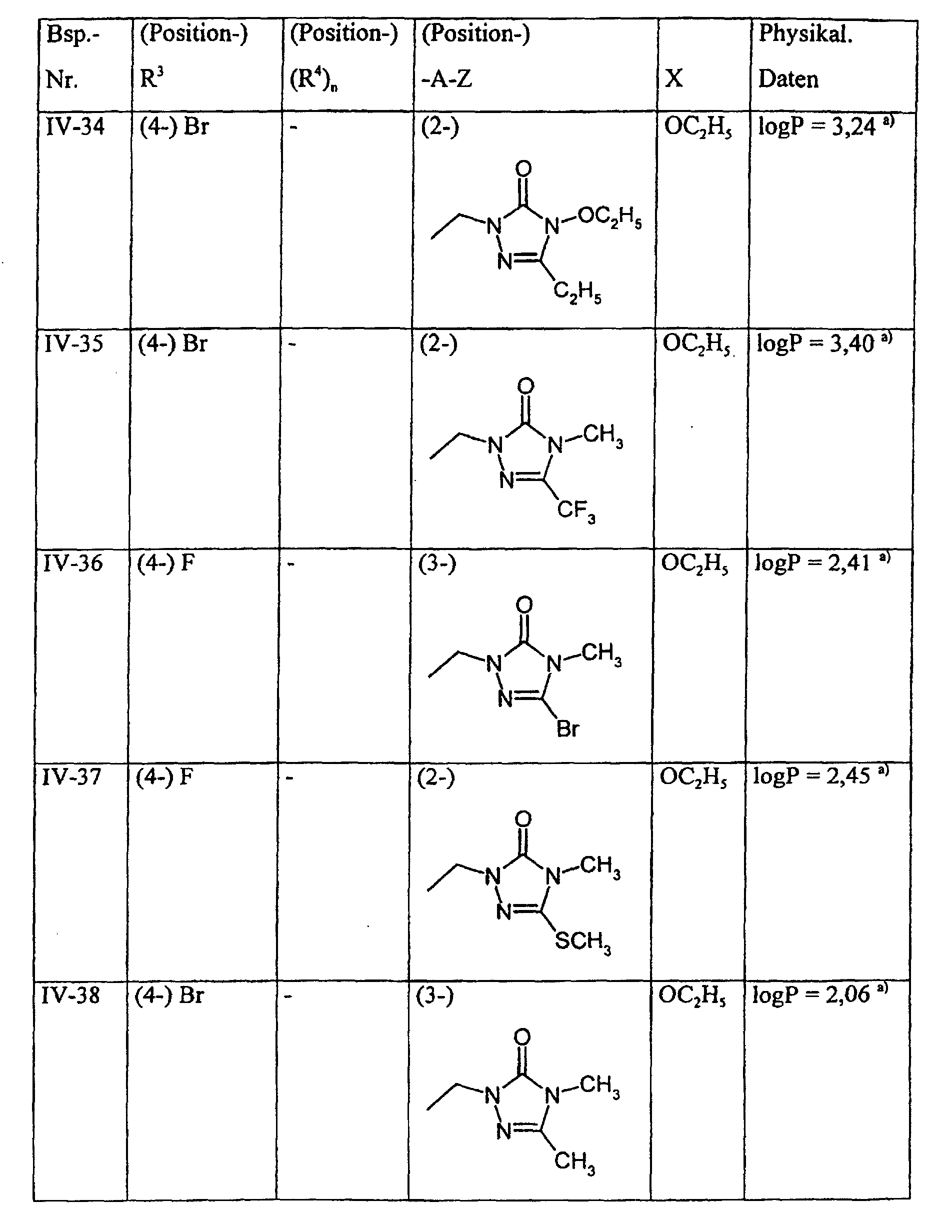

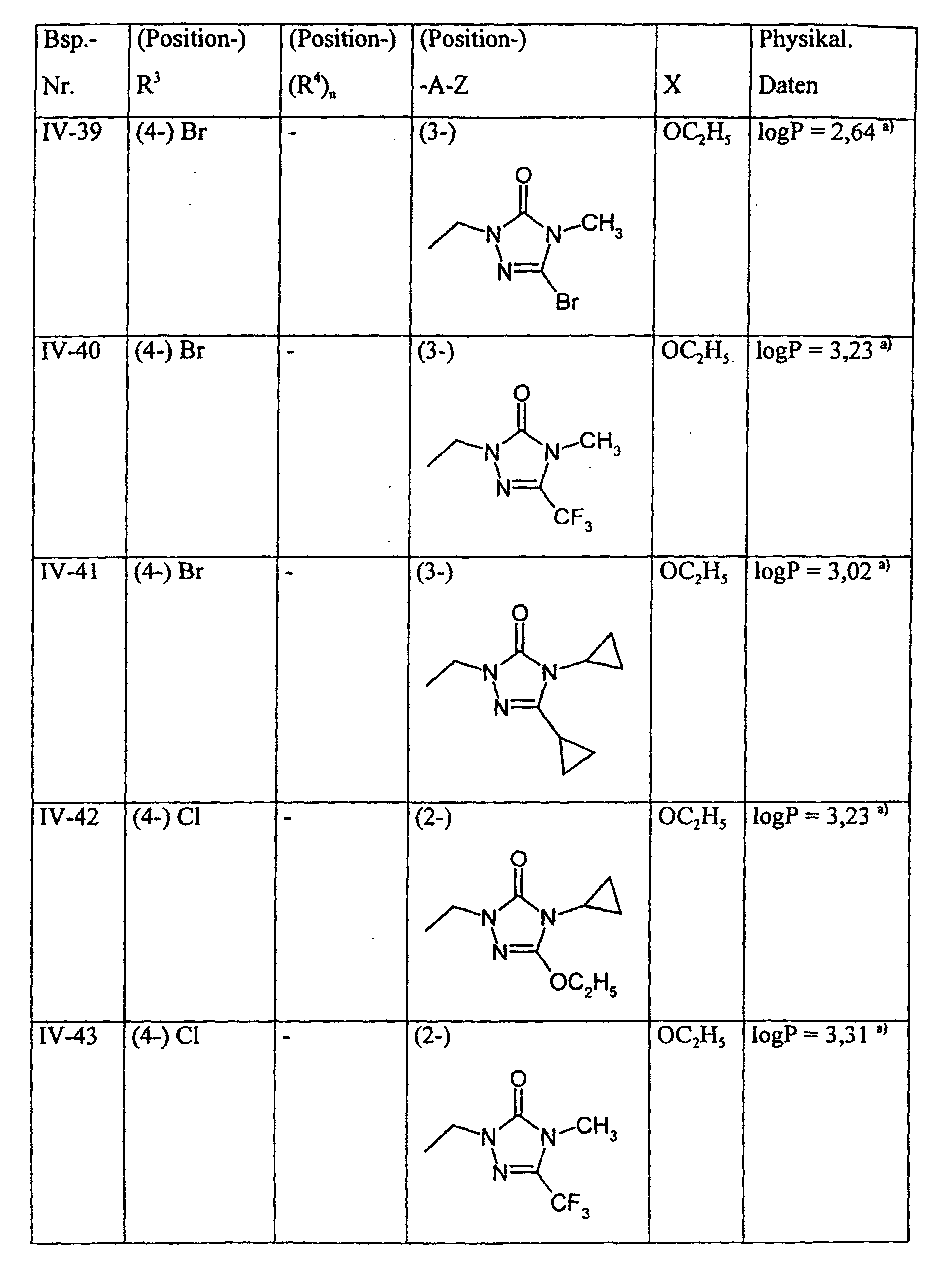

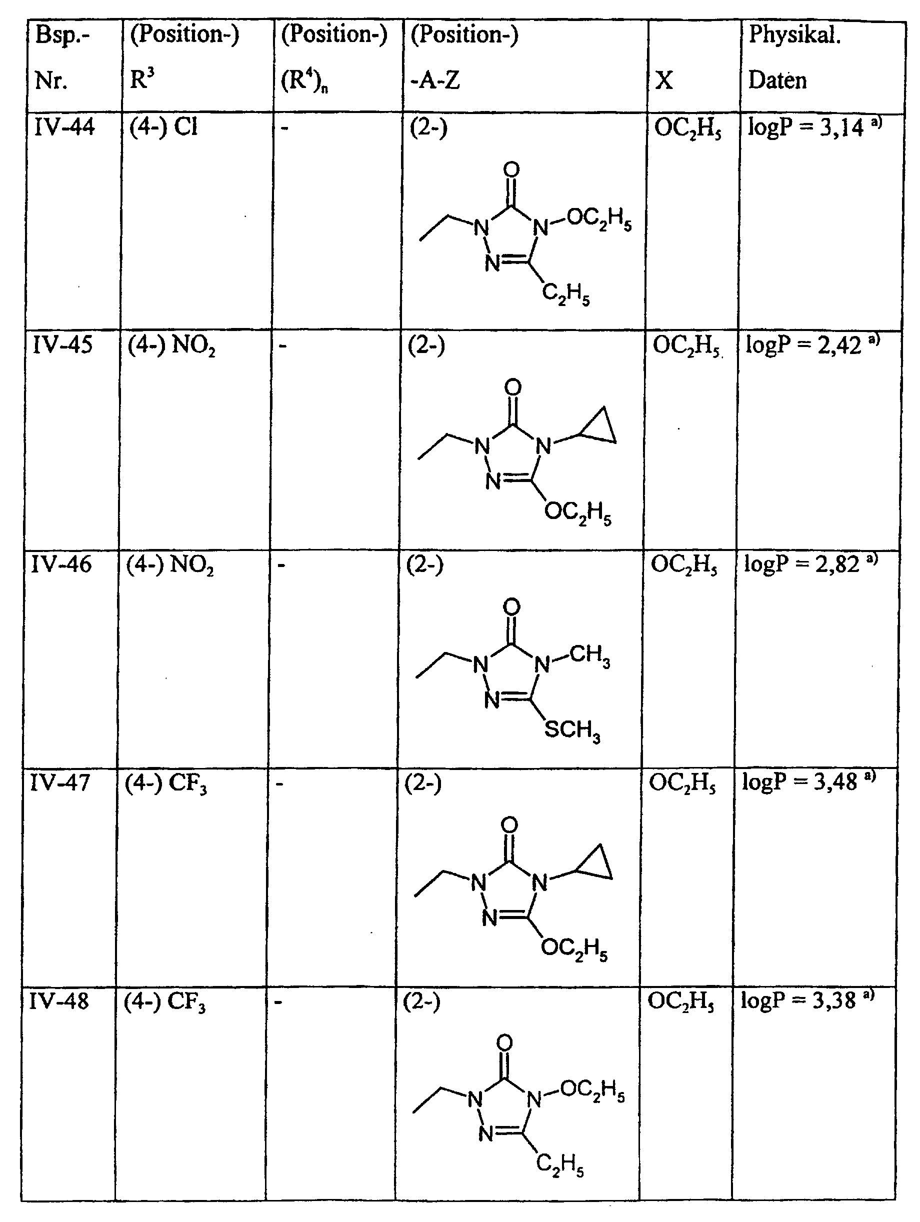

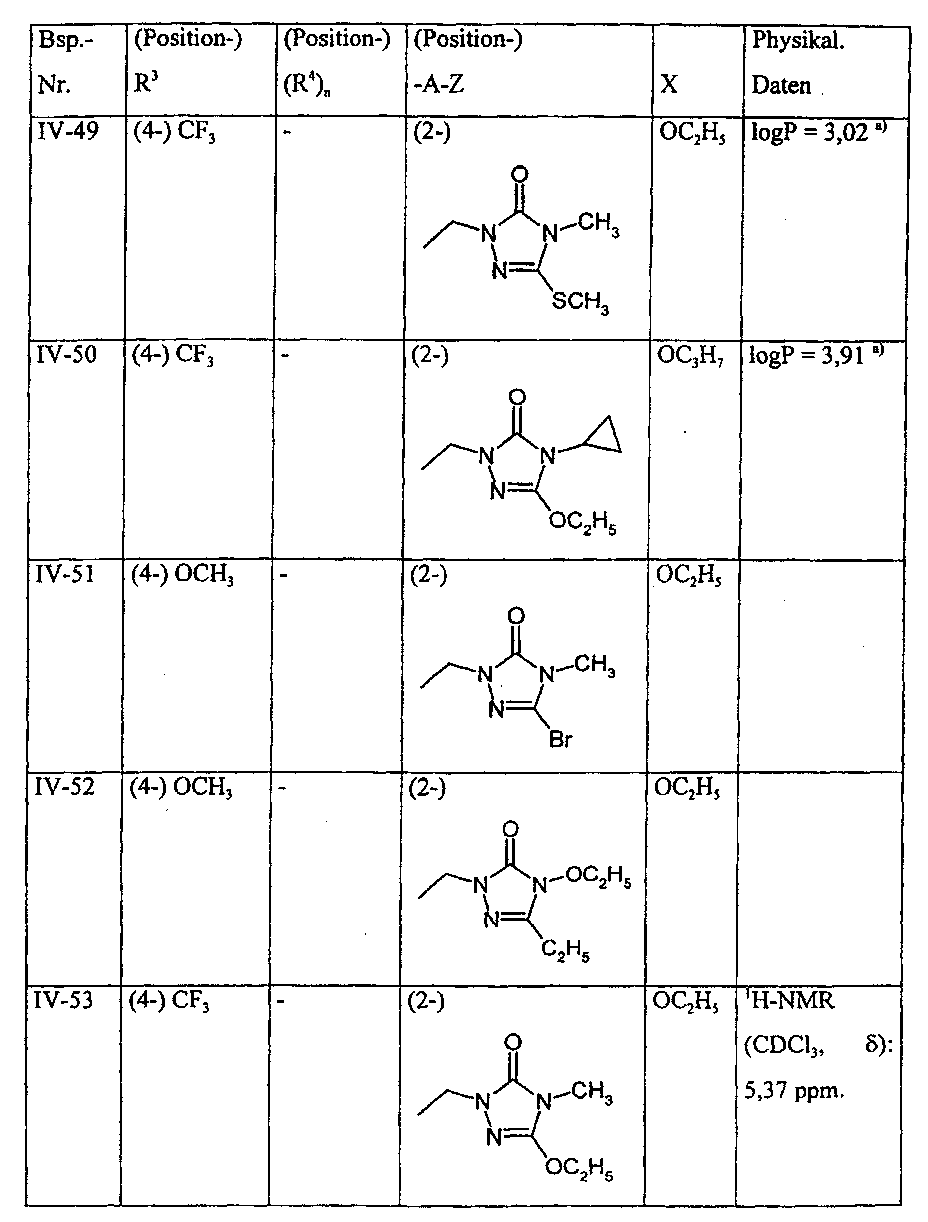

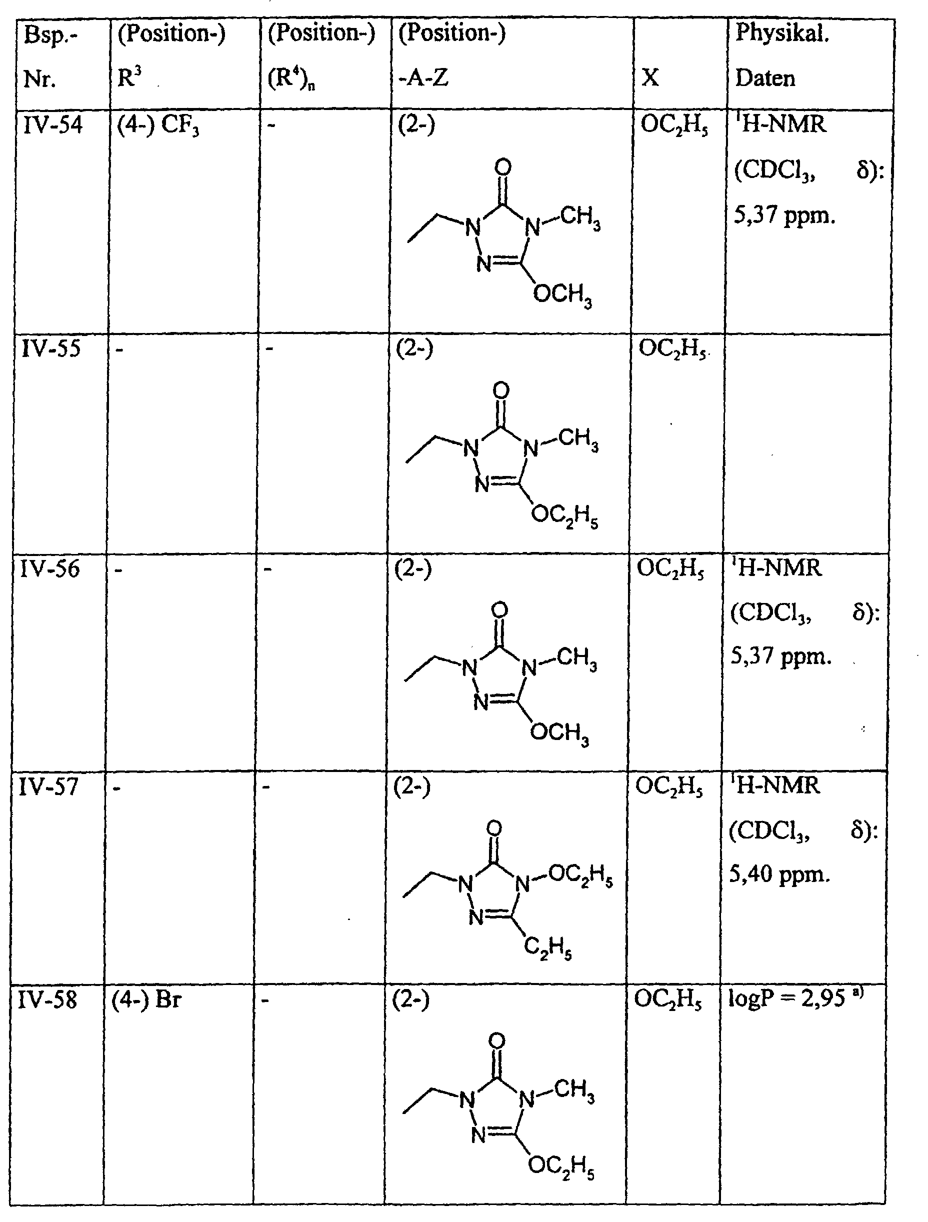

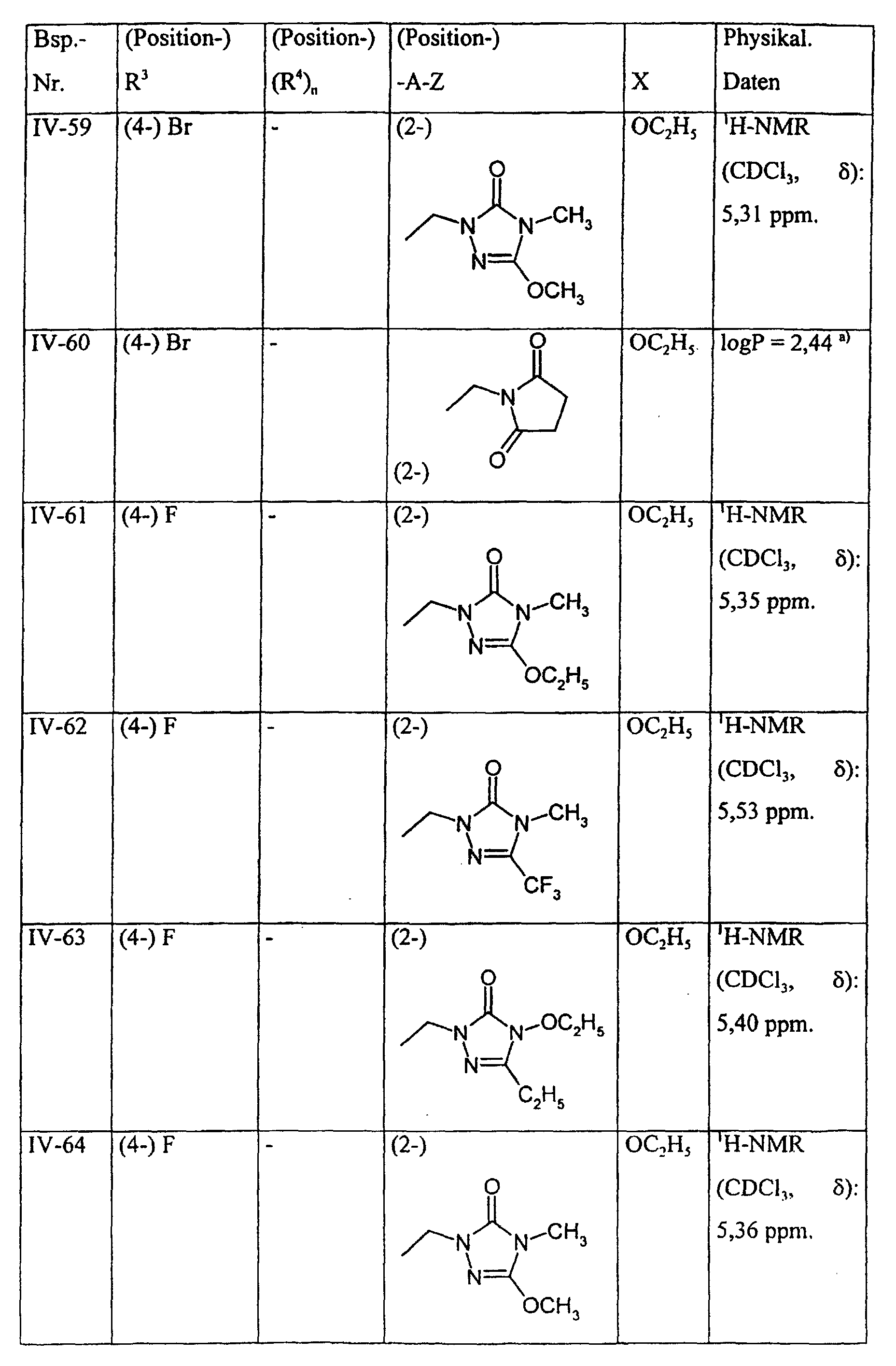

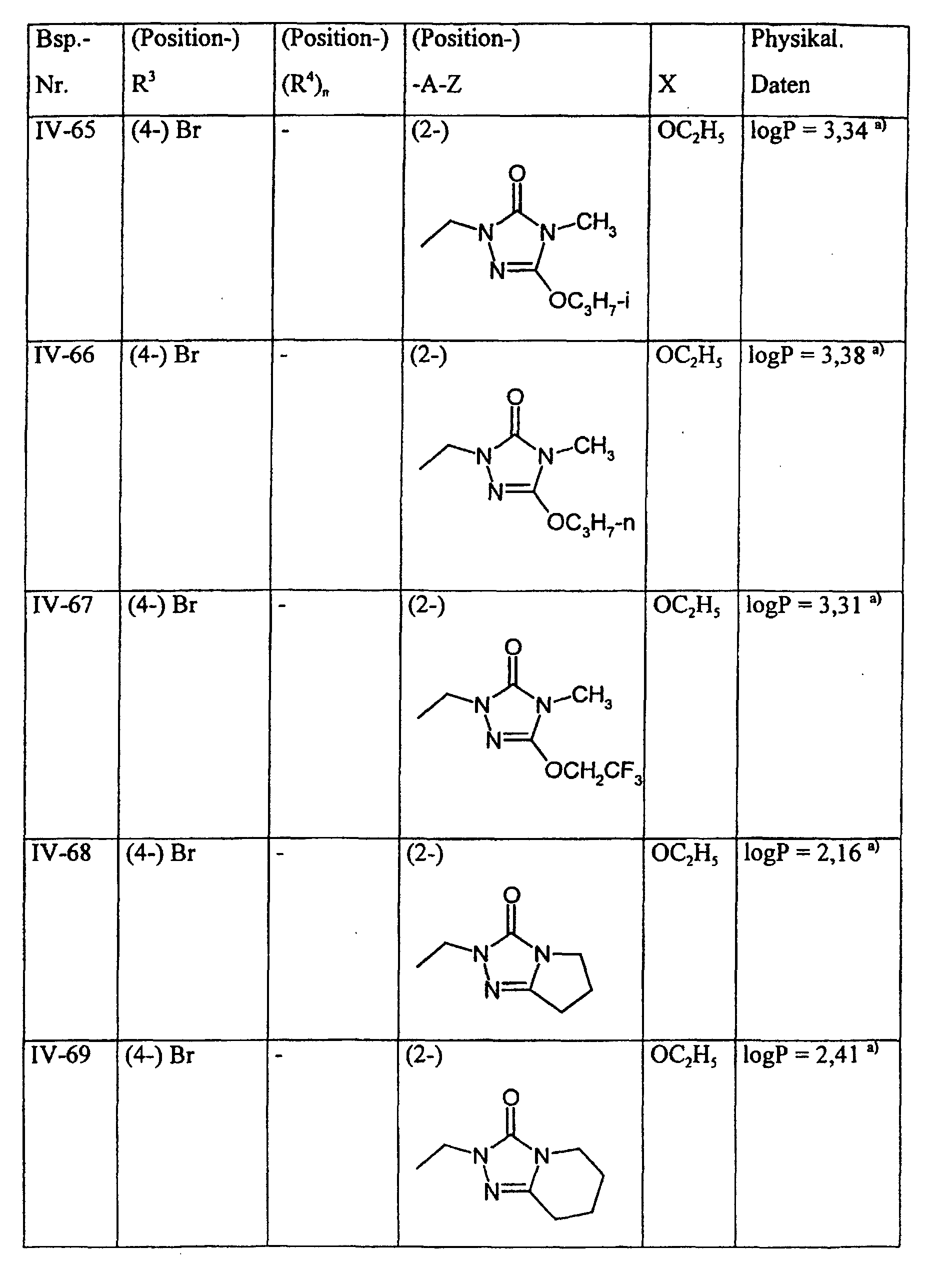

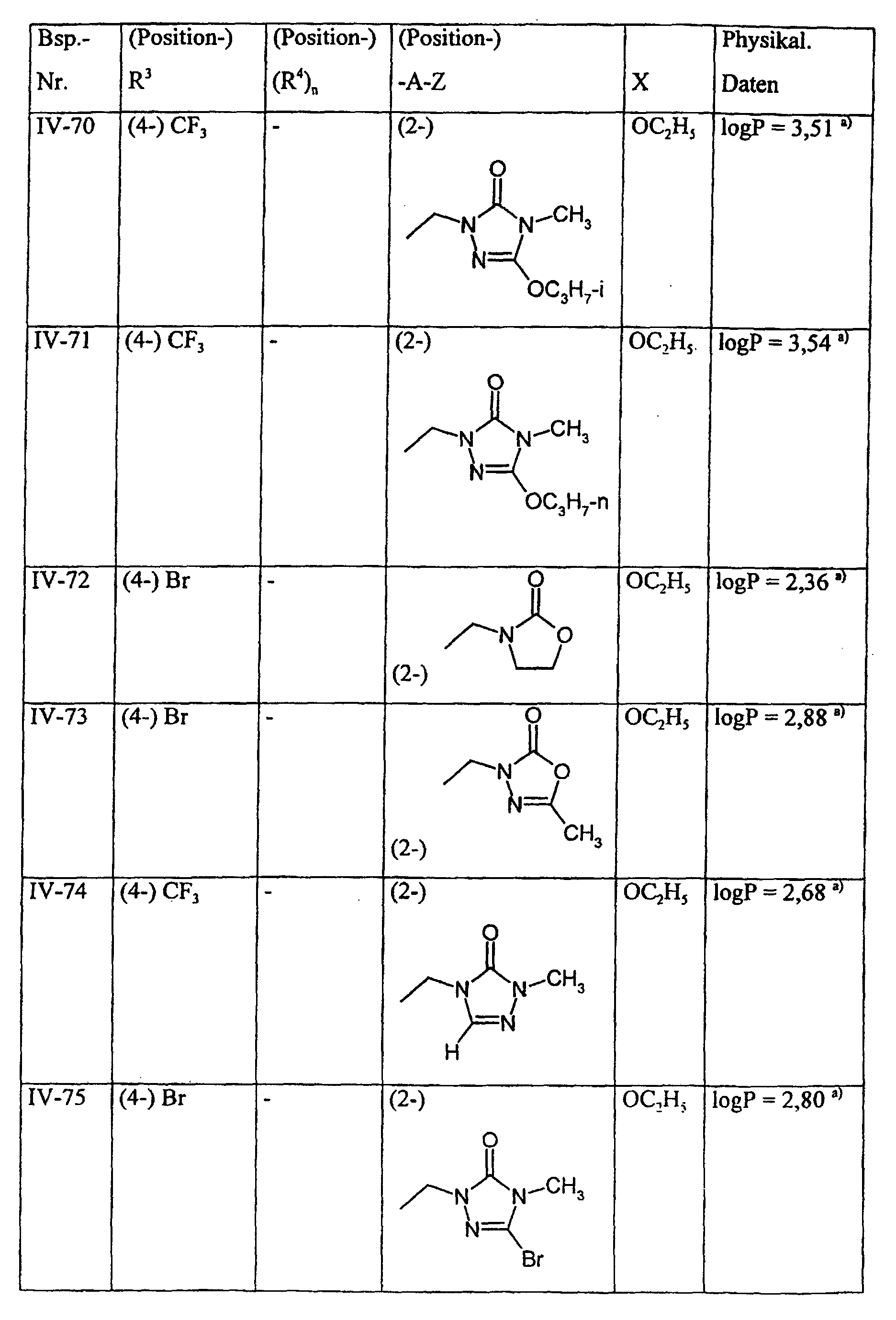

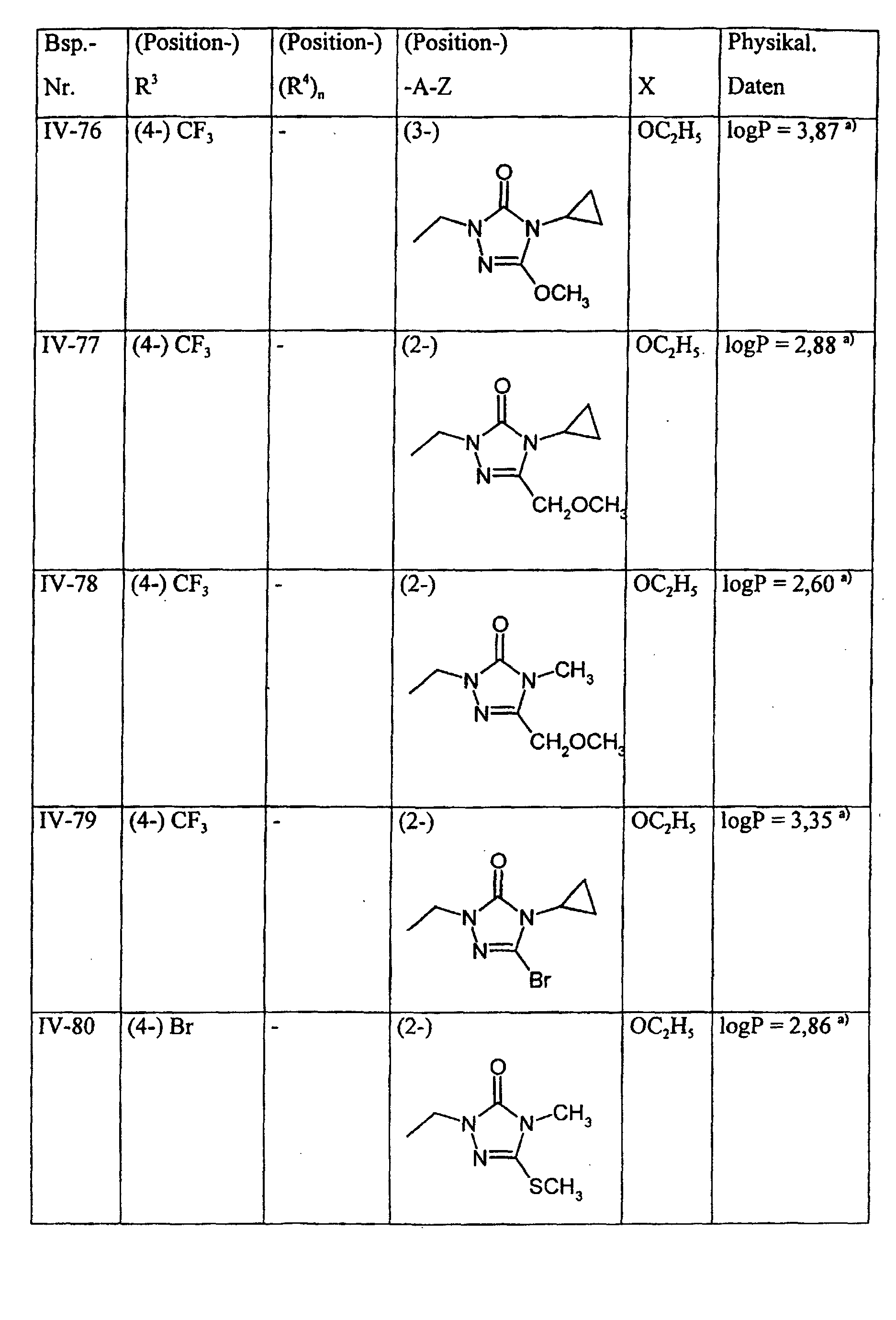

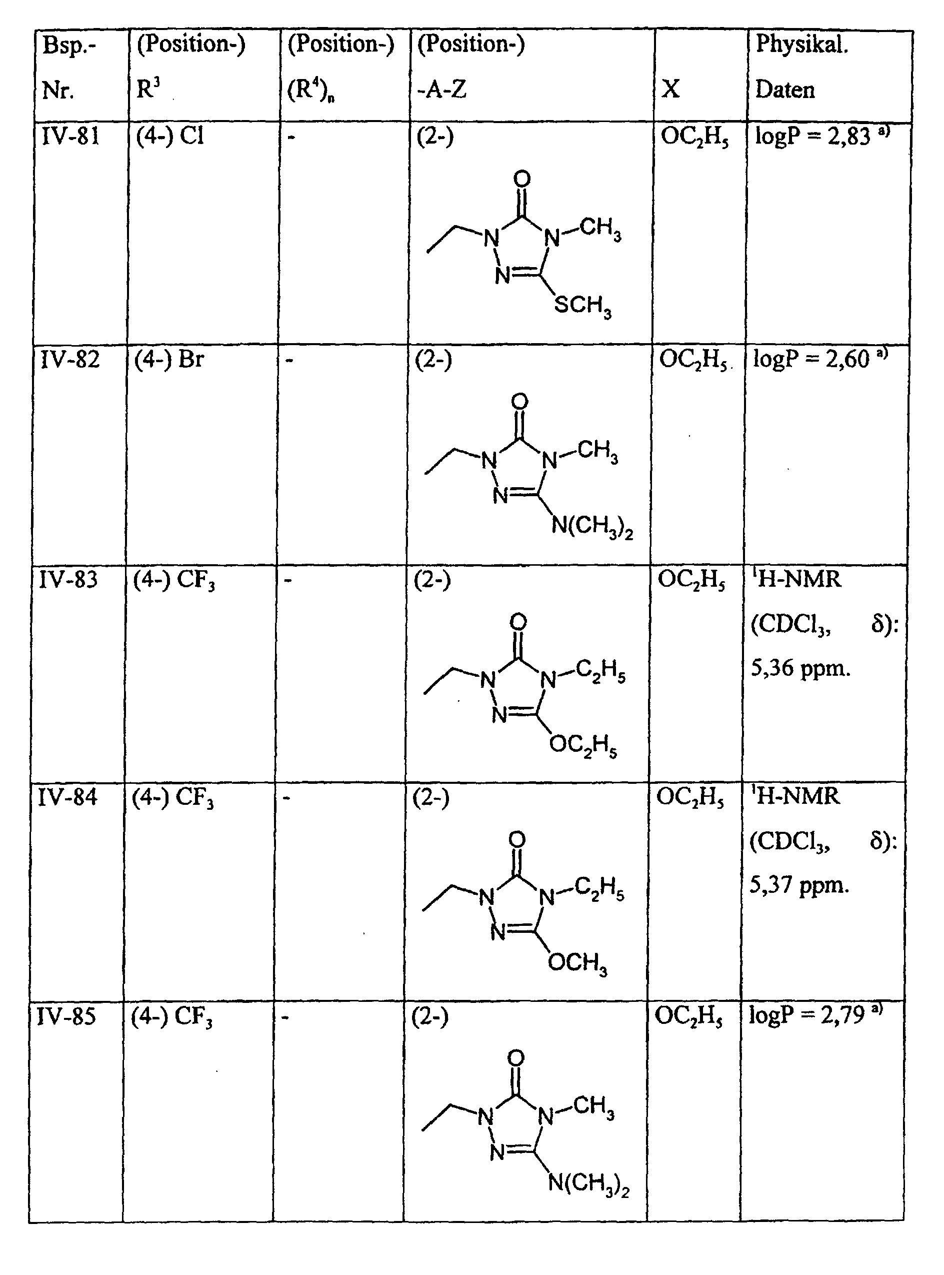

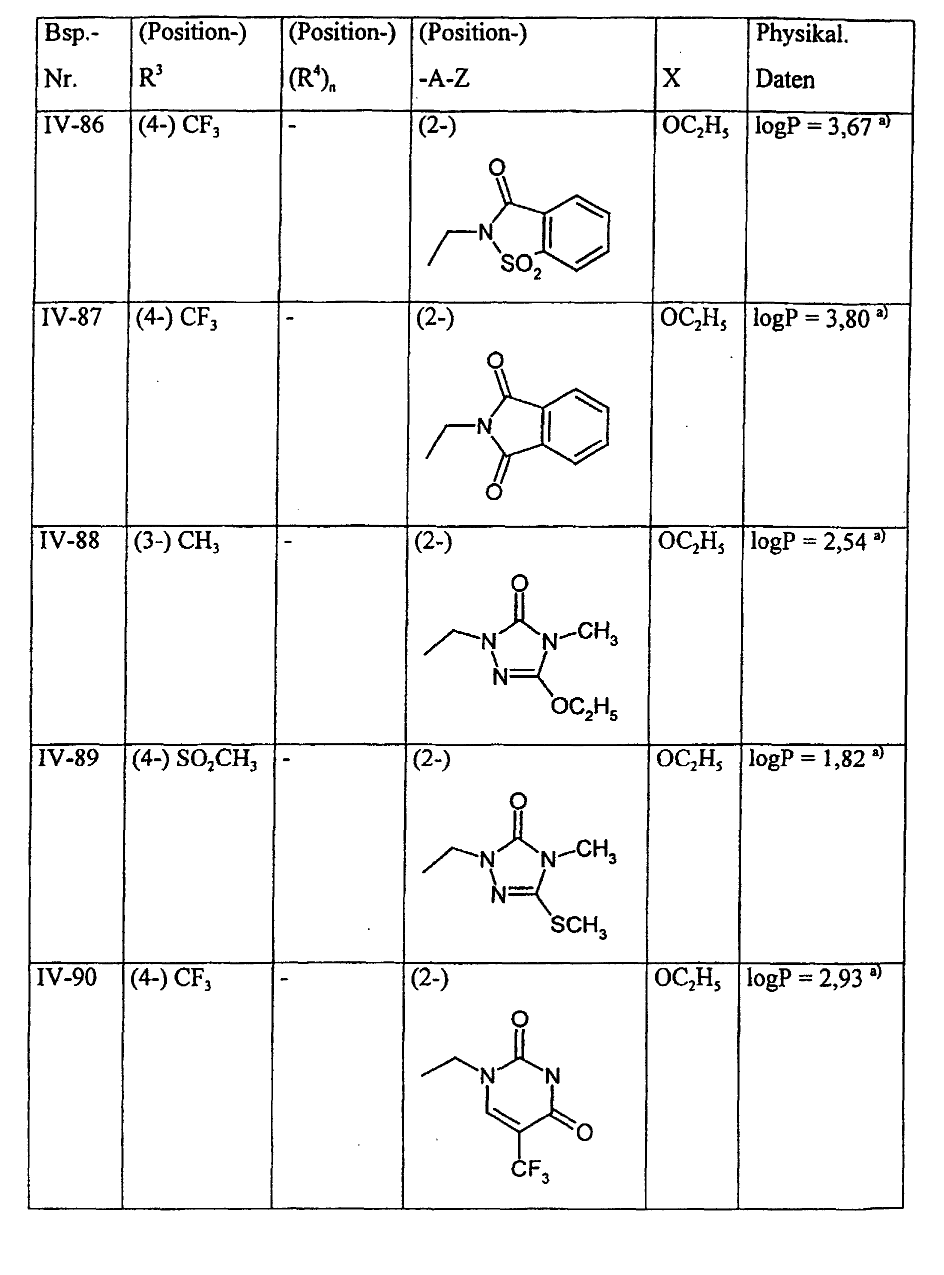

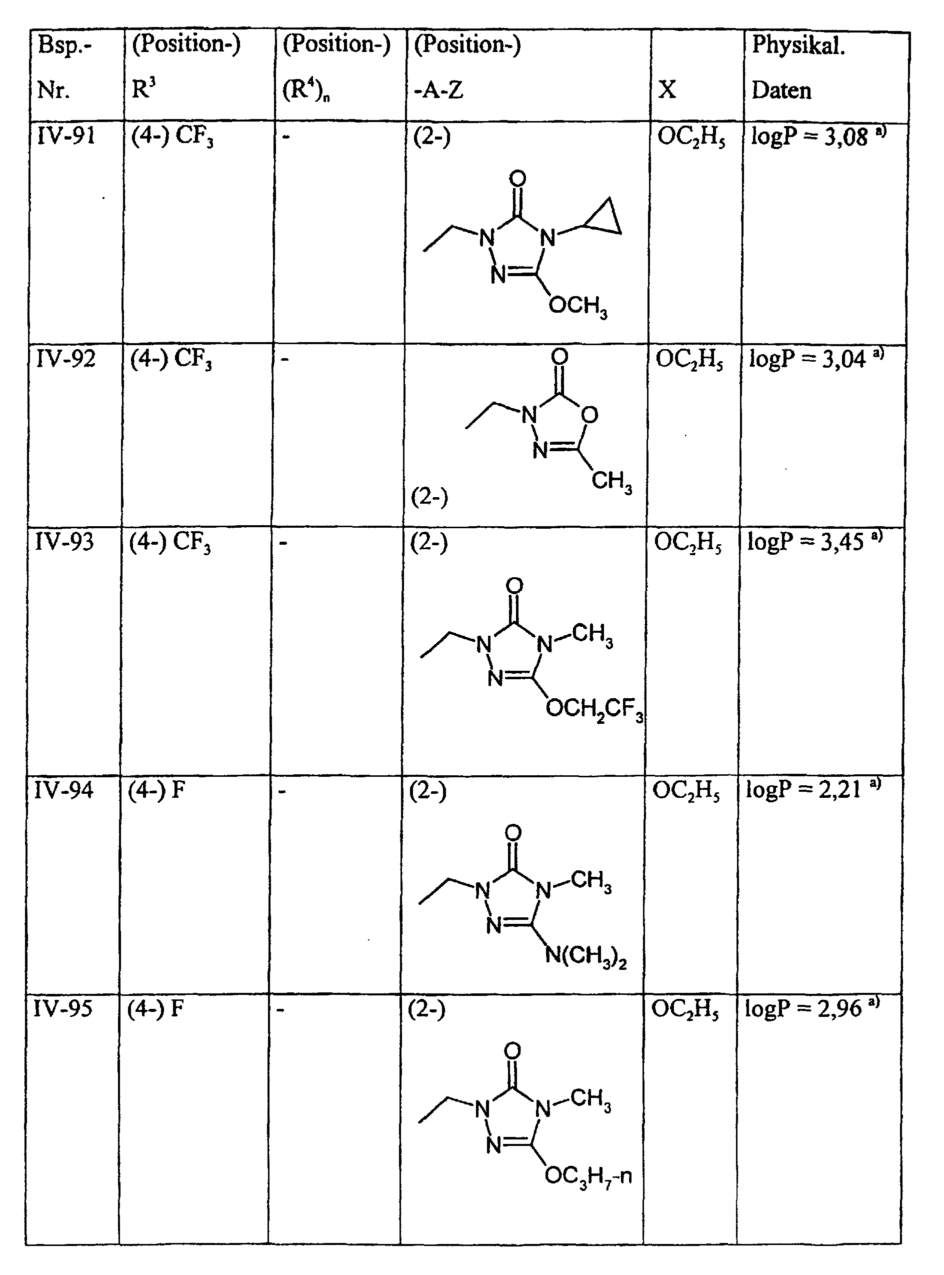

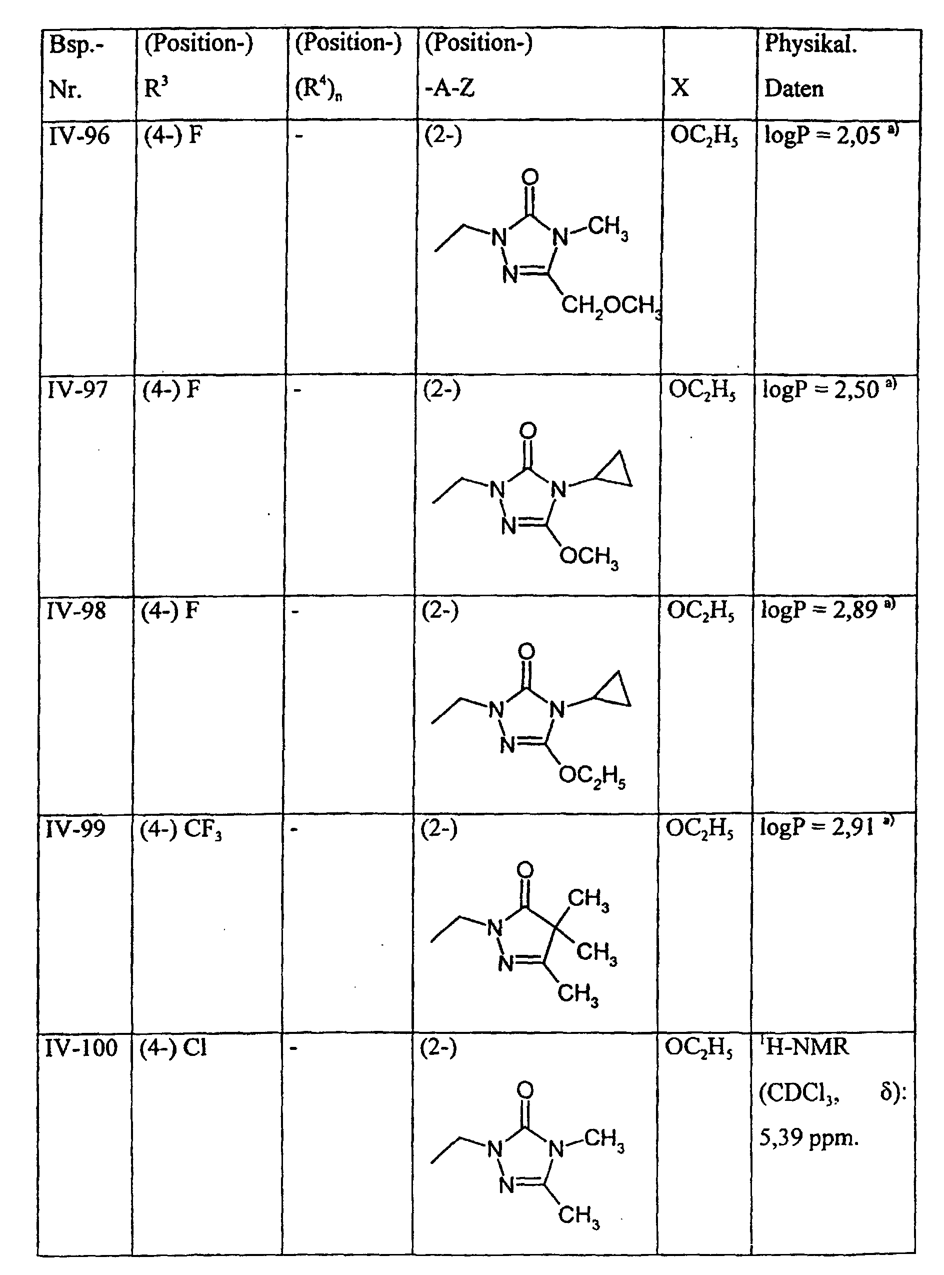

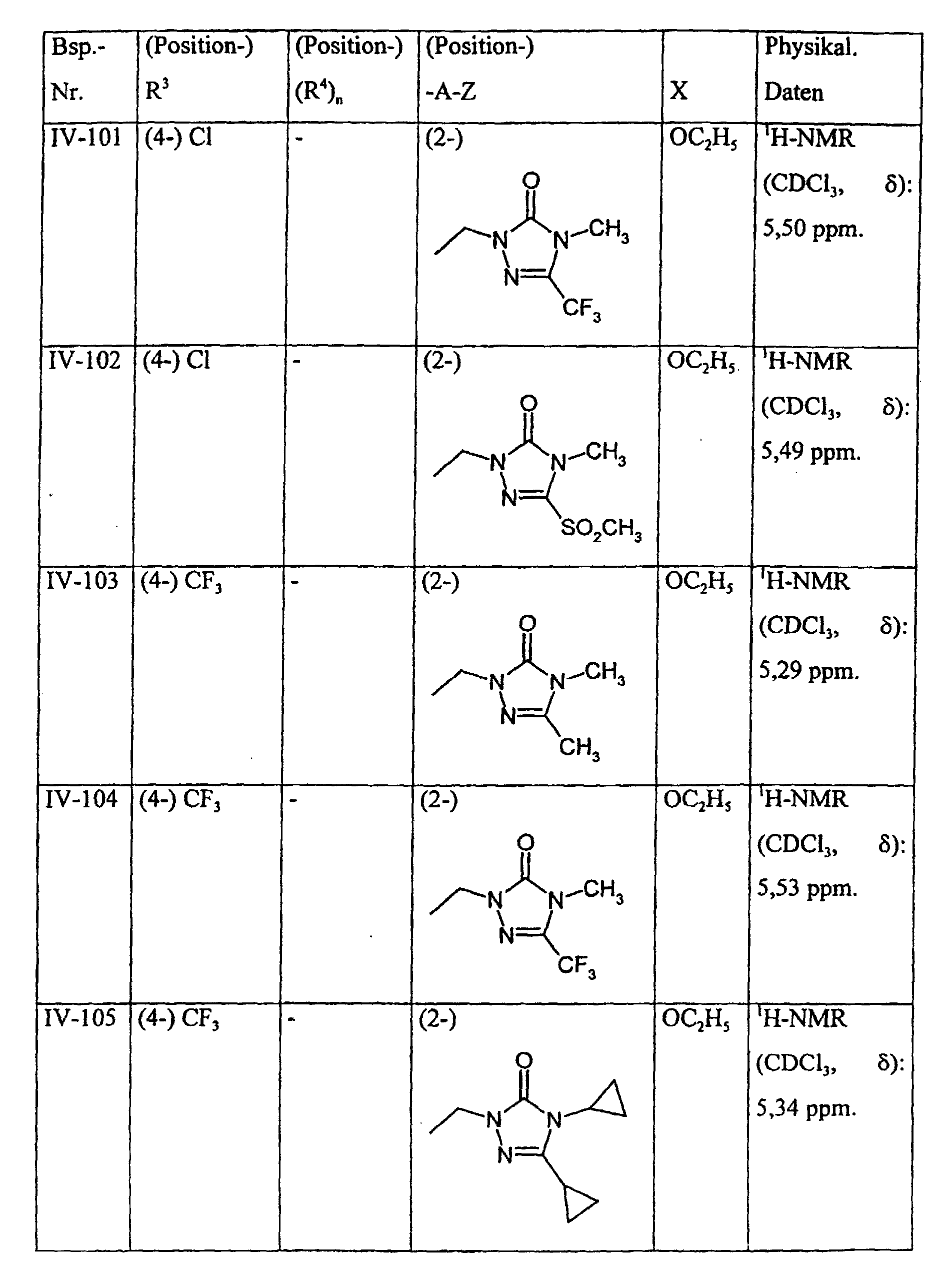

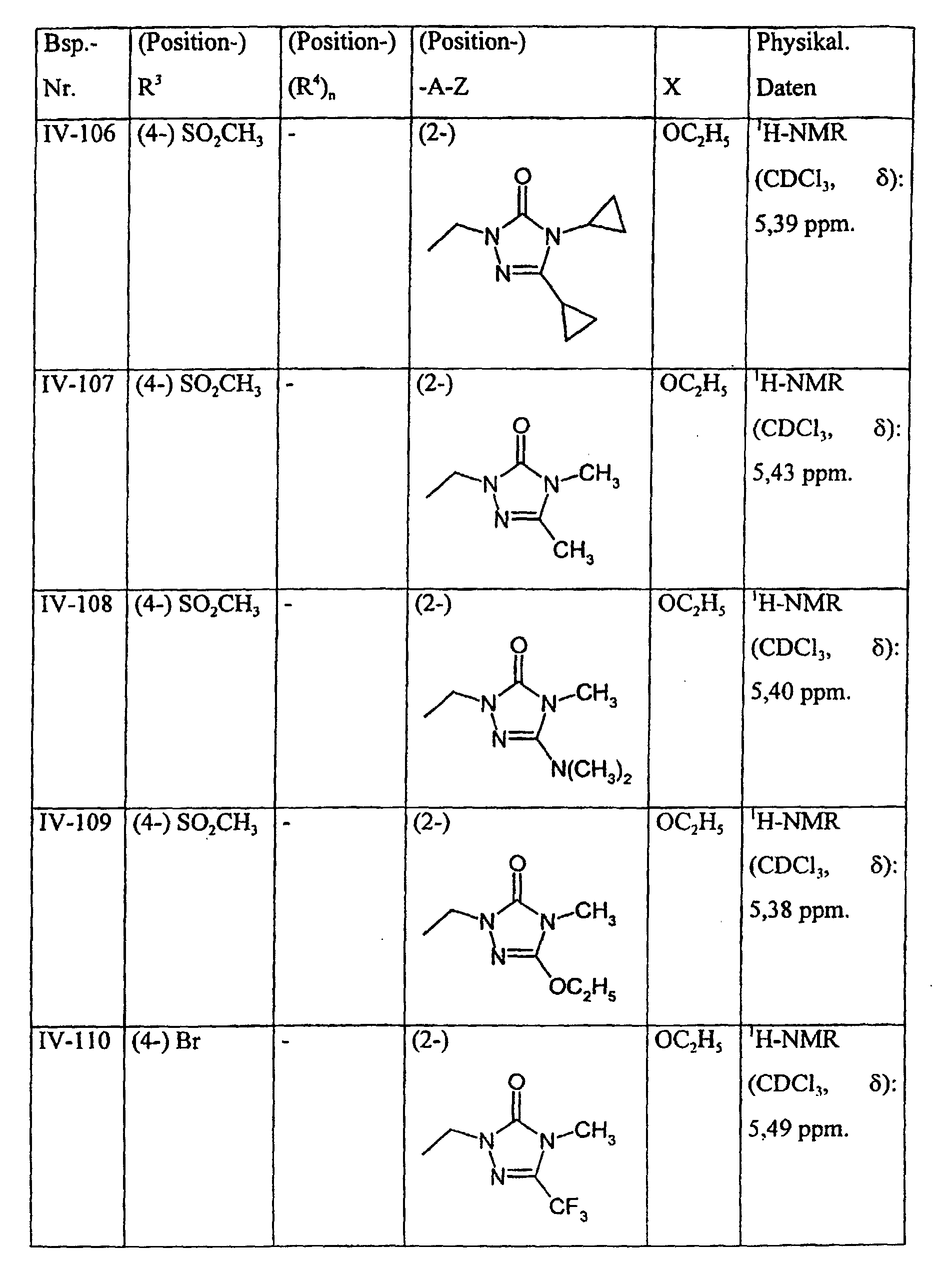

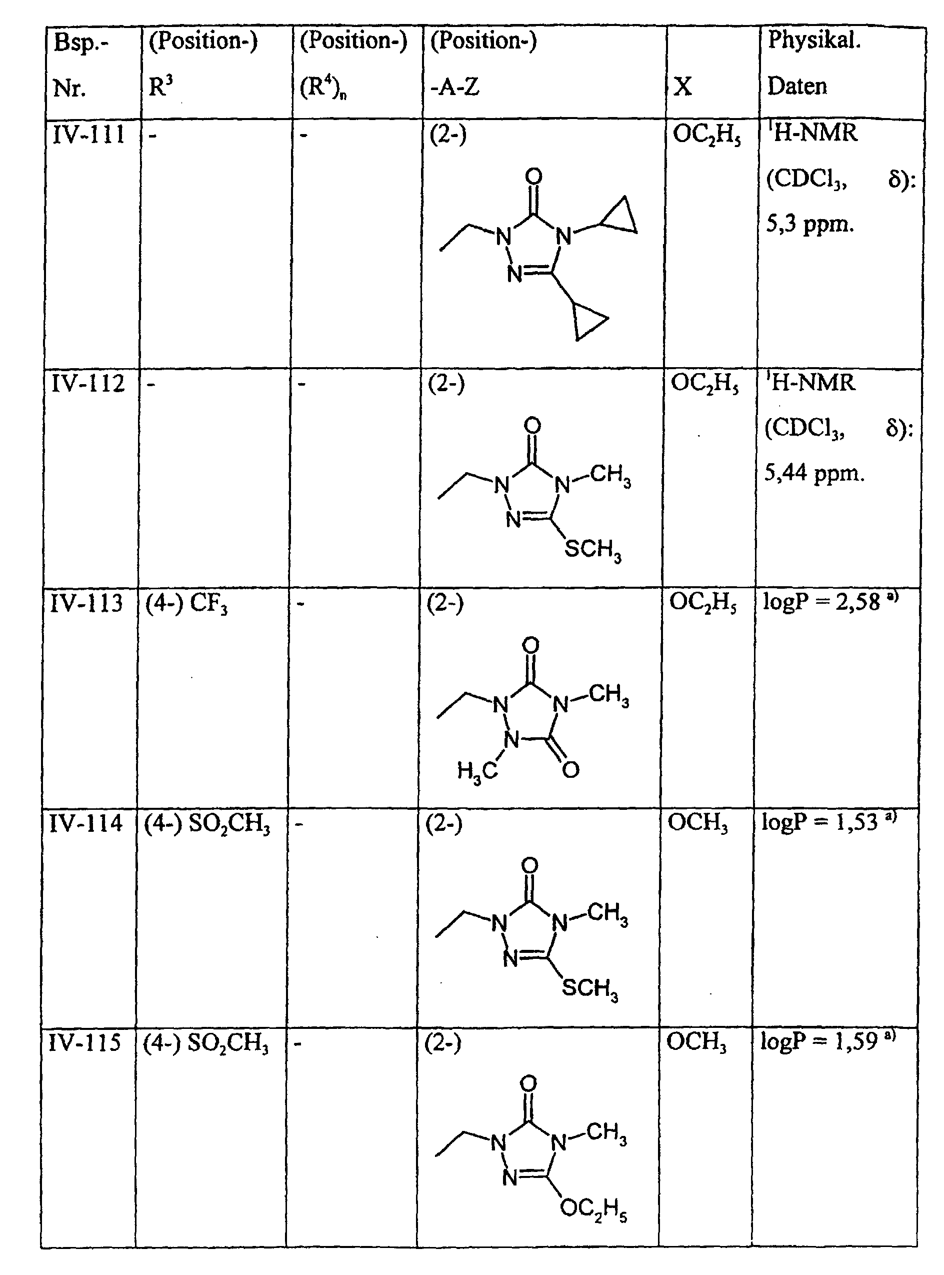

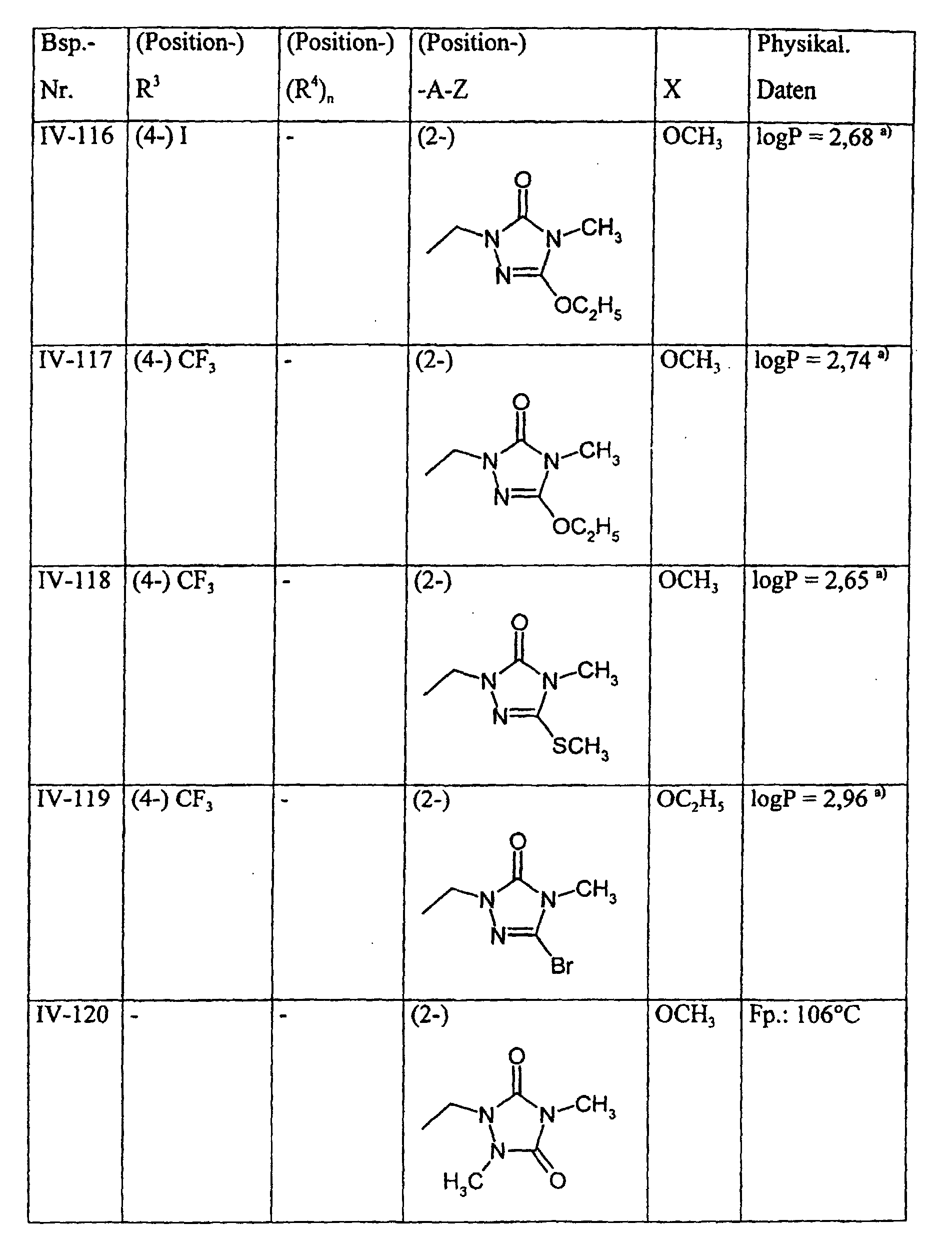

Die Bestimmung der in Tabelle 4 angegebenenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten für die Bestimmung im sauren Bereich: 0,1% wässrige Phosphorsäure, Acetonitril; linearer Gradient von 10% Acetonitril bis 90% Acetonitril - entsprechende Messergebnisse sind in Tabelle 1 mit ^{a)} markiert.
(b) Eluenten für die Bestimmung im neutralen Bereich: 0,01-molare wässrige Phosphatpuffer-Lösung, Acetonitril; linearer Gradient von 10% Acetonitril bis 90% Acetonitril - entsprechende Messergebnisse sind in Tabelle 1 mit ^{b)} markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden so mit der Wirkstoffzubereitung besprüht, dass die jeweils gewünschte Wirkstoffmenge pro Flächeneinheit ausgebracht wird. Die Konzentration der Spritzbrühe wird so gewählt, dass in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle.

Es bedeuten:
- 0 %: = keine Wirkung (wie unbehandelte Kontrolle)
- 100 %: = totale Vernichtung

In diesem Test zeigen beispielsweise die Verbindungen gemäß Herstellungsbeispiel 2 und 3 bei guter Verträglichkeit gegenüber Kulturpflanzen, wie z.B. Mais, starke Wirkung gegen Unkräuter.

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, dass die jeweils gewünschten Wirkstoffmengen pro Flächeneinheit ausgebracht werden. Die Konzentration der Spritzbrühe wird so gewählt, dass in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
- 0 %: = keine Wirkung (wie unbehandelte Kontrolle)
- 100 %: = totale Vernichtung

In diesem Test zeigen beispielsweise die Verbindung gemäß Herstellungsbeispiel 2 und 3 starke Wirkung gegen Unkräuter.

## Patentansprüche

1. Substituierte Benzoylpyrazole der allgemeinen Formel (I), 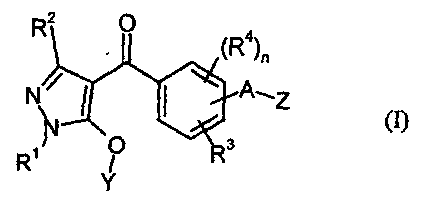 in welcher
n für die Zahlen 0, 1, 2 oder 3 steht,
A für Alkandiyl (Alkylen) steht,
R¹ für jeweils gegebenenfalls substituiertes Alkyl, Alkenyl, Alkinyl oder Cycloalkyl steht,
R² für Wasserstoff, Cyano, Carbamoyl, Thiocarbamoyl, Halogen, oder für jeweils gegebenenfalls substituiertes Alkyl, Alkoxy, Alkylthio, Alkoxycarbonyl oder Cycloalkyl steht,
R³ für Wasserstoff, Nitro, Cyano, Carboxy, Carbamoyl, Thiocarbamoyl, Halogen, oder für jeweils gegebenenfalls substituiertes Alkyl, Alkoxy, Alkylthio, Alkylsulfinyl, Alkylsulfonyl, Alkylamino, Dialkylamino oder Dialkylaminosulfonyl steht,
R⁴ für Nitro, Cyano, Carboxy, Carbamoyl, Thiocarbamoyl, Halogen, oder für jeweils gegebenenfalls substituiertes Alkyl, Alkoxy, Alkylthio, Alkylsulfinyl, Alkylsulfonyl, Alkylamino, Dialkylamino oder Dialkylaminosulfonyl steht,
Y für Wasserstoff oder für jeweils gegebenenfalls substituiertes Alkyl, Alkylcarbonyl, Alkoxycarbonyl, Alkylsulfonyl, Alkylaminocarbonyl, Dialkylaminocarbonyl, Alkenyl, Alkenylcarbonyl, Alkenylsulfonyl, Alkinyl, Alkinylcarbonyl, Cycloalkyl, Cycloalkylcarbonyl, Cycloalkylalkyl, Phenylcarbonyl, Phenylsulfonyl, Phenylalkyl oder Phenylcarbonylalkyl steht, und
Z für eine der nachstehenden heterocyclischen Gruppierungen steht 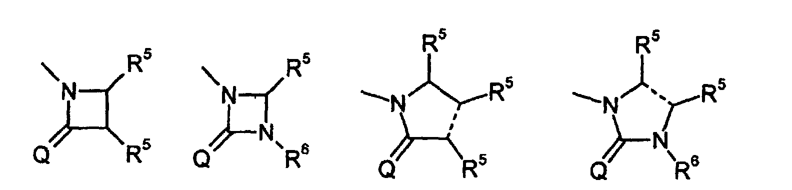 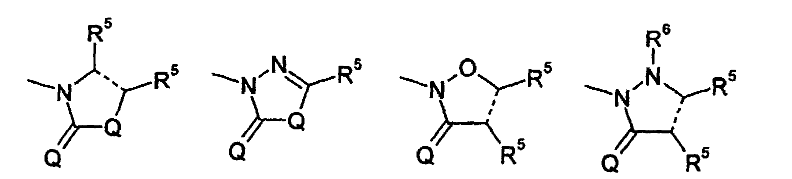 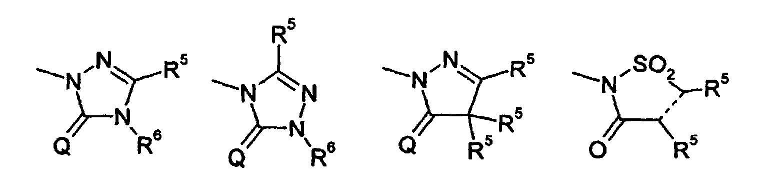 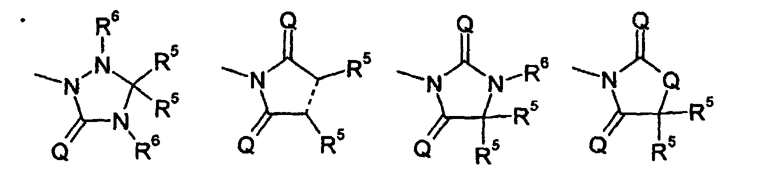 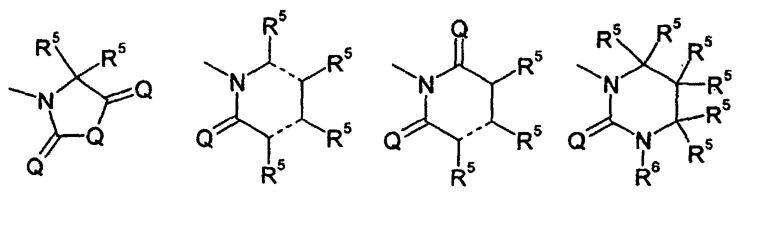 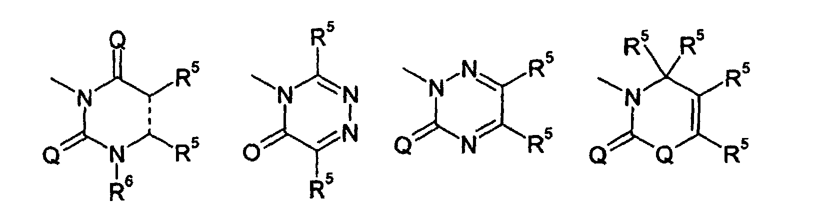 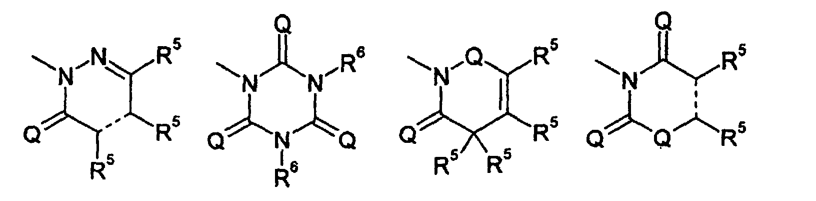 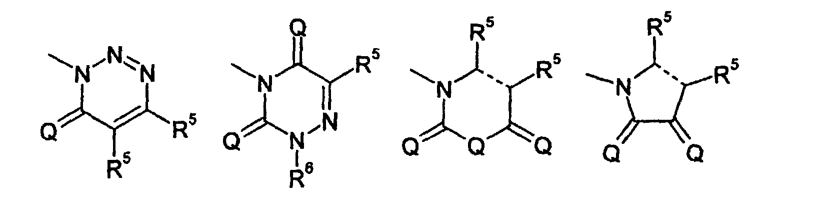 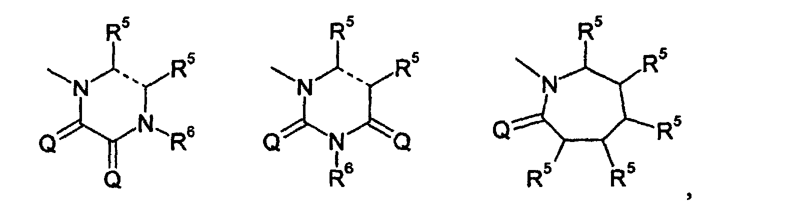 worin jeweils die gestrichelt gezeichnete Bindung eine Einfachbindung oder eine Doppelbindung ist,
Q für Sauerstoff oder Schwefel steht,
R⁵ für Wasserstoff, Hydroxy, Mercapto, Cyano, Halogen, für jeweils gegebenenfalls durch Cyano, Halogen, C₁-C₄-Alkoxy, C₁-C₄-Alkylthio, C₁-C₄-Alkylsulfinyl oder C₁-C₄-Alkylsulfonyl substituiertes Alkyl, Alkylcarbonyl, Alkoxy, Alkoxycarbonyl, Alkylthio, Alkylsulfinyl oder Alkylsulfonyl mit jeweils bis zu 6 Kohlenstoffatomen in den Alkylgruppen, für Propadienylthio, für jeweils gegebenenfalls durch Halogen substituiertes Alkylamino oder Dialkylamino mit jeweils bis zu 6 Kohlenstoffätomen in den Alkylgruppen, für jeweils gegebenenfalls durch Halogen substituiertes Alkenyl, Alkinyl, Alkenyloxy, Alkenylthio oder Alkenylamino mit jeweils bis zu 6 Kohlenstoffatomen in den Alkenyl- bzw. Alkinylgruppen, für jeweils gegebenenfalls durch Halogen substituiertes Cycloalkyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in den Cycloalkylgruppen und gegebenenfalls bis zu 4 Kohlenstoffatomen im Alkylteil, für jeweils gegebenenfalls durch Halogen, C₁-C₄-Alkyl oder C₁-C₄-Alkoxy substituiertes Phenyl, Phenyloxy, Phenylthio, Phenylamino, Benzyl, Benzyloxy, Benzylthio oder Benzylamino steht, für Pyrrolidino, Piperidino oder Morpholino steht, oder - für den Fall, daß zwei benachbarte Reste R⁵ und R⁵ sich an einer Doppelbindung befinden - zusammen mit dem benachbarten Rest R⁵ auch für eine Benzogruppierung steht, und
R⁶ für Wasserstoff, Hydroxy, Amino, Alkylidenamino mit bis zu 4 Kohlenstoffatomen, für jeweils gegebenenfalls durch Halogen oder C₁-C₄-Alkoxy substituiertes Alkyl, Alkoxy, Alkylamino, Dialkylamino oder Alkanoylamino mit jeweils bis zu 6 Kohlenstoffatomen in den Alkylgruppen, für jeweils gegebenenfalls durch Halogen substituiertes Alkenyl, Alkinyl oder Alkenyloxy mit jeweils bis zu 6 Kohlenstoffatomen in den Alkenyl- bzw. Alkinylgruppen, für jeweils gegebenenfalls durch Halogen substituiertes Cycloalkyl, Cycloalkylalkyl oder Cycloalkylamino mit jeweils 3 bis 6 Kohlenstoffatomen in den Cycloalkylgruppen und gegebenenfalls bis zu 3 Kohlenstoffatomen im Alkylteil, oder für jeweils gegebenenfalls durch Halogen, C₁-C₄-Alkyl oder C₁-C₄-Alkoxy substituiertes Phenyl oder Benzyl steht, oder zusammen mit einem benachbarten Rest R⁵ oder R⁶ für gegebenenfalls durch Halogen oder C₁-C₄-Alkyl substituiertes Alkandiyl mit 3 bis 5 Kohlenstoffatomen steht,
wobei die einzelnen Reste R⁵ und R⁶ - soweit mehrere davon an gleiche heterocyclische Gruppierungen gebunden sind - gleiche oder verschiedene Bedeutungen im Rahmen der obigen Definition haben können,
einschließlich aller möglichen tautomeren Formen und der möglichen Salze.

2. Verbindungen gemäß Anspruch 1, **dadurch gekennzeichnet, daß**
n für die Zahlen 0, 1 oder 2 steht,
A für Alkandiyl (Alkylen) mit 1 bis 4 Kohlenstoffatomen steht,
R¹ für gegebenenfalls durch Cyano, Carboxy, Carbamoyl, Halogen, C₁-C₄-Alkoxy, C₁-C₄-Alkyl-carbonyl, C₁-C₄-Alkoxy-carbonyl, C₁-C₄-Alkylthio, C₁-C₄-Alkylsulfinyl oder C₁-C₄-Alkylsulfonyl substituiertes Alkyl mit 1 bis 6 Kohlenstoffatomen, für jeweils gegebenenfalls durch Cyano, Carboxy, Carbamoyl, Halogen oder C₁-C₄-Alkoxycarbonyl substituiertes Alkenyl oder Alkinyl mit jeweils 2 bis 6 Kohlenstoffatomen, oder für gegebenenfalls durch Cyano, Carboxy, Carbamoyl, Halogen, C₁-C₄-Alkyl oder C₁-C₄-Alkoxy-carbonyl substituiertes Cycloalkyl mit 3 bis 6 Kohlenstoffatomen steht,
R² für Wasserstoff, Cyano, Carbamoyl, Thiocarbamoyl, Halogen, für jeweils gegebenenfalls durch Cyano, Halogen oder C₁-C₄-Alkoxy substituiertes Alkyl, Alkoxy oder Alkoxycarbonyl mit jeweils bis zu 6 Kohlenstoffatomen, für gegebenenfalls durch Halogen substituiertes Alkylthio mit 1 bis 6 Kohlenstoffatomen, oder für gegebenenfalls durch Cyano, Halogen oder C₁-C₄-Alkyl substituiertes Cycloalkyl mit 3 bis 6 Kohlenstoffatomen steht,
R³ für Wasserstoff, Nitro, Cyano, Carboxy, Carbamoyl, Thiocarbamoyl, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkoxy, Alkylthio, Alkylsulfinyl oder Alkylsulfonyl mit jeweils bis zu 4 Kohlenstoffatomen in den Alkylgruppen, oder für Alkylamino, Dialkylamino oder Dialkylaminosulfonyl mit jeweils bis zu 4 Kohlenstoffatomen in den Alkylgruppen steht,
R⁴ für Nitro, Cyano, Carboxy, Carbamoyl, Thiocarbamoyl, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkoxy, Alkylthio, Alkylsulfinyl oder Alkylsulfonyl mit jeweils bis zu 4 Kohlenstoffatomen in den Alkylgruppen, oder für Alkylamino, Dialkylamino oder Dialkylaminosulfonyl mit jeweils bis zu 4 Kohlenstoffatomen in den Alkylgruppen steht,
Y für Wasserstoff, für jeweils gegebenenfalls durch Cyano, Carboxy, Carbamoyl, Halogen oder C₁-C₄-Alkoxy-carbonyl substituiertes Alkyl, Alkylcarbonyl oder Alkoxycarbonyl mit jeweils bis zu 6 Kohlenstoffatomen, für jeweils gegebenenfalls durch Halogen substituiertes Alkylsulfonyl, Alkylaminocarbonyl oder Dialkylaminocarbonyl mit jeweils bis zu 6 Kohlenstoffatomen in den Alkylgruppen, für jeweils gegebenenfalls durch Cyano, Carboxy, Carbamoyl, Halogen oder C₁-C₄-Alkoxy-carbonyl substituiertes Alkenyl, Alkenylcarbonyl, Alkinyl oder Alkinylcarbonyl mit jeweils 2 bis 6 Kohlenstoffatomen, für gegebenenfalls durch Halogen substituiertes Alkenylsulfonyl mit bis zu 6 Kohlenstoffatomen, für jeweils gegebenenfalls durch Cyano, Halogen oder C₁-C₄-Alkyl substituiertes Cycloalkyl, Cycloalkylcarbonyl oder Cycloalkylalkyl mit jeweils 3 bis 6 Kohlenstoffatomen in den Cycloalkylgruppen und gegebenenfalls 1 bis 3 Kohlenstoffatomen im Alkylteil, oder für jeweils gegebenenfalls durch Nitro, Cyano, Carboxy, Carbamoyl, Halogen, C₁-C₄-Alkyl, C₁-C₄-Halogenalkyl, C₁-C₄-Alkoxy oder C₁-C₄-Halogenalkoxy substituiertes Phenylcarbonyl, Phenylsulfonyl, Phenyl-C₁-C₄-alkyl oder Phenylcarbonyl-C₁-C₄-alkyl steht, und
Z die in Anspruch 1 angegebene Bedeutung hat.

3. Verbindungen gemäß Anspruch 1 oder 2, **dadurch gekennzeichnet, daß**
n für die Zahlen 0 oder 1 steht,
A Methylen, Ethyliden (Ethan-1,1-diyl) oder Dimethylen (Ethan-1,2-diyl) steht,
R¹ für jeweils gegebenenfalls durch Fluor, Chlor, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, oder für jeweils gegebenenfalls durch Cyano, Fluor, Chlor, Brom, Methyl oder Ethyl substituiertes Cyclopropyl, Cyclobutyl, Cyclopentyl oder Cyclohexyl steht,
R² für Wasserstoff, Cyano, Carbamoyl, Thiocarbamoyl, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, Methoxycarbonyl, Ethoxycarbonyl, n- oder i-Propoxycarbonyl, für jeweils gegebenenfalls durch Fluor und/oder Chlor substituiertes Methylthio, Ethylthio, n- oder i-Propylthio, oder für jeweils gegebenenfalls durch Cyano, Fluor, Chlor, Brom, Methyl oder Ethyl substituiertes Cyclopropyl, Cyclobutyl, Cyclopentyl oder Cyclohexyl steht,
R³ für Wasserstoff, Nitro, Cyano, Carboxy, Carbamoyl, Thiocarbamoyl, Fluor, Chlor, Brom, Iod, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl steht,
R⁴ für Nitro, Cyano, Carboxy, Carbamoyl, Thiocarbamoyl, Fluor, Chlor, Brom, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfmyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl steht,
R⁵ für Wasserstoff, Hydroxy, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Fluorethyl, Chlorethyl, Difluorethyl, Dichlorethyl, Fluor-n-propyl, Fluor-i-propyl, Chlor-n-propyl, Chlor-i-propyl, Methoxymethyl, Ethoxymethyl, Methoxyethyl, Ethoxyethyl, Methoxy, Ethoxy, n- oder i-Propoxy, n-, i-, s- oder t-Butoxy, Fluorethoxy, Chlorethoxy, Difluorethoxy, Dichlorethoxy, Trifluorethoxy, Trichlorethoxy, Chlorfluorethoxy, Chlordifluorethoxy, Fluordichlorethoxy, Methylthio, Ethylthio, n- oder i-Propylthio, Fluorethylthio, Chlorethylthio, Difluorethylthio, Dichlorethylthio, Chlorfluorethylthio, Chlordifluorethylthio, Fluordichlorethylthio, Methylsulfinyl, Ethylsulfinyl, n- oder i-Propylsulfinyl, Methylsulfonyl, Ethylsulfonyl, n- oder i-Propylsulfonyl, Dimethylamino, Propenylthio, Butenylthio, Propinylthio, Butinylthio, Cyclopropyl, Cyclopropylmethyl, Cyclopropylmethoxy, Phenyl oder Phenoxy steht,
R⁶ für Amino, Methyl, Ethyl, n- oder i-Propyl, n-, i-, s- oder t-Butyl, Methoxy, Ethoxy, Methylamino, Dimethylamino, Cyclopropyl oder Cyclopropylmethyl steht, oder zusammen mit R⁵ für Propan-1,3-diyl (Trimethylen), Butan-1,4-diyl (Tetramethylen) oder Pentan-1,5-diyl (Pentamethylen) steht, und
Y für Wasserstoff, für jeweils gegebenenfalls durch Cyano, Fluor, Chlor, Methoxy oder Ethoxy substituiertes Methyl, Ethyl, n- oder i-Propyl, Acetyl, Propionyl, n- oder i-Butyroyl, Methoxycarbonyl oder Ethoxycarbonyl, für jeweils gegebenenfalls durch Fluor, Chlor und/oder Brom substituiertes Methylsulfonyl, Ethylsulfonyl, n- oder i-Propylsulfonyl, n-, i-, s- oder t-Butylsulfonyl, Methylaminocarbonyl, Ethylaminocarbonyl, n- oder i-Propylaminocarbonyl, Dimethylaminocarbonyl oder Diethylaminocarbonyl, für jeweils gegebenenfalls durch Fluor, Chlor oder Brom substituiertes Propenyl, Butenyl, Propenylcarbonyl, Butenylcarbonyl, Propenylsulfonyl, Butenylsulfonyl, Propinyl, Butinyl, Propinylcarbonyl oder Butinylcarbonyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylcarbonyl, Cyclobutylcarbonyl, Cyclopentylcarbonyl, Cyclohexylcarbonyl, Cyclopropylmethyl, Cyclobutylmethyl, Cyclopentylmethyl oder Cyclohexylmethyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder iPropoxy, Difluormethoxy oder Trifluormethoxy substituiertes Phenylcarbonyl, Phenylsulfonyl, Benzyl oder Phenylcarbonylmethyl steht.

4. Verbindungen gemäß einem der Ansprüche 1 bis 3, **dadurch gekennzeichnet, daß**
Z für die folgende Gruppierung steht

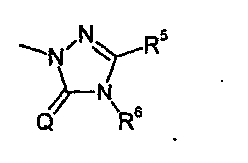

5. Verbindungen gemäß einem der Ansprüche 1 bis 4, **dadurch gekennzeichnet, daß**
Q für Sauerstoff steht.

6. Verbindungen gemäß einem der Ansprüche 1 bis 5, **dadurch gekennzeichnet, daß** n für 0 steht.

7. Verfahren zum Herstellen von Verbindungen gemäß einem der Ansprüche 1 bis 6, **dadurch gekennzeichnet, daß** man
(a) Pyrazole der allgemeinen Formel (II) 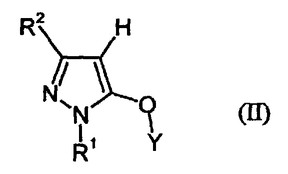 in welcher
R¹, R² und Y die in einem der Ansprüche 1 bis 3 angegebene Bedeutung haben,
mit substituierten Benzoesäuren der allgemeinen Formel (III), 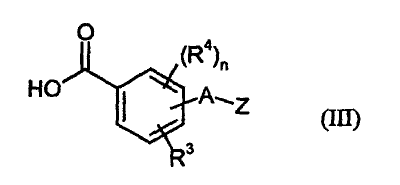 in welcher
n, A, R³, R⁴ und Z die in einem der Ansprüche 1 bis 6 angegebene Bedeutung haben,
in Gegenwart eines Dehydratisierungsmittels, gegebenenfalls in Gegenwart eines oder mehrerer Reaktionshilfsmittel und gegebenenfalls in Gegenwart eines Verdünnungsmittels umsetzt,
oder daß man
(b) Pyrazole der allgemeinen Formel (II) 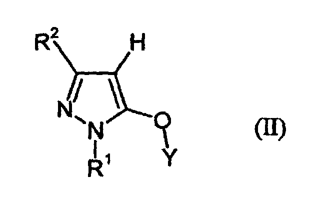 in welcher
R¹, R² und Y die in einem der Ansprüche 1 bis 3 angegebene Bedeutung haben,
mit substituierten Benzoesäurederivaten der allgemeinen Formel (IV) 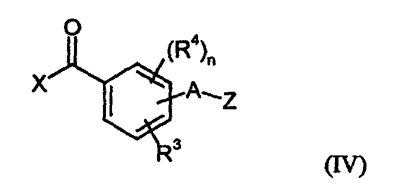 in welcher
n, A, R³, R⁴ und Z die in einem der Ansprüche 1 bis 6 angegebene Bedeutung haben, und
X für Cyano, Halogen oder Alkoxy steht,
- oder mit entsprechenden Carbonsäureanhydriden -
gegebenenfalls in Gegenwart eines oder mehrerer Reaktionshilfsmittel und gegebenenfalls in Gegenwart eines Verdünnungsmittels umsetzt,
oder daß man
(c) substituierte Benzoylpyrazole der allgemeinen Formel (Ia) 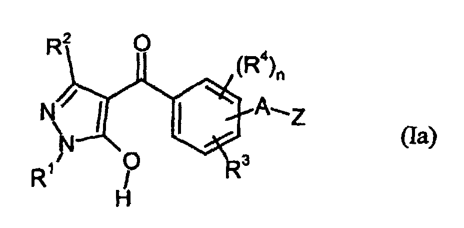 in welcher
n, A, R¹, R², R³, R⁴ und Z die in einem der Ansprüche 1 bis 6 angegebene Bedeutung haben,
mit Verbindungen der allgemeinen Formel (V)
CI-Y (V)
in welcher
Y mit Ausnahme von Wasserstoff die in einem der Ansprüche 1 bis 4 angegebene Bedeutung hat,
- oder gegebenenfalls mit entsprechenden Isocyanaten oder Isothiocyanaten -
gegebenenfalls in Gegenwart eines oder mehrerer Reaktionshilfsmittel und gegebenenfalls in Gegenwart eines Verdünnungsmittels umsetzt,
und gegebenenfalls im Anschluß daran an den so erhaltenen Verbindungen der Formel (I) im Rahmen der Substituentendefinition auf übliche Weise elektrophile oder nucleophile bzw. Oxidations- oder Reduktionsreaktionen durchführt oder die Verbindungen der Formel (I) auf übliche Weise in Salze überführt.

8. Verbindungen der allgemeinen Formel (Ia) 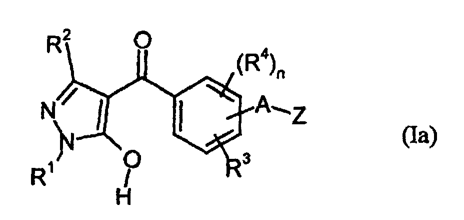 in welcher
n, A, R¹, R², R³, R⁴ und Z die in einem der Ansprüche 1 bis 6 angegebene Bedeutung haben.

9. Herbizide Mittel, **gekennzeichnet durch** den Gehalt mindestens einer Verbindung gemäß einem der Ansprüche 1 bis 6 und üblichen Streckmitteln.

10. Verwendung von mindestens einer Verbindung gemäß einem der Ansprüche 1 bis 6 zur Bekämpfung von unerwünschten Pflanzen.

## Claims

1. Substituted benzoylpyrazoles of the general formula (I), 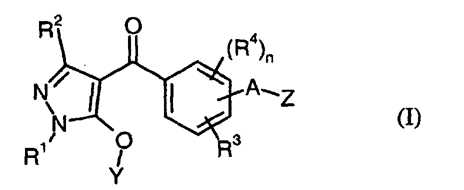 in which
n represents the numbers 0, 1, 2 or 3,
A represents alkanediyl (alkylene),
R¹ represents in each case optionally substituted alkyl, alkenyl, alkinyl or cycloalkyl,
R² represents hydrogen, cyano, carbamoyl, thiocarbamoyl, halogen, or represents in each case optionally substituted alkyl, alkoxy, alkylthio, alkoxycarbonyl or cycloalkyl,
R³ represents hydrogen, nitro, cyano, carboxyl, carbamoyl, thiocarbamoyl, halogen, or represents in each case optionally substituted alkyl, alkoxy, alkylthio, alkylsulfinyl, alkylsulfonyl, alkylamino, dialkylamino or dialkylaminosulfonyl,
R⁴ represents nitro, cyano, carboxyl, carbamoyl, thiocarbamoyl, halogen, or represents in each case optionally substituted alkyl, alkoxy, alkylthio, alkylsulfinyl, alkylsulfonyl, alkylamino, dialkylamino or dialkylaminosulfonyl,
Y represents hydrogen or represents in each case optionally substituted alkyl, alkylcarbonyl, alkoxycarbonyl, alkylsulfonyl, alkylaminocarbonyl, dialkylaminocarbonyl, alkenyl, alkenylcarbonyl, alkenylsulfonyl, alkinyl, alkinylcarbonyl, cycloalkyl, cycloalkylcarbonyl, cycloalkylalkyl, phenylcarbonyl, phenylsulfonyl, phenylalkyl or phenylcarbonylalkyl, and
Z represents one of the heterocyclic groupings below 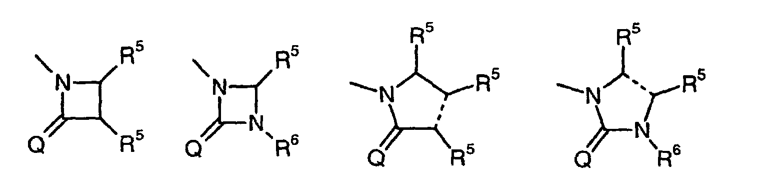 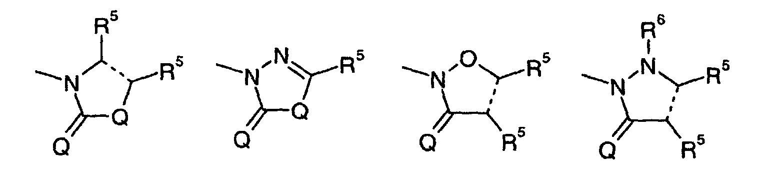 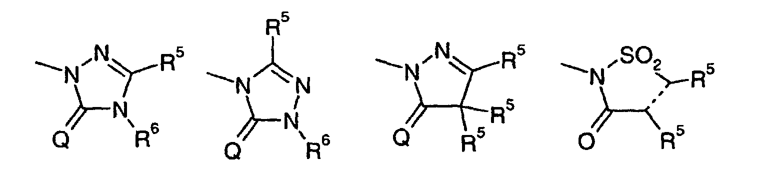 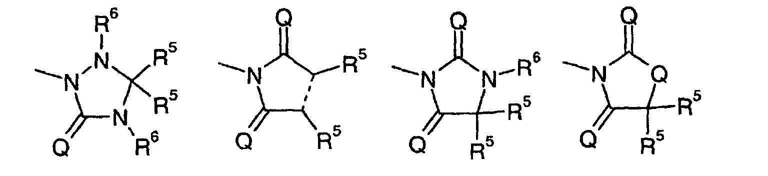 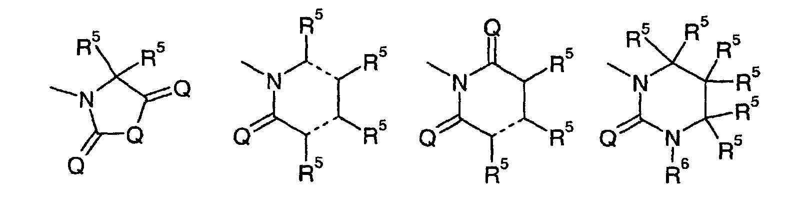 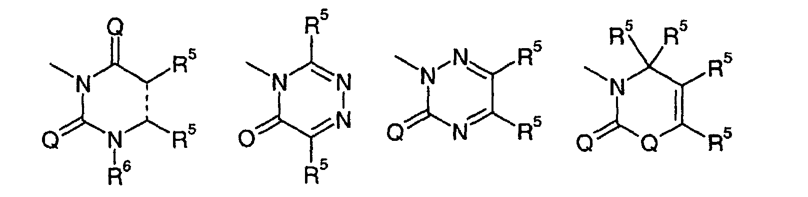 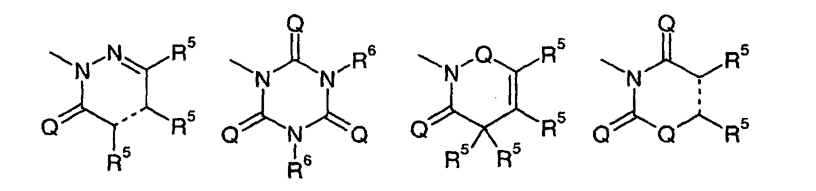 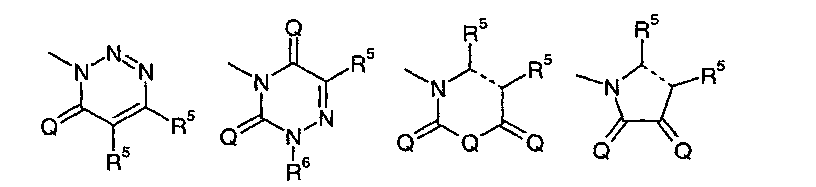 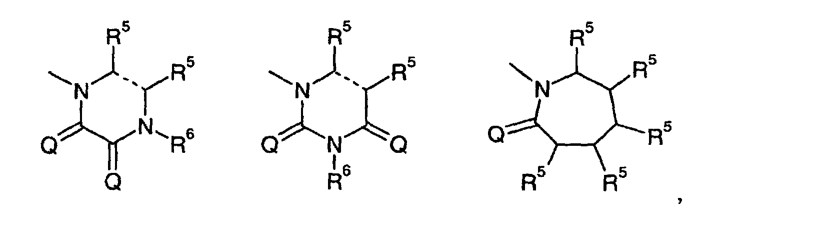 in which in each case the broken bond is a single bond or a double bond,
Q represents oxygen or sulfur,
R⁵ represents hydrogen, hydroxyl, mercapto, cyano, halogen, represents in each case optionally cyano-, halogen-, C₁-C₄-alkoxy-, C₁-C₄-alkylthio-, C₁-C₄-alkylsulfinyl- or C₁-C₄-alkylsulfonyl-substituted alkyl, alkylcarbonyl, alkoxy, alkoxycarbonyl, alkylthio, alkylsulfinyl or alkylsulfonyl having in each case up to 6 carbon atoms in the alkyl groups, represents propadienylthio, represents in each case optionally halogen-substituted alkylamino or dialkylamino having in each case up to 6 carbon atoms in the alkyl groups, represents in each case optionally halogen-substituted alkenyl, alkinyl, alkenyloxy, alkenylthio or alkenylamino having in each case up to 6 carbon atoms in the alkenyl or alkinyl groups, represents in each case optionally halogen-substituted cycloalkyl, cycloalkyloxy, cycloalkylthio, cycloalkylamino, cycloalkylalkyl, cycloalkylalkoxy, cycloalkylalkylthio or cycloalkylalkylamino having in each case 3 to 6 carbon atoms in the cycloalkyl groups and optionally up to 4 carbon atoms in the alkyl moiety, represents in each case optionally halogen-, C₁-C₄-alkyl- or C₁-C₄-alkoxy-substituted phenyl, phenyloxy, phenylthio, phenylamino, benzyl, benzyloxy, benzylthio or benzylamino, represents pyrrolidino, piperidino or morpholino, or - if two adjacent radicals R⁵ and R⁵ are located on a double bond - together with the adjacent radical R⁵ also represents a benzo grouping, and
R⁶ represents hydrogen, hydroxyl, amino, alkylideneamino having up to 4 carbon atoms, represents in each case optionally halogen- or C₁-C₄-alkoxy-substituted alkyl, alkoxy, alkylamino, dialkylamino or alkanoylamino having in each case up to 6 carbon atoms in the alkyl groups, represents in each case optionally halogen-substituted alkenyl, alkinyl or alkenyloxy having in each case up to 6 carbon atoms in the alkenyl or alkinyl groups, represents in each case optionally halogen-substituted cycloalkyl, cycloalkylalkyl or cycloalkylamino having in each case 3 to 6 carbon atoms in the cycloalkyl groups and optionally up to 3 carbon atoms in the alkyl moiety, or represents in each case optionally halogen-, C₁-C₄-alkyl- or C₁-C₄-alkoxy-substituted phenyl or benzyl, or together with an adjacent radical R⁵ or R⁶ represents optionally halogen- or C₁-C₄-alkyl-substituted alkanediyl having 3 to 5 carbon atoms,
where the individual radicals R⁵ and R⁶ - if a plurality of these are attached to the same heterocyclic groupings - may have identical or different meanings within the scope of the above definition,
including all possible tautomeric forms and the possible salts.

2. Compounds according to Claim 1, **characterized in that**
n represents the numbers 0, 1 or 2,
A represents alkanediyl (alkylene) having 1 to 4 carbon atoms,
R¹ represents optionally cyano-, carboxyl-, carbamoyl-, halogen-, C₁-C₄-alkoxy-, C₁-C₄-alkyl-carbonyl-, C₁-C₄-alkoxy-carbonyl-, C₁-C₄-alkylthio-, C₁-C₄-alkylsulfinyl- or C₁-C₄-alkylsulfonyl-substituted alkyl having 1 to 6 carbon atoms, represents in each case optionally cyano-, carboxyl-, carbamoyl-, halogen- or C₁-C₄-alkoxy-carbonyl-substituted alkenyl or alkinyl having in each case 2 to 6 carbon atoms, or represents optionally cyano-, carboxyl-, carbamoyl-, halogen-, C₁-C₄-alkyl- or C₁-C₄-alkoxy-carbonyl-substituted cycloalkyl having 3 to 6 carbon atoms,
R² represents hydrogen, cyano, carbamoyl, thiocarbamoyl, halogen, reprsents in each case optionally cyano-, halogen- or C₁-C₄-alkoxy-substituted alkyl, alkoxy or alkoxycarbonyl having in each case up to 6 carbon atoms, represents optionally halogen-substituted alkylthio having 1 to 6 carbon atoms, or represents optionally cyano-, halogen- or C₁-C₄-alkyl-substituted cycloalkyl having 3 to 6 carbon atoms,
R³ represents hydrogen, nitro, cyano, carboxyl, carbamoyl, thiocarbamoyl, halogen, represents in each case optionally halogen, C₁-C₄-alkoxy-, C₁-C₄-alkylthio-, C₁-C₄-alkylsulfinyl- or C₁-C₄-alkylsulfonyl-substituted alkyl, alkoxy, alkylthio, alkylsulfinyl or alkylsulfonyl having in each case up to 4 carbon atoms in the alkyl groups, or represents alkylamino, dialkylamino or dialkylaminosulfonyl having in each case up to 4 carbon atoms in the alkyl groups,
R⁴ represents nitro, cyano, carboxyl, carbamoyl, thiocarbamoyl, halogen, represents in each case optionally halogen-, C₁-C₄-alkoxy-, C₁-C₄-alkylthio-, C₁-C₄-alkylsulfinyl- or C₁-C₄-alkylsulfonyl-substituted alkyl, alkoxy, alkylthio, alkylsulfinyl or alkylsulfonyl having in each case up to 4 carbon atoms in the alkyl groups, or represents alkylamino, dialkylamino or dialkylaminosulfonyl having in each case up to 4 carbon atoms in the alkyl groups,
Y represents hydrogen, represents in each case optionally cyano-, carboxyl-, carbamoyl-, halogen- or C₁-C₄-alkoxycarbonyl-substituted alkyl, alkylcarbonyl or alkoxycarbonyl having in each case up to 6 carbon atoms, represents in each case optionally halogen-substituted alkylsulfonyl, alkylaminocarbonyl or dialkylaminocarbonyl having in each case up to 6 carbon atoms in the alkyl groups, represents in each case optionally cyano-, carboxyl-, carbamoyl-, halogen- or C₁-C₄-alkoxy-carbonyl-substituted alkenyl, alkenylcarbonyl, alkinyl or alkinylcarbonyl having in each case 2 to 6 carbon atoms, represents optionally halogen-substituted alkenylsulfonyl having up to 6 carbon atoms, represents in each case optionally cyano-, halogen- or C₁-C₄-alkyl-substituted cycloalkyl, cycloalkylcarbonyl or cycloalkylalkyl having in each case 3 to 6 carbon atoms in the cycloalkyl groups and optionally 1 to 3 carbon atoms in the alkyl moiety, or represents in each case optionally nitro-, cyano-, carboxyl-, carbamoyl-, halogen-, C₁-C₄-alkyl-, C₁-C₄-halogenoalkyl-, C₁-C₄-alkoxy- or C₁-C₄-halogenoalkoxy-substituted phenylcarbonyl, phenylsulfonyl, phenyl-C₁-C₄-alkyl or phenylcarbonyl-C₁-C₄-alkyl, and
Z is as defined in Claim 1.

3. Compounds according to claim 1 or 2, **characterized in that**
n represents the numbers 0 or 1,
A represents methylene, ethylidene (ethane-1,1-diyl) or dimethylene (ethane-1,2-diyl),
R¹ represents in each case optionally fluorine-, chlorine-, methoxy-, ethoxy-, n- or i-propoxy-, methylthio-, ethylthio-, n- or i-propylthio-, methylsulfinyl-, ethylsulfinyl-, n- or i-propylsulfinyl-, methylsulfonyl-, ethylsulfonyl-, n- or i-propylsulfonyl-substituted methyl, ethyl, n- or i-propyl, n-, i-, s- or t-butyl, represents in each case optionally fluorine-, chlorine- or bromine-substituted propenyl, butenyl, propinyl or butinyl, or represents in each case optionally cyano-, fluorine-, chlorine-, bromine-, methyl- or ethyl-substituted cyclopropyl, cyclobutyl, cyclopentyl or cyclohexyl,
R² represents hydrogen, cyano, carbamoyl, thiocarbamoyl, represents in each case optionally cyano-, fluorine-, chlorine-, methoxy- or ethoxy-substituted methyl, ethyl, n- or i-propyl, n-, i-, s- or t-butyl, methoxy, ethoxy, n- or i-propoxy, methoxycarbonyl, ethoxycarbonyl, n- or i-propoxycarbonyl, represents in each case optionally fluorine- and/or chlorine-substituted methylthio, ethylthio, n- or i-propylthio, or represents in each case optionally cyano-, fluorine-, chlorine-, bromine-, methyl- or ethyl-substituted cyclopropyl, cyclobutyl, cyclopentyl or cyclohexyl,
R³ represents hydrogen, nitro, cyano, carboxyl, carbamoyl, thiocarbamoyl, fluorine, chlorine, bromine, iodine, represents in each case optionally fluorine- and/or chlorine-, methoxy-, ethoxy-, n- or i-propoxy-, methylthio-, ethylthio-, n- or i-propylthio-, methylsulfinyl-, ethylsulfinyl-, methylsulfonyl- or ethylsulfonyl-substituted methyl, ethyl, n- or i-propyl, n-, i-, s- or t-butyl, represents in each case optionally fluorine- and/or chlorine-, methoxy-, ethoxy-, n- or i-propoxy-substituted methoxy, ethoxy, n- or i-propoxy, represents in each case optionally fluorine- and/or chlorine-substituted methylthio, ethylthio, n- or i-propylthio, methylsulfinyl, ethylsulfinyl, n- or i-propylsulfinyl, methylsulfonyl, ethylsulfonyl, n- or i-propylsulfonyl, or represents methylamino, ethylamino, n- or i-propylamino, dimethylamino, diethylamino, dimethylaminosulfonyl or diethylaminosulfonyl,
R⁴ represents nitro, cyano, carboxyl, carbamoyl, thiocarbamoyl, fluorine, chlorine, bromine, represents in each case optionally fluorine- and/or chlorine-, methoxy-, ethoxy-, n- or i-propoxy-, methylthio-, ethylthio-, n- or i-propylthio-, methylsulfinyl-, ethylsulfinyl-, methylsulfonyl- or ethylsulfonyl-substituted methyl, ethyl, n- or i-propyl, n-, i-, s- or t-butyl, represents in each case optionally fluorine- and/or chlorine-, methoxy-, ethoxy-, n- or i-propoxy-substituted methoxy, ethoxy, n- or i-propoxy, represents in each case optionally fluorine- and/or chlorine-substituted methylthio, ethylthio, n- or i-propylthio, methylsulfinyl, ethylsulfinyl, n- or i-propylsulfinyl, methylsulfonyl, ethylsulfonyl, n- or i-propylsulfonyl, or represents methylamino, ethylamino, n- or i-propylamino, dimethylamino, diethylamino, dimethylaminosulfonyl or diethylaminosulfonyl,
R⁵ represents hydrogen, hydroxyl, chlorine, bromine, methyl, ethyl, n- or i-propyl, n-, i-, s- or t-butyl, difluoromethyl, dichloromethyl, trifluoromethyl, trichloromethyl, chlorodifluoromethyl, fluorodichloromethyl, fluoroethyl, chloroethyl, difluoroethyl, dichloroethyl, fluoro-n-propyl, fluoro-i-propyl, chloro-n-propyl, chloro-i-propyl, methoxymethyl, ethoxymethyl, methoxyethyl, ethoxyethyl, methoxy, ethoxy, n- or i-propoxy, n-, i-, s- or t-butoxy, fluoroethoxy, chloroethoxy, difluoroethoxy, dichloroethoxy, trifluoroethoxy, trichloroethoxy, chlorofluoroethoxy, chlorodifluoroethoxy, fluorodichloroethoxy, methylthio, ethylthio, n- or i-propylthio, fluoroethylthio, chloroethylthio, difluoroethylthio, dichloroethylthio, chlorofluoroethylthio, chlorodifluoroethylthio, fluorodichloroethylthio, methylsulfinyl, ethylsulfinyl, n- or i-propylsulfinyl, methylsulfonyl, ethylsulfonyl, n- or i-propylsulfonyl, dimethylamino, propenylthio, butenylthio, propinylthio, butinylthio, cyclopropyl, cyclopropylmethyl, cyclopropylmethoxy, phenyl or phenoxy,
R⁶ represents amino, methyl, ethyl, n- or i-propyl, n-, i-, s- or t-butyl, methoxy, ethoxy, methylamino, dimethylamino, cyclopropyl or cyclopropylmethyl, or together with R⁵ represents propane-1,3-diyl (trimethylene), butane-1,4-diyl (tetramethylene) or pentane-1,5-diyl (pentamethylene), and
Y represents hydrogen, represents in each case optionally cyano-, fluorine-, chlorine-, methoxy- or ethoxy-substituted methyl, ethyl, n- or i-propyl, acetyl, propionyl, n- or i-butyroyl, methoxycarbonyl or ethoxycarbonyl, represents in each case optionally fluorine-, chlorine- and/or bromine-substituted methylsulfonyl-, ethylsulfonyl-, n- or i-propylsulfonyl-, n-, i-, s- or t-butylsulfonyl-, methylaminocarbonyl, ethylaminocarbonyl, n- or i-propylaminocarbonyl, dimethylaminocarbonyl or diethylaminocarbonyl, represents in each case optionally fluorine-, chlorine- or brominesubstituted propenyl, butenyl, propenylcarbonyl, butenylcarbonyl, propenylsulfonyl, butenylsulfonyl, propinyl, butinyl, propinylcarbonyl or butinylcarbonyl, represents in each case optionally cyano-, fluorine-, chlorine-, methyl- or ethyl-substituted cyclopropyl, cyclobutyl, cyclopentyl, cyclohexyl, cyclopropylcarbonyl, cyclobutylcarbonyl, cyclopentylcarbonyl, cyclohexylcarbonyl, cyclopropylmethyl, cyclobutylmethyl, cyclopentylmethyl or cyclohexylmethyl, or represents in each case optionally nitro-, cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy- or trifluoromethoxy-substituted phenylcarbonyl, phenylsulfonyl, benzyl or phenylcarbonylmethyl.

4. Compounds according to any of claims 1 to 3, **characterized in that**
Z represents the grouping below

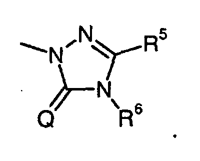

5. Compounds according to any of claims 1 to 4, **characterized in that**
Q represents oxygen.

6. Compounds according to any of claims 1 to 5, **characterized in that** n represents 0.

7. Process for preparing compounds according to any of claims 1 to 6, **characterized in that**
(a) pyrazoles of the general formula (II) 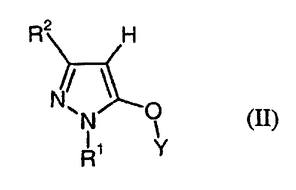 in which
R¹, R² and Y are as defined in any of claims 1 to 3,
are reacted with substituted benzoic acids of the general formula (III), 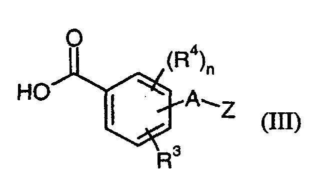 in which
n, A, R³, R⁴ and Z are as defined in any of claims 1 to 6,
in the presence of a dehydrating agent, if appropriate in the presence of one or more reaction auxiliaries and if appropriate in the presence of a diluent,
or that
(b) pyrazoles of the general formula (II) 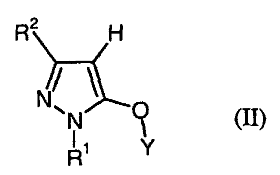 in which
R¹, R² and Y are as defined in any of claims 1 to 3,
are reacted with substituted benzoic acid derivatives of the general formula (IV) 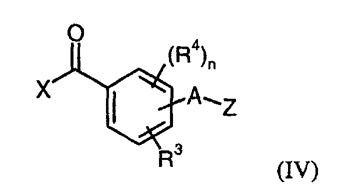 in which
n, A, R³, R⁴ and Z are as defined in any of claims 1 to 6, and
X represents cyano, halogen or alkoxy,
- or with corresponding carboxylic anhydrides -
if appropriate in the presence of one or more reaction auxiliaries and if appropriate in the presence of a diluent,
or that
(c) substituted benzoylpyrazoles of the general formula (Ia) 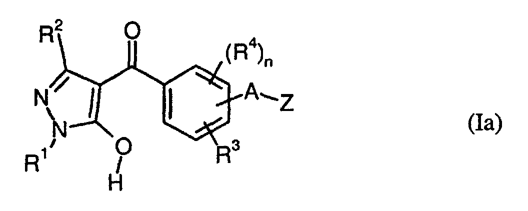 in which
n, A, R¹, R², R³, R⁴ and Z are as defined in any of claims 1 to 6,
are reacted with compounds of the general formula (V)
Cl-Y (V)
in which
Y is as defined in any of claims 1 to 4, except for hydrogen,
- or, if appropriate, with corresponding isocyanates or isothiocyanates -
if appropriate in the presence of one or more reaction auxiliaries and if appropriate in the presence of a diluent,
and, if appropriate, the resulting compounds of the formula (I) are subsequently subjected in a customary manner to electrophilic or nucleophilic and/or oxidation or reduction reactions within the scope of the definition of the substituents, or the compounds of the formula (I) are converted in a customary manner into salts.

8. Compounds of the general formula (Ia) 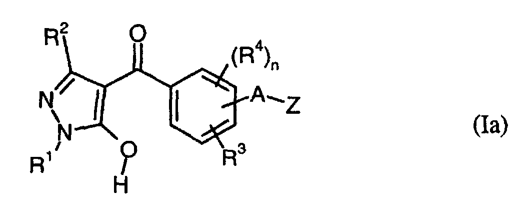 in which
n, A, R¹, R², R³, R⁴ and Z are as defined in any of claims 1 to 6.

9. Herbicidal compositions, **characterized in that** they comprise at least one of the compounds according to any of claims 1 to 6 and customary extenders.

10. Use of at least one compound according to any of claims 1 to 6 for controlling undesirable plants.

## Revendications

1. Benzoylpyrazoles substitués de formule générale (I) 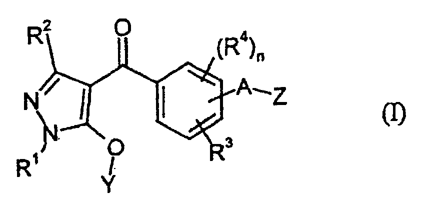 dans laquelle
n représente les nombres 0, 1, 2 ou 3,
A est un reste alcanediyle (alkylène),
R¹ est un reste alkyle, alcényle, alcinyle ou cyloalkyle dont chacun est éventuellement substitué,
R² représente l'hydrogène, un groupe cyano, carbamoyle, thiocarbamoyle, un halogène, ou un reste alkyle, alkoxy, alkylthio, alkoxycarbonyle ou cycloalkyle dont chacun est éventuellement substitué,
R³ représente l'hydrogène, un groupe nitro, cyano, carboxy, carbamoyle, thiocarbamoyle, un halogène, ou un groupe alkyle, alkoxy, alkylthio, alkylsulfinyle, alkylsulfonyle, alkylamino, dialkylamino ou dialkylaminosulfonyle dont chacun est éventuellement substitué,
R⁴ est un groupe nitro, cyano, carboxy, carbamoyle, thiocarbamoyle, un halogène ou un groupe alkyle, alkoxy, alkylthio, alkylsulfinyle, alkylsulfonyle, alkylamino, dialkylamino ou dialkylaminosulfonyle dont chacun est éventuellement substitué,
Y représente l'hydrogène ou un groupe alkyle, alkylcarbonyle, alkoxycarbonyle, alkylsulfonyle, alkylaminocarbonyle, dialkylaminocarbonyle, alcényle, alcénylcarbonyle, alcénylsulfonyle, alcinyle, alcinylcarbonyle, cycloalkyle, cycloalkylcarbonyle, cycloalkylalkyle, phénylcarbonyle, phénylsulfonyle, phénylalkyle ou phénylcarbonylalkyle dont chacun est éventuellement substitué, et
Z représente l'un des groupements hétérocycliques suivants : 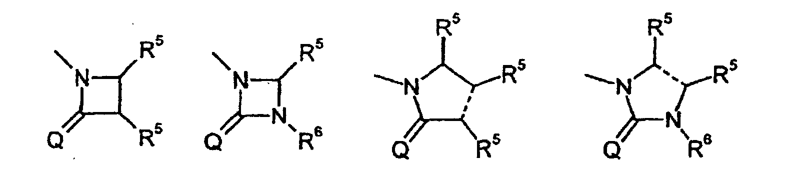 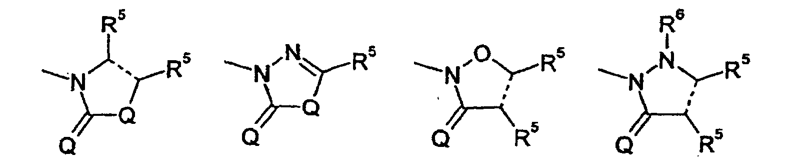 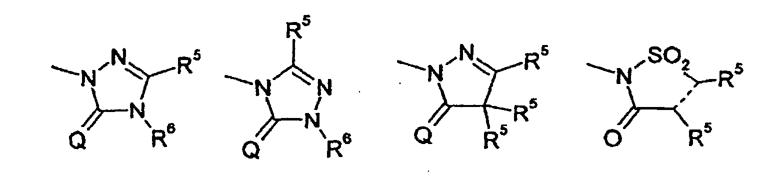 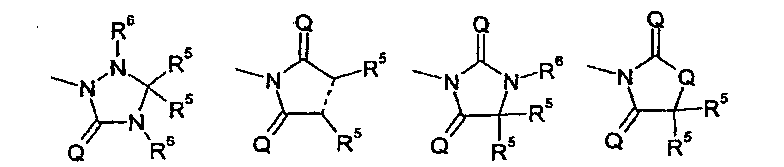 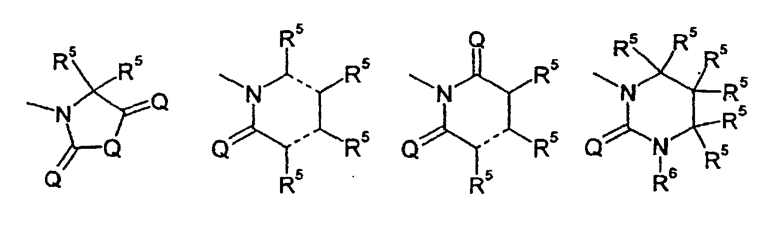 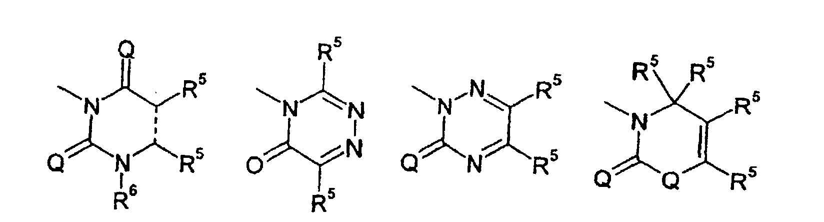 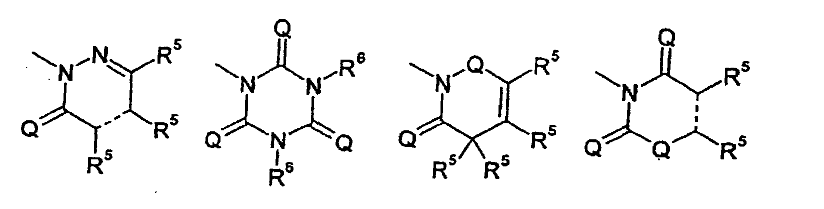 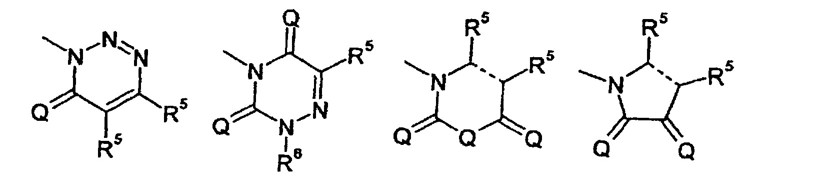 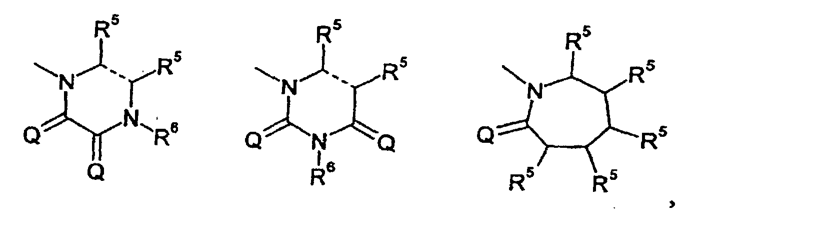
dans chacun desquels la liaison représentée en traits interrompus est une liaison simple ou une liaison double,
Q représente l'oxygène ou le soufre,
R⁵ est l'hydrogène, un groupe hydroxy, mercapto, cyano, un halogène, un groupe alkyle, alkylcarbonyle, alkoxy, alkoxycarbonyle, alkylthio, alkylsulfinyle ou alkylsulfonyle ayant chacun jusqu'à 6 atomes de carbone dans les groupes alkyle et chacun portant éventuellement un substituant cyano, halogéno, alkoxy en C₁ à C₄, alkylthio en C₁ à C₄, alkylsulfinyle en C₁ à C₄ ou alkylsulfonyle en C₁ à C₄, un reste propadiénylthio, un reste alkylamino ou dialkylamino ayant chacun jusqu'à 6 atomes de carbone dans les groupes alkyle et portant éventuellement un substituant halogéno, un reste alcényle, alcinyle, alcényloxy, alcénylthio ou alcénylamino ayant chacun jusqu'à 4 atomes de carbone dans les groupes alcényle ou alcinyle et portant chacun le cas échéant un substituant halogéno, un reste cycloalkyle, cycloalkyloxy, cycloalkylthio, cycloalkylamino, cycloalkylalkyle, cycloalkylalkoxy, cycloalkylalkylthio ou cycloalkylalkylamino ayant chacun 3 à 6 atomes de carbone dans les groupes cycloalkyle et le cas échéant jusqu'à 3 atomes de carbone dans la partie alkyle et portant chacun le cas échéant un substituant halogéno, alkyle en C₁ à C₄ ou alcoxy en C₁ à C₄, un reste phényle, phényloxy, phénylthio, phénylamino, benzyle, benzyloxy, benzylthio ou benzylamino portant chacun le cas échéant un substituant halogéno, un reste pyrrolidino, pipéridino ou morpholino, ou bien - au cas où deux restes R⁵ et R⁵ contigus se trouvent sur une double liaison - R⁵ forme aussi un groupement benzo conjointement avec le reste R⁵ contigu, et est
R⁶ l'hydrogène, un groupe hydroxy, amino, un reste alkylidène-amino ayant jusqu'à 4 atomes de carbone, un reste alkyle, alkoxy, alkylamino, dialkylamino ou alcanolyamino ayant chacun jusqu'à 6 atomes de carbone dans les groupes alkyle et portant chacun le cas échéant un substituant halogéno, ou alkoxy en alkyle en C₁ à C₄ ou alcoxy en C₁ à C_{4,} un reste alcényle, alcinyle ou alcényloxy ayant chacun jusqu'à 6 atomes de carbone dans les groupes alcényle ou alcinyle et portant chacun le cas échéant un substituant halogéno, un reste cycloalkyle, cycloalkylalkyle ou cycloalkylamino ayant chacun 3 à 6 atomes de carbone dans les groupes cycloalkyle et le cas échéant jusqu'à 3 atomes de carbone dans la partie alkyle et portant chacun le cas échéant un substituant halogéno, ou bien un reste phényle ou benzyle portant chacun le cas échéant un substituant halogéno, alkyle en C₁ à C₄ ou alkoxy en C₁ à C₄, ou forment conjointement avec un reste R⁵ ou R⁶ contigus un reste alcanediyle de 3 à 5 atomes de carbone portant éventuellement un substituant halogéno,
les restes R⁵ et R⁶ individuels - dans la mesure où plusieurs d'entre eux sont liés à des groupements hétérocycliques identiques - peuvent avoir des significations identiques ou différentes dans le cadre de la définition donnée ci-dessus,
y compris toutes les formes tautomères possibles et tous les sels possibles.

2. Composés suivant la revendication 1, **caractérisés en ce que**
n représente les nombres 0, 1 ou 2,
A est un reste alcanediyle (alkylène) ayant 1 à 4 atomes de carbone,
R¹ est un reste alkyle ayant 1 à 6 atomes de carbone portant éventuellement un substituant cyano, carboxy, carbamoyle, halogéno, alkoxy en C₁ à C₄, (alkyle en C₁ à C₄)-carbonyle, (alkoxy en C₁ à C₄)-carbonyle, alkylthio en C₁ à C₄, alkylsulfinyle en C₁ à C₄ ou alkylsulfonyle en C₁ à C₄, un reste alcényle ou alcinyle ayant chacun 2 à 6 atomes de carbone et portant chacun le cas échéant un substituant cyano, carboxy, carbamoyle, halogéno ou (alkoxy en C₁ à C₄)-carbonyle, ou un reste cyloalkyle ayant 3 à 6 atomes de carbone portant éventuellement un substituant cyano, carboxy, carbamoyle, halogéno, alkyle en C₁ à C₄ ou (alkoxy en C₁ à C₄)-carbonyle,
R² représente l'hydrogène, un groupe cyano, carbamoyle, thiocarbamoyle, un halogène, un reste alkyle, alkoxy ou alkoxycarbonyle ayant chacun jusqu'à 6 atomes de carbone et portant chacun le cas échéant un substituant cyano, halogéno ou alkoxy en C₁ à C₄, un reste alkylthio ayant 1 à 6 atomes de carbone et portant éventuellement un substituant halogéno, ou un reste cycloalkyle ayant 3 à 6 atomes de carbone portant éventuellement un substituant cyano, halogéno ou alkyle en C₁ à C₄,
R³ représente l'hydrogène, un groupe nitro, cyano, carboxy, carbamoyle, thiocarbamoyle, un halogène, un reste alkyle, alkoxy, alkylthio, alkylsulfinyle ou alkylsulfonyle ayant chacun jusqu'à 4 atomes de carbone dans les groupes alkyle et portant chacun le cas échéant un substituant halogéno, alkoxy en C₁ à C₄, alkylthio en C₁ à C₄, alkylsulfinyle en C₁ à C₄ ou alkylsulfonyle en C₁ à C₄, ou un reste alkylamino, dialkylamino ou dialkylaminosulfonyle ayant chacun jusqu'à 4 atomes de carbone dans les groupes alkyle,
R⁴ est un groupe nitro, cyano, carboxy, carbamoyle, thiocarbamoyle, un halogène, un reste alkyle, alkoxy, alkylthio, alkylsulfinyle ou alkylsulfonyle ayant chacun jusqu'à 4 atomes de carbone dans les groupes alkyle et portant chacun le cas échéant un substituant halogéno, alkoxy en C₁ à C₄, alkylthio en C₁ à C₄, alkylsulfinyle en C₁ à C₄ ou alkylsulfonyle en C₁ à C₄, ou bien un reste alkylamino, dialkylamino ou dialkylaminosulfonyle ayant chacun jusqu'à 4 atomes de carbone dans les groupes alkyle,
Y représente l'hydrogène, un reste alkyle, alkylcarbonyle ou alkoxycarbonyle ayant chacun jusqu'à 6 atomes de carbone et portant chacun le cas échéant un substituant cyano, carboxy, carbamoyle, halogéno ou (alkoxy en C₁ à C₄)-carbonyle, un reste alkyl-sulfonyle, alkylaminocarbonyle ou dialkylaminocarbonyle ayant chacun jusqu'à 6 atomes de carbone dans les groupes alkyle et portant chacun le cas échéant un substituant halogéno, un reste alcényle, alcénylcarbonyle, alcinyle ou alcinylcarbonyle ayant chacun 2 à 6 atomes de carbone et portant chacun le cas échéant un substituant cyano, carboxy, carbamoyle, halogéno ou (alkoxy en C₁ à C₄)-carbonyle, un reste alcénylsulfonyle ayant jusqu'à 6 atomes de carbone et portant éventuellement un substituant halogéno, un reste cycloalkyle, cycloalkylcarbonyle ou cycloalkylalkyle ayant chacun 3 à 6 atomes de carbone dans les groupes cyclo-alkyle et le cas échéant 1 à 3 atomes de carbone dans la partie alkyle et portant chacun le cas échéant un substituant cyano, halogéno ou alkyle en C₁ à C₄, ou bien un reste phénylcarbonyle, phénylsulfonyle, phényl-(alkyle en C₁ à C₄) ou phénylcarbonyl-(alkyle en C₁ à C₄) portant chacun le cas échéant un substituant nitro, cyano, carboxy, carbamoyle, halogéno, alkyle en C₁ à C₄, halogénalkyle en C₁ à C₄, alkoxy en C₁ à C₄ ou halogénalkoxy en C₁ à C₄, et
Z a la définition indiquée dans la revendication 1.

3. Composés suivant la revendication 1 ou 2, **caractérisés en ce que**
n représente le nombre 0 ou 1,
A est un reste méthylène, éthylidène-(éthane-1,1-diyle) ou diméthylène-(éthane-1,2-diyle),
R¹ est un reste méthyle, éthyle, n-propyle, iso-propyle, n-butyle, iso-butyle, sec.-butyle ou tertio-butyle portant chacun le cas échéant un substituant fluoro, chloro, méthoxy, éthoxy, n-propoxy, iso-propoxy, méthylthio, éthylthio, n-propylthio, iso-propylthio, méthylsulfinyle, éthylsulfinyle, n-propylsulfinyle, iso-propylsulfinyle, méthylsulfonyle, éthylsulfonyle, n-propylsulfonyle ou iso-propylsulfonyle, un reste propényle, butényle, propynyle ou butynyle portant chacun le cas échéant un substituant fluoro, chloro ou bromo, ou bien un reste cyclopropyle, cyclobutyle, cyclopentyle ou cyclohexyle portant chacun le cas échéant un substituant cyano, fluoro, chloro, bromo, méthyle ou éthyle,
R² représente l'hydrogène, un groupe cyano, carbamoyle, thiocarbamoyle, un reste méthyle, éthyle, n-propyle, iso-propyle, n-butyle, iso-butyle, sec.-butyle, tertio-butyle, méthoxy, éthoxy, n-propoxy, iso-propoxy, méthoxycarbonyle, éthoxycarbonyle, n-propoxycarbonyle ou iso-propoxycarbonyle portant chacun le cas échéant un substituant cyano, fluoro, chloro, méthoxy ou éthoxy, un reste méthylthio, éthylthio, n-propylthio ou iso-propylthio portant chacun le cas échéant un substituant fluoro et/ou chloro, ou un reste cyclopropyle, cyclobutyle, cyclopentyle ou cyclohexyle portant chacun le cas échéant un substituant cyano, fluoro, chloro, bromo, méthyle ou éthyle,
R³ représente l'hydrogène, un groupe nitro, cyano, carboxy, carbamoyle, thiocarbamoyle, le fluor, le chlore, le brome, l'iode, un reste méthyle, éthyle, n-propyle, iso-propyle, n-butyle, iso-butyle, sec.-butyle ou tertio-butyle portant chacun le cas échéant un substituant fluoro et/ou chloro, méthoxy, éthoxy, n-propoxy, iso-propoxy, méthylthio, éthylthio, n-propylthio, iso-propylthio, méthylsulfinyle, éthylsulfinyle, méthylsulfonyle ou éthylsulfonyle, un reste méthoxy, éthoxy, n-propoxy ou iso-propoxy portant chacun le cas échéant un substituant fluoro et/ou chloro, méthoxy, éthoxy, n-propoxy ou isopropoxy, un reste méthylthio, éthylthio, n-propylthio, iso-propylthio, méthylsulfinyle, éthylsulfinyle, n-propylsulfinyle, iso-propylsulfinyle, méthylsulfonyle, éthylsulfonyle, n-propylsulfonyle, iso-propylsulfonyle portant chacun le cas échéant un substituant fluoro et/ou chloro, ou un reste méthylamino, éthylamino, n-propylamino, iso-propylamino, diméthylamino, diéthylamino, diméthylaminosulfonyle ou diéthylaminosulfonyle,
R⁴ est un groupe nitro, cyano, carboxy, carbamoyle, thiocarbamoyle, le fluor, le chlore, le brome, un reste méthyle, éthyle, n-propyle, iso-propyle, n-butyle, iso-butyle, sec.-butyle ou tertio-butyle portant chacun le cas échéant un substituant fluoro et/ou chloro, méthoxy, éthoxy, n-propoxy, isopropoxy, méthylthio, éthylthio, n-propylthio, isopropylthio, méthylsulfinyle, éthylsulfinyle, méthylsulfonyle ou éthylsulfonyle, un reste méthoxy, éthoxy, n-propoxy ou iso-propoxy portant chacun le cas échéant un substituant fluoro et/ou chloro, méthoxy, éthoxy, n-propoxy ou iso-propoxy, un reste méthylthio, éthylthio, n-propylthio, iso-propylthio, méthylsulfinyle, éthylsulfinyle, n-propylsulfinyle, iso-propylsulfinyle, méthylsulfonyle, éthylsulfonyle, n-propylsulfonyle ou iso-propylsulfonyle portant chacun le cas échéant un substituant fluoro et/ou chloro, ou un reste méthylamino, éthylamino, n-propylamino, iso-propylamino, diméthylamino, diéthylamino, diméthylaminosulfonyle ou diéthylaminosulfonyle,
R⁵ est l'hydrogène, un groupe hydroxy, le chlore, le brome, un reste méthyle, éthyle, n-propyle, iso-propyle, n-butyle, iso-butyle, sec.-butyle, tertiobutyle, difluorométhyle, dichlorométhyle, trifluorométhyle, trichlorométhyle, chlorodifluorométhyle, fluorodichlorométhyle, fluoréthyle, chloréthyle, difluoréthyle, dichloréthyle, fluoro-n-propyle, fluor-iso-propyle, chloro-n-propyle, chlorisopropyle, méthoxyméthyle, éthoxyméthyle, méthoxyéthyle, éthoxyéthyle, méthoxy, éthoxy, n-propoxy, iso-propoxy, n-butoxy, iso-butoxy, sec.-butoxy, tertio-butoxy, fluoréthoxy, chloréthoxy, difluoréthoxy, dichloréthoxy, trifluoréthoxy, trichloréthoxy, chlorofluoréthoxy, chlorodifluoréthoxy, fluorodichloréthoxy, méthylthio, éthylthio, n-propylthio, iso-propylthio, fluoréthylthio, chloréthylthio, difluoréthylthio, dichloréthylthio, chlorofluoréthylthio, chlorodifluoréthylthio, fluorodichloréthylthio, méthylsulfinyle, éthylsulfinyle, n-propylsulfinyle, iso-propylsulfinyle, méthylsulfonyle, éthylsulfonyle, n-propylsulfonyle, iso-propylsulfonyle, diméthylamino, propénylthio, buténylthio, propynylthio, butynylthio, cyclopropyle, cyclopropylméthyle, cyclopropylméthoxy, phényle ou phénoxy,
R⁶ est un groupe amino, un reste méthyle, éthyle, n-propyle, iso-propyle, n-butyle, iso-butyle, sec.-butyle, tertio-butyle, méthoxy, éthoxy, méthylamino, diméthylamino, cyclopropyle ou cyclopropylméthyle, ou forme conjointement avec R⁵ un reste propane-1,3-diyle (triméthylène), butane-1,4-diyle (tétraméthylène) ou pentane-1,5-diyle (pentaméthylène), et
Y représente l'hydrogène, un reste méthyle, éthyle, n-propyle, iso-propyle, acétyle, propionyle, n-butyroyle, iso-butyroyle, méthoxycarbonyle ou éthoxycarbonyle portant chacun le cas échéant un substituant cyano, fluoro, chloro, méthoxy ou éthoxy, un reste méthylsulfonyle, éthylsulfonyle, n-propylsulfonyle, iso-propylsulfonyle, n-butylsulfonyle, isobutylsulfonyle, sec.-butylsulfonyle, tertio-butylsulfonyle, méthylaminocarbonyle, éthylaminocarbonyle, n-propylaminocarbonyle, iso-propylaminocarbonyle, diméthylaminocarbonyle ou diéthylaminocarbonyle portant chacun le cas échéant un substituant fluoro, chloro ou bromo, un reste propényle, butényle, propényl-carbonyle, buténylcarbonyle, propénylsulfonyle, buténylsulfonyle, propynyle, butynyle, propynyl-carbonyle ou butynylcarbonyle portant chacun le cas échéant un substituant fluoro, chloro ou bromo, un reste cyclopropyle, cyclobutyle, cyclopentyle, cyclo-hexyle, cyclopropylcarbonyle, cyclobutylcarbonyle, cyclopentylcarbonyle, cyclohexylcarbonyle, cyclopropylméthyle, cyclobutylméthyle, cyclopentylméthyle ou cyclohexylméthyle portant chacun le cas échéant un substituant cyano, fluoro, chloro, méthyle ou éthyle, ou un reste phénylcarbonyle, phénylsulfonyle, benzyle ou phénylcarbonylméthyle portant chacun le cas échéant un substituant nitro, cyano, fluoro, chloro, bromo, méthyle, éthyle, n-propyle, iso-propyle, n-butyle, iso-butyle, sec.-butyle, tertio-butyle, trifluorométhyle, méthoxy, éthoxy, n-propoxy, iso-propoxy, difluorométhoxy ou trifluorométhoxy.

4. Composés suivant l'une des revendications 1 à 3, **caractérisés en ce que**
Z représente le groupement suivant

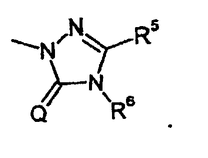

5. Composés suivant l'une des revendications 1 à 4, **caractérisés en ce que**
Q représente l'oxygène.

6. Composés suivant l'une des revendications 1 à 5, **caractérisés en ce que** n est égal à 0.

7. Procédé de production de composés suivant l'une des revendications 1 à 6, **caractérisé en ce que** :
(a) on fait réagir des pyrazoles de formule générale (II) 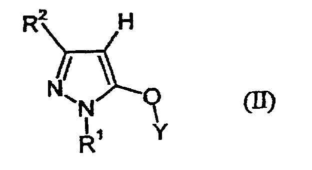 dans laquelle
R¹, R² et Y ont la définition indiquée dans les revendications 1 à 3,
avec des acides benzoïques substitués de formule générale (III) 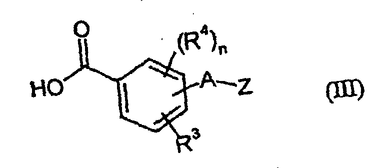 dans laquelle
n, A, R³, R⁴ et Z ont la définition indiquée dans les revendications 1 à 6,
en présence d'un agent déshydratant, le cas échéant en présence d'un ou plusieurs auxiliaires de réaction et en présence éventuelle d'un diluant,
ou **en ce que**
(b) on fait réagir des pyrazoles de formule générale (II) 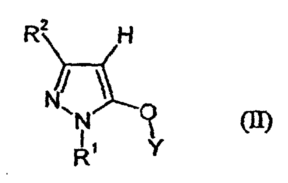 dans laquelle
R¹, R² et Y ont la définition indiquée dans l'une des revendications 1 à 3,
avec des dérivés substitués d'acide benzoïque de formule générale (IV) 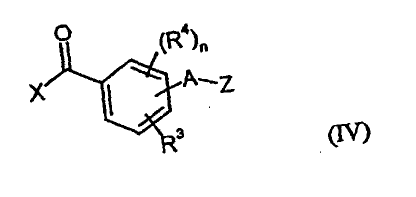 dans laquelle
n, A, R³, R⁴ et Z ont la définition indiquée dans l'une des revendications 1 à 6, et
X est un groupe cyano, un halogène ou un groupe alkoxy,
ou avec des anhydrides d'acides carboxyliques correspondants
le cas échéant en présence d'un auxiliaire de réaction et en présence éventuelle d'un diluant,
ou **en ce que**
(c) on fait réagir des benzoylpyrazoles substituées de formule générale (Ia) 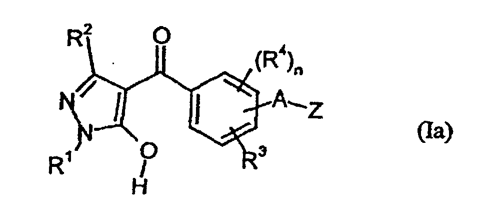 dans laquelle
n, A, R¹, R², R³, R⁴ et Z ont la définition indiquée dans l'une des revendications 1 à 6,
avec des composés de formule générale (V)
CI-Y (V)
dans laquelle
Y a la définition indiquée dans l'une des revendications 1 à 4, à l'exception de l'hydrogène,
- ou le cas échéant avec des isocyanates ou des isothiocyanates correspondants -
le cas échéant en présence d'un auxiliaire de réaction et en présence éventuelle d'un diluant,
et après quoi le cas échéant on conduit d'une façon simple des réactions électrophiles ou nucléophiles ou réactions d'oxydation ou de réduction sur les composés de formule (I) ainsi obtenus dans le cadre de la définition des substituants, ou bien on transforme en sels d'une manière connue les composés de formule (I).

8. Composés de formule générale (Ia) 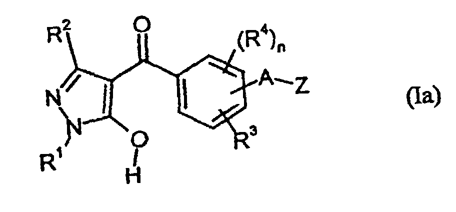 dans laquelle
n, A, R¹, R², R³, R⁴ et Z ont la définition indiquée dans l'une des revendications 1 à 6.

9. Compositions herbicides, **caractérisées en ce qu'**elles contiennent au moins un composé suivant l'une des revendications 1 à 6 et des diluants usuels.

10. Utilisation d'au moins un composé suivant l'une des revendications 1 à 6 pour combattre une végétation indésirable.
